# EUROPEAN PATENT SPECIFICATION

(11) **EP 4 410 776 B1**
(45) Date of publication and mention of the grant of the patent: **09.12.2026**
(21) Application number: 22875174.9
(22) Date of filing: 30.09.2022
(51) Int. Cl.: C07D 213/04, C07C 229/34, A61K 31/4418, A61K 31/4427, A61K 31/195, A61P 7/00, A61P 11/00, A61P 9/00, A61P 13/00, A61P 25/00, A61P 37/00

(54) **COMPOUND AS INHIBITOR OF COMPLEMENT FACTOR D, AND PHARMACEUTICAL COMPOSITION AND USE THEREOF**
VERBINDUNG ALS INHIBITOR DES KOMPLEMENTFAKTORS D SOWIE PHARMAZEUTISCHE ZUSAMMENSETZUNG UND VERWENDUNG DAVON
COMPOSÉ SERVANT D'INHIBITEUR DU FACTEUR D DU COMPLÉMENT, COMPOSITION PHARMACEUTIQUE ET UTILISATION ASSOCIÉES

(30) Priority: 30.09.2021 CN 202111165838; 22.09.2022 CN 202211160701
(43) Date of publication of application: 07.08.2024
(73) Proprietor: Wuhan Createrna Science and Technology Co.,Ltd., Wuhan, Hubei 430075 (CN)
(72) Inventor: LOU, Jun, Wuhan, Hubei 430075 (CN); WU, Jingkang, Wuhan, Hubei 430075 (CN); LIU, Li, Wuhan, Hubei 430075 (CN); SUN, Youyou, Wuhan, Hubei 430075 (CN); CHEN, Yongkai, Wuhan, Hubei 430075 (CN); ZHANG, Yihan, Wuhan, Hubei 430075 (CN); LU, Xiaoqin, Wuhan, Hubei 430075 (CN); ZHOU, Feng, Wuhan, Hubei 430075 (CN); CHEN, Ying, Wuhan, Hubei 430075 (CN); ZHANG, Liqian, Wuhan, Hubei 430075 (CN); YANG, Fan, Wuhan, Hubei 430075 (CN); WANG, Chaodong, Wuhan, Hubei 430075 (CN)
(74) Representative: Murgitroyd & Company
(86) International application number: PCT/CN2022/123316
(87) International publication number: WO 2023/051793

(56) References cited:
- WO-A1-2020/051538
- CN-A- 105 555 766
- CN-A- 106 413 707
- CN-A- 112 839 945
- KARKI RAJESHRI G. ET AL: "Design, Synthesis, and Preclinical Characterization of Selective Factor D Inhibitors Targeting the Alternative Complement Pathway", vol. 62, no. 9, 9 May 2019 (2019-05-09), US, pages 4656 - 4668, XP093229704, ISSN: 0022-2623, Retrieved from the Internet <URL:https://pubs.acs.org/doi/pdf/10.1021/acs.jmedchem.9b00271> DOI: 10.1021/acs.jmedchem.9b00271
- VELCICKY JURAJ ET AL: "Discovery and Optimization of Novel SUCNR1 Inhibitors: Design of Zwitterionic Derivatives with a Salt Bridge for the Improvement of Oral Exposure", JOURNAL OF MEDICINAL CHEMISTRY, vol. 63, no. 17, 28 August 2020 (2020-08-28), US, pages 9856 - 9875, XP093109240, ISSN: 0022-2623, DOI: 10.1021/acs.jmedchem.0c01020
- LORTHIOIS EDWIGE ET AL: "Structure-Based Design and Preclinical Characterization of Selective and Orally Bioavailable Factor XIa Inhibitors: Demonstrating the Power of an Integrated S1 Protease Family Approach", vol. 63, no. 15, 13 August 2020 (2020-08-13), US, pages 8088 - 8113, XP093229705, ISSN: 0022-2623, Retrieved from the Internet <URL:https://pubs.acs.org/doi/pdf/10.1021/acs.jmedchem.0c00279> DOI: 10.1021/acs.jmedchem.0c00279

## Description

The present application claims the right of the priorities of Chinese patent application 2021111658382 filed on September 30, 2021 and Chinese patent application 2022111607012 filed on September 22, 2022.

### TECHNICAL FIELD

The present disclosure relates to the field of medicine, in particular to a compound capable of inhibiting the activity of complement factor D, a pharmaceutical composition thereof, and a use thereof.

### BACKGROUND

Complement is a kind of protein that widely exists in the serum, tissue fluid, and cell membrane surface of humans and vertebrates, and mediates immune and inflammatory responses. It is mostly glycoproteins, produced by various cells such as hepatocytes, macrophages, and intestinal mucosal epithelial cells. Complement is a necessary supplementary condition for antibodies to achieve their cytolytic effect, hence it is named complement, but it actually mediates specific and non-specific immunity. The three activation pathways of the complement system include the classical pathway, the mannanbinding lectin pathway (MBL), and the alternative (bypass) pathway. Complement factor D plays an early and central role in the activation of the complement bypass pathway cascade. Activation of the bypass complement pathway is triggered by spontaneous hydrolysis of the thioester bond within C3 to produce C3 (H2O), and the C3 (H2O) combines with factor B to form the C3 (H2O) B complex. The role of complement factor D is to break factor B within the C3 (H2O) B complex to form Ba and Bb. In addition to combining with C3b to form C3 convertase, Bb also participates in the proliferation of preactivated B lymphocytes, while Ba inhibits their proliferation. Factor D, which is expressed at a high level in adipose, can stimulate glucose transport, promote the accumulation of triglycerides in adipocytes, and inhibit lipolysis.

Dysregulation of the complement system plays an important role in the pathogenesis of IgA nephropathy (IgAN), lupus nephritis (LN), and paroxysmal nocturnal hemoglobinuria (PNH), and the deposition of complement components and immune complex are often found in the renal pathological tests of IgAN and LN. Complement is the direct cause of hemolysis in PNH, and C5aR is involved in the amplification of complement system damage. CFB and CFD are key components of the complement bypass pathway and are directly involved in the regulation of complement activation. Therefore, C5aR, CFB, and CFD are closely related to the pathogenesis of IgAN, LN, and PNH.

Paroxysmal nocturnal hemoglobinuria (PNH) is a rare and life-threatening hematological disorder, characterized by complement-driven hemolysis, thrombosis, and impaired bone marrow function, leading to anemia, fatigue, and other debilitating symptoms, which will seriously affect the quality of life of patients. The drugs currently on the market for PNH are mainly monoclonal antibody drugs Soliris and Ultomiris. Among them, Soliris was first approved for marketing in 2007 and has been approved for a variety of super rare diseases, including paroxysmal nocturnal hemoglobinuria (PNH), atypical hemolytic uremic syndrome (aHUS), generalized myasthenia gravis (gMG), and neuromyelitis optica spectrum disorder (NMOSD). Ultomiris is an upgraded product of Soliris, a second-generation and long-acting C5 complement inhibitor, which was first approved for marketing at the end of 2018. The approved indications include: PNH and aHUS. Despite treatment with current anti-C5 standard-of-care therapies, a large proportion of PNH patients remain anemic and transfusion dependent.

CN105555766 A discloses a compound of formula I for use as a complement factor D inhibitors:

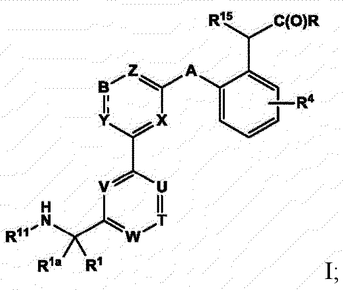

At present, there are no drugs on the market for small molecule inhibitors of complement factor D, and the target is complement alternative pathway, which is a key driver of complement-driven renal disease (CDRD). Therefore, it is of positive significance to develop small molecule inhibitors with good biological activity for the treatment of the above diseases.

### CONTENT OF THE PRESENT INVENTION

The present disclosure aims to address the technical problem that there are fewer types of existing small molecule inhibitors of complement factor D. Therefore, the present disclosure provides a compound as an inhibitor of complement factor D, a pharmaceutical composition thereof, and a use thereof. The compound has good inhibitory activity on complement factor D, and has excellent pharmacokinetic and pharmacodynamic activities.

The present disclosure provides a compound of formula (I), a tautomer thereof, a stereoisomer thereof, or a pharmaceutically acceptable salt of any one of the foregoing (referring to the foregoing compound of formula (I), the tautomer thereof, the stereoisomer thereof), or a solvate of any one of the foregoing (referring to the foregoing compound of formula (I), the tautomer thereof, the stereoisomer thereof, or the pharmaceutically acceptable salt of any one of the foregoing): 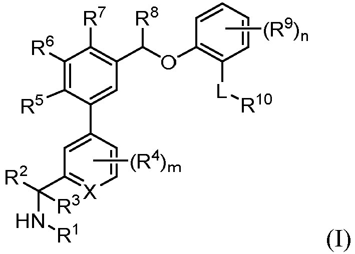 wherein
R¹ is H, D, C₁₋₆ alkyl, or C₁₋₆ alkyl substituted by 1, 2, or 3 R¹⁻¹; each R¹⁻¹ is independently halogen, -CN, -OH, C₁₋₆ alkoxy, or -NH₂;
R² and R³ are each independently H, D, halogen, C₁₋₆ alkyl, or C₁₋₆ alkyl substituted by 1, 2, or 3 R²⁻¹; each R²⁻¹ is independently halogen, -CN, -OH, C₁₋₆ alkoxy, or -NH₂;
R⁴ is H or halogen; m is 0, 1, 2, or 3;
R⁵ and R⁷ are each independently H, halogen, -CN, C₁₋₆ alkyl, or C₁₋₆ alkoxy;
R⁶ is a 8- to 11-membered heterocycloalkyl with 1, 2, or 3 heteroatoms selected from 1, 2, or 3 types of N, O, and S, or a 8- to 11-membered heterocycloalkyl with 1, 2, or 3 heteroatoms selected from 1, 2, or 3 types of N, O, and substituted by 1, 2, or 3 R⁶⁻²:
R⁶⁻² are each independently hydroxyl, oxo (=O), halogen, C₁₋₆ alkyl, C₁₋₆ alkoxy, C₁₋₆ haloalkyl, C₁₋₆ haloalkoxy, C₃₋₆ cycloalkyl, or -(CH₂)ₚ-C₃₋₆ cycloalkyl; p is 1, 2, 3, or 4;
in R⁶⁻², the cycloalkyl is independently a monocyclic ring, a bridged ring, or a spiro ring;
in R⁶, the heterocycloalkyl is a bridged ring, or a spiro ring;
R⁸ is H, halogen, or C₁₋₆ alkyl;
R⁹ is H, halogen, C₁₋₆ alkyl, or C₁₋₆ alkyl substituted by 1, 2, or 3 R⁹⁻¹;
each R⁹⁻¹ is independently halogen, -CN, -OH, or -NH₂; n is 0, 1, 2, 3, or 4;
L is -(CR^{a}R^{b})_{q}-; q is 0, 1, 2, or 3;
R^{a} and R^{b} are each independently H, D, or halogen, or R^{a} and R^{b} are connected together to form a C₃₋₆ cycloalkylene, and the formed cycloalkylene is a monocyclic ring, a bridged ring, or a spiro ring;
R¹⁰ is -COOH;
X is CR^{d} or N; R^{d} is H, halogen, or C₁₋₆ alkyl.

In a certain embodiment of the present disclosure, the compound of formula (I) has a structure of formula (I-1): 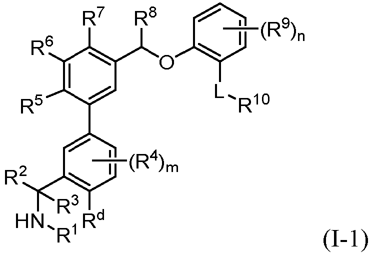 wherein R¹, R², R³, R⁴, R⁵, R⁶, R⁷, R⁸, R⁹, R¹⁰, R^{d}, L, m, and n are defined as in any one of the embodiments of the present disclosure.

In a certain embodiment of the present disclosure, the compound of formula (I) has a structure of formula (I-2): 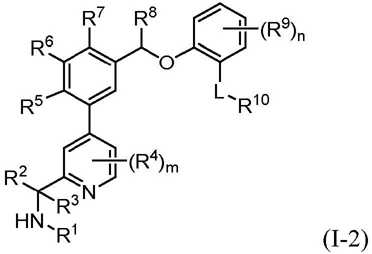 wherein R¹, R², R³, R⁴, R⁵, R⁶, R⁷, R⁸, R⁹, R¹⁰, L, m, and n are defined as in any one of the embodiments of the present disclosure.

In a certain embodiment of the present disclosure, the compound of formula (I) has a structure of formula (I-3): 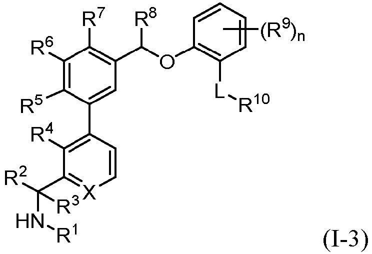 wherein X, R¹, R², R³, R⁴, R⁵, R⁶, R⁷, R⁸, R⁹, R¹⁰, L, and n are defined as in any one of the embodiments of the present disclosure.

In a certain embodiment of the present disclosure, the compound of formula (I) has a structure of formula (I-4): 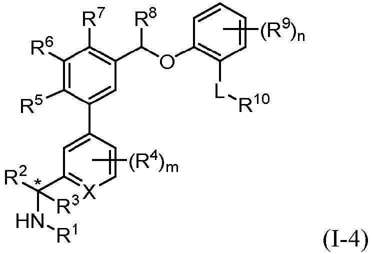 wherein X, R¹, R², R³, R⁴, R⁵, R⁶, R⁷, R⁸, R⁹, R¹⁰, L, m, and n are defined as in any one of the embodiments of the present disclosure; when a carbon atom marked with " 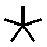 " is a chiral carbon atom, it represents an R configuration, an S configuration, or a mixture thereof.

In some preferred embodiments of the present disclosure, some groups in the compound of formula (I), the tautomer thereof, the stereoisomer thereof, or the pharmaceutically acceptable salt of any one of the foregoing (referring to the foregoing compound of formula (I), the tautomer thereof, the stereoisomer thereof), or the solvate of any one of the foregoing (referring to the foregoing compound of formula (I), the tautomer thereof, the stereoisomer thereof, or the pharmaceutically acceptable salt of any one of the foregoing) are defined as follows, and unmentioned groups are as described in any one of the embodiments of the present disclosure (referred to as "in a certain embodiment of the present disclosure").

In a certain embodiment of the present disclosure, R¹ is H.

In a certain embodiment of the present disclosure, R² and R³ are each independently H, C₁₋₆ alkyl, or C₁₋₆ alkyl substituted by 1, 2, or 3 R²⁻¹; each R²⁻¹ is independently halogen or -OH, and the halogen is preferably F.

In a certain embodiment of the present disclosure, R² and R³ are each independently H, C₁₋₃ alkyl, or C₁₋₃ alkyl substituted by 1, 2, or 3 R²⁻¹; each R²⁻¹ is independently halogen or -OH, and the halogen is preferably F.

In a certain embodiment of the present disclosure, m is 0 or 1.

In a certain embodiment of the present disclosure, R⁶ is "8- to 11-membered heterocycloalkyl with 1, 2, or 3 heteroatoms selected from 1, 2, or 3 types of N, O, and S" or "8- to 11-membered heterocycloalkyl with 1, 2, or 3 heteroatoms selected from 1, 2, or 3 types of N, O, and S" substituted by 1, 2, or 3 R⁶⁻², wherein the 8- to 11-membered heterocycloalkyl is 6-azaspiro[2.5]octyl, 5-azaspiro[2.5]octyl, 6-azaspiro[3.4]octyl, 2-azaspiro[3.4]octyl, 2-oxa-6-azaspiro[3.4]octyl, 6-oxa-2-azaspiro[3.4]octyl, 4-oxa-7-azaspiro[2.5]octyl, 2-azaspiro[4.4]nonyl, 2-azaspiro[3.5]nonyl, 2-oxa-7-azaspiro[3.5]nonyl, 1-oxa-7-azaspiro[3.5]nonyl, 7-azaspiro[3.5]nonyl, 2,7-diazaspiro[3.5]nonyl, 2-oxa-8-azaspiro[4.5]decyl, 3-oxa-9-azaspiro[5.5]undecyl, 2-oxa-9-azaspiro[5.5]undecyl, 3,9-diazaspiro[5.5]undecyl, or 3-azabicyclo[3.2.1]octyl.

In a certain embodiment of the present disclosure, R⁶ is a 8- to 11-membered heterocycloalkyl with 1, 2, or 3 heteroatoms selected from 1, 2, or 3 types of N, O, and S or a 8- to 11-membered heterocycloalkyl with 1, 2, or 3 heteroatoms selected from 1, 2, or 3 types of N, O, and S substituted by 1, 2, or 3 R⁶⁻², wherein the 8- to 11-membered heterocycloalkyl is 6-azaspiro[2.5]octyl, 5-azaspiro[2.5]octyl, 6-azaspiro[3.4]octyl, 2-azaspiro[3.4]octyl, 2-oxa-6-azaspiro[3.4]octyl, 6-oxa-2-azaspiro[3.4]octyl, 4-oxa-7-azaspiro[2.5]octyl, 2-azaspiro[4.4]nonyl, 2-azaspiro[3.5]nonyl, 2-oxa-7-azaspiro[3.5]nonyl, 1-oxa-7-azaspiro[3.5]nonyl, 7-azaspiro[3.5]nonyl, 2,7-diazaspiro[3.5]nonyl, 2-oxa-8-azaspiro[4.5]decyl, 3-oxa-9-azaspiro[5.5]undecyl, 2-oxa-9-azaspiro[5.5]undecyl, 3,9-diazaspiro[5.5]undecyl, 3-azabicyclo[3.2.1]octyl, 3-azaspiro[5,5]undecyl, 8-azaspiro[4.5]decyl, or 1-oxa-6-azaspiro[3,4]octyl.

In a certain embodiment of the present disclosure, R⁵ and R⁷ are each independently H or halogen, and the halogen is preferably F.

In a certain embodiment of the present disclosure, each R⁶⁻² is independently hydroxyl or C₁₋₆ alkyl, and the C₁₋₆ alkyl is preferably methyl.

In a certain embodiment of the present disclosure, each R⁶⁻² is independently hydroxyl or C₁₋₃ alkyl, preferably, each R⁶⁻² is independently hydroxyl, methyl, ethyl, n-propyl, or isopropyl.

In a certain embodiment of the present disclosure, R⁸ is H.

In a certain embodiment of the present disclosure, R⁹ is H or halogen, and the halogen is preferably F.

In a certain embodiment of the present disclosure, n is 0 or 1.

In a certain embodiment of the present disclosure, q is 1.

In a certain embodiment of the present disclosure, R^{a} and R^{b} are each independently H.

In the present invention, R¹⁰ is -COOH.

In a certain embodiment of the present disclosure, R^{d} is H.

In the present invention, R⁶ is a 8- to 10-membered heterocycloalkyl with 1, 2, or 3 heteroatoms selected from 1, 2, or 3 types of N, O, and S or a 8- to 10-membered heterocycloalkyl with 1, 2, or 3 heteroatoms selected from 1, 2, or 3 types of N, O, and S substituted by 1, 2, or 3 R⁶⁻², wherein the heterocycloalkyl is a bridged ring or a spiro ring.

In the present invention, R⁶ is a 8- to 11-membered heterocycloalkyl with 1, 2, or 3 heteroatoms selected from 1, 2, or 3 types of N, O, and S or a 8- to 11-membered heterocycloalkyl with 1, 2, or 3 heteroatoms selected from 1, 2, or 3 types of N, O, and S substituted by 1, 2, or 3 R⁶⁻², wherein the heterocycloalkyl is a bridged ring or a spiro ring.

In a certain embodiment of the present disclosure, R¹ is H;
R² and R³ are each independently H, C₁₋₃ alkyl, or C₁₋₃ alkyl substituted by 1, 2, or 3 R²⁻¹; each R²⁻¹ is independently halogen or -OH;
R⁴ is H or halogen; m is 0 or 1;
R⁵ and R⁷ are each independently H or halogen;
R⁶ is a 8- to 11-membered heterocycloalkyl with 1, 2, or 3 heteroatoms selected from 1, 2, or 3 types of N, O, and S or a 8- to 11-membered heterocycloalkyl with 1, 2, or 3 heteroatoms selected from 1, 2, or 3 types of N, O, and S substituted by 1, 2, or 3 R⁶⁻², wherein the heterocycloalkyl is a bridged ring or a spiro ring;
each R⁶⁻² is independently hydroxyl or C₁₋₃ alkyl;
R⁸ is H;
R⁹ is H or halogen; n is 0 or 1;
L is -(CR^{a}R^{b})_{q}-, q is 1; R^{a} and R^{b} are each independently H;
R¹⁰ is -COOH;
X is CR^{d} or N; R^{d} is H.

In a certain embodiment of the present disclosure, in R¹, R², R³, R⁵, R⁷, R⁶⁻², R⁸, R⁹, and R^{d}, each alkyl is independently methyl, ethyl, n-propyl, isopropyl, n-butyl, isobutyl, or tert-butyl, preferably methyl or ethyl.

In a certain embodiment of the present disclosure, in R¹⁻¹, R²⁻¹, R⁵, R⁷, and R⁶⁻², each alkoxy is independently methoxy, ethoxy, n-propoxy, isopropoxy, n-butoxy, isobutoxy, or tertbutoxy.

In a certain embodiment of the present disclosure, in R¹⁻¹, R², R³, R²⁻¹, R⁴, R⁵, R⁷, R⁶⁻², R⁸, R⁹, R⁹⁻¹, R^{a}, R^{b}, and R^{d}, each halogen is independently F, Cl, Br, or I, preferably F.

In the present invention in R⁶, each heterocycloalkyl is independently a 8- to 11-membered heterocycloalkyl with 1 or 2 heteroatoms selected from 1, 2, or 3 types of N, O, and S, and the heterocycloalkyl is a bridged ring or a spiro ring; preferably, the heterocycloalkyl is connected to the parent through an N atom; the heterocycloalkyl is preferably

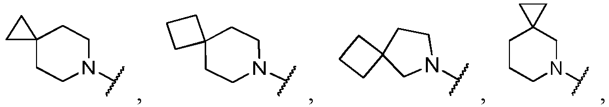

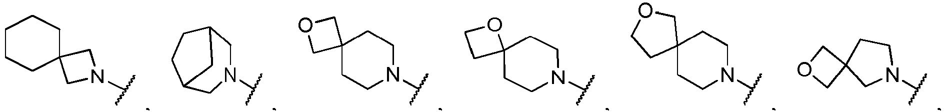

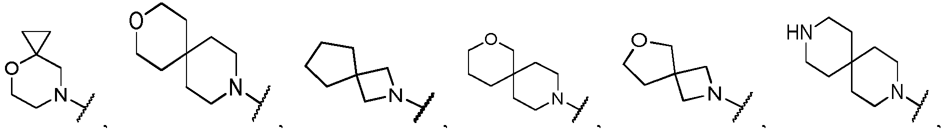

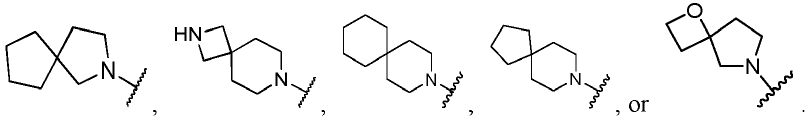

In a certain embodiment of the present disclosure, R⁶ is

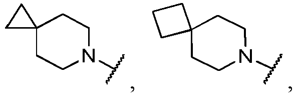

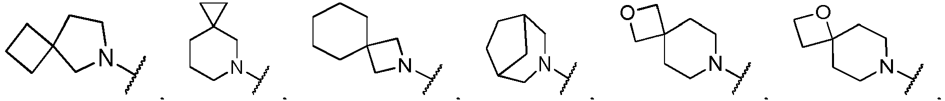

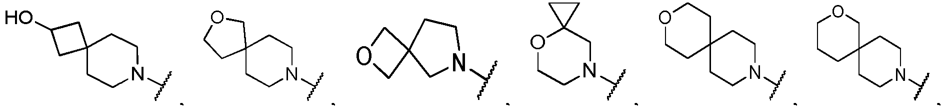

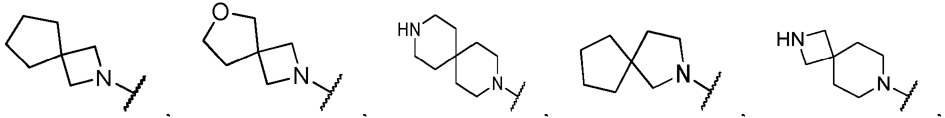

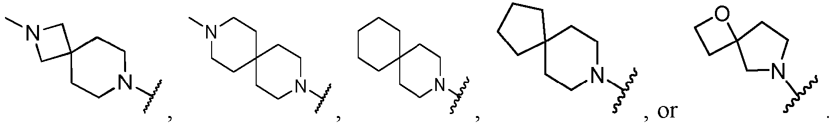

In a certain embodiment of the present disclosure, R² is H, R³ is H, -CH₃, -CH₂OH, - CH₂CH₂OH, -CH₂F, or -CF₂H.

In a certain embodiment of the present disclosure, R¹ is H;
R² is H, R³ is H, -CH₃, or -CH₂F;
R⁴ is H or halogen, m is 0 or 1;
R⁵ and R⁷ are each independently H or F;
R⁶ is a 8- to 11-membered heterocycloalkyl with 1, 2, or 3 heteroatoms selected from 1, 2, or 3 types of N, O, and S or a 8- to 11-membered heterocycloalkyl with 1, 2, or 3 heteroatoms selected from 1, 2, or 3 types of N, O, and S substituted by 1, 2, or 3 R⁶⁻²:
each R⁶⁻² is independently hydroxyl or C₁₋₃ alkyl;
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d} or N; R^{d} is H.

In a certain embodiment of the present disclosure, R¹ is H;
R² is H, R³ is H, -CH₃, or -CH₂F;
R⁴ is H or halogen, m is 0 or 1;
R⁵ and R⁷ are each independently H;
R⁶ is a 8- to 10-membered heterocycloalkyl with 1, 2, or 3 heteroatoms selected from 1, 2, or 3 types of N, O, and S or a 8- to 10-membered heterocycloalkyl with 1, 2, or 3 heteroatoms selected from 1, 2, or 3 types of N, O, and S substituted by 1, 2, or 3 R⁶⁻²;
R⁶⁻² is hydroxyl;
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d}; R^{d} is H.

In a certain embodiment of the present disclosure, R¹ is H;
R² is H, R³ is H, -CH₃, or -CH₂F;
R⁴ is H or halogen, m is 0 or 1;
R⁵ and R⁷ are each independently H or F;
R⁶ is a 8- to 10-membered heterocycloalkyl with 1, 2, or 3 heteroatoms selected from 1, 2, or 3 types of N, O, and S or a 8- to 10-membered heterocycloalkyl with 1, 2, or 3 heteroatoms selected from 1, 2, or 3 types of N, O, and S substituted by 1, 2, or 3 R⁶⁻²;
each R⁶⁻² is independently C₁₋₃ alkyl;
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d} or N; R^{d} is H.

In a certain embodiment of the present disclosure, R¹ is H;
R² is H, R³ is H, -CH₃, or -CH₂F;
R⁴ is H or halogen, m is 0 or 1;
R⁵ and R⁷ are each independently H or F;
R⁶ is "8- to 10-membered heterocycloalkyl with 1, 2, or 3 heteroatoms selected from 1, 2, or 3 types of N, O, and S" or "8- to 10-membered heterocycloalkyl with 1, 2, or 3 heteroatoms selected from 1, 2, or 3 types of N, O, and S" substituted by 1, 2, or 3 R⁶⁻²;
each R⁶⁻² is independently hydroxyl or C₁₋₃ alkyl;
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d} or N; R^{d} is H.

In a certain embodiment of the present disclosure, R¹ is H;
R² is H, R³ is H, -CH₃, or -CH₂F;
R⁴ is H or halogen, m is 0 or 1;
R⁵ and R⁷ are each independently H;
R⁶ is 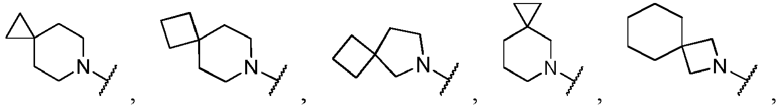 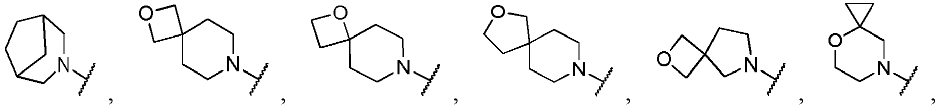 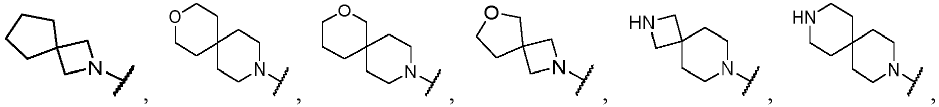 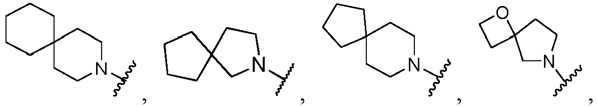 or the following group substituted by 1 R⁶⁻²: 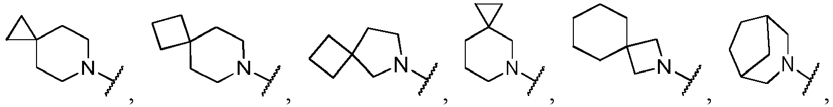 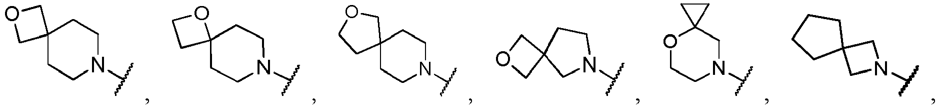 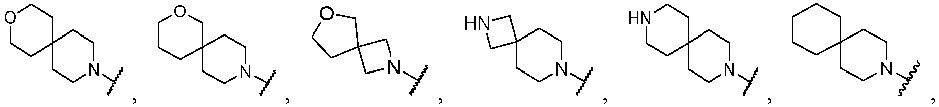 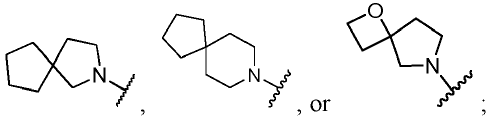
R⁶⁻² is hydroxyl;
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d}; R^{d} is H.

In a certain embodiment of the present disclosure, R¹ is H;
R² is H, R³ is H, -CH₃, or -CH₂F;
R⁴ is H or halogen, m is 0 or 1;
R⁵ and R⁷ are each independently H or F;
R⁶ is 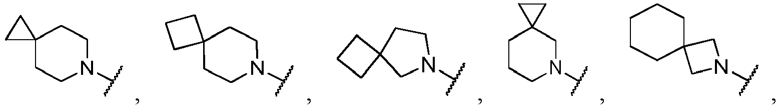 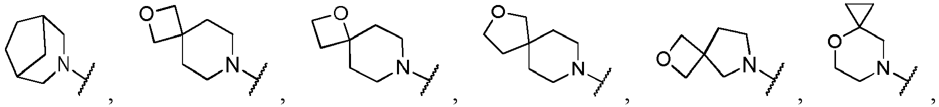 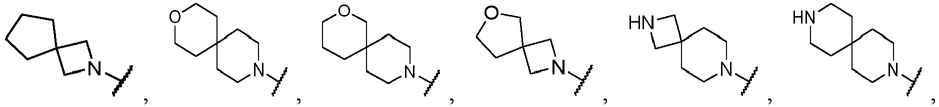 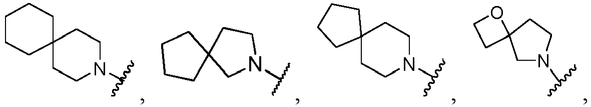 or the following group substituted by 1 R⁶⁻²: 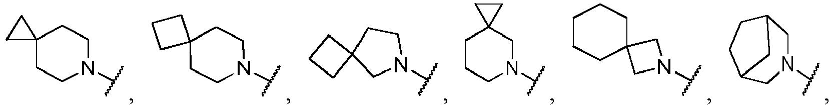 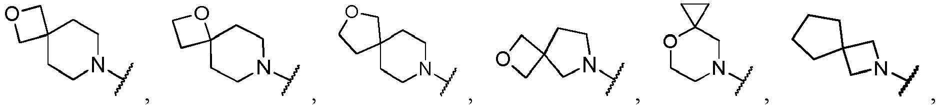 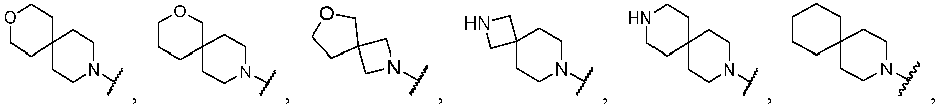 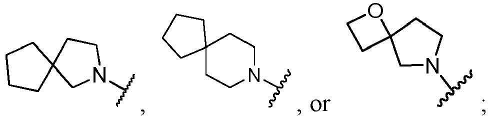
R⁶⁻² is methyl;
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d} or N; R^{d} is H.

In a certain embodiment of the present disclosure, R¹ is H;
R² is H, R³ is H, -CH₃, or -CH₂F;
R⁴ is H or halogen, m is 0 or 1;
R⁵ and R⁷ are each independently H or F;
R⁶ is 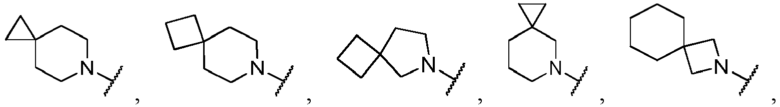 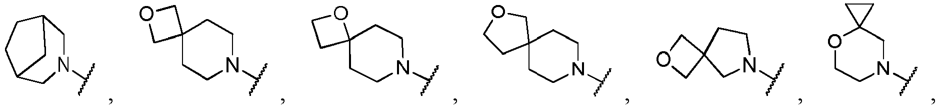 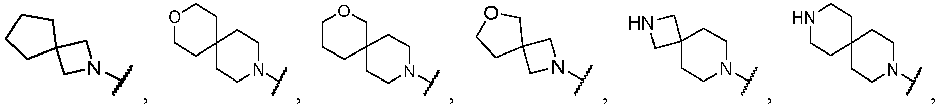 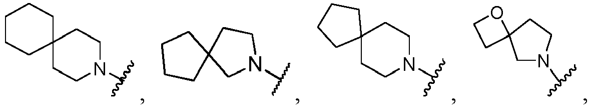 or the following group substituted by 1 R⁶⁻²: 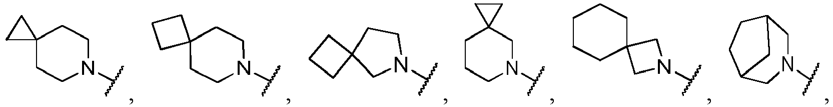 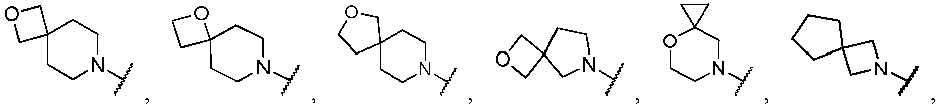 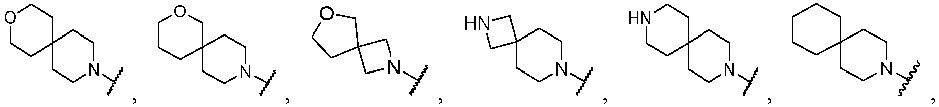 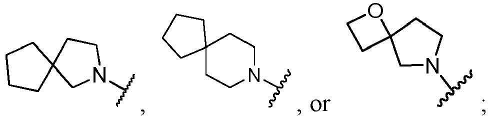
R⁶⁻² is hydroxyl or methyl;
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d} or N; R^{d} is H.

In a certain embodiment of the present disclosure, R¹ is H;
R² is H, R³ is H, -CH₃, or -CH₂F;
R⁴ is H or F, m is 0 or 1;
R⁵ and R⁷ are each independently H or F;
R⁶ is 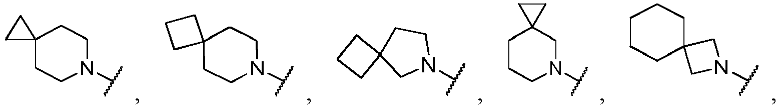 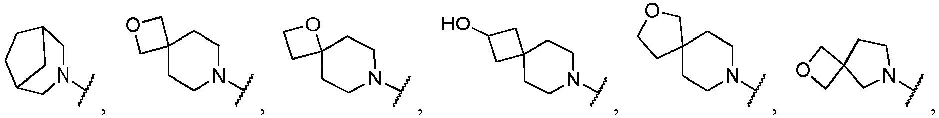 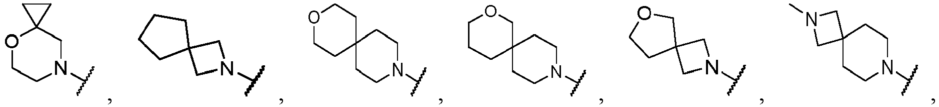 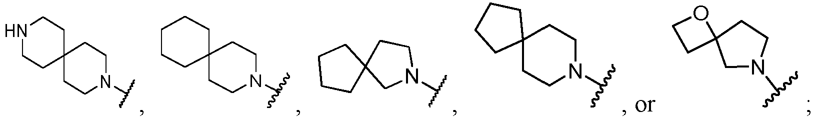
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d} or N; R^{d} is H.

In a certain embodiment of the present disclosure, the compound of formula (I) is selected from any one of the following compounds:

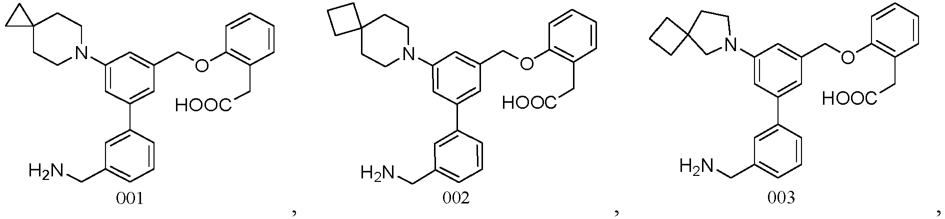

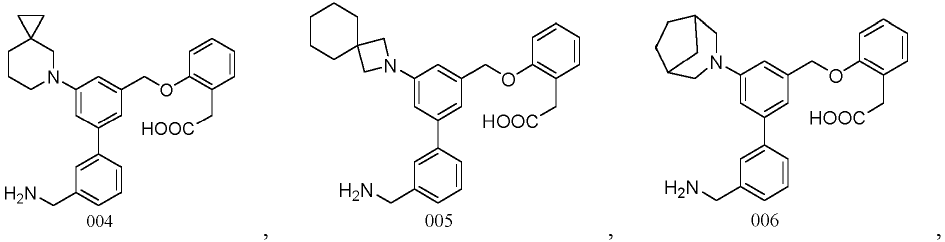

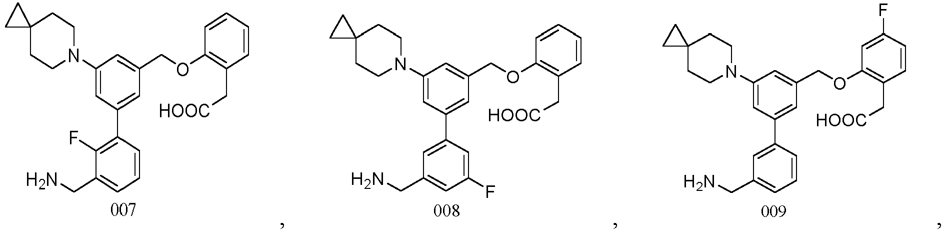

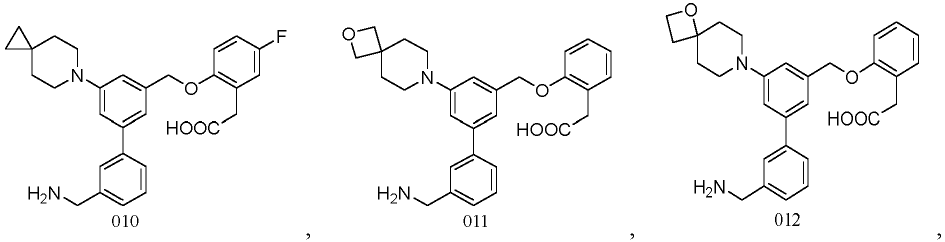

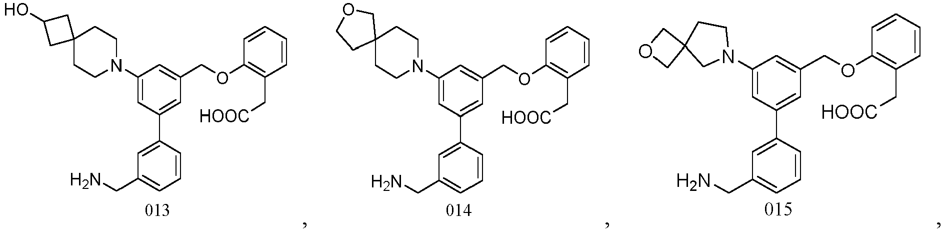

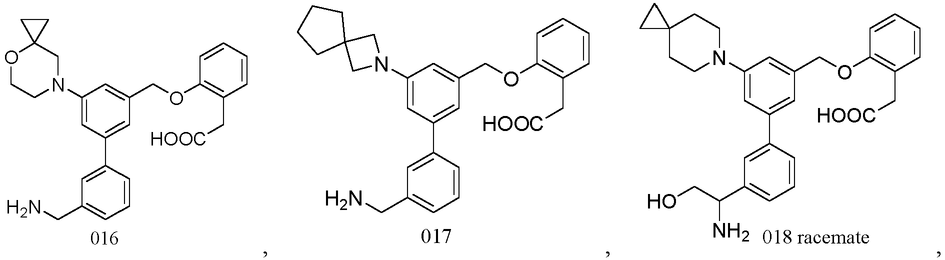

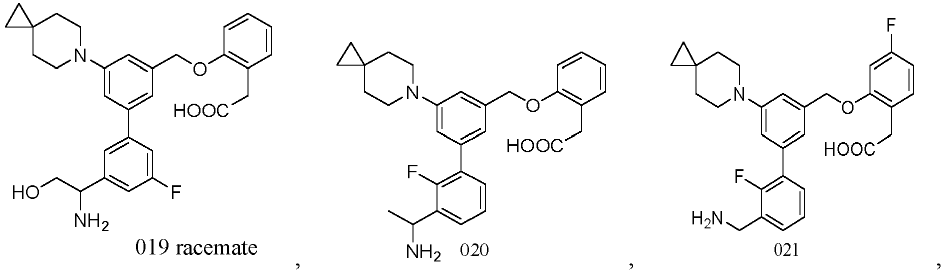

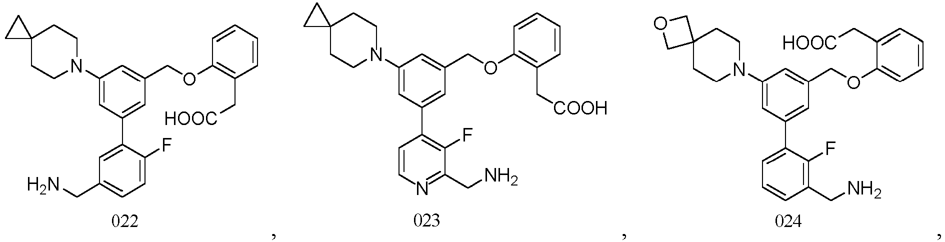

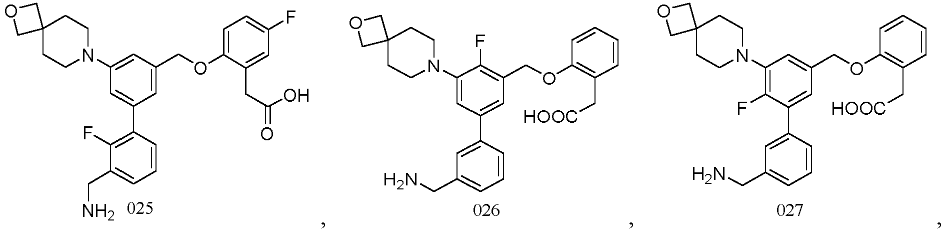

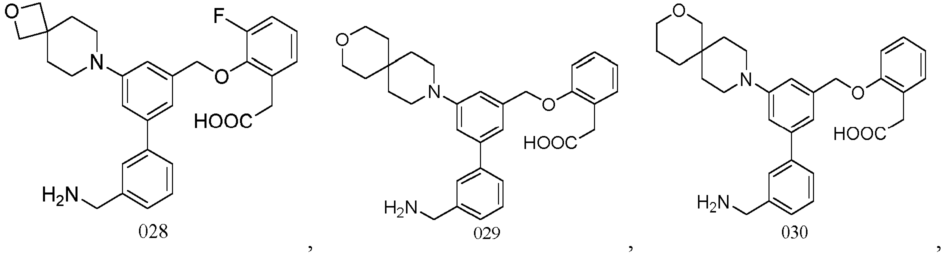

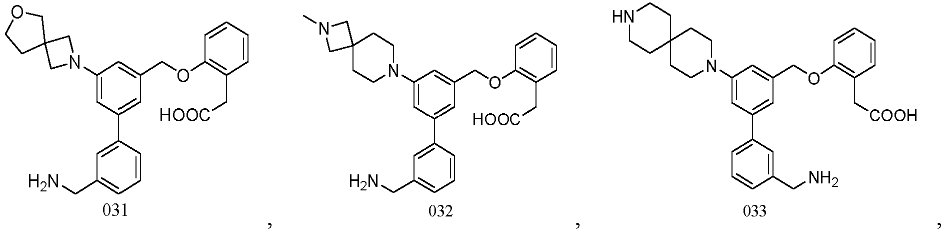

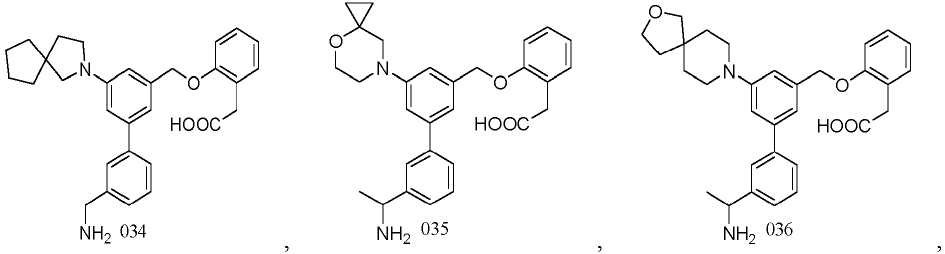

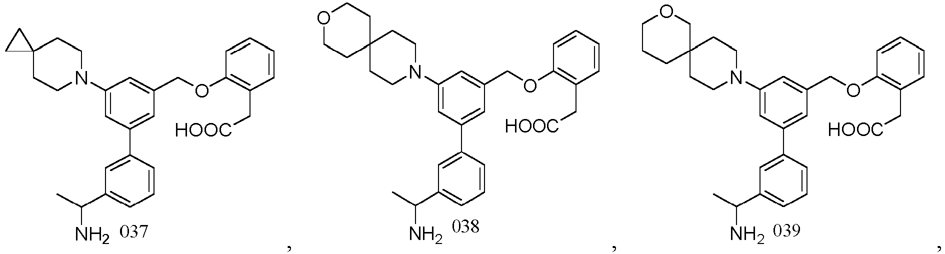

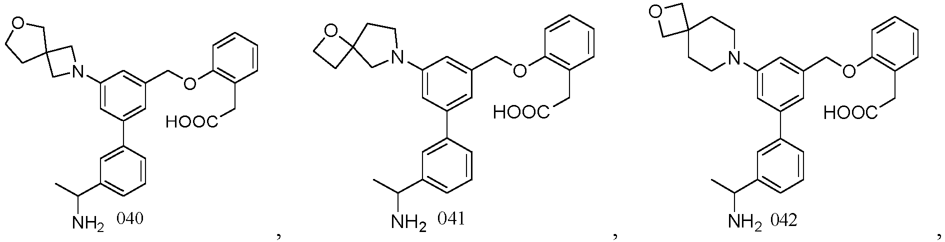

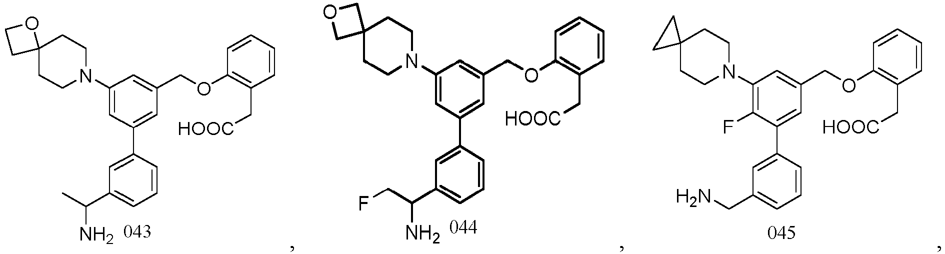

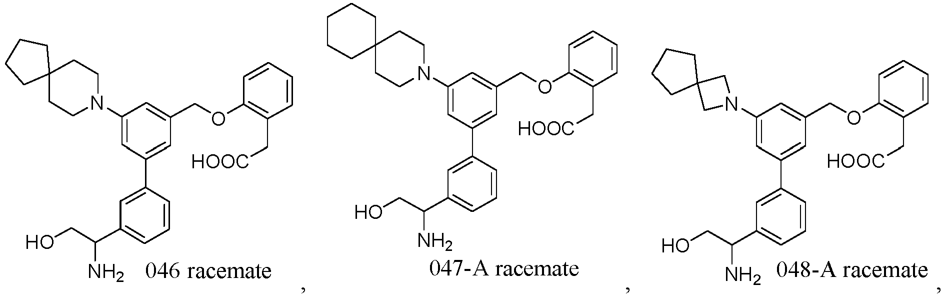

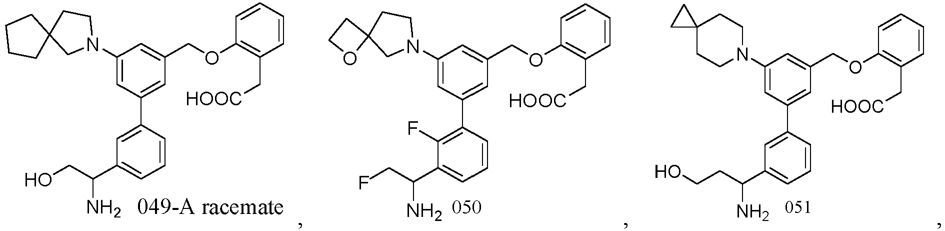

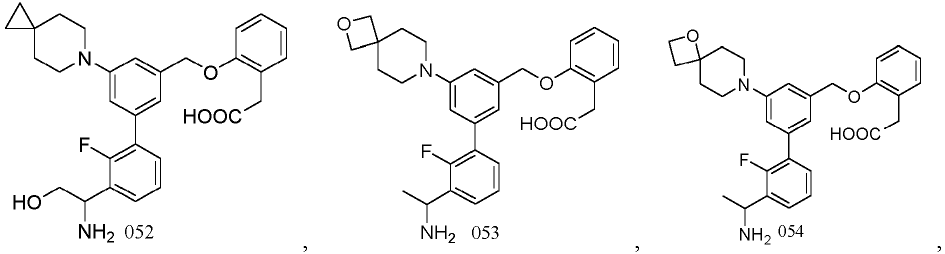

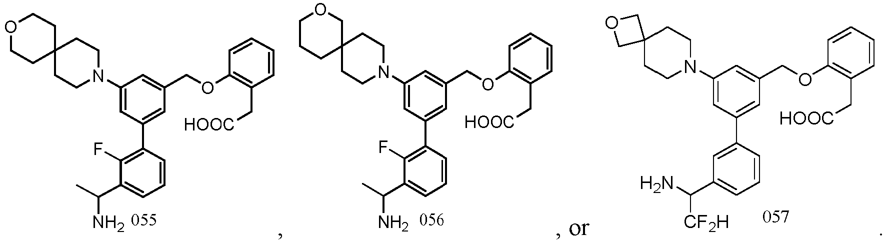

In a certain embodiment of the present disclosure, the compound of formula (I) is selected from any one of the following compounds:

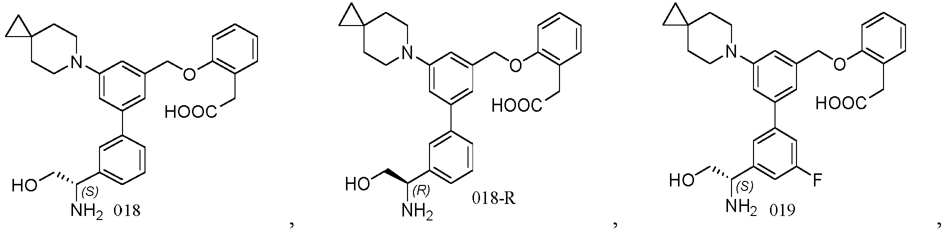

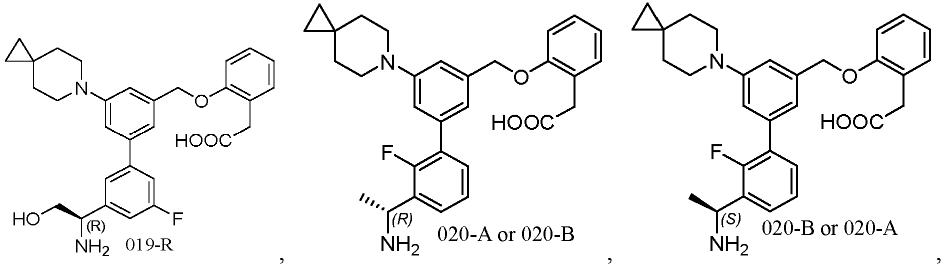

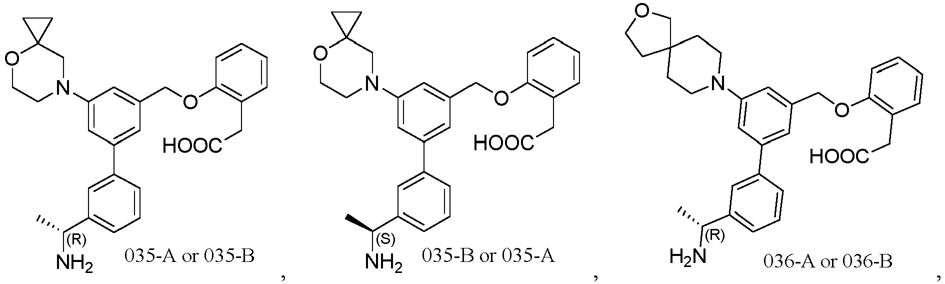

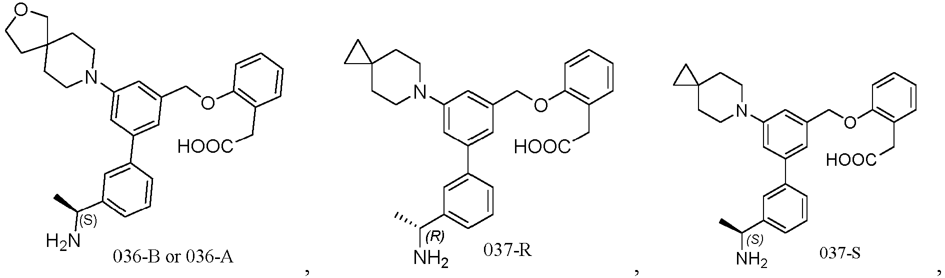

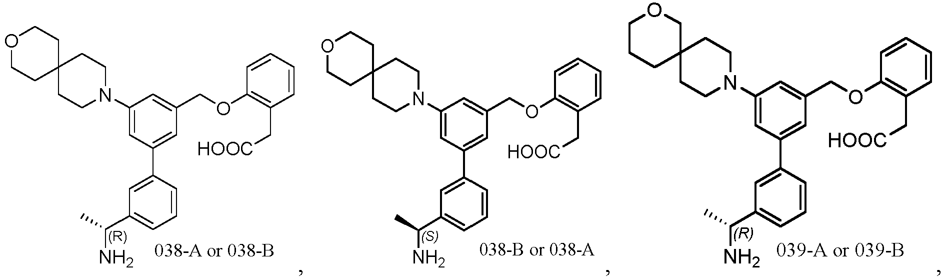

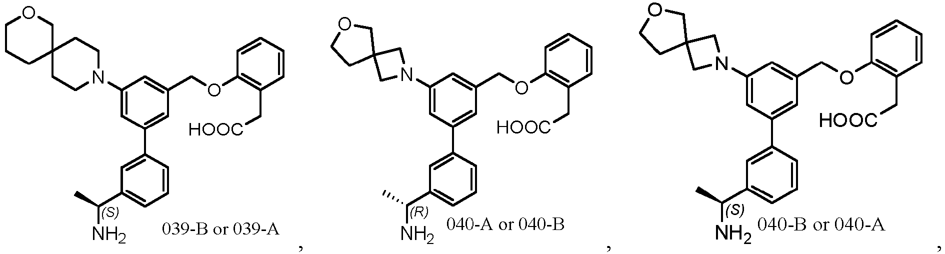

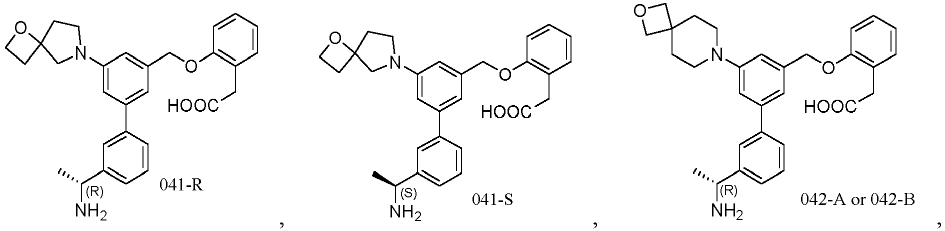

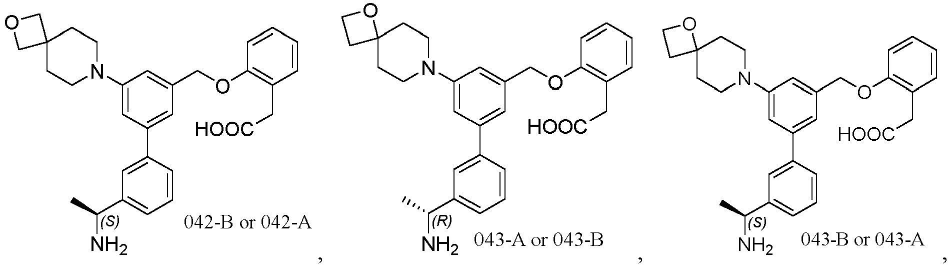

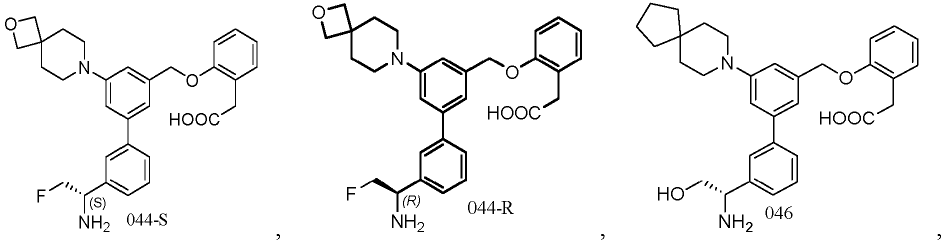

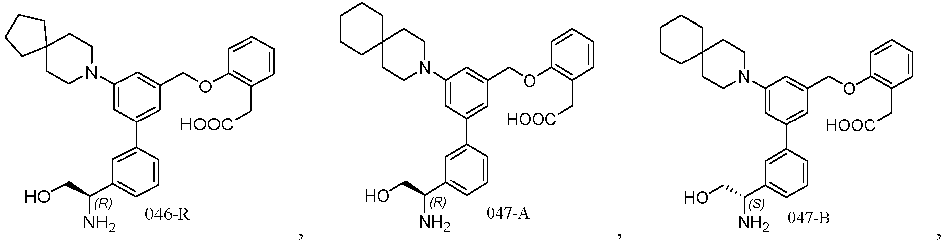

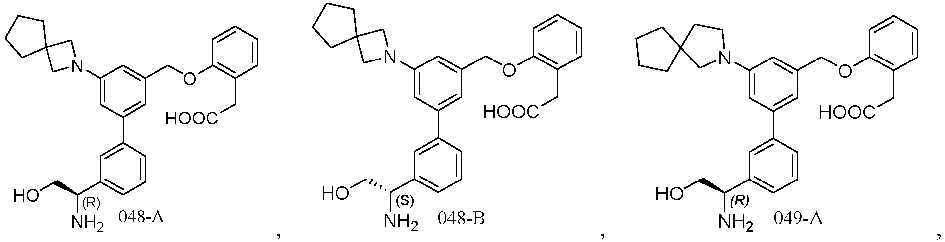

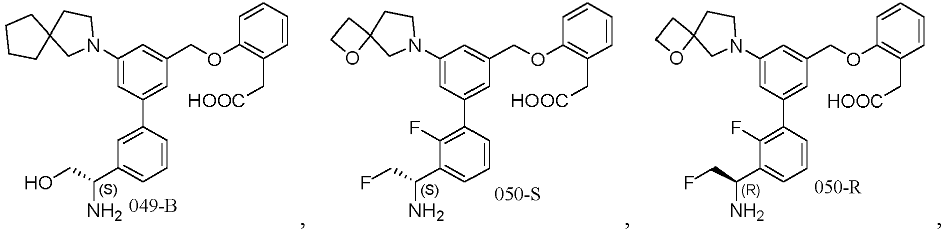

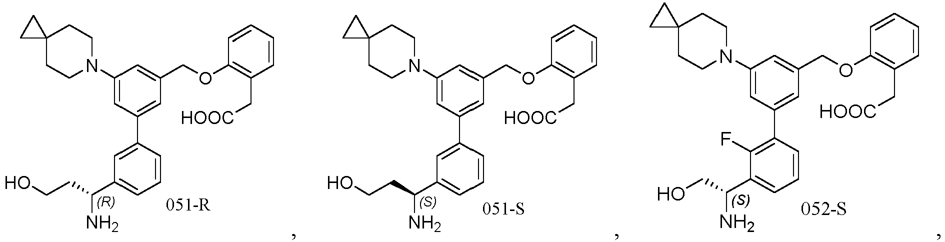

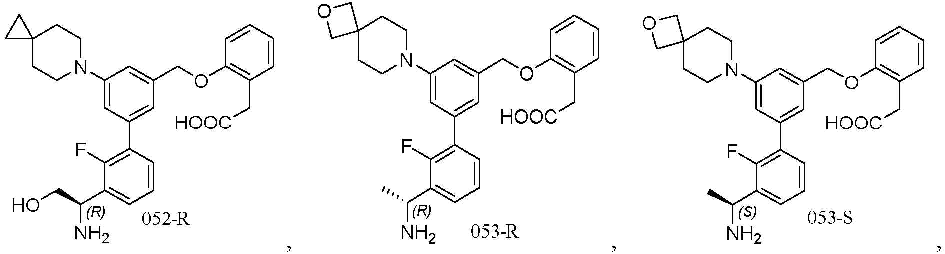

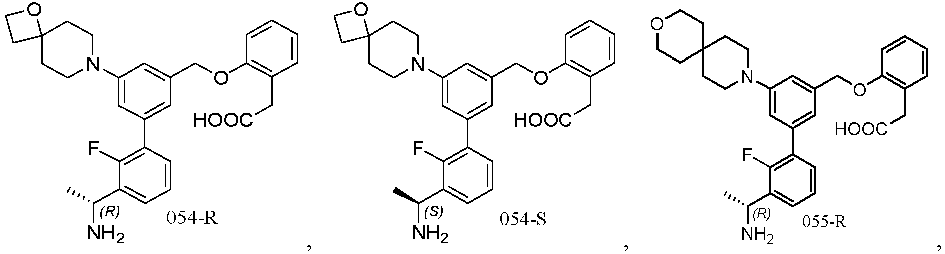

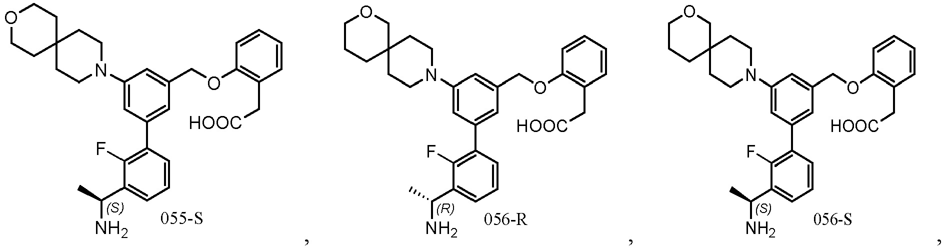

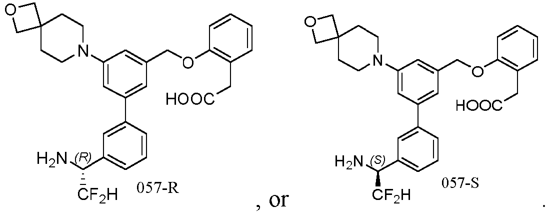

The present disclosure also provides a preparation method for a compound of formula (I), which comprises the following steps:
(1) subjecting a compound of formula II-3 to a deprotection reaction to obtain a compound of formula II-4; 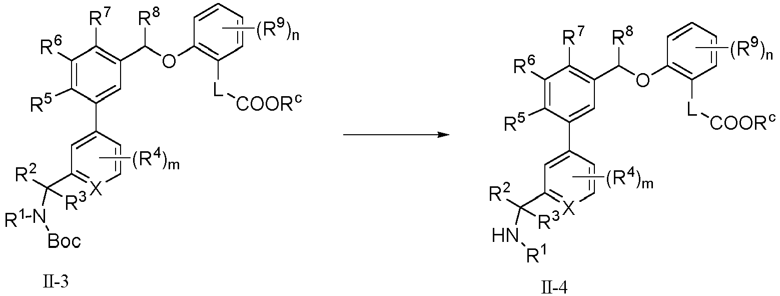
(2) subjecting the compound of formula II-4 to a hydrolysis reaction to obtain the compound of formula (I); 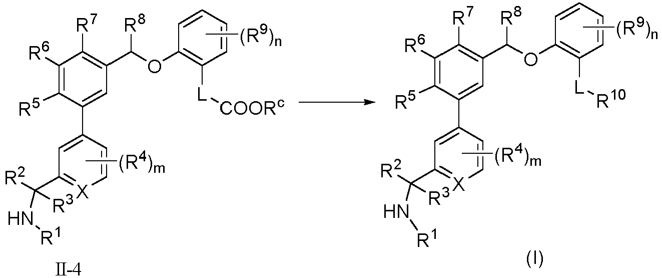
wherein R¹⁰ is -COOH, R^{c} is C₁₋₆ alkyl or C₁₋₆ alkyl substituted by 1, 2, or 3 R^{c-1}; each R^{c-1} is independently halogen, -OH, or -C(=O)OC(CH₃)₃, and R¹, R², R³, R⁴, R⁵, R⁶, R⁷, R⁸, R⁹, X, L, m, and n are defined as in any one of the embodiments of the present disclosure, and the conditions and operations of the deprotection reaction and hydrolysis reaction can be the same as those commonly used for such reactions in the art; preferably, R¹ is H.

In a certain embodiment of the present disclosure, the preparation method for the compound of formula (I) comprises the following steps: 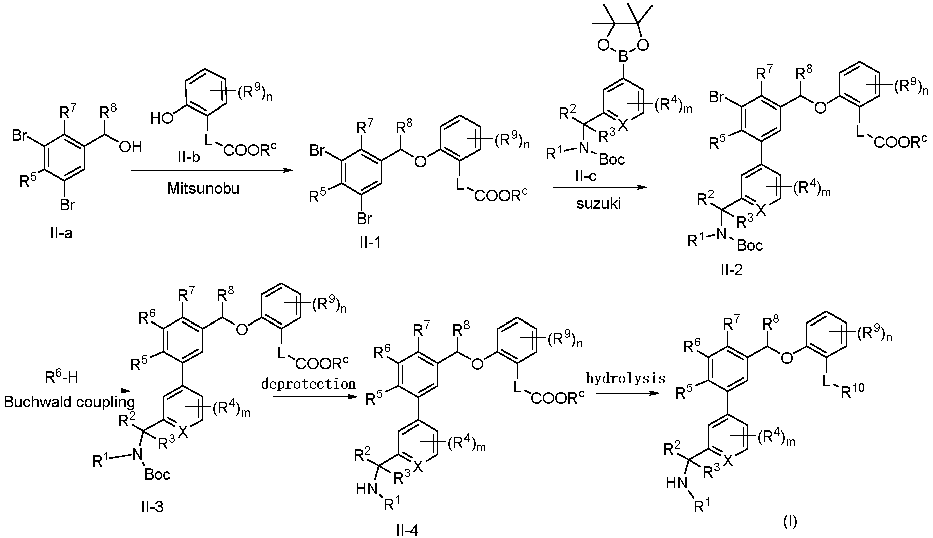 wherein R¹⁰ is -COOH, R^{c} is C₁₋₆ alkyl or C₁₋₆ alkyl substituted by 1, 2, or 3 R^{c-1}; each R^{c-1} is independently halogen, -OH, or -C(=O)OC(CH₃)₃, and R¹, R², R³, R⁴, R⁵, R⁶, R⁷, R⁸, R⁹, X, L, m, and n are defined as in any one of the embodiments of the present disclosure, and the conditions and operations of the above reactions can be the same as those commonly used for such reactions in the art; preferably, R¹ is H.

The present disclosure also provides a compound of formula II-3 or formula II-4: 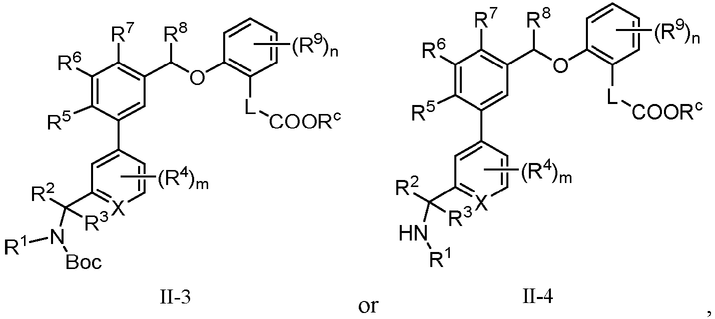 wherein R^{c} is C₁₋₆ alkyl or C₁₋₆ alkyl substituted by 1, 2, or 3 R^{c-1}; each R^{c-1} is independently halogen, -OH, or -C(=O)OC(CH₃)₃, and R¹, R², R³, R⁴, R⁵, R⁶, R⁷, R⁸, R⁹, X, L, m, and n are defined as in any one of the embodiments of the present disclosure.

In a certain embodiment of the present disclosure, in the compound of formula II-3 or formula II-4, R¹ is H.

The present disclosure also provides a compound of any one of the following: 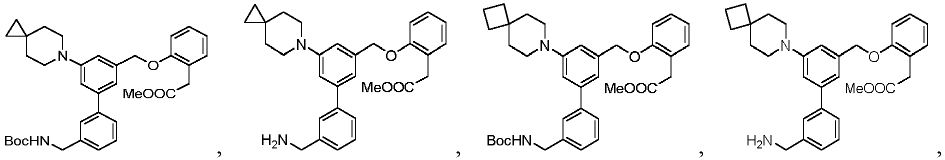 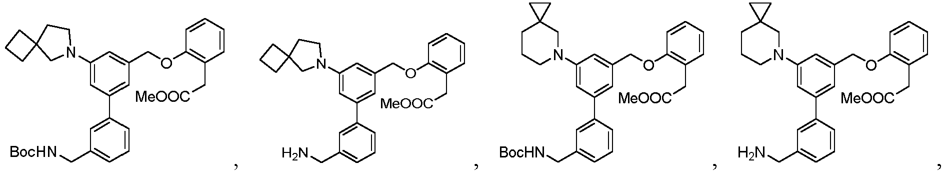 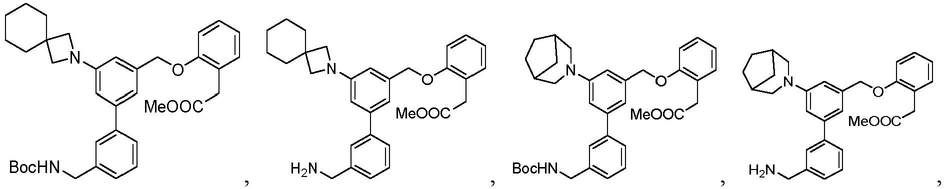 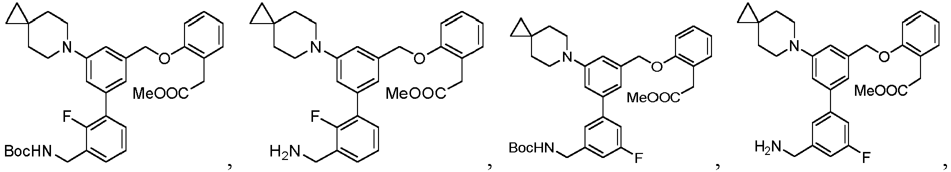 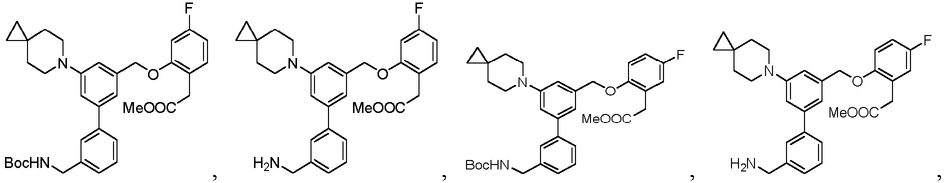 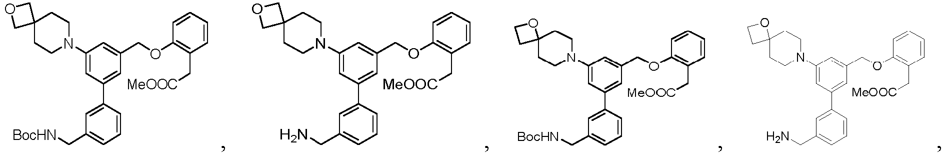 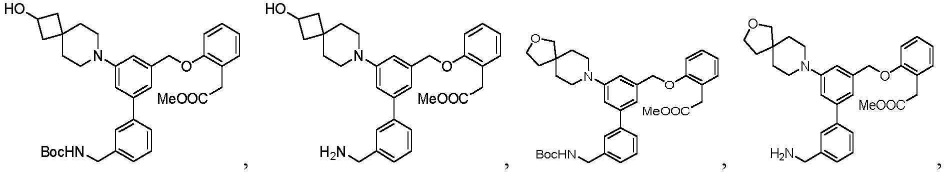 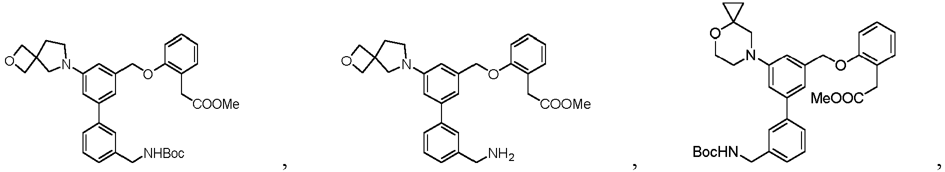 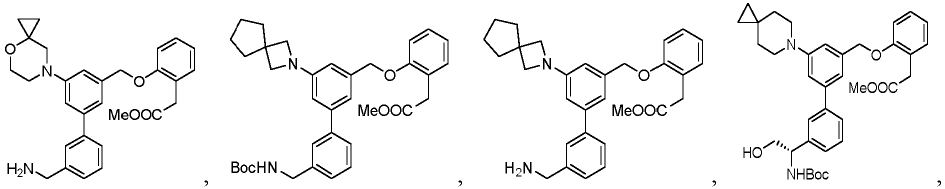 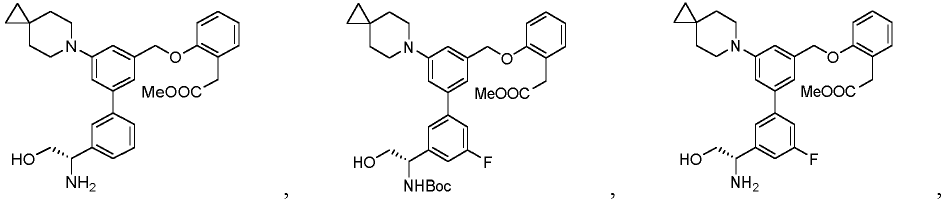 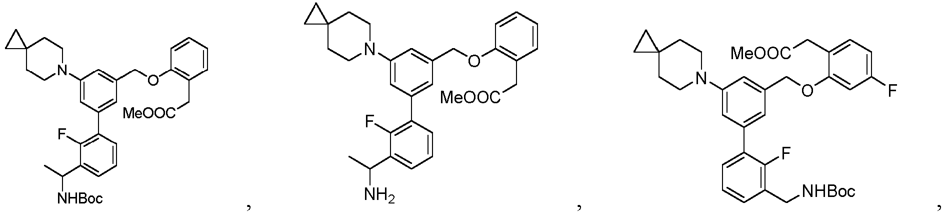 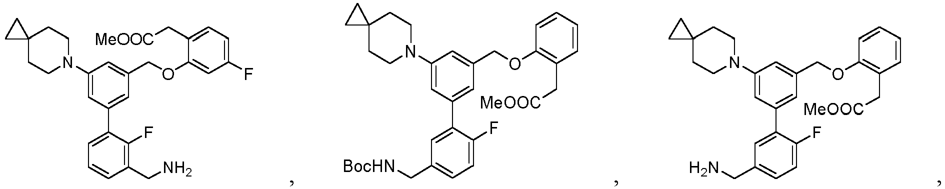 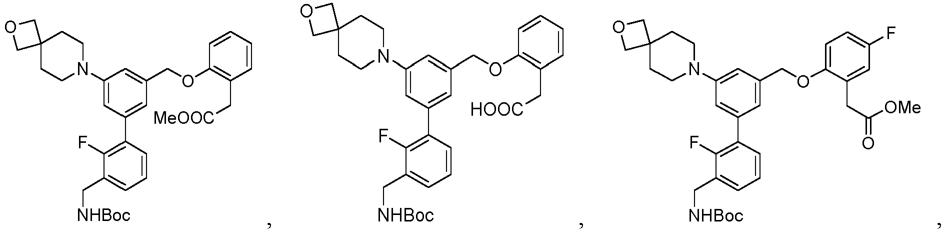 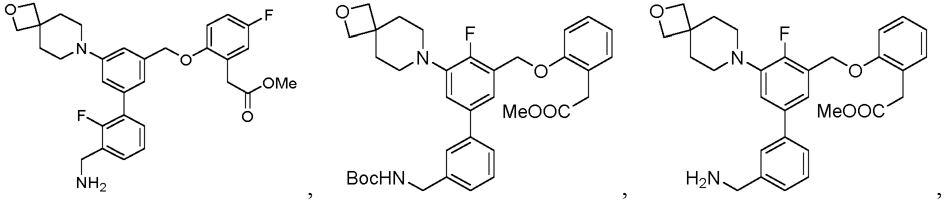 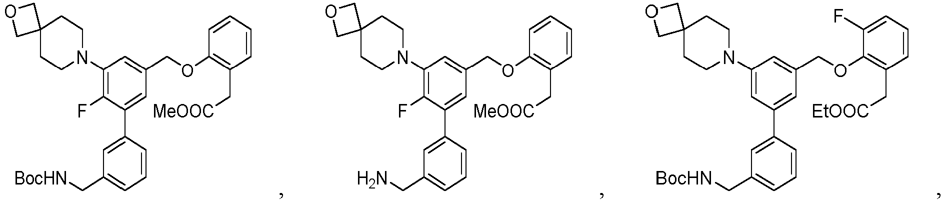 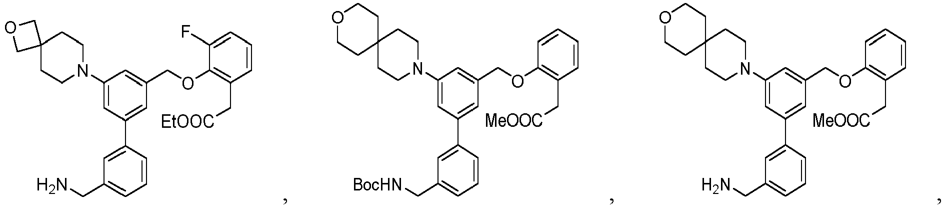 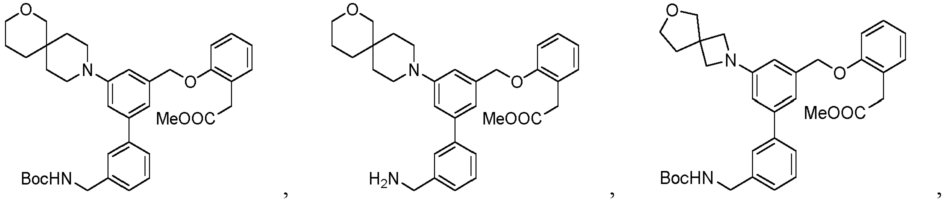 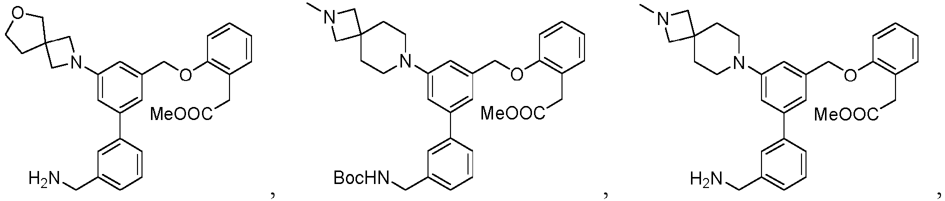 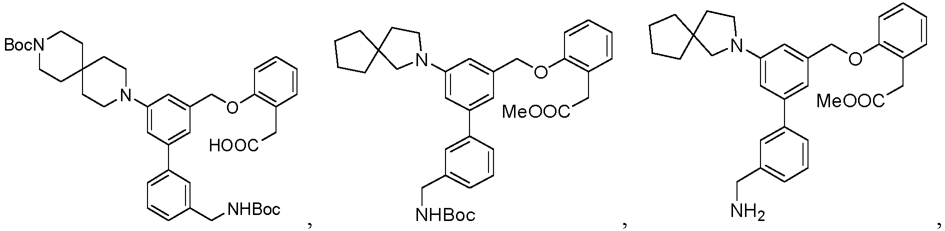 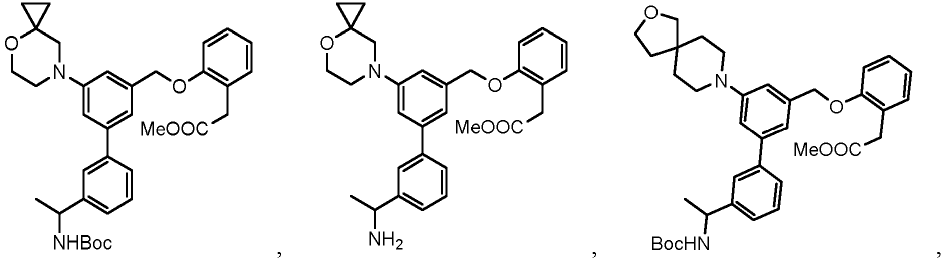 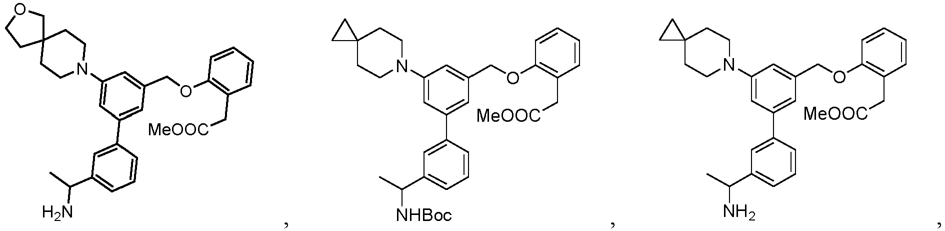 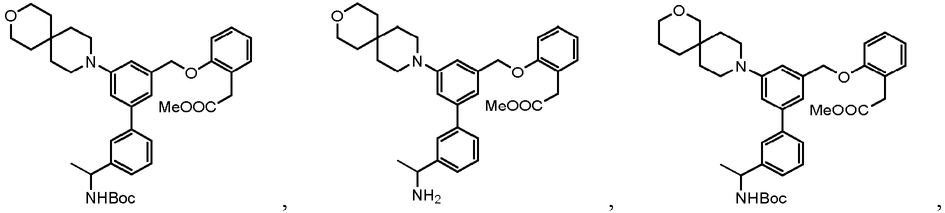 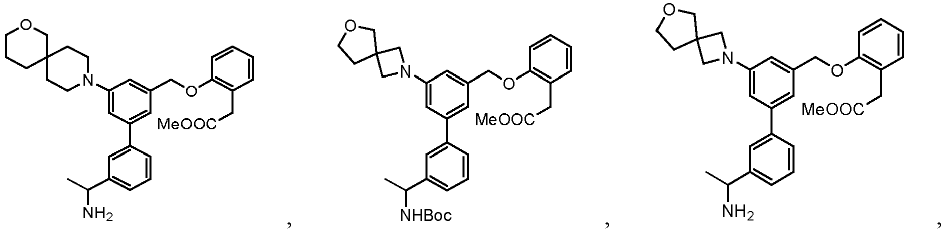 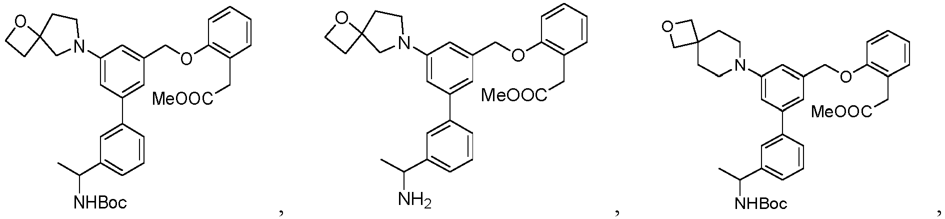 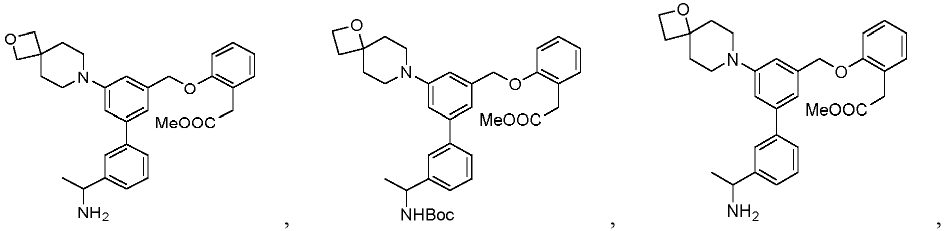 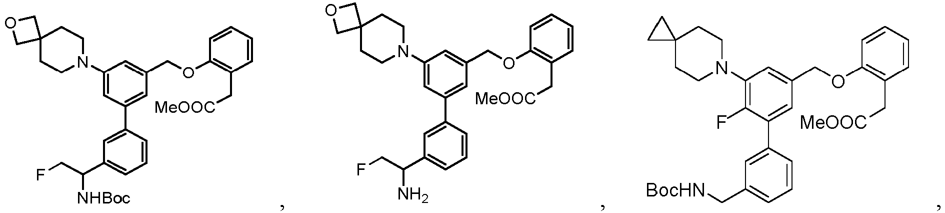 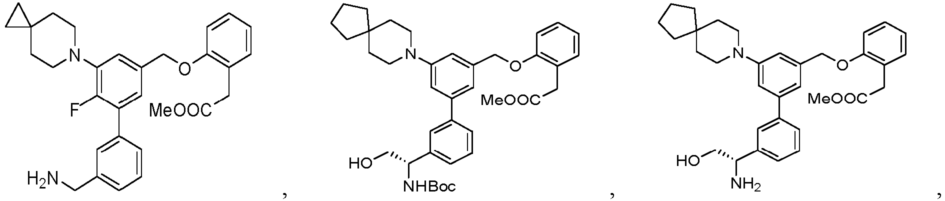 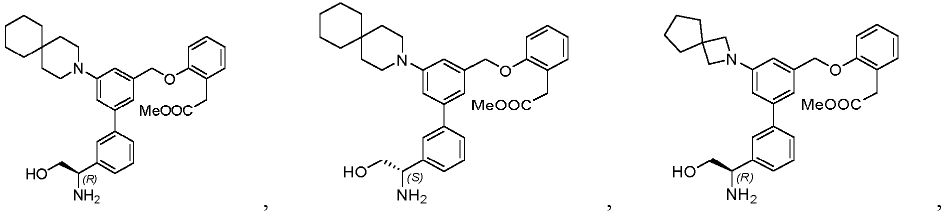 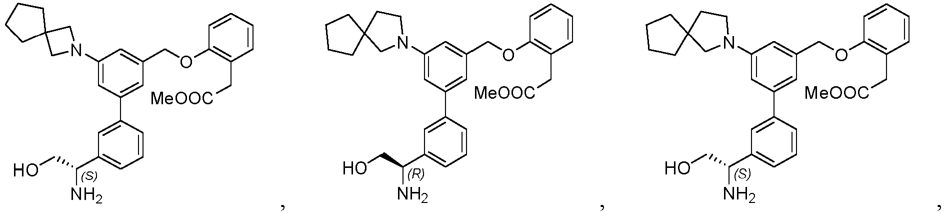 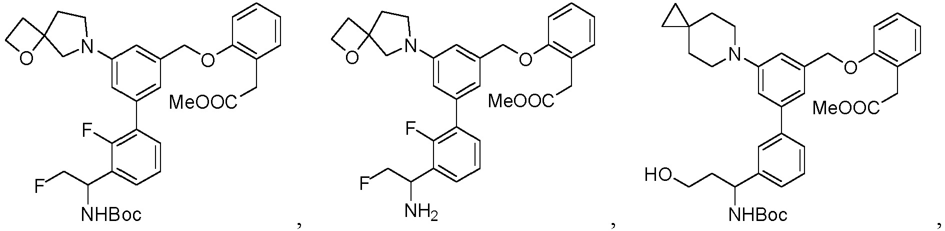 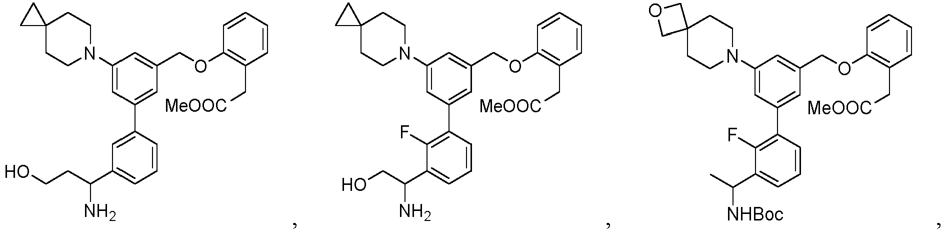 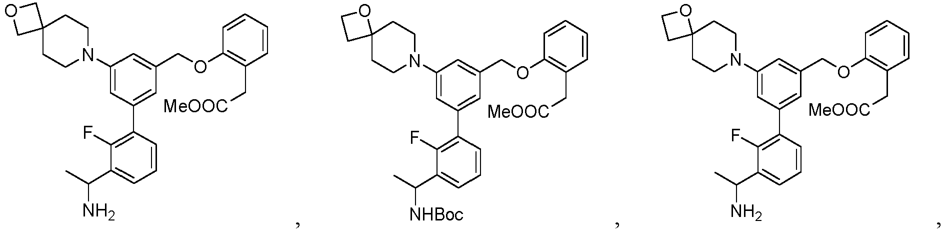 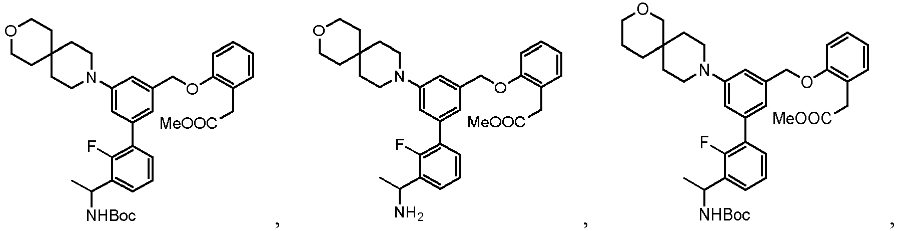 or

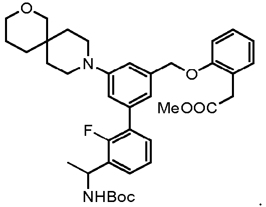

The present disclosure also provides a pharmaceutical composition, which comprises:
(1) the compound of formula (I), the tautomer thereof, the stereoisomer thereofor the pharmaceutically acceptable salt of any one of the foregoing (referring to the foregoing compound of formula (I), the tautomer thereof, the stereoisomer thereof), or the solvate of any one of the foregoing (referring to the foregoing compound of formula (I), the tautomer thereof, the stereoisomer thereof, or the pharmaceutically acceptable salt of any one of the foregoing) as described in any one of the foregoing; and
(2) a pharmaceutically acceptable carrier.

The present disclosure also provides a use of the compound of formula (I), the tautomer thereof, the stereoisomer thereof, or the pharmaceutically acceptable salt of any one of the foregoing (referring to the foregoing compound of formula (I), the tautomer thereof, the stereoisomer thereof), or the solvate of any one of the foregoing (referring to the foregoing compound of formula (I), the tautomer thereof, the stereoisomer thereof, or the pharmaceutically acceptable salt of any one of the foregoing) as described in any one of the foregoing, or the pharmaceutical composition as described in any one of the foregoing in the manufacture of a medicament for the treatment and/or prevention of a disease mediated by complement factor D.

The present disclosure also provides a method for treating and/or preventing a disease mediated by complement factor D, which comprises: administering to an individual in need thereof a therapeutically effective amount of a substance X or the pharmaceutical composition as described in any one of the foregoing, wherein the substance X is the compound of formula (I), the tautomer thereof, the stereoisomer thereof, or the pharmaceutically acceptable salt of any one of the foregoing (referring to the foregoing compound of formula (I), the tautomer thereof, the stereoisomer thereof), or the solvate of any one of the foregoing (referring to the foregoing compound of formula (I), the tautomer thereof, the stereoisomer thereof, or the pharmaceutically acceptable salt of any one of the foregoing) as described in any one of the foregoing. Preferably, the individual is a patient according to the present disclosure.

In a certain embodiment of the present disclosure, the disease mediated by complement factor D comprises a hematological disorder, a renal disease, a cardiovascular disease, an immunological disorder, a disease of the central nervous system, a respiratory disease, a urogenital system disease, or an ocular disorder. Preferably, the disease mediated by complement factor D is, for example, a hematological disorder, a renal disease, a cardiovascular disease, an immunological disorder, a disease of the central nervous system, or an ocular disorder.

In a certain embodiment of the present disclosure, the disease mediated by complement factor D is cold agglutinin disease, catastrophic antiphospholipid syndrome, hemolytic anemia, antineutrophil cytoplasmic antibody (ANCA)-associated vasculitis (AAV), warm autoimmune hemolytic anemia, paroxysmal nocturnal hemoglobinuria, IgA nephropathy, lupus nephritis, atypical hemolytic uremic syndrome, membranoproliferative glomerulonephritis (MPGN), dense-deposit disease, C3 glomerulonephritis, focal segmental glomerulosclerosis, diabetic nephropathy, systemic lupus or lupus erythematosus, rheumatoid arthritis, inflammatory bowel disease, psoriasis, multiple sclerosis, organ transplant rejection, myasthenia gravis, Alzheimer's disease, respiratory distress syndrome, asthma, chronic obstructive pulmonary disease, emphysema, coronavirus infection (such as SARS-CoV, MERS-CoV, or SARS-CoV-2 infection), macular degeneration, age-related macular degeneration (AMD), macular edema, diabetic macular edema, choroidal neovascularization (CNV), uveitis, Behcet's uveitis, proliferative diabetic retinopathy, non-proliferative diabetic retinopathy, glaucoma, hypertensive retinopathy, corneal neovascularization disease, postcorneal transplant rejection, corneal dystrophy disease, autoimmune dry eye disease, Stevens-Johnson syndrome, Sjögren's syndrome, environmental dry eye disease, Fuchs' endothelial dystrophy, retinal vein occlusion, or postoperative inflammation.

In other embodiments of the present disclosure, the immunological disorder is: lupus, allograft rejection, autoimmune thyroid disease (such as Graves' disease and Hashimoto's thyroiditis), autoimmune uveoretinitis, giant cell arteritis, inflammatory bowel disease (including Crohn's disease, ulcerative colitis, limited enteritis, granulomatous enteritis, distal ileitis, limited ileitis, and terminal ileitis), diabetes, multiple sclerosis, pernicious anemia, psoriasis, rheumatoid arthritis, sarcoidosis, scleroderma, etc.

Further, the disease mediated by complement factor D includes, but is not limited to, paroxysmal nocturnal hemoglobinuria, IgA nephropathy, lupus nephritis, atypical hemolytic uremic syndrome, organ transplant rejection, myasthenia gravis, neuromyelitis optica, membranoproliferative glomerulonephritis, dense deposit disease, cold agglutinin disease and catastrophic antiphospholipid syndrome, C3 glomerulonephritis and focal segmental glomerulosclerosis, macular degeneration, age-related macular degeneration (AMD), macular edema, diabetic macular edema, etc.

The present disclosure also provides a use of the compound of formula (I), the tautomer thereof, the stereoisomer thereof, or the pharmaceutically acceptable salt of any one of the foregoing (referring to the foregoing compound of formula (I), the tautomer thereof, the stereoisomer thereof), or the solvate of any one of the foregoing (referring to the foregoing compound of formula (I), the tautomer thereof, the stereoisomer thereof, or the pharmaceutically acceptable salt of any one of the foregoing) as described in any one of the foregoing, or the pharmaceutical composition as described in any one of the foregoing in the manufacture of a complement factor D inhibitor medicament.

In the use, the complement factor D inhibitor medicament can be used in mammalian organisms *in vivo;* it can also be used *in vitro,* mainly for experimental purposes, such as providing comparison as a standard sample or a control sample, or making a kit according to the conventional method in the art to provide rapid detection for the inhibition effect of complement factor D.

Unless otherwise specified, the terms used in the present disclosure have the following meanings:
Those skilled in the art can understand that according to the conventions used in the art, the " 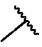 '' " used in the structural formula of the group described in the present disclosure means that the corresponding group is connected to other moieties and groups in the compound through this site.

The substituent used herein may be preceded by a single dash "-" to indicate that the named substituent is connected to the parent moiety through a single bond. When the linking groups listed in the present disclosure do not specify the linking direction, the linking direction is in the same direction as the reading order from left to right.

The term "pharmaceutically acceptable" means that salts, solvents, excipients, etc. are generally non-toxic, safe, and suitable for use by patients. The "patient" is preferably a mammal, more preferably a human. The term "mammal" includes any mammal. Examples of mammals include, but are not limited to, cows, horses, sheep, pigs, cats, dogs, mice, rats, rabbits, guinea pigs, monkeys, humans, etc.

The term "pharmaceutically acceptable salt" refers to the salt prepared by the compound of the present disclosure and a relatively nontoxic and pharmaceutically acceptable acid or base. When the compound of the present disclosure contains a relatively acidic functional group, a base addition salt can be obtained by bringing the neutral form of the compound into contact with a sufficient amount of the pharmaceutically acceptable base in a pure solution or a suitable inert solvent. Pharmaceutically acceptable base addition salts include, but are not limited to, lithium salts, sodium salts, potassium salts, calcium salts, aluminum salts, magnesium salts, zinc salts, bismuth salts, ammonium salts, and diethanolamine salts. When the compound of the present disclosure contains a relatively basic functional group, an acid addition salt can be obtained by bringing the neutral form of the compound into contact with a sufficient amount of pharmaceutically acceptable acid in a pure solution or a suitable inert solvent. The pharmaceutically acceptable acids include inorganic acids, including but not limited to: hydrochloric acid, hydrobromic acid, hydroiodic acid, nitric acid, carbonic acid, phosphoric acid, phosphorous acid, sulfuric acid, etc. The pharmaceutically acceptable acids include organic acids, including but not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acid citric acid, oleic acid, tannic acid, pantothenic acid, hydrogen tartrate, ascorbic acid, gentisic acid, fumaric acid, gluconic acid, sugar acid, formic acid, ethanesulfonic acid, pamoic acid (i.e., 4,4'-methylene-bis(3-hydroxy-2-naphthoic acid)), amino acids (e.g., glutamic acid, arginine), etc. When the compounds of the present disclosure contain relatively acidic and relatively basic functional groups, they can be converted into base addition salts or acid addition salts. For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to a substance formed by combining the compound of the present disclosure with a stoichiometric or non-stoichiometric solvent. Solvent molecules in the solvate can exist in an ordered or unordered arrangement. The solvent includes, but is not limited to, water, methanol, ethanol, etc.

The "pharmaceutically acceptable salt" and "solvate" in the term "solvate of pharmaceutically acceptable salt" are defined as above, and the "solvate of pharmaceutically acceptable salt" refers to a substance formed by combining the compound of the present disclosure: 1, with a relatively nontoxic and pharmaceutically acceptable acid or base, 2, with a stoichiometric or non-stoichiometric solvent. The "solvate of pharmaceutically acceptable salt" includes, but is not limited to, the hydrochloride monohydrate of the compound of the present disclosure.

When an arbitrary variable (e.g., R¹⁻¹) appears many times in the definition of a compound, the definition of each occurrence of the variable has nothing to do with the definitions of other occurrences, and their meanings are independent of each other and have no influence on each other. Therefore, if a group is substituted by 1, 2, or 3 R¹⁻¹ groups, that is, the group may be substituted by up to 3 R¹⁻¹ groups, and the definition of R¹⁻¹ groups of this position and the definition of R¹⁻¹ groups of the remaining positions are independent of each other. Additionally, combinations of substituents and/or variables are permissible only if such combinations result in stable compounds.

The term "A-membered", where A is an integer, usually describes that the number of ring-forming atoms is A. For example, piperidinyl is an example of 6-membered heterocycloalkyl, cyclopropyl is an example of 3-membered cycloalkyl, phenyl is an example of 6-membered aryl, etc.

The term "A- to B-membered", where A and B are integers, usually describes that the number of ring-forming atoms is in the range of A to B.

The term "halogen" refers to fluorine, chlorine, bromine, or iodine.

The term "alkyl" refers to a linear or branched saturated hydrocarbon group with a specified number of carbon atoms. In some embodiments, the alkyl is C₁₋₆ alkyl, such as C₁₋₅ alkyl, C₁₋₄ alkyl, C₁₋₃ alkyl, and C₁₋₂ alkyl; in other embodiments, the alkyl is C₁₋₃ alkyl. Examples of alkyl include, but are not limited to, methyl, ethyl, n-propyl, isopropyl, n-butyl, tert-butyl, isobutyl, sec-butyl, n-pentyl, n-hexyl, and similar alkyl groups.

The term "alkoxy" refers to the group -O-R^{X}, wherein R^{X} is the alkyl as defined above.

The term "cycloalkyl" refers to a saturated, monocyclic, bridged, or spirocyclic cyclic group with a specified number of ring carbon atoms (e.g., C₃₋₆, C₇₋₁₂), the ring atoms only consisting of carbon atoms. Monocycloalkyl includes, but is not limited to, cyclopropyl, cyclobutyl, cyclopentyl, cyclohexyl, etc.

The term "cycloalkylene" refers to a group formed by the elimination of two hydrogens from a saturated, monocyclic, bridged, or spirocyclic cyclic alkane with a specified number of ring carbon atoms (e.g., C₃₋₆, C₇₋₁₂), the ring atoms only consisting of carbon atoms, and the two eliminated hydrogens can be on the same atom or on different atoms. Monocycloalkylene includes, but is not limited to, cyclopropylene (e.g., 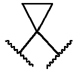 or 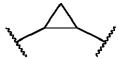 ), cyclobutylene (e.g., 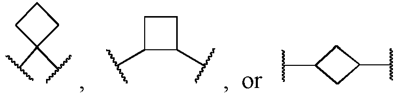 ), cyclopentylene (e.g., 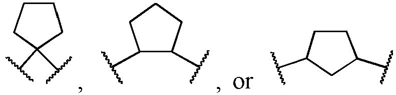 ), cyclohexylene (e.g., 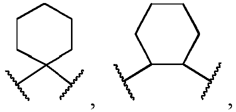 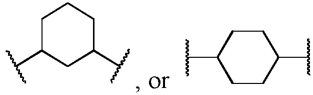 ), etc.

The term "heterocycloalkyl" refers to a saturated cyclic group with a specified number of ring atoms (for example, 7 to 12 ring atoms) containing at least 1 heteroatom independently selected from nitrogen, oxygen, and sulfur, which is a monocyclic, bridged, or spirocyclic system. Preferably, the number of heteroatoms in the heterocycloalkyl is 1, 2, or 3. The carbon atoms and heteroatoms of heterocycloalkyl may optionally be oxidized to form oxo or sulfide groups or other oxidative bonds (e.g., C(=O), S(=O), S(=O)₂, or N-oxides, etc.), or the nitrogen atoms may be quaternized. Heterocycloalkyl can be linked to other moieties and groups in the compound through ring-forming carbon atoms or ring-forming heteroatoms. In some embodiments, the heterocycloalkyl is 8- to 11-membered heterocycloalkyl with 1 or 2 heteroatoms selected from 1, 2, or 3 types of N, O, and S; in other embodiments, the heterocycloalkyl is 8- to 10-membered heterocycloalkyl with 1 or 2 heteroatoms selected from 1, 2, or 3 types of N, O, and S. Heterocycloalkyl includes, but is not limited to, azetidinyl, tetrahydropyrrolyl, tetrahydrofuranyl, morpholinyl, piperidinyl, 6-azaspiro[2.5]octyl, 5-azaspiro[2.5]octyl, 6-azaspiro[3.4]octyl, 2-azaspiro[3.4]octyl, 2-oxa-6-azaspiro[3.4]octyl, 6-oxa-2-azaspiro[3.4]octyl, 4-oxa-7-azaspiro[2.5]octyl, 2-azaspiro[4.4]nonyl, 2-azaspiro[3.5]nonyl, 2-oxa-7-azaspiro[3.5]nonyl, 1-oxa-7-azaspiro[3.5]nonyl, 7-azaspiro[3.5]nonyl, 2,7-diazaspiro[3.5]nonyl, 2-oxa-8-azaspiro[4.5]decyl, 3-oxa-9-azaspiro[5.5]undecyl, 2-oxa-9-azaspiro[5.5]undecyl, 3,9-diazaspiro[5.5]undecyl, 3-azabicyclo[3.2.1]octyl, 3-azaspiro[5,5]undecyl, 8-azaspiro[4.5]decyl, 1-oxa-6-azaspiro[3,4]octyl, etc.

The term "pharmaceutically acceptable carrier" refers to the excipients and additives used in the manufacture of drugs and the formulation of prescriptions, which are all substances contained in pharmaceutical preparations except active ingredients. Available in the Pharmacopoeia of the People's Republic of China (2015 Edition) Part IV, or, Handbook of Pharmaceutical Excipients (Raymond C Rowe, 2009 Sixth Edition).

The term "treatment" refers to therapeutic therapy. When referring to a specific disorder, treatment refers to: (1) ameliorating one or more biological manifestations of the disease or disorder, (2) interfering with (a) one or more points in the biological cascade leading to or causing the disorder or (b) one or more biological manifestations of the disorder, (3) ameliorating one or more symptoms, effects, or side effects associated with the disorder, or one or more symptoms, effects or side effects associated with the disorder or its treatment, or (4) slowing the progression of the disorder or one or more biological manifestations of the disorder.

The term "prevention" refers to the reduction of the risk of acquiring or developing diseases or disorders.

The term "therapeutically effective amount" refers to the amount of a compound that is sufficient to effectively treat the diseases or disorders described herein when administered to a patient. The "therapeutically effective amount" will vary according to the compound, the disease and its severity, and the age of the patient to be treated, but it can be adjusted by those skilled in the art as needed.

On the basis of not violating the common sense in the field, the preferred conditions above can be arbitrarily combined to obtain the preferred examples of the present disclosure.

The reagents and raw materials used in the present disclosure are commercially available.

The positive and progressive effect of the present disclosure is that the compound of the present disclosure has good inhibitory activity on complement factor D (the IC₅₀ value of the example compound for C3b is 0.01-100 nM), good inhibitory effect on rabbit erythrocyte hemolysis (the IC₅₀ value of rabbit erythrocyte hemolysis is 0.1-500nM), as well as excellent pharmacokinetics and pharmacodynamic activity.

### DETAILED DESCRIPTION OF THE PREFERRED EMBODIMENT

The present disclosure is further described below by the way of examples, but the present disclosure is not thereby limited to the scope of the described examples. The experimental methods for which the specific conditions are not specified in the following examples are selected according to the conventional methods and conditions, or according to the commodity instructions.

The terms used in the following specific experimental descriptions refer to (unless otherwise stated) the following reagents or operations:
(Boc)₂O: di-tert-butyl dicarbonate; B₂Pin₂: bis(pinacolato)diboron; DIAD: diisopropyl azodicarboxylate; DMF: N,N-dimethylformamide; DCM: dichloromethane; DIEA: N,N-diisopropylethylamine; Et₃N: triethylamine; EA: ethyl acetate; IV: intravenous injection; KOAc: potassium acetate; MeOH: methanol; MeCN: acetonitrile; PPh₃: triphenylphosphine; Pd₂(dba)₃: tris(dibenzylideneacetone)dipalladium; Pd(dppf)Cl₂: [1,1'-bis(diphenylphosphino)ferrocene]dichloropalladium(II); Pd(PPh₃)₄: tetrakis(triphenylphosphine)palladium; PO: oral administration; TEA: triethylamine; TFA: trifluoroacetic acid; THF: tetrahydrofuran; X-Phos: 2-dicyclohexylphosphino-2',4',6'-triisopropylbiphenyl;

### <Preparation example>

### Example 1. Synthesis of 2-(2-((3'-(aminomethyl)-5-(6-azaspiro[2.5]octan-6-yl)-[1,1'-biphenyl]-3-yl)methoxy)phenyl)acetic acid (compound 001)

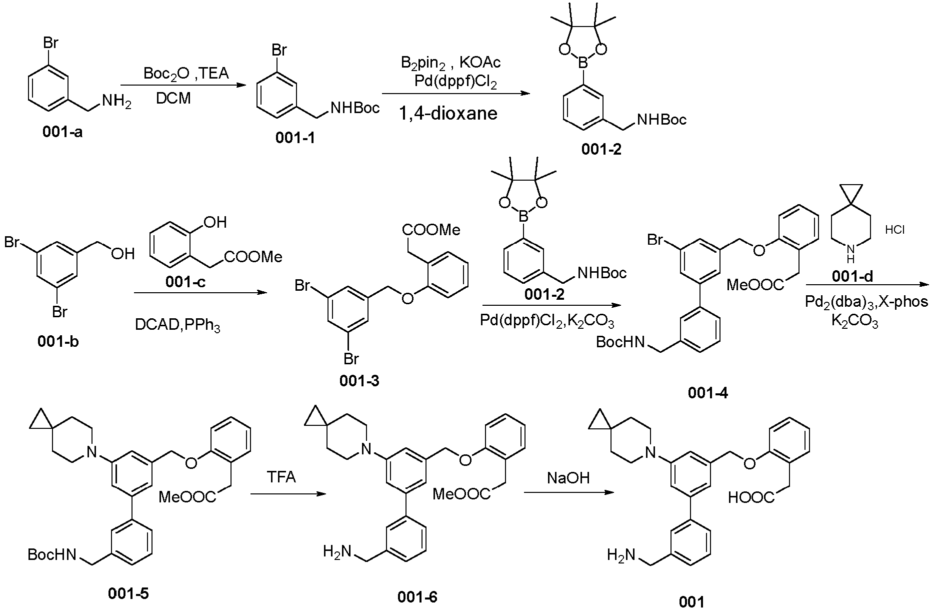

### 1.1 Synthesis of compound 001-1

To a 500 mL reaction flask were added 001-a (11.16 g, 60 mmol), triethylamine (11.00 g, 108 mmol), and dichloromethane (120 mL). To the above system was added di-tert-butyl dicarbonate (15.71 g, 72 mmol) under an ice bath. The reaction mixture was reacted at 0°C for 1 hour, then concentrated under reduced pressure, transferred to a 500 mL separatory funnel, and added with 150 mL of ethyl acetate and 200 mL of water. The resulting mixture was extracted and the phases were separated to separate the organic phase. The aqueous phase was then extracted with ethyl acetate (50 mL × 2). The combined organic phases were washed with saturated sodium chloride solution, dried over anhydrous sodium sulfate, filtered, and the filtrate was concentrated under reduced pressure. The residue was purified by silica gel column chromatography (petroleum ether/ethyl acetate = 10/1 (v/v)) to obtain compound 001-1, which was directly used in the next step.

### 1.2 Synthesis of compound 001-2

To a 500 mL reaction flask were sequentially added compound 001-1 (18.02 g, 60 mmol) obtained in step 1.1, bis(pinacolato)diboron (18.28 g, 72 mmol), potassium acetate (11.78 g, 120 mmol), [1,1'-bis(diphenylphosphino)ferrocene]dichloropalladium(II) (4.40 g, 6 mmol), and 1,4-dioxane (120 mL). After the system was replaced with nitrogen three times, the resulting reaction mixture was heated in an oil bath at 80°C and reacted for 4 hours. The reaction mixture was cooled to room temperature and filtered. After the filtrate was concentrated under reduced pressure, 150 mL of ethyl acetate and 200 mL of water were added thereto, then the mixture was extracted and the phases were separated to separate the organic phase. The aqueous phase was then extracted with ethyl acetate (50 mL × 2). The combined organic phases were washed with saturated sodium chloride solution, dried over anhydrous sodium sulfate, filtered, and the filtrate was concentrated under reduced pressure. The residue was purified by silica gel column chromatography (petroleum ether/ethyl acetate = 8/1 (v/v)) to obtain compound 001-2, which could be directly used in the next step. LC/MS(ESI+)m/z: [M+H-t-Bu]⁺=278.15.

### 1.3 Synthesis of compound 001-3

001-b (5.00 g, 18.8 mmol), 001-c (3.12 g, 18.8 mmol), and triphenylphosphine (9.87 g, 37.6 mmol) were sequentially dissolved in dichloromethane (70 mL), and a solution of bis(4-chlorobenzyl) azodicarboxylate (13.7 g, 37.6 mmol) in dichloromethane (70 mL) was added dropwise to the above system under an ice bath. After the dropwise addition, the reaction was continued for 2 hours at 0°C. The reaction mixture was filtered, and the filtrate was concentrated under reduced pressure. The residue was purified by silica gel column chromatography (petroleum ether/ethyl acetate = 20/1 to 10/1 (v/v)) to obtain compound 001-3. LC/MS(ESI+) m/z: [M+H]⁺=414.90.

### 1.4 Synthesis of compound 001-4

To 1,4-dioxane/water (30 mL/3 mL) were sequentially added 001-3 (2.00 g, 4.8 mmol), 001-2 (1.30 g, 3.9 mmol), potassium carbonate (1.30 g, 9.6 mmol), [1,1'-bis(diphenylphosphino)ferrocene]dichloropalladium(II) (0.35 g, 0.48 mmol). After the system was replaced with nitrogen three times, the reaction mixture was reacted at 80°C for 2 hours, then filtered, and the filtrate was diluted with water (30 mL) and then extracted with ethyl acetate (30 mL × 2). The combined organic phases were washed with saturated sodium chloride solution, dried over anhydrous sodium sulfate, filtered, and the filtrate was concentrated under reduced pressure. The residue was purified by silica gel column chromatography (petroleum ether/ethyl acetate = 10/1 to 5/1 (v/v)) to obtain compound 001-4. LC/MS(ESI+) m/z: [M+H-Boc]⁺=439.95.

### 1.5 Synthesis of compound 001-5

To 1,4-dioxane (50 mL) were sequentially added 001-4 (4.3 g, 7.96 mmol), 6-azaspiro[2.5]octane hydrochloride (compound 001-d, 1.4 g, 9.55 mmol), potassium carbonate (3.3 g, 23.87 mmol), tris(dibenzylideneacetone)dipalladium (400 mg, 10 wt%) and 2-dicyclohexylphosphino-2',4',6'-triisopropylbiphenyl (400 mg, 10 wt%). The system was replaced with nitrogen three times, and the reaction mixture was reacted at 90°C for 4 hours. The reaction mixture was filtered, then the filtrate was concentrated under reduced pressure, and the residue was purified by silica gel column chromatography to obtain compound 001-5. LC/MS(ESI+) m/z: [M+H]⁺ =571.15.

### 1.6 Synthesis of compound 001-6

001-5 (3.5 g, 6.1 mmol) was dissolved in dichloromethane (27 mL), then added with trifluoroacetic acid (9 mL), and the reaction was carried out at room temperature for 1 hour. The pH of the reaction mixture was adjusted to 8 by adding saturated sodium bicarbonate aqueous solution, and then the reaction mixture was extracted with ethyl acetate (30 mL × 3). The combined organic phases were washed with saturated brine, dried over anhydrous sodium sulfate, filtered, and the filtrate was concentrated under reduced pressure to obtain compound 001-6 (3.2 g, crude product), which could be used directly in the next step. LC/MS(ESI+) m/z: [M+H]⁺ =471.10.

### 1.7 Synthesis of compound 001

To tetrahydrofuran (10 mL), methanol (5 mL), and water (5 mL) were added 001-6 (3.1 g, 6.5 mmol) and sodium hydroxide (1.05 g, 26.3 mmol), and the reaction mixture was reacted at 60°C for 4 hours. The reaction mixture was purified by preparative highperformance liquid chromatography and then lyophilized to obtain the target compound 001.

¹H NMR (400 MHz, DMSO-*d₆*): δ 8.27 (s, 0.59H), 8.10 (s, 1H), 7.67 (d, *J* = 7.6 Hz, 1H), 7.37-7.40 (m, 2H), 7.28 (d, *J =* 7.2 Hz, 1H), 7.08-7.14 (m, 3H), 7.00 (s , 1H), 6.92 (d, *J* = 8.0 Hz, 1H), 6.81 (t, *J =* 7.6 Hz, 1H), 5.13 (s, 2H), 3.97 (s, 2H), 3.43 (s, 2H), 3.30 (t, *J =* 5.2 Hz, 4H), 1.48 (t, *J =* 5.2 Hz, 4H), 0.35 (s, 4H); LC/MS(ESI+) m/z: [M+H]⁺ =457.10.

### Example 2. Synthesis of 2-(2-((3'-(aminomethyl)-5-(7-azaspiro[3.5]nonan-7-yl)-[1,1'-biphenyl]-3-yl)methoxy)phenyl)acetic acid (compound 002)

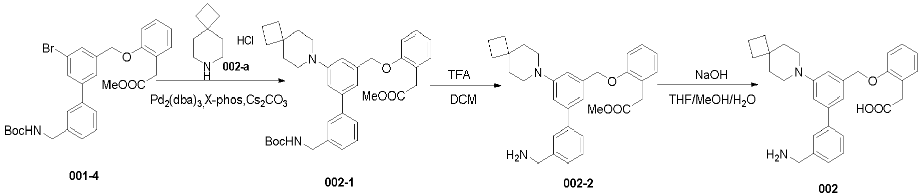

The synthesis of compound 002 refers to the synthesis method of compound 001, which only needs to replace the raw material 001-d with the hydrochloride of 7-azaspiro[3.5]nonane (002-a).

¹H NMR (400 MHz, DMSO-*d₆*): δ 8.27 (br.s, 0.6H), 7.82-7.92 (m, 1H), 7.5 (dd, *J* = 7.6, 20.0 Hz, 1H), 7. 40 (br.s, 0.6H), 7.33 (t, *J* = 7.6 Hz, 1H), 7.22-7.27 (m, 1H), 6.99-7.12 (m, 4H), 6.87-6.92 (m, 1H), 6.72-6.79 (m, 1H), 5.11 (d, *J =* 5.2 Hz, 2H), 4.21 (s, 1H), 3.83 (s, 1H), 3.36 (s, 1H), 3.31 (s, 1H), 3.15 (t, *J =* 5.2 Hz, 4H), 1.83-1.88 (m, 2H), 1.76-1.78 (m, 4H), 1.64-1.66 (m, 4H); LC/MS(ESI+) m/z: [M+H]⁺ =471.10.

### Example 3. Synthesis of 2-(2-((3'-(aminomethyl)-5-(6-azaspiro[3.4]octan-6-yl)-[1,1'-biphenyl]-3-yl)methoxy)phenyl)acetic acid (compound 003)

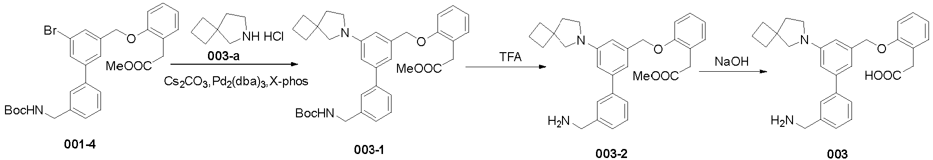

The synthesis of compound 003 refers to the synthesis method of compound 001, which only needs to replace the raw material 001-d with the hydrochloride of 6-azaspiro[3.4]octane (003-a).

¹H NMR (400 MHz, DMSO-*d₆*): δ 9.51 (s, 1H), 7.98 (d, *J* = 114.8 Hz, 1H), 7.59 (dd, *J* = 48.4, 7.6 Hz, 1H), 7.36 (t, *J =* 7.6 Hz, 1H), 7.31 - 7.22 (m, 2H), 7.11 - 7.01 (m, 2H), 6.91 - 6.67 (m, 3H), 6.51 (d, *J =* 10.4 Hz, 1H), 5.15 (d, *J =* 23.2 Hz, 2H), 4.23 (s, 1H), 3.92 (s, 1H), 3.39 (s, 2H), 3.34 - 3.31 (m, 4H), 2.06 - 2.00 (m, 4H), 1.99 - 1.85 (m, 4H); LC/MS(ESI+) m/z: [M+H]⁺ =457.05.

### Example 4. Synthesis of 2-(2-((3'-(aminomethyl)-5-(5-azaspiro[2.5]octan-5-yl)-[1,1'-biphenyl]-3-yl)methoxy)phenyl)acetic acid (compound 004)

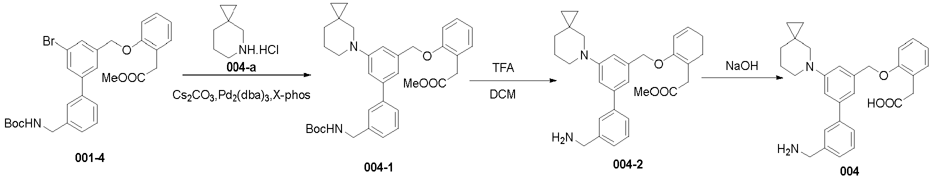

The synthesis of compound 004 refers to the synthesis method of compound 001, which only needs to replace the raw material 001-d with the hydrochloride of 5-azaspiro[2.5]octane (004-a).
¹H NMR (400 MHz, DMSO-*d₆*): δ 8.33 (s, 1H), 8.04 (s, 1H), 7.67 (d, *J* = 8.0 Hz, 1H), 7.39 (t, *J =* 7.6 Hz, 1H), 7.35 - 7.27 (m, 2H), 7.12 (t, *J* = 7.6 Hz, 2H), 7.08 (s, 1H), 6.98 - 6.90 (m, 2H), 6.82 (t, *J* = 7.6 Hz, 1H), 5.12 (s, 2H), 3.98 (s, 2H), 3.45 (s, 2H), 3.30 - 3.23 (m, 2H), 3.00 (s, 2H), 1.80 - 1.74 (m, 2H), 1.45 - 1.36 (m, 2H), 0.48 (t, *J* = 5.6 Hz, 2H), 0.33 (t, *J* = 4.8 Hz, 2H);
LC/MS(ESI+) m/z: [M+H]⁺ =457.10.

### Example 5. Synthesis of 2-(2-((3'-(aminomethyl)-5-(2-azaspiro[3.5]nonan-2-yl)-[1,1'-biphenyl]-3-yl)methoxy)phenyl)acetic acid (compound 005)

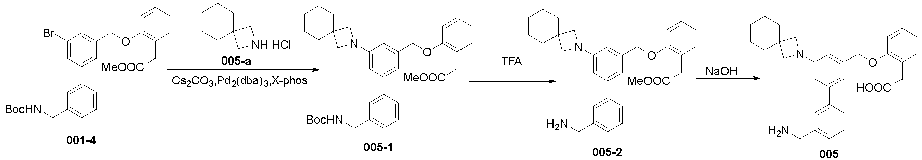

The synthesis of compound 005 refers to the synthesis method of compound 001, which only needs to replace the raw material 001-d with the hydrochloride of 2-azaspiro[3.5]nonane (005-a).

¹H NMR (400 MHz, DMSO-*d₆*): δ 9.54 (s, 0.25H), 7.98 (d, *J =* 104.4 Hz, 1H), 7.55 (dd, *J =* 44.8, 8.0 Hz, 1H), 7.39 - 7.22 (m, 3H), 7.14 - 7.00 (m, 2H), 6.90 - 6.74 (m, 2H), 6.60 (s, 1H), 6.42 (d, *J =* 13.2 Hz, 1H), 5.13 (d, *J =* 25.2 Hz, 2H), 4.07 (d, *J =* 134 Hz, 2H), 3.58 (d, *J =* 3.2 Hz, 4H), 3.38 (d, *J =* 4.4 Hz, 2H), 1.67 (s, 4H), 1.42 (d, *J* = 26.4 Hz, 6H); LC/MS(ESI+) m/z: [M+H]⁺ =471.10.

### Example 6. Synthesis of 2-(2-((3'-(aminomethyl)-5-(3-azabicyclo[3.2.1]octan-3-yl)-[1,1'-biphenyl]-3-yl)methoxy)phenyl)acetic acid (compound 006)

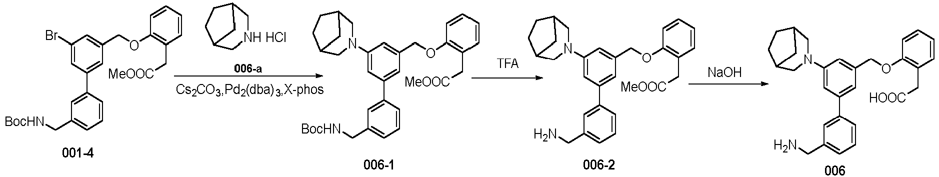

The synthesis of compound 006 refers to the synthesis method of compound 001, which only needs to replace the raw material 001-d with the hydrochloride of 3-azabicyclo[3.2.1]octane (006-a).

¹H NMR (400 MHz, DMSO-*d₆*): δ 7.76 (d, *J =* 54.4 Hz, 1H), 7.50 (dd, *J =* 14.4, 7.6 Hz, 1H), 7.39 - 7.32 (m, 1H), 7.27 (t, *J* = 7.2 Hz, 1H), 7.20 - 6.89 (m, 5H), 6.84 - 6.73 (m, 2H), 5.11 (d, *J* = 54.4 Hz, 2H), 4.00 (d, *J* = 184.4 Hz, 2H), 3.60 (d, *J* = 10.0 Hz, 2H), 3.38 (s, 2H), 3.27 (s, 2H), 2.85 - 2.75 (m, 2H), 2.37 (s, 2H), 1.59 (s, 2H), 1.55 (s, 2H); LC/MS(ESI+) m/z: [M+H]⁺ =457.05.

### Example 7. Synthesis of 2-(2-((3'-(aminomethyl)-2'-fluoro-5-(6-azaspiro[2.5]octan-6-yl)-[1,1'-biphenyl]-3-yl)methoxy)phenyl)acetic acid (compound 007)

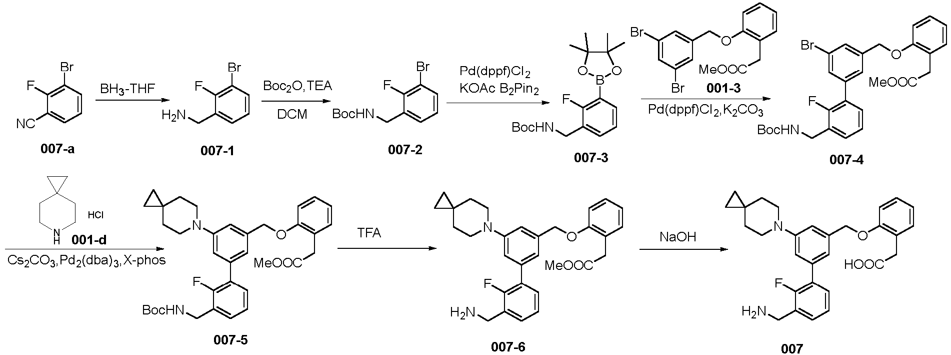

### 7.1 Synthesis of compound 007-1

007-a (1.0 g, 5 mmol) was dissolved in tetrahydrofuran (5 mL), and 1 M borane solution in tetrahydrofuran (20 mL, 20 mmol) was slowly added dropwise thereto under nitrogen atmosphere. After the addition, the reaction mixture was heated to 60°C and reacted for 5 hours. The reaction mixture was cooled to room temperature, quenched by slowly adding dropwise with methanol (20 mL), then added with 1 M dilute hydrochloric acid (10 mL), and the reaction mixture was extracted with ethyl acetate (20 mL × 3). The pH of the aqueous phase was adjusted with saturated sodium bicarbonate aqueous solution to 8-10, and the aqueous phase was extracted with ethyl acetate (20 mL × 3). The combined organic phases were washed with saturated brine, dried over anhydrous sodium sulfate, filtered, and the filtrate was concentrated under reduced pressure to obtain compound 007-1 (crude product). LC/MS(ESI+) m/z: [M+H]⁺ =204.00.

### 7.2 Synthesis of compound 007-2

007-1 (600 mg, crude product), di-tert-butyl dicarbonate (704 mg, 3.23 mmol), and triethylamine (594 mg, 5.88 mmol) were sequentially dissolved in dichloromethane (5 mL), and the reaction mixture was reacted overnight at room temperature. The reaction mixture was concentrated under reduced pressure, and the residue was purified by silica gel column chromatography (petroleum ether/ethyl acetate = 8/1) to obtain compound 007-2. LC/MS(ESI+) m/z: [M+H]⁺ =236.00.

### 7.3 Synthesis of compound 007-3

To 1,4-dioxane (5 mL) were sequentially added 007-2 (600 mg, 1.97 mmol), bis(pinacolato)diboron (601 mg, 2.37 mmol), potassium acetate (387 mg, 3.95 mmol), and [1,1'-bis(diphenylphosphino)ferrocene]dichloropalladium(II) (144 mg, 0.2 mmol). The system was replaced with nitrogen three times, and the reaction mixture was reacted at 100°C for 2 hours. The reaction mixture was cooled to room temperature, concentrated under reduced pressure, and the residue was purified by silica gel column chromatography (petroleum ether/ethyl acetate = 12/1) to obtain compound 007-3. LC/MS(ESI+) m/z: [M+H-t-Bu]⁺ =296.05.

### 7.4 Synthesis of compound 007-4

To 1,4-dioxane (5 mL) and water (1 mL) were sequentially added 007-3 (510 mg, 1.45 mmol), 001-3 (721 mg, 1.74 mmol), potassium carbonate (401 mg, 2.90 mmol), and [1,1'-bis(diphenylphosphino)ferrocene]dichloropalladium(II) (106 mg, 0.145 mmol), and the reaction mixture was reacted at 100°C for 2 hours. The reaction mixture was cooled to room temperature, added with water (10 mL), and extracted with ethyl acetate (15 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 10/1) to obtain compound 007-4.

### 7.5 Synthesis of compound 007

The synthesis of compound 007 refers to the synthesis method of compound 001, which only needs to replace the intermediate 001-4 with 007-4.

¹H NMR (400 MHz, DMSO-*d₆*): δ 7.49 - 7.32 (m, 2H), 7.32 - 7.15 (m, 2H), 7.15 - 7.04 (m, 2H), 7.04 - 6.96 (m, 2H), 6.93 (d, *J =* 7.6 Hz, 1H), 6.83 - 6.74 (m, 1H), 5.10 (d, *J =* 49.6 Hz, 2H), 4.16 (s, 1H), 3.79 (s, 1H), 3.29 (d, *J =* 4.4 Hz, 4H), 3.26 (s, 2H), 1.46 (dd, *J =* 13.2, 8 Hz, 4H), 0.33 (d, *J =* 4.8 Hz, 4H); LC/MS(ESI+) m/z: [M+H]⁺ =475.05.

### Example 8. Synthesis of 2-(2-((3'-(aminomethyl)-5'-fluoro-5-(6-azaspiro[2.5]octan-6-yl)-[1,1'-biphenyl]-3-yl)methoxy)phenyl)acetic acid (compound 008)

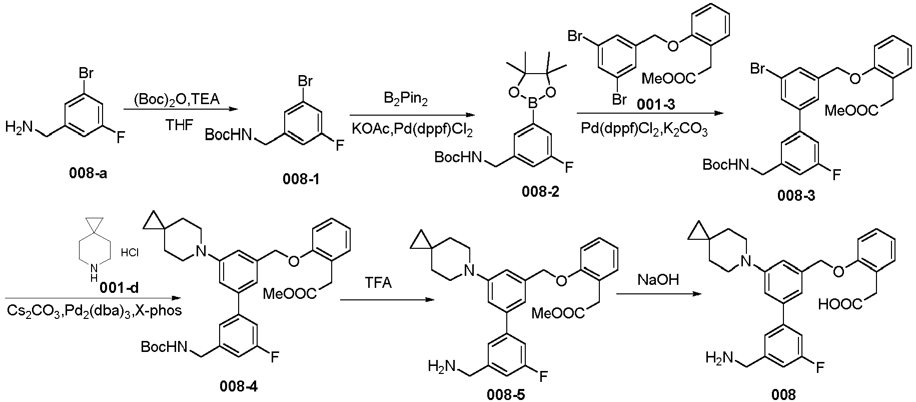

The synthesis of compound 008 refers to the synthesis method of compound 007, which only needs to replace the intermediate 007-1 with (3-bromo-5-fluorophenyl)methanamine (008-a).

¹H NMR (400 MHz, DMSO-*d₆*): δ 9.46 (s, 1H), 7.74 (d, *J* = 29.6 Hz, 1H), 7.37 (d, *J* = 40.4 Hz, 2H), 7.28 - 6.94 (m, 5H), 6.94 - 6.67 (m, 2H), 5.14 (d, *J =* 36.8 Hz, 2H), 4.24 (s, 1H), 3.86 (s, 1H), 3.40 (s, 1H), 3.35 (s, 1H), 3.31 (d, *J* = 4.8 Hz, 4H), 1.48 (s, 4H), 0.34 (s, 4H); LC/MS(ESI+) m/z: [M+H]⁺ =475.10.

### Example 9. Synthesis of 2-(2-((3'-(aminomethyl)-5-(6-azaspiro[2.5]octan-6-yl)-[1,1'-biphenyl]-3-yl)methoxy)-4-fluorophenyl)acetic acid (compound 009)

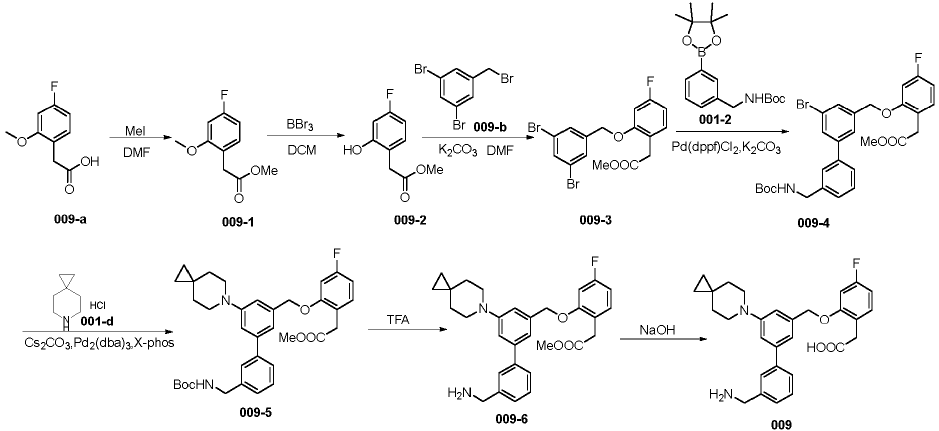

### 9.1 Synthesis of compound 009-1

009-a (1.0 g, 5.42 mmol) was dissolved in N,N-dimethylformamide (15 mL), then cesium carbonate (2.6 g, 8.1 mmol) and methyl iodide (1.16 g, 8.1 mmol) were added thereto, and the reaction mixture was reacted at 25°C for 1 hour. The reaction mixture was added with water (10 mL) and extracted with ethyl acetate (10 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 009-1. LC/MS(ESI+) m/z: [M+H]⁺ =199.10.

### 9.2 Synthesis of compound 009-2

009-1 (1.0 g, 5.04 mmol) was dissolved in dichloromethane (20 mL) under nitrogen atmosphere. The reaction system was cooled to -20°C, and boron tribromide (20 mmol, 15.12 mmol) was slowly added dropwise thereto. After the addition, the reaction mixture was maintained at this temperature and reacted for 2 hours. The reaction was quenched by dropwise addition of methanol (6 mL) at -20°C, and the reaction mixture was added with water and extracted with dichloromethane (10 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 009-2.

### 9.3 Synthesis of compound 009-3

009-b (1.45 g, 4.42 mmol) and 009-2 (740 mg, 4.02 mmol) were dissolved into N,N-dimethylformamide (20 mL), then potassium carbonate (1.12 g, 8.04 mmol) was added thereto, and the reaction was carried out at room temperature for 2 hours. The reaction mixture was added with water and extracted with ethyl acetate (10 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 009-3.

### 9.4 Synthesis of compound 009-4

To 1,4-dioxane (20 mL) and water (2 mL) were sequentially added 009-3 (750 mg, 1.74 mmol), 001-2 (578 mg, 1.74 mmol), potassium carbonate (480 mg, 3.48 mmol), and [1,1'-bis(diphenylphosphino)ferrocene]dichloropalladium(II) (127 mg, 0.174 mmol), and the reaction mixture was reacted at 100°C for 2 hours. The reaction mixture was cooled to room temperature, added with water, and extracted with ethyl acetate (10 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 009-4.

### 9.5 Synthesis of compound 009

The synthesis of compound 009 refers to the synthesis method of compound 001, which only needs to replace the raw material 001-4 with 009-4.

¹H NMR (400 MHz, DMSO-*d₆*): δ 9.49 (s, 1H), 7.88 (d, *J* = 24.0 Hz, 1H), 7.56 (t,*J* = 8.0 Hz, 1H), 7.39-7.34 (m, 1H), 7.32 - 7.07 (m, 4H), 7.06-6.99 (m, 1H), 6.82-6.79 (m, 1H), 6.68 - 6.56 (m, 1H), 5.17 (d, *J =* 44.0 Hz, 2H), 4.24 (s, 1H), 3.85 (s, 1H), 3.36 (s, 1H), 3.31 (s, 4H), 3.28 (s, 1H), 1.49 (d, *J =* 4.0 Hz, 4H), 0.35 (s, 4H); LC/MS(ESI+) m/z: [M+H]⁺ =475.05.

### Example 10. Synthesis of 2-(2-((3'-(aminomethyl)-5-(6-azaspiro[2.5]octan-6-yl)-[1,1'-biphenyl]-3-yl)methoxy)-5-fluorophenyl)acetic acid (compound 010)

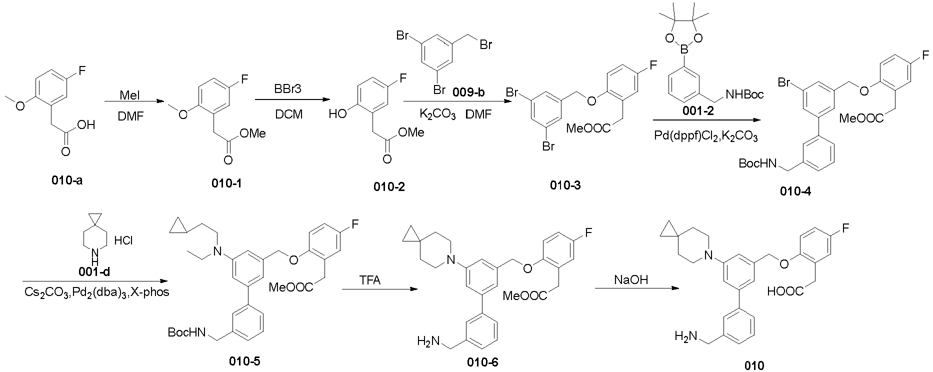

The synthesis of compound 010 refers to the synthesis method of compound 009, which only needs to replace the raw material 2-(4-fluoro-2-methoxyphenyl)acetic acid (009-a) with 2-(5-fluoro-2-methoxyphenyl)acetic acid (010-a).

¹H NMR (400 MHz, DMSO-*d₆*): δ 8.13 (s, 1H), 7.82 (s, 1H), 7.66 (d, *J* = 8.0 Hz, 1H), 7.54 (d, *J =* 8.0 Hz, 1H), 7.47 - 7.30 (m, 2H), 7.30-7.25 (m, 1H), 7.14 (s, 1H), 6.98-6.92 (m, 2H), 6.89 - 6.72 (m, 2H), 5.15 (d, *J* = 20.0 Hz, 2H), 4.24 (s, 1H), 3.95 (s, 1H), 3.38 (s, 2H), 3.29 (d, *J* = 8.0 Hz, 4H), 1.49-1.48 (m, 4H), 0.35 (s, 4H); LC/MS(ESI+) m/z: [M+H]⁺ =475.05.

### Example 11. Synthesis of 2-(2-((3'-(aminomethyl)-5-(2-oxa-7-azaspiro[3.5]nonan-7-yl)-[1,1'-biphenyl]-3-yl)methoxy)phenyl)acetic acid (compound 011)

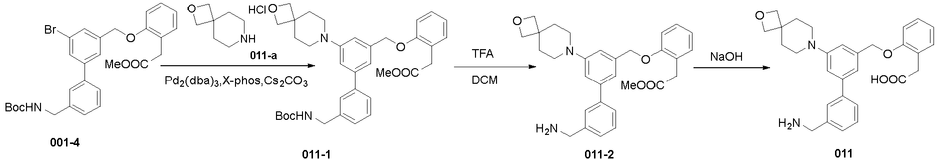

The synthesis of compound 011 refers to the synthesis method of compound 001, which only needs to replace the raw material 001-d with the hydrochloride of 2-oxa-7-azaspiro[3.5]nonane (011-a).

¹H NMR (400 MHz, DMSO-*d₆*): δ 7.71 (s, 1H), 7.50 (d, *J* = 7.6 Hz, 1H), 7.35 (t, *J* = 7.6 Hz, 1H), 7.27 (d, *J* = 7.6 Hz, 1H), 7.18 (d, *J =* 8.8 Hz, 2H), 7.04-7.08 (m, 3H), 6.92 (d, *J =* 8.0 Hz, 1H), 6.80 (t, *J =* 7.6 Hz, 1H), 5.05 (s, 2H), 4.36 (d, *J* = 4.0 Hz, 4H)), 3.77 (s, 2H), 3.39 (s, 2H), 3.18 (t, *J =* 5.6 Hz, 4H), 1.90 (t, *J =* 5.6 Hz, 4H); LC/MS(ESI+) m/z: [M+H]⁺ =473.05.

### Example 12. Synthesis of 2-(2-((3'-(aminomethyl)-5-(1-oxa-7-azaspiro[3.5]nonan-7-yl)-[1,1'-biphenyl]-3-yl)methoxy)phenyl)acetic acid (compound 012)

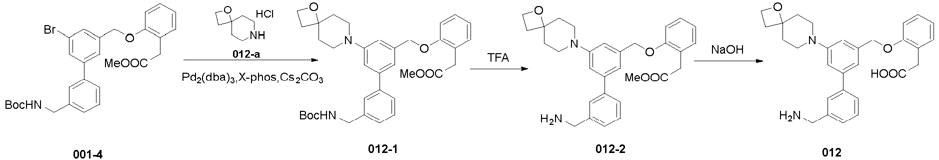

The synthesis of compound 012 refers to the synthesis method of compound 001, which only needs to replace the raw material 001-d with the hydrochloride of 1-oxa-7-azaspiro[3.5]nonane (012-a).

¹H NMR (400 MHz, DMSO-*d₆*): δ 9.53 (s, 1H), 7.85-8.01 (m, 1H), 7.62-7.53 (m, 1H), 7.43 (s, 1H), 7.39 - 7.32 (m, 1H), 7.30 - 7.23 (m, 1H), 7.14 - 6.96 (m, 4H), 6.91-6.74 (m, 2H), 5.15 (d, *J =* 32.0 Hz, 2H), 4.43 (t, *J* =8.0 Hz, 2H), 4.23 (s, 1H), 3.87 (s, 1H), 3.37 (d, *J =* 16.0 Hz, 4H), 3.20-3.14 (m, 2H), 2.39 (t, *J* = 8.0 Hz, 2H), 1.98 - 1.79 (m, 4H); LC/MS(ESI+) m/z: [M+H]⁺ =473.05.

### Example 13. Synthesis of 2-(2-((3'-(aminomethyl)-5-(2-hydroxy-7-azaspiro[3.5]nonan-7-yl)-[1,1'-biphenyl]-3-yl)methoxy)phenyl)acetic acid (compound 013)

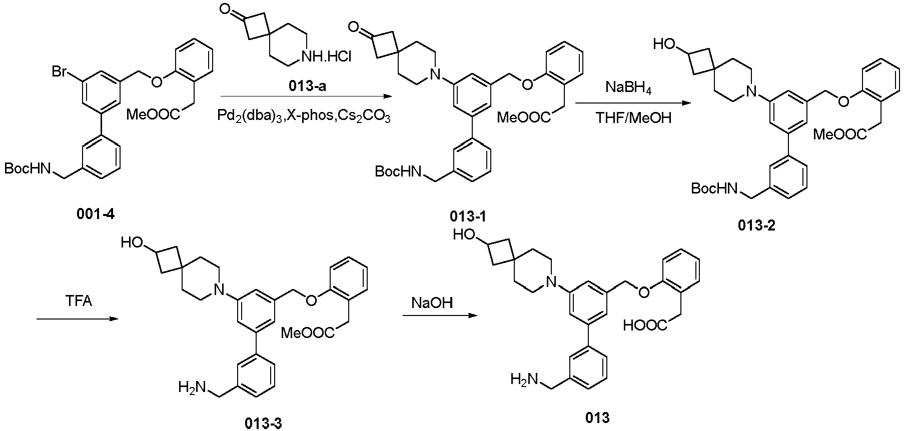

### 13.1 Synthesis of compound 013-1

001-4 (400 mg, 0.881 mmol) was weighed into a reaction flask, and then 013-a (174 mg, 0.940 mmol), cesium carbonate (1 g, 3.4 mmol), tris(dibenzylideneacetone)dipalladium (140 mg, 0.088 mmol), 2-dicyclohexylphosphino-2',4',6'-triisopropylbiphenyl (90 mg, 0.088 mmol), and 1,4-dioxane (5 mL) were sequentially added thereto. The system was replaced with nitrogen three times, and the reaction mixture was reacted at 100°C for 4 hours. The reaction mixture was filtered, and the filtrate was concentrated under reduced pressure. The residue was purified by silica gel column chromatography (petroleum ether/ethyl acetate = 3/1) to obtain compound 013-1. LC/MS(ESI+) m/z: [M+H]⁺ =599.15.

### 13.2 Synthesis of compound 013-2

013-1 (200 mg, 340 µmol) was dissolved in methanol (3 mL) and tetrahydrofuran (3 mL), then sodium borohydride (68 mg, 680 µmol) was added thereto in batches at 0°C, and the reaction mixture was reacted at 0°C for another 0.5 hours. The reaction mixture was quenched by adding ammonium chloride aqueous solution (5 mL), and extracted with ethyl acetate (10 mL × 2). The combined organic phases were washed with saturated brine, dried over anhydrous sodium sulfate, filtered, and the filtrate was concentrated under reduced pressure to obtain 013-2 (crude product), which could be used directly in the next step. LC/MS(ESI+) m/z: [M+H]⁺ =601.10.

### 13.3 Synthesis of compound 013

The synthesis of compound 013 refers to the synthesis method of compound 001, which only needs to replace the intermediate 001-5 with 013-2.

¹H NMR (400 MHz, DMSO-*d₆*): δ 7.77 (d, *J =* 20.0 Hz, 1H), 7.48 (d, *J =* 8.0 Hz, 1H), 7.33(m, 1H), 7.24(m, 1H), 7.18,s, 1H) , 7.13(m, 1H), 7.05(m, 2H), 7.00(m, 1H) , 6.88 (d, *J =* 8.0 Hz, 1H), 6.74 (m, 1H), 5.14 (m, 2H), 4.20 (s, 1H), 4.12 (t, *J =* 16.0 Hz, 1H), 3.76 (s, 2H), 3.28 (s, 2H), 3.12(m, 4H), 2.45 (m,2H), 2.12 (m, 2H), 1.57 (d, *J* = 4.5 Hz,4H); LC/MS(ESI+) m/z: [M+H]⁺ =487.10.

### Example 14. Synthesis of 2-(2-((3'-(aminomethyl)-5-(2-oxa-8-azaspiro[4.5]decan-8-yl)-[1,1'-biphenyl]-3-yl)methoxy)phenyl)acetic acid (compound 014)

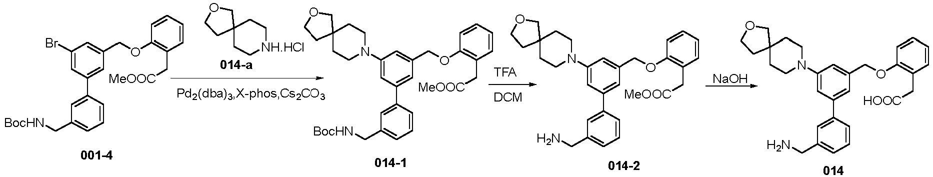

The synthesis of compound 014 refers to the synthesis method of compound 001, which only needs to replace the intermediate 001-d with the hydrochloride of 2-oxa-8-azaspiro[4.5]decane (014-a).

¹H NMR (400 MHz, DMSO-*d₆*): δ 7.86 (s, 1H), 7.55 (d, *J* = 8.0 Hz, 1H), 7.35 (t, *J* = 16.0 Hz, 1H), 7.26 (m, 2H), 7.15(m, 1H), 7.08(m, 3H), 6.91 (d, *J* = 8.0 Hz, 1H), 6.79 (t, *J* = 12.0Hz, 1H), 5.08 (s, 2H), 3.89 (m, 2H), 3.76(m, 4H), 3.49 (m, 2H), 3.26 (m, 4H), 1.74 (t, *J* =12.0Hz, 2H), 1.64 (t, *J =* 12.0 Hz, 4H); LC/MS(ESI+) m/z: [M+H]⁺ =487.15.

### Example 15. Synthesis of 2-(2-((3'-(aminomethyl)-5-(2-oxa-6-azaspiro[3.4]octan-6-yl)-[1,1'-biphenyl]-3-yl)methoxy)phenyl)acetic acid (compound 015) trifluoroacetate

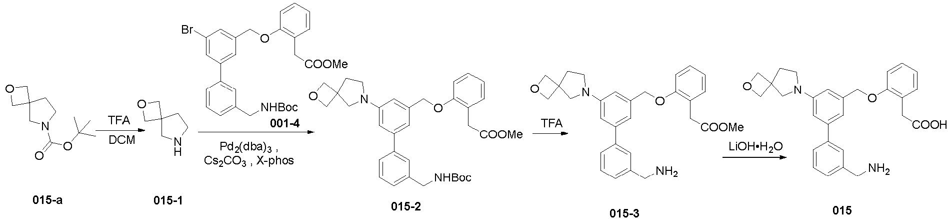

### 15.1 Synthesis of compound 015-1

015-a (86 mg, 0.4 mmol) was dissolved in dichloromethane (1 mL), then the system was added with trifluoroacetic acid (2 mL) at room temperature, and the reaction mixture was heated at 60°C for 2 hours. The reaction mixture was cooled to room temperature and concentrated under reduced pressure to obtain compound 015-1 (crude product), which could be used directly in the next step.

### 15.2 Synthesis of trifluoroacetate of compound 015

Referring to the synthesis method of compound 001, it is only necessary to replace the intermediate 001-d with 015-1. The preparation method is to lyophilize under trifluoroacetic acid conditions to obtain the trifluoroacetate of compound 015.

¹H NMR (600 MHz, DMSO-*d₆*): δ 12.20 (s, 1H), 8.17 (s, 2H), 7.78 (s, 1H), 7.69 (d, *J* = 7.8 Hz, 1H), 7.50 (t, *J* = 7.8 Hz, 1H), 7.43 (d, *J =* 7.8 Hz, 1H), 7.22 (t, *J* = 7.8 Hz, 2H), 7.03 (d, *J =* 7.8 Hz, 1H), 6.99 (s, 1H), 6.90 (t, *J =* 7.8 Hz, 1H), 6.72 (s, 1H), 6.68 (s, 1H), 5.11 (s, 2H), 4.61 (d, *J =* 6.0 Hz, 2H), 4.56 (d, *J =* 6.0 Hz, 2H), 4.12 (d, *J =* 4.8 Hz, 2H), 3.60 (d, *J =* 5.4 Hz, 2H), 3.38 (s, 2H), 2.29 (t, *J =* 6.6 Hz, 2H ), 2.02-1.96 (m, 2H); LC/MS(ESI+) m/z: [M+H]⁺ =459.05.

### Example 16. Synthesis of 2-(2-((3'-(aminomethyl)-5-(4-oxa-7-azaspiro[2.5]octan-7-yl)-[1,1'-biphenyl]-3-yl)methoxy)phenyl)acetic acid (compound 016) trifluoroacetate

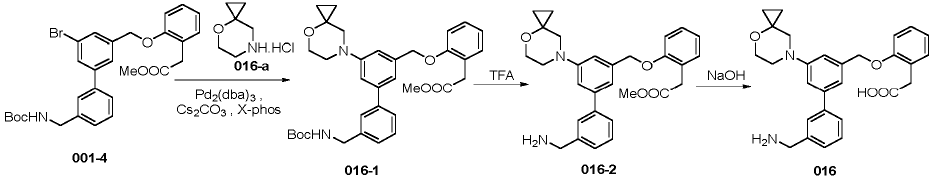

The synthesis of compound 016 refers to the synthesis method of compound 001, which only needs to replace the intermediate 001-d with the hydrochloride of 4-oxa-7-azaspiro[2.5]octane (016-a).
¹H NMR (600 MHz, DMSO-*d₆*): δ 9.54 (s, 1H), 7.86 (s, 1H), 7.56 (d, *J* = 7.9 Hz, 1H), 7.48 (s, 1H), 7.28 (t, *J =* 7.6 Hz, 1H), 7.13 (m, 2H), 7.05 (m, 3H), 6.94 (s, 1H), 6.76 (t, *J =* 16.0Hz, 2H), 5.20 (s, 2H), 4.24 (s, 2H), 3.83(m, 2H), 3.39 (s, 2H), 3.29 (m, 2H), 3.21 (s, 2H), 0.77 (m, 2H), 0.69 (m, 2H);
LC/MS(ESI+) m/z: [M+H]⁺ =459.05.

### Example 17. Synthesis of 2-(2-((3'-(aminomethyl)-5-(2-azaspiro[3.4]octan-2-yl)-[1,1'-biphenyl]-3-yl)methoxy)phenyl)acetic acid (compound 017)

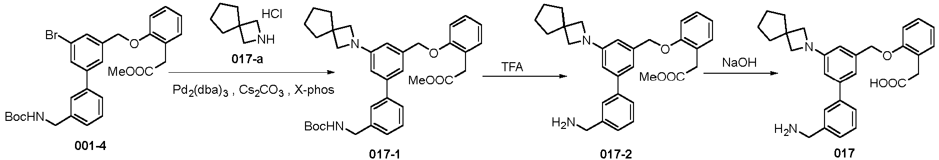

The synthesis of compound 017 refers to the synthesis method of compound 001, which only needs to replace the intermediate 001-d with the hydrochloride of 2-azaspiro[3.4]octane (017-a).
¹H NMR (600 MHz, DMSO-*d₆*): δ 8.15 (s, 1H), 7.60 (d, *J =* 8.0 Hz, 1H), 7.39-7.31 (m, 2H), 7.23 (d, *J* = 8.0 Hz, 1H), 7.03-7.08 (m, 2H), 6.86 (d, *J* = 8.0 Hz, 1H), 6.77 (t, *J =* 7.2 Hz, 1H), 6.59 (s, 1H), 6.41 (s, 1H), 5.10 (s, 2H), 3.91 (s, 2H), 3.72 (s, 4H), 3.37 (s, 2H), 1.80 (t, *J =* 6.8 Hz, 4H), 1.57-1.60 (m, 4H);
LC/MS(ESI+) m/z: [M+H]⁺ =457.05.

### Example 18. Synthesis of (S)-2-(2-((3'-(1-amino-2-hydroxyethyl)-5-(6-azaspiro[2.5]octan-6-yl)-[1,1'-biphenyl]-3-yl)methoxy)phenyl)acetic acid (compound 018)

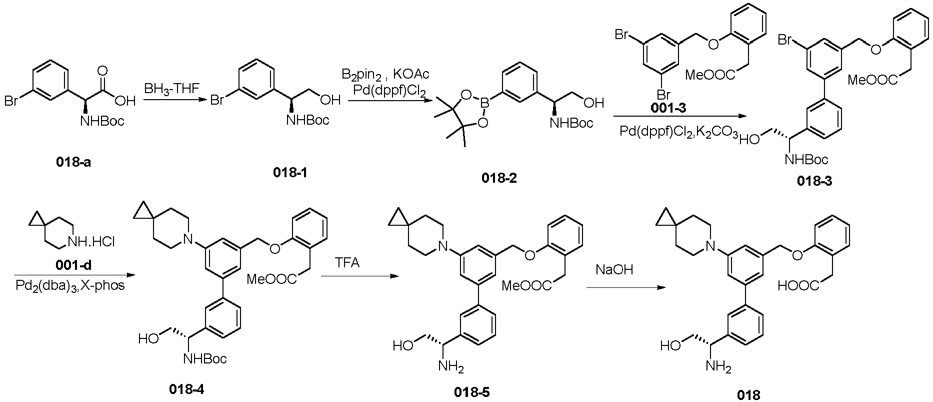

### 18.1 Synthesis of compound 018-1

018-a (3.5 g, 10.6 mmol) was dissolved in tetrahydrofuran (35 mL), and 1 M borane solution in tetrahydrofuran (52.5 mL, 53.1 mmol) was added dropwise thereto at room temperature. After the addition, the reaction mixture was reacted at 80°C for 1 hour. The reaction mixture was cooled to room temperature, quenched with methanol, and concentrated under reduced pressure. To the residue was added 1 N dilute hydrochloric acid (10 mL), then the mixture was stirred at room temperature for 10 min, and extracted with ethyl acetate (20 mL × 2). The pH of the aqueous phase was adjusted to be alkaline with saturated sodium bicarbonate aqueous solution, and then the aqueous phase was extracted with ethyl acetate (20 mL × 2). The combined organic phases were washed with saturated brine, dried over anhydrous sodium sulfate, filtered, and the filtrate was concentrated under reduced pressure to obtain compound 018-1 (crude product), which could be used directly in the next step.

### 18.2 Synthesis of compound 018-2

To 1,4-dioxane (10 mL) were sequentially added 018-1 (1.7 g, 5.4 mmol), bis(pinacolato)diboron (2.0 g, 6.5 mmol), potassium acetate (1.5 g, 9.2 mmol), and [1,1'-bis(diphenylphosphino)ferrocene]dichloropalladium(II) (500 mg, 460 µmol). After the system was replaced with nitrogen three times, the reaction mixture was reacted at 80°C for 3 hours. The reaction mixture was diluted with water (30 mL) and then extracted with ethyl acetate (20 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 10/1) to obtain compound 018-2.

### 18.3 Synthesis of compound 018

The synthesis of compound 018 refers to the synthesis method of compound 001, which only needs to replace the intermediate 001-2 with 018-2.

¹H NMR (400 MHz, DMSO-*d₆*): δ 9.48 (s, 1H), 7.89 (d, *J* = 8.0 Hz, 1H), 7.54 (s, 1H), 7.43 (s, 1H), 7.29 (t, *J* = 4.0Hz, 2H), 7.15 (m, 3H), 7.04 (m, 2H), 6.90 (d, *J* =4.0 Hz, 1H), 6.79 (d, *J =* 4.0 Hz, 1H), 5.13 (m, 2H), 4.75 - 3.84 (m, 1H), 3.53 (m, 2H), 3.96(m, 2H), 3.29 (s, 4H), 1.49 (s, 4H), 0.35 (s, 4H); LC/MS(ESI+) m/z: [M+H]⁺ =487.05.

### Example 19. Synthesis of (S)-2-(2-((3'-(1-amino-2-hydroxyethyl)-5'-fluoro-5-(6-azaspiro[2.5]octan-6-yl)-[1,1'-biphenyl]-3-yl)methoxy)phenyl)acetic acid (compound 019)

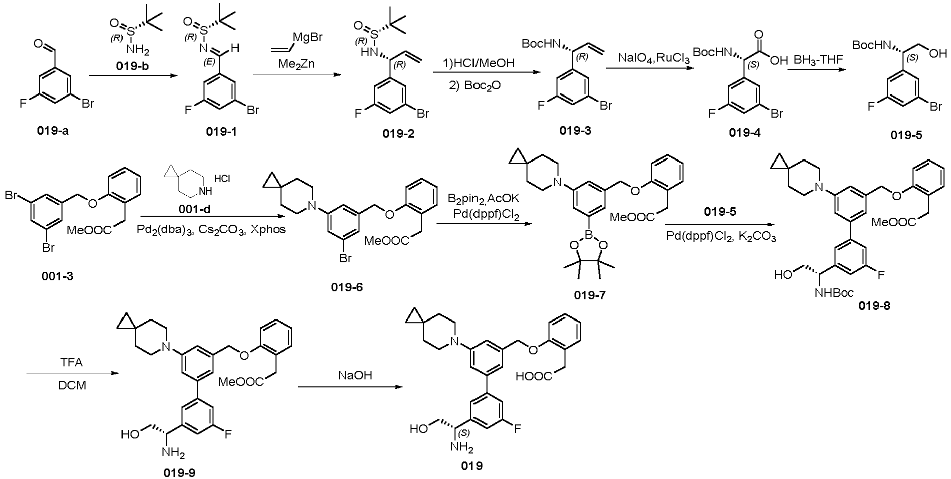

### 19.1 Synthesis of compound 019-1

019-a (3.0 g, 14.8 mmol) was dissolved in dichloromethane (25 mL), then 019-b (1.8 g, 14.8 mmol) and cesium carbonate (4.8 g, 14.8 mmol) were added thereto, and the reaction mixture was reacted at 25°C for 12 hours. The reaction mixture was added with water (30 mL) and extracted with ethyl acetate (15 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 019-1. LC/MS(ESI+) m/z: [M+H]⁺ =305.90.

### 19.2 Synthesis of compound 019-2

Vinylmagnesium bromide (16.7 mL, 16.7 mmol) and dimethylzinc reagent (16.7 mL, 16.7 mmol) were mixed under nitrogen atmosphere, and the reaction was carried out at room temperature for 0.5 hours. The system was cooled to -78°C, and 019-1 (3.0 g, 9.81 mmol) was slowly added dropwise thereto. After the addition, the reaction mixture was maintained at this temperature and reacted for 2 hours. After the reaction was quenched by dropwise addition of saturated ammonium chloride aqueous solution (26 mL) at -78°C, the reaction mixture was extracted with ethyl acetate (30 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 019-2. LC/MS(ESI+) m/z: [M+H]⁺ =333.90.

### 19.3 Synthesis of compound 019-3

019-2 (2.5 g, 7.5 mmol) was dissolved in methanol (20 mL), then 4 N hydrochloric acid (5 mL, 7.5 mmol) was added thereto, and the reaction was carried out at room temperature for 1 hour. The reaction mixture was concentrated under reduced pressure, and dichloromethane (20 mL) was added thereto, then di-tert-butyl dicarbonate (3.07 g, 14.08 mmol) was added thereto, and the reaction was carried out at room temperature for 2 hours. The reaction mixture was concentrated under reduced pressure, and the residue was purified by silica gel column chromatography (petroleum ether/ethyl acetate = 3/1 to 1/1) to obtain compound 019-3. LC/MS(ESI+) m/z: [M+H-t-Bu]⁺ =273.90.

### 19.4 Synthesis of compound 019-4

019-3 (2.5 g, 7.58 mmol) was dissolved in a mixed system of carbon tetrachloride (20 mL), acetonitrile (20 mL), and water (30 mL) under nitrogen atmosphere, then sodium periodate (3.4 g, 15.9 mmol) and ruthenium trichloride (158 mg, 0.758 mmol) were added thereto, and the reaction mixture was reacted at room temperature for 2 hours. The reaction mixture was extracted with ethyl acetate (30 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 10/1 to 2/1) to obtain compound 019-4.

### 19.5 Synthesis of compound 019-5

019-4 (130 mg, 0.432 mmol) was weighed into a reaction flask, and 1 M borane solution in tetrahydrofuran (1.5 mL, 0.864 mmol) was added thereto under nitrogen atmosphere. The reaction mixture was reacted at 25°C for 12 hours. The reaction was quenched by the addition of methanol (5 mL), and the insoluble substance was removed by filtration. The filtrate was concentrated under reduced pressure, and the residue was purified by silica gel column chromatography (petroleum ether/ethyl acetate = 3/1 to 1/1) to obtain compound 019-5. LC/MS(ESI+) m/z: [M+H-t-Bu]⁺ =277.95.

### 19.6 Synthesis of compound 019-6

To 1,4-dioxane (10 mL) were sequentially added 001-3 (1.0 g, 2.42 mmol), 001-d (320 mg, 2.16 mmol), tris(dibenzylideneacetone)dipalladium (240 mg, 0.24 mmol), 2-dicyclohexylphosphino-2',4',6'-triisopropylbiphenyl (120 mg, 0.24 mmol), and cesium carbonate (1.6 g, 4.84 mmol). The system was replaced with nitrogen three times, and the reaction mixture was reacted at 100°C for 4 hours. The reaction mixture was filtered, and the filtrate was concentrated under reduced pressure. The residue was purified by silica gel column chromatography (petroleum ether/ethyl acetate = 3/1 to 1/1) to obtain compound 019-6. LC/MS(ESI+) m/z: [M+H]⁺ =443.95.

### 19.7 Synthesis of compound 019-7

To 1,4-dioxane (10 mL) were sequentially added 019-6 (170 mg, 2.7 mmol), bis(pinacolato)diboron (822 mg, 3.24 mmol), potassium acetate (540 mg, 5.4 mmol), and [1,1'-bis(diphenylphosphino)ferrocene]dichloropalladium(II) (240 mg, 300 µmol). The system was replaced with nitrogen three times, and the reaction mixture was reacted at 100°C for 4 hours. The reaction mixture was diluted with water (10 mL) and then extracted with ethyl acetate (10 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 10/1) to obtain compound 019-7. LC/MS(ESI+) m/z: [M+H]⁺ =492.10.

### 19.8 Synthesis of compound 019-8

To 1,4-dioxane (4 mL) and water (1 mL) were sequentially added 019-7 (50 mg, 0.045 mmol), 019-5 (38 mg, 0.054 mmol), potassium carbonate (30 mg, 0.09 mmol), and [1,1'-bis(diphenylphosphino)ferrocene]dichloropalladium(II) (8.5 mg, 0.05 mmol). The system was replaced with nitrogen three times, and the reaction mixture was reacted at 100°C for 2 hours. The reaction mixture was added with water and extracted with ethyl acetate (10 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 019-8. LC/MS(ESI+) m/z: [M+H]⁺ =619.10.

### 19.9 Synthesis of compound 019-9

019-8 (35 mg, 0.057 mmol) was dissolved in dichloromethane (5 mL) and trifluoroacetic acid (1 mL), and the reaction was carried out at room temperature for 0.5 hours. The pH of the reaction mixture was adjusted to 7-8 by adding saturated sodium bicarbonate aqueous solution (6 mL), and the reaction mixture was extracted with dichloromethane (10 mL × 3). The combined organic phases were washed with saturated brine, dried over anhydrous sodium sulfate, filtered, and the filtrate was concentrated under reduced pressure to obtain compound 019-9 (crude product), which could be used directly in the next step. LC/MS(ESI+) m/z: [M+H]⁺ =519.10.

### 19.10 Synthesis of compound 019

019-9 (22 mg, 0.067 mmol) and sodium hydroxide (11 mg, 0.27 mmol) were dissolved in a mixture of tetrahydrofuran (1 mL), methanol (0.5 mL), and water (1 mL), and the reaction was carried out at 60°C for 2 hours. The reaction mixture was purified by preparative highperformance liquid chromatography and lyophilized to obtain the target compound 019.

¹H NMR (400 MHz, DMSO-*d₆*): δ 7.97 (s, 1H), 7.55 (d, *J =* 12.0 Hz, 1H), 7.38 (s, 1H), 7.18 - 7.09 (m, 4H), 7.05 (s, 1H), 6.94 (d, *J* = 8.0 Hz, 1H), 6.81 (t, *J* = 8.0 Hz, 1H), 5.13 (s, 2H), 4.13 (s, 1H), 3.73 - 3.61 (m, 2H), 3.48 (d, *J* = 16.0 Hz, 1H), 3.39 (s, 1H), 3.34 - 3.30 (m, 4H), 1.50 - 1.46 (m, 4H), 0.35 (s, 4H); LC/MS(ESI+) m/z: [M+H]⁺ =505.05.

### Example 20. Synthesis of (R)-2-(2-((3'-(1-aminoethyl)-2'-fluoro-5-(6-azaspiro[2.5]octan-6-yl)-[1,1'-biphenyl]-3-yl)methoxy)phenyl)acetic acid (compound 020-A or 020-B) and (S)-2-(2-((3'-(1-aminoethyl)-2'-fluoro-5-(6-azaspiro[2.5]octan-6-yl)-[1,1'-biphenyl]-3-yl)methoxy)phenyl)acetic acid (compound 020-B or 020-A)

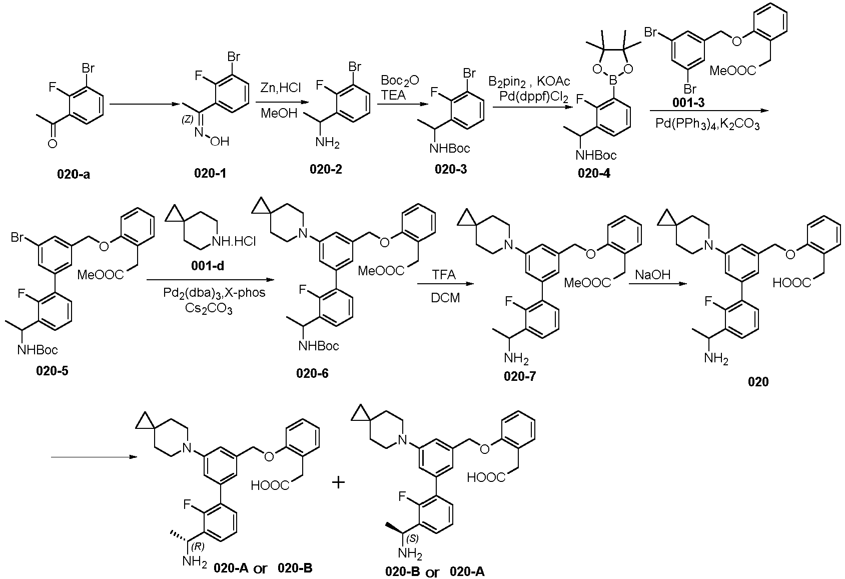

### 20.1 Synthesis of compound 020-1

020-a (2 g, 5.6 mmol) and hydroxylamine hydrochloride (4.5 g, 37.6 mmol) were dissolved in pyridine (10 mL), and the reaction mixture was reacted at 45°C for 1 hour. The pH of the reaction mixture was adjusted to 2 with 1 N hydrochloric acid, and the reaction mixture was extracted with ethyl acetate (20 mL × 2). The combined organic phases were washed with saturated brine, dried over anhydrous sodium sulfate, filtered, and the filtrate was concentrated under reduced pressure to obtain compound 020-1 (crude product), which could be used directly in the next step. LC/MS(ESI+) m/z: [M+H]⁺ =234.10.

### 20.2 Synthesis of compound 020-2

To methanol (10 mL) were added 020-1 (1.16 g, 0.22 mmol) and zinc powder (2 g, 2.2 mmol), then 6 N hydrochloric acid (10 mL) was added dropwise thereto, and the reaction mixture was reacted at 70°C for 1 hour. The pH of the reaction mixture was adjusted to 8 with sodium bicarbonate aqueous solution (20 mL), and the reaction mixture was filtered. The filter cake was rinsed with water (10 mL), and then the filtrate was extracted with ethyl acetate (30 mL × 2). The combined organic phases were washed with saturated brine, dried over anhydrous sodium sulfate, filtered, and the filtrate was concentrated under reduced pressure to obtain compound 020-2 (crude product). LC/MS(ESI+) m/z: [M+H]⁺ =220.00.

### 20.3 Synthesis of compound 020-3

The synthesis method of compound 020-3 refers to the synthesis method of compound 001-1 in Example 1.

### 20.4 Synthesis of compound 020-4

The synthesis method of compound 020-4 refers to the synthesis method of compound 001-2 in Example 1.

### 20.5 Synthesis of compounds 020-5 to 020-7

The synthesis method of compounds 020-5 to 020-7 refers to the synthesis method of compounds 001-4 to 001-6 in Example 1.

### 20.6 Synthesis of compounds 020-A and 020-B

020-7 (75 mg, 150 µmol) and sodium hydroxide (300 mg, 3.1 mmol) were dissolved in a mixture of tetrahydrofuran (3 mL), methanol (0.5 mL), and water (1 mL), and the reaction mixture was reacted at 60°C for 2 hours. The reaction mixture was purified by preparative high-performance liquid chromatography and lyophilized to obtain compound 020, and the compound 020 was subjected to chiral resolution (AD-H column, n-hexane: [(ethanol: methanol = 3:1)] = 6:4 isocratic elution) to obtain the target compounds 020-A (retention time = 7.640 min) and 020-B (retention time = 13.087 min).

### Compound 020-A:

¹H NMR (400 MHz, DMSO-*d₆*): δ 7.53(m, 1H), 7.35(t, *J* = 4.0 Hz, 1H), 7.24(m, 3H), 7.06 (m, 2H), 6.99 (d, *J =* 4.0 Hz, 2H), 6.89 (m, 1H), 5.11 (s, 2H), 4.36 (s, 1H), 3.57 (s, 2H),3.29 (m 4H), 1.47 (m, 4H), 1.35 (s, 3H), 0.34 (s, 4H); LC/MS(ESI+) m/z: [M+H]⁺ =489.10.

### Compound 020-B:

¹H NMR (400 MHz, DMSO-*d*₆): δ 7.53(m, 1H), 7.35(t, *J* = 4.0 Hz, 1H), 7.24(m, 3H), 7.06 (m, 2H), 6.99 (d, *J =* 4.0 Hz, 2H), 6.89 (m, 1H), 5.11 (s, 2H), 4.36 (s, 1H), 3.57 (s, 2H),3.29 (m 4H), 1.47 (m, 4H), 1.35 (s, 3H), 0.34 (s, 4H); LC/MS(ESI+) m/z: [M+H]⁺ =489.10.

### Example 21. Synthesis of 2-(2-((3'-(aminomethyl)-2'-fluoro-5-(6-azaspiro[2.5]octan-6-yl)-[1,1'-biphenyl]-3-yl)methoxy)-4-fluorophenyl)acetic acid (compound 021)

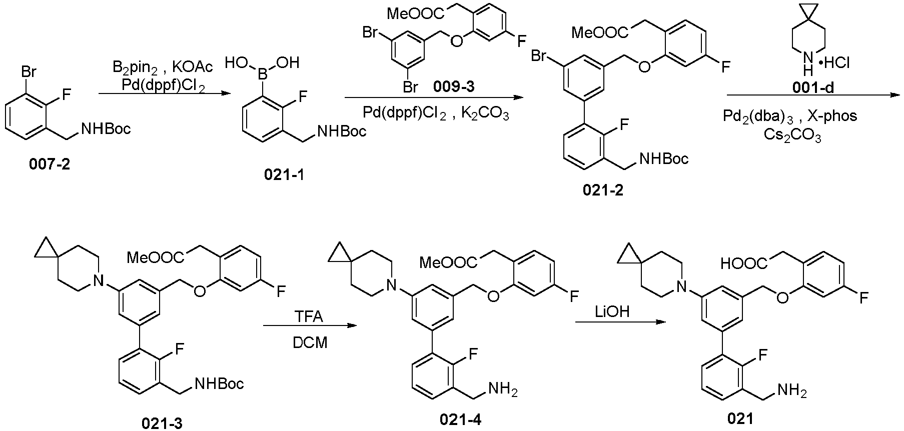

The synthesis of compound 021 refers to the synthesis method of compound 007, which only needs to replace the intermediate 001-3 with 009-3.

¹H NMR (600 MHz, DMSO-*d₆*): δ 8.30 (s, 1H), 7.45 (q, *J* = 8.0 Hz, 2H), 7.25 (t, *J* = 8.0 Hz, 1H), 7.20 (t, *J =* 8.0 Hz, 1H), 7.06 (s, 1H), 7.02 (s, 1H), 6.99 (s, 1H), 6.91 (dd, *J₁* = 11.6 Hz, *J₂* = 2.4 Hz, 1H), 6.70 (td, *J₁* = 8.4 Hz, *J₂* = 2.4 Hz, 1H), 5.14 (s, 2H), 3.91 (s, 2H), 3.50 (s, 2H), 3.28 (t, *J =* 5.2Hz, 4H), 1.46 (t, *J =* 5.2Hz, 4H), 0.34 (s, 4H); LC/MS(ESI+) m/z: [M+H]⁺ =493.05.

### Example 22. Synthesis of 2-(2-((5'-(aminomethyl)-2'-fluoro-5-(6-azaspiro[2.5]octan-6-yl)-[1,1'-biphenyl]-3-yl)methoxy)phenyl)acetic acid (compound 022)

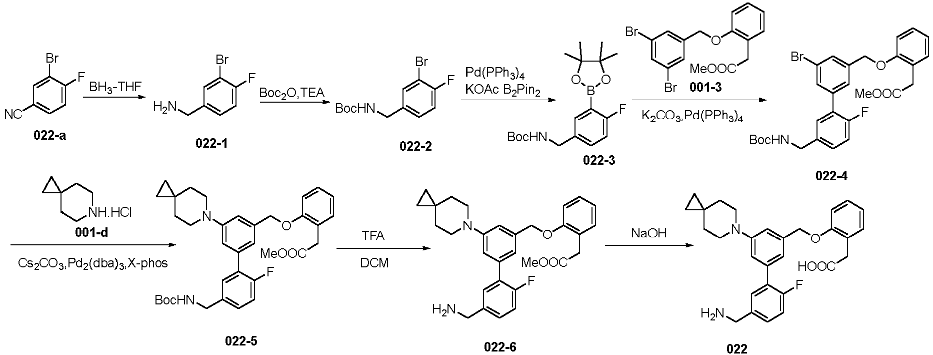

The synthesis of compound 022 refers to the synthesis method of compound 007, which only needs to replace the starting material 007-a with 3-bromo-4-fluorobenzonitrile (022-a).

¹H NMR (400 MHz, DMSO-*d₆*): δ 7.69 (d, *J* = 8.0 Hz, 1H), 7.27 (m, 1H), 7.09 (m, 6H), 6.83 (m, 2H), 5.20 (s, 2H), 5.08 (s, 2H), 4.19 (s, 1H), 3.79 (s, 1H), 3.37 (s, 2H), 3.28 (s, 4H), 1.48 (d, *J* = 4.0 Hz, 4H), 0.35 (s, 4H); LC/MS(ESI+) m/z: [M+H]⁺ =475.05.

### Example 23. Synthesis of 2-(2-((3-(2-(aminomethyl)-3-fluoropyridin-4-yl)-5-(6-azaspiro[2.5]octan-6-yl)benzyl)oxy)phenyl)acetic acid (compound 023)

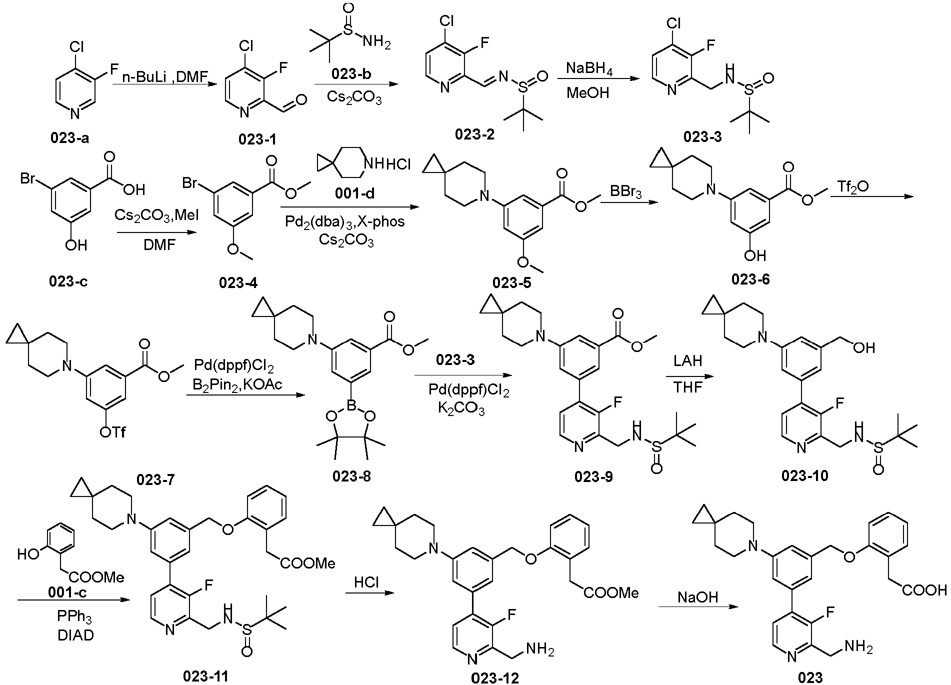

### 23.1 Synthesis of compound 023-1

Triacetonamine (12.9 g, 91.2 mmol) was weighed into a reaction flask, then 50 mL of dry tetrahydrofuran was added thereto, and n-butyllithium (57 mL, 91.2 mmol) was slowly added dropwise thereto under nitrogen atmosphere at 0°C. The reaction was carried out at 0°C for 1 hour. Then the reaction mixture was cooled to -78°C and slowly added dropwise with 023-a (10 g, 76 mmol), and the temperature was kept below -65°C and the reaction was carried out for 10 min. Then N,N-dimethylformamide (16.7 g, 228.1 mmol) was added dropwise thereto, and the temperature was kept below -65°C and the reaction was carried out for 10 min. Then glacial acetic acid (6.85 g, 114 mmol) and acetic anhydride (11.6 g, 114 mmol) were added dropwise thereto, and then the reaction mixture was warmed to 0°C and added with water (100 mL). The pH of the reaction mixture was adjusted to 8, and the reaction mixture was extracted with ethyl acetate (100 mL × 3). The organic phases were combined, dried over anhydrous sodium sulfate, and filtered. The filtrate was concentrated under reduced pressure, and the residue was purified by silica gel column chromatography (petroleum ether/ethyl acetate = 2/1 to 1/1) to obtain compound 023-1.

### 23.2 Synthesis of compound 023-2

023-1 (8.4 g, 52.7 mmol) and 023-b (7.66 g, 63.2 mmol) were weighed into a reaction flask, then dichloromethane (50 mL) and cesium carbonate (34.3 g, 105.3 mmol) were added thereto, and the reaction was carried out at room temperature for 2 hours. The reaction mixture was added with water (100 mL) and extracted with ethyl acetate (50 mL × 3). The organic phases were combined, dried over anhydrous sodium sulfate, filtered, and the filtrate was concentrated under reduced pressure. The residue was purified by silica gel column chromatography (petroleum ether/ethyl acetate = 2/1 to 1/1) to obtain compound 023-2. LC/MS(ESI+)m/z: [M+H]⁺=262.95.

### 23.3 Synthesis of compound 023-3

023-2 (8.0 g, 30.45 mmol) was weighed into a reaction flask, then methanol (60 mL) was added thereto. To the above system was slowly added sodium borohydride (4.61 g, 121.80 mmol) under nitrogen atmosphere, and the reaction was carried out at room temperature for 2 hours. The reaction mixture was concentrated under reduced pressure, and then added with ethyl acetate (50 mL) and saturated sodium bicarbonate aqueous solution (50 mL). The organic phase was separated, and the aqueous phase was extracted with ethyl acetate (50 mL × 3). The organic phases were combined, dried over anhydrous sodium sulfate, and filtered. The filtrate was concentrated under reduced pressure, and the residue was purified by silica gel column chromatography (petroleum ether/ethyl acetate = 2/1 to 1/1) to obtain compound 023-3. LC/MS(ESI+) m/z: [M+H]⁺=265.05.

### 23.4 Synthesis of compound 023-4

023-c (10 g, 46.1 mmol) was weighed into a reaction flask, then N,N-dimethylformamide (50 mL), cesium carbonate (45 g, 138.2 mmol), and methyl iodide (16.4 g, 115.2 mmol) were added thereto, and the reaction was carried out at room temperature overnight. The reaction mixture was added with water and extracted with ethyl acetate (50 mL × 3). The organic phases were combined, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 023-4.

### 23.5 Synthesis of compound 023-5

023-4 (5 g, 20.4 mmol) was weighed into a reaction flask, then 001-d (3.68 g, 23 mmol), cesium carbonate (20 g, 61.21 mmol), 1,4-dioxane (30 mL), tris(dibenzylideneacetone)dipalladium (200 mg, 2.04 mmol), and 2-dicyclohexylphosphino-2',4',6'-triisopropylbiphenyl (100 mg, 2.04 mmol) were added thereto, and the reaction was carried out at 100°C for 12 hours. The reaction mixture was filtered and concentrated, and the residue was purified by silica gel column chromatography (petroleum ether/ethyl acetate = 3/1 to 1/1) to obtain compound 023-5. LC/MS(ESI+) m/z: [M+H]⁺ =276.05.

### 23.6 Synthesis of compound 023-6

023-5 (6.0 g, 14.5 mmol) was weighed into a reaction flask, then dichloromethane (30 mL) was added thereto, and boron tribromide (7.28 g, 29.1 mmol) was slowly added dropwise thereto at 0°C. After the addition, the reaction mixture was warmed to room temperature and reacted for 2 hours. The reaction was slowly quenched by adding methanol (30 mL), then the reaction mixture was added with water (30 mL) and extracted with dichloromethane (50 mL × 3). The organic phases were combined, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 023-6. LC/MS(ESI+) m/z: [M+H]⁺ =262.05.

### 23.7 Synthesis of compound 023-7

023-6 (2.4 g, 10 mmol) was weighed into a reaction flask, then dichloromethane (30 mL) was added thereto. Pyridine (870 mg, 6.26 mmol) was added thereto, and trifluoromethanesulfonic anhydride (3.4 g, 12 mmol) was added dropwise thereto at 0°C. After the addition, the reaction mixture was warmed to room temperature and reacted for 2 hours. The reaction mixture was added with water (30 mL) and extracted with dichloromethane (50 mL × 3). The organic phases were combined, dried over anhydrous sodium sulfate, filtered, and the filtrate was concentrated under reduced pressure. The residue was purified by silica gel column chromatography (petroleum ether/ethyl acetate = 5/1 to 1/1) to obtain compound 023-7.

### 23.8 Synthesis of compound 023-8

023-7 (1.7 g, 4.32 mmol) was weighed into a reaction flask, then 1,4-dioxane (50 mL), bis(pinacolato)diboron (1.7 g, 6.5 mmol), potassium acetate (1.3 g, 13 mmol), and [1,1'-bis(diphenylphosphino)ferrocene]dichloropalladium(II) (323 mg, 0.44 mmol) were added thereto. The reaction mixture was refluxed at 80°C for 12 hours under nitrogen atmosphere. The reaction mixture was concentrated, and the residue was purified by silica gel column chromatography (petroleum ether/ethyl acetate = 5/1 to 1/1) to obtain compound 023-8. LC/MS(ESI+) m/z: [M+H]⁺ =372.00.

### 23.9 Synthesis of compound 023-9

023-8 (1.5 g, 4.05 mmol) was weighed into a reaction flask, and 023-3 (962 mg, 5.3 mmol), 1,4-dioxane (30 mL), and water (5 mL) were added thereto, and then [1,1'-bis(diphenylphosphino)ferrocene]dichloropalladium(II) (356 mg, 0.405 mmol) and potassium carbonate (1.68 g, 12.12 mmol) were added thereto. The reaction mixture was refluxed at 100°C for 2 hours under nitrogen atmosphere. The reaction mixture was concentrated, and the residue was purified by silica gel column chromatography (petroleum ether/ethyl acetate = 5/1 to 1/1) to obtain compound 023-9. LC/MS(ESI+) m/z: [M+H]⁺ =474.00.

### 23.10 Synthesis of compound 023-10

023-9 (1.7 g, 3.59 mmol) was weighed into a reaction flask, then tetrahydrofuran (15 mL) was added thereto. The reaction mixture was cooled to 0°C, slowly added with lithium aluminum hydride (408 mg, 10.77 mmol), and the reaction was carried out at 0°C for 1 hour. The reaction mixture was added with water (20 mL) and extracted with ethyl acetate (50 mL × 3). The organic phases were combined, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 023-10. LC/MS(ESI+) m/z: [M+H]⁺ =446.00.

### 23.11 Synthesis of compound 023-11

023-10 (700 mg, 1.57 mmol) was weighed into a reaction flask, then 001-c (313 mg, 1.89 mmol), triphenylphosphine (824 mg, 3.14 mmol), and dichloromethane (10 mL) were added thereto, and diisopropyl azodicarboxylate (635 mg, 3.14 mmol) was slowly added dropwise thereto at 0°C under nitrogen atmosphere. After the addition, the reaction mixture was warmed to room temperature and reacted for 2 hours. The reaction mixture was concentrated, and the residue was purified by silica gel column chromatography (petroleum ether/ethyl acetate = 3/1 to 1/1) to obtain compound 023-11. LC/MS(ESI+) m/z: [M+H]⁺ =594.00.

### 23.12 Synthesis of compound 023-12

023-11 (400 mg, 1.674 mmol) was weighed into a reaction flask, then dichloromethane (2 mL) and a solution of hydrogen chloride in 1,4-dioxane (4N, 2 mL) were added thereto, and the reaction was carried out at room temperature for 2 hours. The reaction mixture was concentrated to obtain compound 023-12 (crude product), which could be used directly in the next step.

### 23.13 Synthesis of compound 023

To methanol (2 mL) and water (2 mL) were sequentially added 023-12 (300 mg, 0.613 mmol) and sodium hydroxide (246 mg, 1.84 mmol), and the reaction was carried out at 60°C for 2 hours. The reaction mixture was purified by preparative high-performance liquid chromatography and lyophilized to obtain the target compound 023.

¹H NMR (400 MHz, DMSO-*d₆*): δ 8.52 (d, *J* = 8.0 Hz, 1H), 8.43 (s, 2H), 7.69 (t, *J* = 4.0 Hz, 1H), 7.26 - 7.20 (m, 3H), 7.14 (d, *J =* 12.0 Hz, 2H), 7.04 (d, *J =* 8.0 Hz, 1H), 6.91 (t, *J* = 4.0 Hz, 1H), 5.15 (s, 2H), 4.34 (d, *J =* 4.0 Hz, 2H), 3.59 (s, 2H), 3.37 - 3.30 (m, 4H), 1.51 - 1.44 (m, 4H), 0.35 (s, 4H); LC/MS(ESI+) m/z: [M+H]⁺ =476.00.

### Example 24. Synthesis of 2-(2-((3'-(aminomethyl)-2'-fluoro-5-(2-oxa-7-azaspiro[3.5]nonan-7-yl)-[1,1'-biphenyl]-3-yl)methoxy)phenyl)acetic acid (compound 024) trifluoroacetate

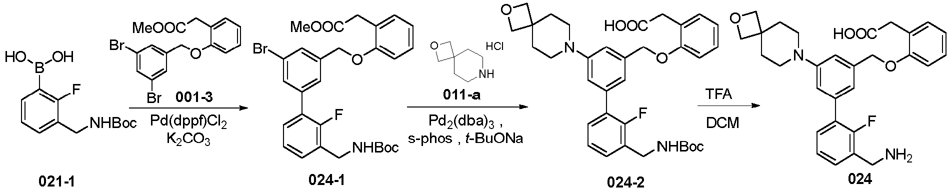

### 24.1 Synthesis of compound 024-1

To a 50 mL reaction flask were added 021-1 (407 mg, 1.51 mmol), potassium carbonate (626 mg, 4.53 mmol), 001-3 (1.13 g, 2.72 mmol), [1,1'-bis(diphenylphosphino)ferrocene]dichloropalladium(II) (110 mg, 0.15 mmol), 1,4-dioxane (9 mL), and water (1 mL). After the system was replaced with nitrogen three times, the reaction mixture was refluxed at 110°C for 2 hours. The reaction mixture was cooled to room temperature, filtered, and the filtrate was diluted with water (60 mL) and extracted with ethyl acetate (20 mL × 3). The combined organic phases were washed with saturated sodium chloride solution, dried over anhydrous sodium sulfate, filtered, and the filtrate was concentrated under reduced pressure. The residue was purified by silica gel column chromatography (petroleum ether/ethyl acetate = 4/1) to obtain compound 024-1. LC/MS(ESI+)m/z: [M+H-Boc]⁺=457.90.

### 24.2 Synthesis of compound 024-2

To a 50 mL reaction flask were added 024-1 (303 mg, 0.54 mmol), sodium tert-butoxide (156 mg, 1.63 mmol), 011-a (121 mg, 0.705 mmol), tris(dibenzylideneacetone)dipalladium (50 mg, 54.3 µmol), 2-dicyclohexylphosphino-2',6'-dimethoxybiphenyl (45 mg, 108.5 µmol), and toluene (10 mL). After the system was replaced with nitrogen three times, the reaction mixture was refluxed at 110°C for 2 hours. The reaction mixture was cooled to room temperature, added with 0.5 M dilute hydrochloric acid to adjust the pH to 2, filtered, and the filtrate was diluted with water (60 mL) and extracted with ethyl acetate (20 mL × 3). The combined organic phases were washed with saturated sodium chloride aqueous solution, dried over anhydrous sodium sulfate, filtered, and the filtrate was concentrated under reduced pressure. The residue was purified by silica gel column chromatography to obtain compound 024-2. LC/MS(ESI+)m/z: [M+H]⁺=591.05.

### 24.3 Synthesis of trifluoroacetate of compound 024

024-2 (59 mg, 0.1 mmol) and dichloromethane (2 mL) were placed in a flask, then the system was added with trifluoroacetic acid (0.4 mL) at room temperature, and the reaction mixture was stirred at room temperature for 2 hours. The reaction mixture was concentrated under reduced pressure, and the residue was purified by preparative high-performance liquid chromatography and lyophilized to obtain the trifluoroacetate of compound 024.

¹H NMR (600 MHz, DMSO-*d₆*): δ 8.25 (s, 2H), 7.57 (dt, *J1* = 7.6 Hz, *J2* = 1.6 Hz, 1H), 7.51 (t, *J* = 7.6 Hz, 1H), 7.34 (t, *J* = 7.6 Hz, 1H), 7.22 (d, *J =* 7.2 Hz, 2H), 7.12 (s, 1H), 7.04-7.0 (m, 3H), 6.90 (t, *J* = 7.6 Hz, 1H), 5.11 (s, 2H), 4.35 (s, 4H), 4.15 (d, *J* = 6.0 Hz, 2H), 3.58 (s, 2H), 3.17 (t, *J* = 5.6 Hz, 3H), 1.89 (t, *J* = 5.6 Hz, 3H), 1.23 (s, 2H); LC/MS(ESI+) m/z: [M+H]⁺ =491.05.

### Example 25. Synthesis of 2-(2-((3'-(aminomethyl)-2'-fluoro-5-(2-oxa-7-azaspiro[3.5]nonan-7-yl)-[1,1'-biphenyl]-3-yl)methoxy)-5-fluorophenyl)acetic acid (compound 025)

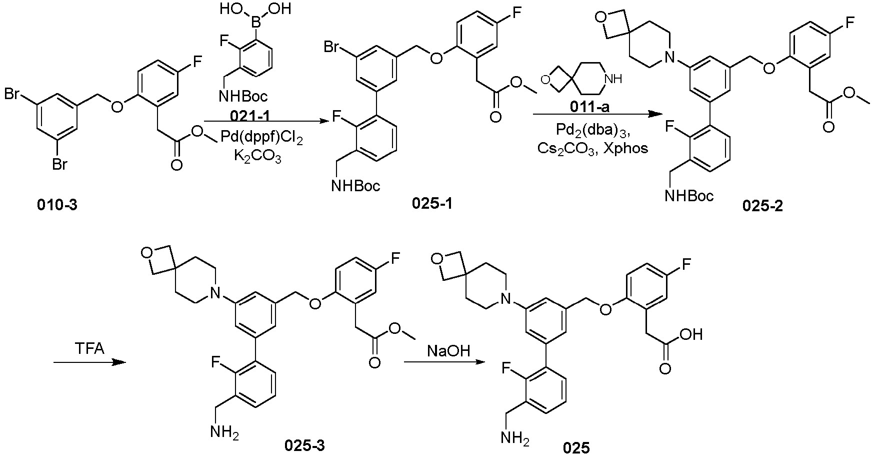

### 25.1 Synthesis of compound 025-1

010-3 (300 mg, 0.69 mmol), 021-1 (244 mg, 0.69 mmol), potassium carbonate (192 mg, 1.39 mmol), and [1,1'-bis(diphenylphosphino)ferrocene]dichloropalladium(II) (52 mg, 0.07 mmol) were dissolved in 1,4-dioxane (10 mL) and water (1 mL) under nitrogen atmosphere, and the resulting mixture was reacted at 100°C for 2 hours. The reaction mixture was cooled to room temperature, added with water, extracted with ethyl acetate (10 mL × 3). The combined organic phases were dried over anhydrous sodium sulfate, filtered, and the filtrate was concentrated under reduced pressure. The residue was purified by silica gel column chromatography (petroleum ether/ethyl acetate = 3/1 to 1/1) to obtain compound 025-1. LC/MS(ESI+)m/z: [M+H-Boc]⁺=475.85.

### 25.2 Synthesis of compound 025-2

025-1 (300 mg, 0.52 mmol) was weighed into a reaction flask, then 011-a (110 mg, 0.52 mmol), cesium carbonate (680 mg, 2.08 mmol), 1,4-dioxane (10 mL), tris(dibenzylideneacetone)dipalladium (80 mg, 0.06 mmol), and 2-dicyclohexylphosphino-2',4',6'-triisopropylbiphenyl (150 mg, 0.06 mmol) were added thereto, and the reaction was carried out at 100°C for 4 hours. The reaction mixture was filtered, and the filtrate was concentrated under reduced pressure. The residue was purified by silica gel column chromatography (petroleum ether/ethyl acetate = 3/1 to 1/1) to obtain compound 025-2. LC/MS(ESI+)m/z: [M+H]⁺=623.00.

### 25.3 Synthesis of compound 025

The synthesis of compound 025 refers to the synthesis method of steps 1.6 and 1.7 in Example 1.

¹H NMR (600 MHz, DMSO-*d₆*): δ 7.43 (t, *J* = 8.0 Hz, 1H), 7.41 - 7.36 (m, 1H), 7.21 (t, J = 8.0 Hz, 1H), 7.08 - 7.04 (m, 2H), 7.02 (s, 1H), 7.00 - 6.94 (m, 3H), 5.10 (s, 2H), 4.34 (s, 4H), 3.83 (s, 2H), 3.51 (s, 2H), 3.18 - 3.12 (m, 4H), 1.90 - 1.87 (m, 4H); LC/MS(ESI+) m/z: [M+H]⁺ =509.05.

### Example 26. Synthesis of 2-(2-((3'-(aminomethyl)-4-fluoro-5-(2-oxa-7-azaspiro[3.5]nonan-7-yl)-[1,1'-biphenyl]-3-yl)methoxy)phenyl)acetic acid (compound 026)

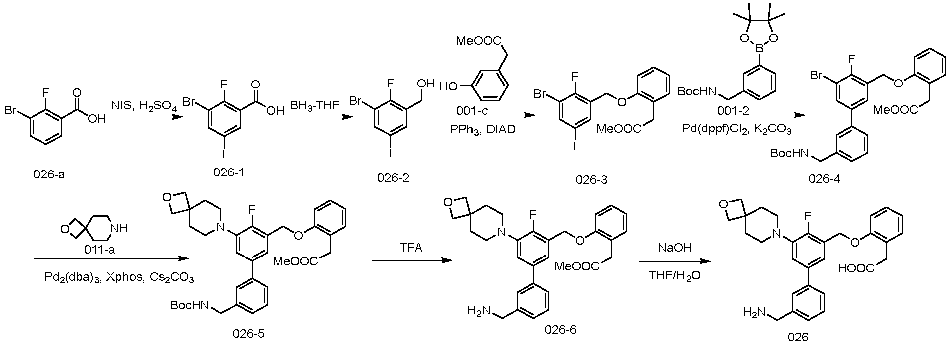

### 26.1 Synthesis of compound 026-1

026-a (3.0 g, 13.7 mmol) was weighed into a reaction flask, then sulfuric acid (10 mL) was added thereto, and N-iodosuccinimide (2.5 g, 14.4 mmol) was slowly added thereto at 0°C. Then the reaction mixture was warmed to room temperature and reacted overnight. The reaction mixture was poured into ice water to precipitate a solid, filtered, and the solid was dried to obtain compound 026-1.

### 26.2 Synthesis of compound 026-2

026-1 (3.6 g, 10.44 mmol) was weighed into a reaction flask, then tetrahydrofuran (20 mL) was added thereto, and borane tetrahydrofuran (31.3 mL, 31.30 mmol, 1 M) was slowly added dropwise thereto under nitrogen atmosphere at 0°C. The reaction mixture was reacted for 10 min, then heated to 60°C, and then reacted for 2 hours. The reaction mixture was cooled to room temperature, and the reaction was quenched by slow dropwise addition of methanol (20 mL). After quenching, the reaction mixture was concentrated, and the residue was purified by silica gel column chromatography (petroleum ether/ethyl acetate = 10/1 to 1/1) to obtain compound 026-2.

### 26.3 Synthesis of compound 026-3

026-2 (1 g, 3.02 mmol) was weighed into a reaction flask, then 001-c (551 mg, 3.02 mmol), dichloromethane (20 mL), and triphenylphosphine (1.6 g, 6.04 mmol) were added thereto. The system was replaced with nitrogen three times, and diisopropyl azodicarboxylate (2.2 g, 6.04 mmol) was slowly added dropwise thereto at 0°C, and then the reaction was carried out at room temperature for 2 hours. The reaction mixture was concentrated, and the residue was purified by silica gel column chromatography (petroleum ether/ethyl acetate = 10/1 to 1/1) to obtain compound 026-3. LC/MS(ESI+)m/z: [M+H]⁺ = 480.75.

### 26.4 Synthesis of compound 026-4

026-3 (540 mg, 1.13 mmol), 001-2 (376 mg, 1.13 mmol), potassium carbonate (312 mg, 2.25 mmol), and [1,1'-bis(diphenylphosphino)ferrocene]dichloropalladium(II) (83 mg, 0.113 mmol) were dissolved in 1,4-dioxane (10 mL) and water (1 mL) under nitrogen atmosphere, and the reaction was carried out at 100°C for 2 hours. The reaction mixture was cooled to room temperature, added with water, and extracted with ethyl acetate (10 mL × 3). The organic phases were combined, dried over anhydrous sodium sulfate, filtered, and the filtrate was concentrated under reduced pressure. The residue was purified by silica gel column chromatography (petroleum ether/ethyl acetate = 3/1 to 1/1) to obtain compound 026-4. LC/MS(ESI+)m/z: [M+H]⁺ = 459.90.

### 26.5 Synthesis of compound 026-5

026-4 (400 mg, 0.72 mmol) was weighed into a reaction flask, then 011-a (110 mg, 0.72 mmol), cesium carbonate (480 mg, 1.4 mmol), 1,4-dioxane (10 mL), tris(dibenzylideneacetone)dipalladium (66 mg, 0.07 mmol), and 2-dicyclohexylphosphino-2',4',6'-triisopropylbiphenyl (32 mg, 0.07 mmol) were added thereto, and the reaction was carried out at 100°C for 4 hours. The reaction mixture was cooled to room temperature, diluted with water (20 mL), and extracted with ethyl acetate (20 mL × 3). The organic phase was concentrated, and the residue was purified by silica gel column chromatography (petroleum ether/ethyl acetate = 3/1 to 1/1) to obtain compound 026-5. LC/MS(ESI+)m/z: [M+H]⁺ = 605.10.

### 26.6 Synthesis of compound 026

The synthesis of compound 026 refers to the synthesis method of steps 1.6 and 1.7 in Example 1.

¹H NMR (400 MHz, DMSO-*d₆*): δ 8.14 (s, 1H), 7.66 (d, *J* = 4.0 Hz, 2H), 7.37 (t, *J* = 8.0 Hz, 1H), 7.27-7.22 (m, 2H), 7.12-7.07 (m , 2H), 6.89 (d, *J* = 8.0 Hz, 1H), 6.81 (t, *J* = 8.0 Hz, 1H), 5.25 (s, 2H), 4.37 (s, 4H), 3.93 (s, 2H), 3.39 (s, 2H), 3.00 (s, 4H), 2.07-1.86 (m, 4H); LC/MS(ESI+) m/z: [M+H]⁺ =491.05.

### Example 27. Synthesis of 2-(2-((3'-(aminomethyl)-6-fluoro-5-(2-oxa-7-azaspiro[3.5]nonan-7-yl)-[1,1'-biphenyl]-3-yl)methoxy)phenyl)acetic acid (compound 027)

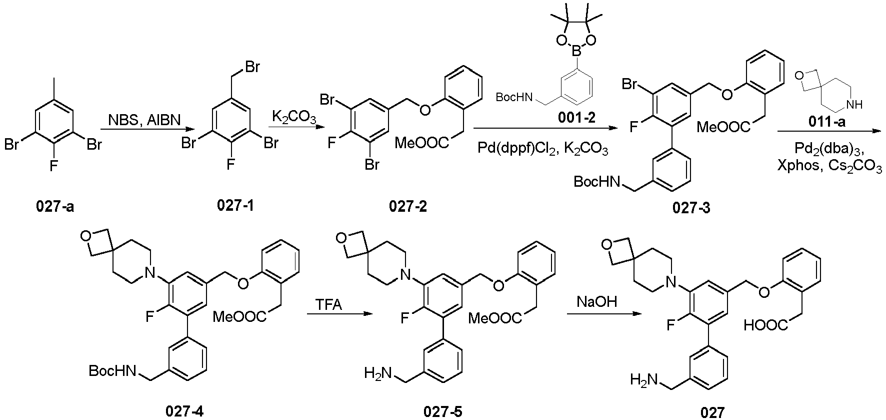

### 27.1 Synthesis of compound 027-1

027-a (2.0 g, 7.4 mmol) was weighed and dissolved in acetonitrile (20 mL), then N-bromosuccinimide (1.31 g, 7.4 mmol) and azobisisobutyronitrile (120 mg, 0.74 mmol) were added thereto, and the reaction was carried out at 80°C for 2 hours. The reaction mixture was concentrated, and the residue was purified by silica gel column chromatography to obtain compound 027-1.

### 27.2 Synthesis of compound 027-2

027-1 (1.8 g, 5.19 mmol) was weighed and dissolved in acetonitrile (20 mL), then potassium carbonate (1.43 g, 10.38 mmol) and methyl o-hydroxyphenylacetate (905.5 mg, 5.45 mmol) were added thereto, and the reaction was carried out at 25°C for 2 hours. The reaction mixture was filtered, then the filtrate was concentrated, and the residue was purified by silica gel column chromatography to obtain compound 027-2. LC/MS(ESI+) m/z: [M+H]⁺ = 432.8.

### 27.3 Synthesis of compound 027-3

027-2 (1.0 g, 2.3 mmol) was weighed into a reaction flask, then potassium carbonate (640 mg, 4.6 mmol), 001-2 (0.77 g, 2.3 mmol), [1,1'-bis(diphenylphosphino)ferrocene]dichloropalladium(II) (100 mg, 10 wt%), 1,4-dioxane (10 mL), and water (1 mL) were added thereto. The reaction was carried out at 90°C for 2 hours under nitrogen atmosphere. The reaction mixture was cooled to room temperature, filtered, then the filtrate was concentrated under reduced pressure, and the residue was purified by silica gel column chromatography to obtain compound 027-3. LC/MS(ESI+) m/z: [M+H]⁺= 558.1.

### 27.4 Synthesis of compound 027-4

027-3 (120 mg, 0.21 mmol) was weighed and dissolved in 1,4-dioxane (5 mL), then 011-a (48.7 mg, 0.25 mmol), cesium carbonate (210 mg, 0.64 mmol), tris(dibenzylideneacetone)dipalladium (12 mg, 10 wt%), and 2-dicyclohexylphosphino-2',4',6'-triisopropylbiphenyl (12 mg, 10 wt%) were added thereto, and the reaction was carried out at 100°C for 2 hours under nitrogen atmosphere. The reaction mixture was cooled to room temperature, filtered, then the filtrate was concentrated, and the residue was purified by silica gel column chromatography to obtain compound 027-4. LC/MS(ESI+) m/z: [M+H]⁺ = 605.1.

### 27.5 Synthesis of compound 027

The synthesis of compound 027 refers to the synthesis method of steps 1.6 and 1.7 in Example 1.

¹H NMR (400 MHz, DMSO-*d₆*): δ 7.98 (s, 1H), 7.61 (d, *J* = 7.2 Hz, 1H), 7. 43 (d, *J* = 5.6 Hz, 1H), 7.38 (t, *J* = 8.0 Hz, 1H), 7.30 (d, *J* = 7.6 Hz, 1H), 7.10-7.08 (m, 3H), 6.90 (d, *J* = 8.4 Hz, 1H), 6.80 (t, *J* = 7.6 Hz, 1H), 5.10 (s, 2H), 4.37 (s, 4H), 3.94 (s, 2H), 3.36 (s, 2H), 2.95 (t, *J =* 5.2 Hz, 4H), 1.95 (t, *J* = 5.6 Hz, 4H); LC/MS(ESI+) m/z: [M+H]⁺ =491.0.

### Example 28. Synthesis of 2-(2-((3'-(aminomethyl)-5-(2-oxa-7-azaspiro[3.5]nonan-7-yl)-[1,1'-biphenyl]-3-yl)methoxy)-3-fluorophenyl)acetic acid (compound 028)

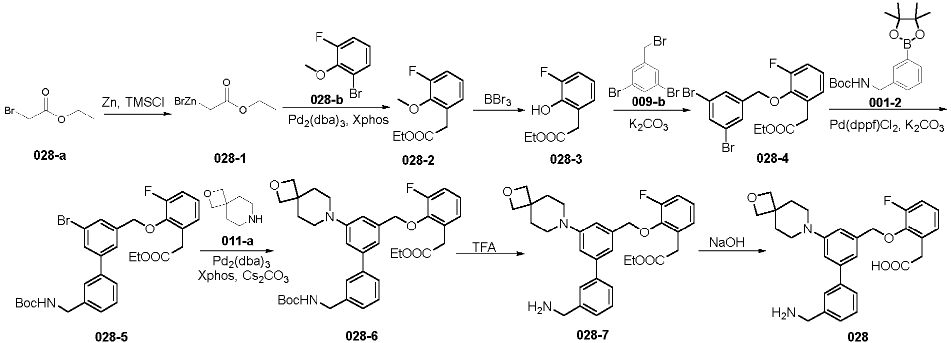

### 28.1 Synthesis of compound 028-1

Zinc powder (4.7 g, 71.86 mmol) was weighed into a reaction flask, then tetrahydrofuran (10 mL) was added thereto, and trimethylchlorosilane (390 mg, 3.59 mmol) was added thereto under nitrogen atmosphere, and the reaction was carried out at 50°C for 0.5 hours. Then 028-a (6.0 g, 35.93 mmol) was slowly added dropwise thereto. The reaction mixture was reacted at 70°C for 2 hours to obtain a solution of compound 028-1, which was used directly in the next step.

### 28.2 Synthesis of compound 028-2

028-b (2.0 g, 9.75 mmol) was weighed into a reaction flask, then tetrahydrofuran (10 mL), tris(dibenzylideneacetone)dipalladium (1.0 g, 0.98 mmol), and 2-dicyclohexylphosphino-2',4',6'-triisopropylbiphenyl (500 mg, 0.95 mmol) were added thereto, and the solution of compound 028-1 (12 mL) obtained in step 28.1 was slowly added dropwise thereto under nitrogen atmosphere. The reaction was carried out at 70°C for 2 hours. The reaction mixture was quenched by the addition of saturated ammonium chloride aqueous solution (10 mL) and extracted with ethyl acetate (30 mL × 3). The combined organic phases were dried over anhydrous sodium sulfate, filtered, and the filtrate was concentrated under reduced pressure. The residue was purified by silica gel column chromatography (petroleum ether/ethyl acetate = 10/1 to 1/1) to obtain compound 028-2. LC/MS(ESI+) m/z: [M+H]⁺=213.10

### 28.3 Synthesis of compound 028-3

028-2 (1.8 g, 8.5 mmol) was weighed into a reaction flask, then dichloromethane (20 mL) was added thereto, and boron tribromide (18 mL, 17.0 mmol) was slowly added dropwise thereto under nitrogen atmosphere at 0°C. The reaction mixture was reacted for 10 min, then transferred to room temperature, and the reaction was continued for 10 min. The reaction was quenched by slow dropwise addition of ethanol (10 mL), and the reaction mixture was diluted with water (10 mL) and extracted with dichloromethane (30 mL × 3). The organic phase was dried over anhydrous sodium sulfate, filtered, and the filtrate was concentrated under reduced pressure. The residue was purified by silica gel column chromatography (petroleum ether/ethyl acetate = 10/1 to 1/1) to obtain compound 028-3. LC/MS(ESI+) m/z: [M+H]⁺ = 199.10.

### 28.4 Synthesis of compound 028-4

028-3 (0.75 g, 3.65 mmol) was weighed into a reaction flask, then 009-b (1.2 g, 3.65 mmol), potassium carbonate (1.1 g, 7.3 mmol), and N,N-dimethylformamide (20 mL) were added thereto, and the reaction was carried out at room temperature for 2 hours. The reaction mixture was diluted with water (20 mL) and extracted with ethyl acetate (30 mL × 3). The organic phase was dried over anhydrous sodium sulfate, filtered, and the filtrate was concentrated under reduced pressure. The residue was purified by silica gel column chromatography (petroleum ether/ethyl acetate = 10/1 to 1/1) to obtain compound 028-4. LC/MS(ESI+) m/z: [M+H]⁺= 446.75.

### 28.5 Synthesis of compound 028

The synthesis of compound 028 refers to the synthesis method of compound 027, which only needs to replace the 027-2 in the synthesis step with 028-4.

¹H NMR (400 MHz, DMSO-*d₆*): δ 8.18 (s, 1H), 7.68 (d, *J* = 8.0 Hz, 1H), 7.53 (s, 1H), 7.39 (t, *J* = 8.0 Hz, 1H), 7.28 (d, *J* = 8.0 Hz, 1H), 7.18 (s, 1H), 7.09-7.03 (m, 1H), 7.00 - 6.93 (m, 3H), 5.09 (s, 2H), 4.35 (s, 4H), 3.97 (s, 2H), 3.43 (s, 2H), 3.21 - 3.16 (m, 4H), 1.93 - 1.88 (m, 4H); LC/MS(ESI+) m/z: [M+H]⁺ =491.05.

### Example 29. Synthesis of 2-(2-((3'-(aminomethyl)-5-(3-oxa-9-azaspiro[5.5]undecan-9-yl)-[1,1'-biphenyl]-3-yl)methoxy)phenyl)acetic acid (compound 029)

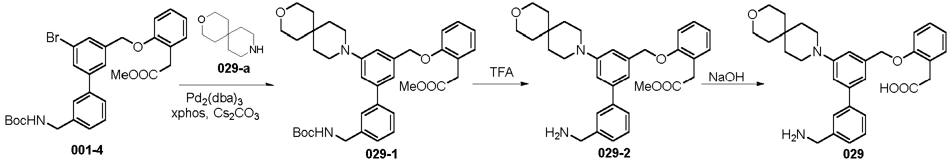

The synthesis of compound 029 refers to the synthesis method of compound 001, which only needs to replace the raw material in the step from 001-d to 3-oxa-9-azaspiro[5.5]undecane (029-a).

¹H NMR (600 MHz, DMSO-*d₆*): δ 8.25 (s, 2H), 7.79 (s, 1H), 7.70 (d, *J* = 5.6 Hz, 1H), 7.50 (t, *J* = 5.2 Hz, 1H), 7.43 (d, *J* = 5.2 Hz, 1H), 7.25 - 7.18 (m, 4H), 7.15 (d, *J* = 2.0 Hz, 1H), 7.03 (d, *J* = 5.2 Hz, 1H), 6.93 - 6.87 (m, 1H), 5.13 (s, 2H), 4.12 (dd, *J* = 4.0 Hz, *J* = 7.6 Hz, 2H), 3.60 (s, 2H), 3.60 - 3.57 (m, 4H), 3.32 - 3.25 (m, 4H), 1.69 - 1.62 (m, 4H), 1.51 - 1.46 (m, 4H); LC/MS(ESI+) m/z: [M+H]⁺ =501.10.

### Example 30. Synthesis of 2-(2-((3'-(aminomethyl)-5-(2-oxa-9-azaspiro[5.5]undecan-9-yl)-[1,1'-biphenyl]-3-yl)methoxy)phenyl)acetic acid (compound 030)

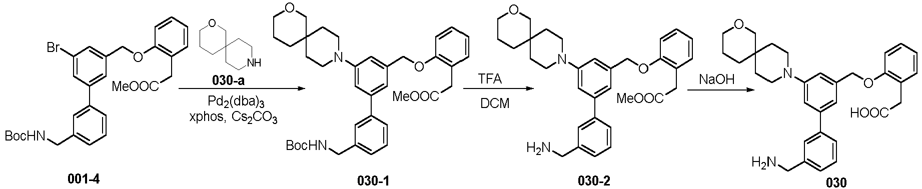

The synthesis of compound 030 refers to the synthesis method of compound 001, which only needs to replace the raw material in the step from 001-d to 2-oxa-9-azaspiro[5.5]undecane (030-a).

¹H NMR (400 MHz, DMSO-*d₆*): δ 8.17 (s, 1H), 7.67 (d, *J =* 8.0 Hz, 1H), 7.41 (s, 1H), 7.37 (t, *J =* 7.6 Hz, 1H), 7.26 (d, *J* = 7.6 Hz, 1H), 7.14 - 7.05 (m, 3H), 6.97 (s, 1H), 6.90 (d, *J* = 8.0 Hz, 1H), 6.80 (t, *J* = 7.2 Hz, 1H), 5.14 (s, 2H), 3.94 (s, 2H), 3.55 (s, 2H), 3.40 (d, *J* = 2.4 Hz, 4H), 3.23 (t, *J =* 5.6 Hz, 4H), 1.62 - 1.51 (m, 8H); LC/MS(ESI+) m/z: [M+H]⁺ =501.15.

### Example 31. Synthesis of 2-(2-((3'-(aminomethyl)-5-(6-oxa-2-azaspiro[3.4]octan-2-yl)-[1,1'-biphenyl]-3-yl)methoxy)phenyl)acetic acid (compound 031)

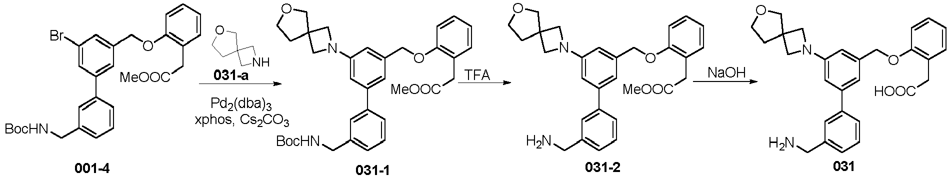

The synthesis of compound 031 refers to the synthesis method of compound 001, which only needs to replace the raw material 001-d with 6-oxa-2-azaspiro[3.4]octane (031-a).

¹H NMR (400 MHz, DMSO-*d₆*): δ 9.47 (s, 1H), 7.92 (d, *J* = 59.6 Hz, 1H), 7.53 (dd, *J* = 30.0, 8 Hz, 1H), 7.39 - 7.33 (m, 1H), 7.30 - 7.24 (m, 2H), 7.17 - 7.00 (m, 2H), 6.92 - 6.74 (m, 2H), 6.64 (d, *J* = 5.6 Hz, 1H), 6.48 (d, *J =* 23.2 Hz, 1H), 5.14 (d, *J* = 32.0 Hz, 2H), 4.24 (s, 1H), 3.86 (s, 4H), 3.83 (d, *J* = 3.2 Hz, 2H), 3.74 (t, *J* = 7.2 Hz, 3H), 3.38 (d, *J* = 13.2 Hz, 2H), 2.18 - 2.13 (m, 2H); LC/MS(ESI+) m/z: [M+H]⁺ =459.05.

### Example 32. Synthesis of 2-(2-((3'-(aminomethyl)-5-(2-methyl-2,7-diazaspiro[3.5]nonan-7-yl)-[1,1'-biphenyl]-3-yl)methoxy)phenyl)acetic acid (compound 032)

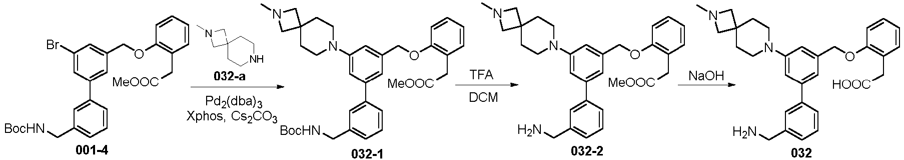

The synthesis of compound 032 refers to the synthesis method of compound 001, which only needs to replace the raw material 001-d with 2-methyl-2,7-diazaspiro[3.5]nonane (032-a).

¹H NMR (400 MHz, DMSO-*d₆*): δ 11.67 - 11.47 (m, 1H), 10.08 (s, 1H), 8.23 (s, 2H), 7.78 (s, 1H), 7.69 (d, *J* = 7.6 Hz, 1H), 7.50 (t, *J* = 7.6 Hz, 1H), 7.43 (d, *J* = 7.6 Hz, 1H), 7.23 (t, *J* = 7.6 Hz, 2H), 7.14 (d, *J =* 5.8 Hz, 2H), 7.07 (s, 1H), 7.03 (d, *J =* 8.2 Hz, 1H), 6.90 (t, *J =* 7.4 Hz, 1H), 5.12 (s, 2H), 4.11 (d, *J* = 5.4 Hz, 4H), 3.88 - 3.75 (m, 2H), 3.60 (s, 2H), 3.23 (d, *J* = 36.8 Hz, 4H), 2.87 (d, *J* = 4.2 Hz, 3H), 1.90 (s, 4H); LC/MS(ESI+) m/z: [M+H]⁺ =486.05.

### Example 33. Synthesis of 2-(2-((3'-(aminomethyl)-5-(3,9-diazaspiro[5.5]undecan-3-yl)-[1,1'-biphenyl]-3-yl)methoxy)phenyl)acetic acid (compound 033) trifluoroacetate

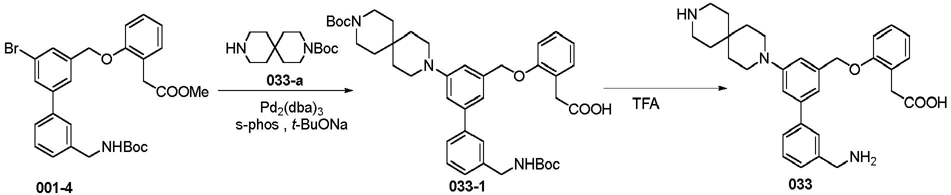

### 33.1 Synthesis of compound 033-1

To a 50 mL reaction flask were added 001-4 (216 mg, 0.4 mmol), sodium tert-butoxide (77 mg, 0.8 mmol), 033-a (122 mg, 0.48 mmol), tris(dibenzylideneacetone)dipalladium (37 mg, 40 µmol), 2-dicyclohexylphosphino-2',6'-dimethoxybiphenyl (33 mg, 80 µmol), and toluene (4 mL). After the system was replaced with nitrogen three times, the reaction mixture was refluxed at 110°C for 2 hours. The reaction mixture was cooled to room temperature, added with 0.5 M dilute hydrochloric acid to adjust the pH to 2, filtered, and the filtrate was diluted with water (60 mL) and extracted with ethyl acetate (20 mL × 3). The combined organic phases were washed with saturated sodium chloride solution, dried over anhydrous sodium sulfate, filtered, and the filtrate was concentrated under reduced pressure. The residue was purified by silica gel column chromatography to obtain compound 033-1. LC/MS(ESI+) m/z: [M+H]⁺ = 700.10.

### 33.2 Synthesis of trifluoroacetate of compound 033

033-1 (78 mg, 0.11 mmol) and dichloromethane (2 mL) were placed in a flask, then the system was added with 4-fluorophenylboronic acid (31 mg, 0.22 mmol) at room temperature. The reaction mixture was stirred at room temperature for 2 hours to remove nitrogen oxides, then the system was added with trifluoroacetic acid (0.2 mL), and the reaction was carried out at room temperature for 1 hour. The reaction mixture was concentrated under reduced pressure, and the residue was purified by preparative high-performance liquid chromatography to obtain the trifluoroacetate of compound 033.

¹H NMR (400 MHz, DMSO-*d₆*): δ 8.49 (s, 2H), 8.26 (s, 3H), 7.78 (s, 1H), 7.69 (d, *J* = 7.6 Hz, 1H), 7.50 (t, *J* = 7.6 Hz, 1H), 7.43 (d, *J* = 7.6 Hz, 1H), 7.22 (d, *J* = 7.2 Hz, 2H), 7.15 (d, *J =* 5.6 Hz, 2H), 7.10 (s, 1H), 7.04 (d, *J* = 8.0 Hz, 1H), 6.91 (t, *J* = 7.6 Hz, 1H), 5.13 (s, 2H), 4.12 (q, *J* = 5.6 Hz, 2H), 3.60 (s, 2H), 3.27 (t, *J* = 6.0 Hz, 4H ), 3.09 (s, 4H), 1.64 (d, *J* = 5.6 Hz, 8H); LC/MS(ESI+) m/z: [M+H]⁺ =500.10.

### Example 34. Synthesis of 2-(2-((3'-(aminomethyl)-5-(2-azaspiro[4.4]nonan-2-yl)-[1,1'-biphenyl]-3-yl)methoxy)phenyl)acetic acid (compound 034)

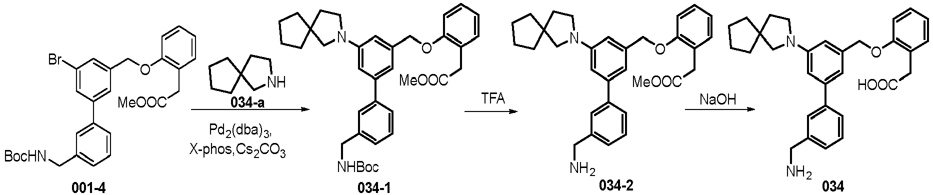

The synthesis of compound 034 refers to the synthesis method of compound 001, which only needs to replace the raw material 001-d with 2-azaspiro[4.4]nonane (034-a).

¹H NMR (400 MHz, DMSO-*d₆*): δ 8.11 (s, 1H), 7.66 (d, *J* = 6.0 Hz, 1H), 7.37 (t, *J* = 6.0 Hz, 1H), 7.30 - 7.22 (m, 2H), 7.15 - 7.05 (m, 2H), 6.90 (d, *J* = 12.0 Hz, 1H), 6.80 (t, *J =* 6.0 Hz, 1H), 6.68 (s, 1H), 6.53 (s, 1H), 5.13 (s, 2H), 3.94 (s, 2H), 3.42 (s, 2H), 3.39-3.37 (m, 2H), 3.21 (s, 2H), 1.88 (t, *J* = 6.0 Hz, 2H), 1.70 - 1.65 (m, 4H), 1.64 - 1.56 (m, 4H); LC/MS(ESI+) m/z: [M+H]⁺ =471.10.

### Example 35. Synthesis of (S)-2-(2-((3'-(1-aminoethyl)-5-(4-oxa-7-azaspiro[2.5]octan-7-yl)-[1,1'-biphenyl]-3-yl)methoxy)phenyl)acetic acid (compound 035-A or 035-B) and (R)-2-(2-((3'-(1-aminoethyl)-5-(4-oxa-7-azaspiro[2.5]octan-7-yl)-[1,1'-biphenyl]-3-yl)methoxy)phenyl)acetic acid (compound 035-B or 035-A)

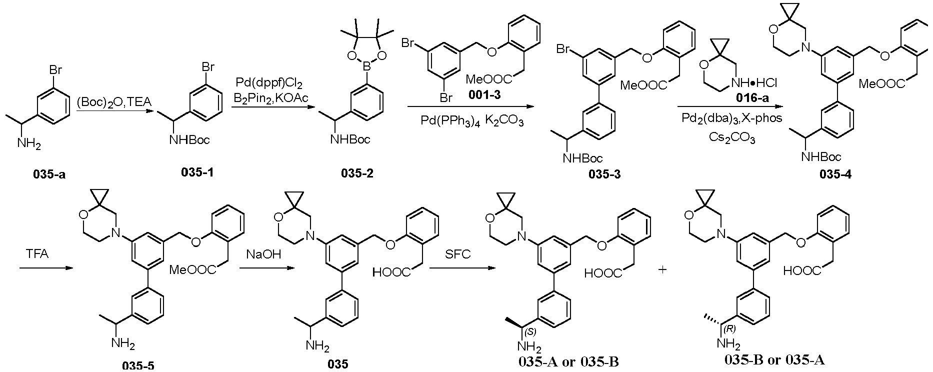

### 35.1 Synthesis of compound 035-1

035-a (500 mg, 2.5 mmol) was weighed into a reaction flask, then di-tert-butyl dicarbonate (599.95 mg, 2.75 mmol), triethylamine (505.77 mg, 5 mmol), and dichloromethane (5 mL) were added thereto, and the reaction was carried out at room temperature for 2 hours. The reaction mixture was concentrated, and then the residue was purified by silica gel column chromatography (petroleum ether/ethyl acetate = 20/1 to 15/1) to obtain compound 035-1, which was used directly in the next step. LC/MS(ESI+) m/z: [M+H-tBu]⁺ =244.0.

### 35.2 Synthesis of compound 035-2

035-1 (7.50 g, 24.98 mmol), bis(pinacolato)diboron (7.61 g, 29.98 mmol), potassium acetate (4.90 g, 49.97 mmol), and [1,1'-bis(diphenylphosphino)ferrocene]dichloropalladium(II) (0.75 g, 2.50 mmol) were dissolved in 1,4-dioxane (80 mL). The system was replaced with nitrogen three times, and the reaction was carried out at 85°C for 10 hours. The reaction mixture was cooled to room temperature, diluted with water (80 mL), and extracted with ethyl acetate (100 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 10/1 to 3/1) to obtain compound 035-2. LC/MS(ESI+) m/z: [M+H-tBu]⁺ =292.05.

### 35.3 Synthesis of compound 035-3

001-3 (3.2 g, 7.74 mmol), 035-2 (1.42 g, 6.45 mmol), potassium carbonate (1.78 g, 12.89 mmol), and tetrakis(triphenylphosphine)palladium (0.7 g, 0.64 mmol) were dissolved in a mixture of 1,4-dioxane and water (40 mL, 1,4-dioxane/water = 4/1). The system was replaced with nitrogen three times, and the reaction was carried out at 80°C for 16 hours. The reaction mixture was cooled to room temperature and extracted with ethyl acetate (50 mL × 3). The combined organic phases were washed with saturated brine, dried over anhydrous sodium sulfate, filtered, and the filtrate was concentrated under reduced pressure. The residue was purified by silica gel column chromatography (petroleum ether/ethyl acetate = 8/1 to 3/1) to obtain compound 035-3. LC/MS(ESI+) m/z: [M+H-Boc]⁺ =453.95.

### 35.4 Synthesis of compound 035-4

035-3 (0.2 g, 0.36 mmol), 016-a (65 mg, 0.43 mmol), tris(dibenzylideneacetone)dipalladium (20 mg, 0.04 mmol), 2-dicyclohexylphosphino-2',4',6'-triisopropylbiphenyl (20 mg, 0.04 mmol), and cesium carbonate (0.5 g, 1.44 mmol) were dissolved in 1,4-dioxane (3 mL). The system was replaced with nitrogen three times, and the reaction was carried out at 100°C for 16 hours. The reaction mixture was cooled to room temperature, diluted with water (15 mL), and then extracted with ethyl acetate (10 mL × 3). The combined organic phases were washed with saturated brine (10 mL), dried over anhydrous sodium sulfate, filtered, and the filtrate was concentrated under reduced pressure. The residue was purified by silica gel column chromatography (petroleum ether/ethyl acetate = 10/1 to 1/1) to obtain compound 035-4. LC/MS(ESI+) m/z: [M+H]⁺ =587.15.

### 35.5 Synthesis of compounds 035-A and 035-B

The synthesis of compound 035 refers to the synthesis method of steps 1.6 and 1.7 in Example 1. 035 was further subjected to chiral resolution by supercritical fluid chromatography (Method: AD-3-IPA + CAN(DEA)-40-3 mL-35 T) to obtain 035-A (retention time Rt = 1.097 min) and 035-B (retention time Rt = 1.773 min).

### 035-A (retention time Rt = 1.097 min):

¹H NMR (400 MHz, DMSO-*d₆*): δ 8.27 (s, 1H), 7.69 (d, *J* = 7.8 Hz, 1H), 7.54 (s, 1H), 7.38 (t, *J* = 7.6 Hz, 1H), 7.26 (d, *J* = 7.4 Hz, 1H), 7.13 (s, 1H), 7.09 (d, *J* = 4.8 Hz, 2H), 6.96 (s, 1H), 6.89 (d, *J* = 8.6 Hz, 1H), 6.80 (t, *J* = 7.4 Hz, 1H), 5.11 (s, 2H), 4.25 (d, *J* = 6.2 Hz, 1H), 3.83 (s, 2H), 3.43 - 3.26 (m, 4H), 3.20 (s, 2H), 1.50 (d, *J* = 6.4 Hz, 3H), 0.80 - 0.61 (m, 4H); LC/MS(ESI+) m/z: [M+H]⁺ =473.05.

### 035-B (retention time Rt = 1.773 min):

¹H NMR (400 MHz, DMSO-*d₆*): δ 8.25 (s, 1H), 7.69 (d, *J* = 7.8 Hz, 1H), 7.53 (s, 1H), 7.39 (t, *J =* 7.6 Hz, 1H), 7.27 (d, *J* = 7.6 Hz, 1H), 7.13 (s, 1H), 7.10 (dt, *J* = 4.0, 3.6 Hz, 2H), 6.97 (s, 1H), 6.90 (d, *J* = 8.4 Hz, 1H), 6.81 (t, *J* = 7.4 Hz, 1H), 5.12 (s, 2H), 4.26 (d, *J* = 6.6 Hz, 1H), 3.87 - 3.80 (m, 2H), 3.40 (dd, *J* = 17.8, 12.0 Hz, 2H), 3.31 - 3.26 (m, 2H), 3.20 (s, 2H), 1.50 (d, *J=* 6.8 Hz, 3H), 0.77 - 0.65 (m, 4H); LC/MS(ESI+) m/z: [M+H]⁺ =473.05.

### Example 36. Synthesis of (S)-2-(2-((3'-(1-aminoethyl)-5-(2-oxa-8-azaspiro[4.5]decan-8-yl)-[1,1'-biphenyl]-3-yl)methoxy)phenyl)acetic acid (compound 036-A or 036-B) and (R)-2-(2-((3'-(1-aminoethyl)-5-(2-oxa-8-azaspiro[4.5]decan-8-yl)-[1,1'-biphenyl]-3-yl)methoxy)phenyl)acetic acid (compound 036-B or 036-A)

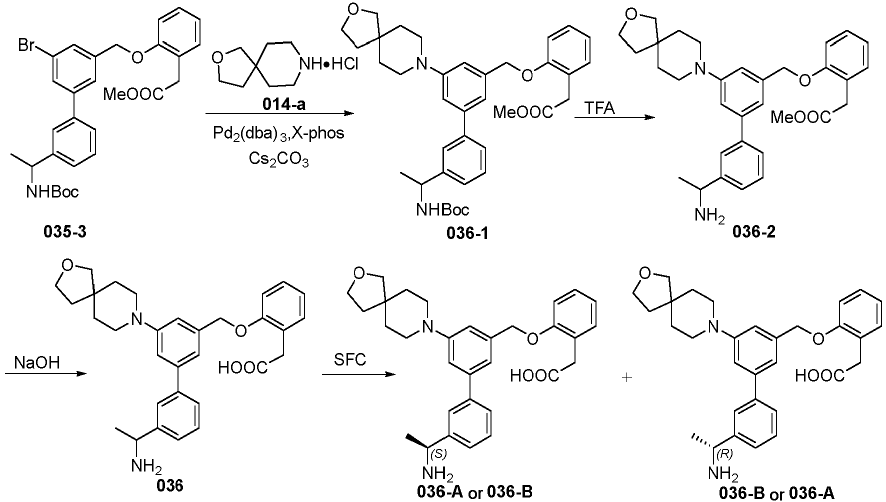

### 36.1 Synthesis of compound 036-1

035-3 (0.1 g, 0.18 mmol), 014-a (36 mg, 0.19 mmol), tris(dibenzylideneacetone)dipalladium (10 mg, 0.02 mmol), 2-dicyclohexylphosphino-2',4',6'-triisopropylbiphenyl (10 mg, 0.02 mmol), and cesium carbonate (0.3 g, 0.91mmol) were dissolved in 1,4-dioxane (3 mL). The system was replaced with nitrogen three times, and the reaction was carried out at 100°C for 16 hours. The reaction mixture was cooled to room temperature, diluted with water (10 mL), and extracted with ethyl acetate (10 mL × 3), and the organic phases were combined. The organic phases were washed with saturated brine (10 mL), dried over anhydrous sodium sulfate, filtered, and the filtrate was concentrated under reduced pressure. The residue was purified by silica gel column chromatography (petroleum ether/ethyl acetate = 10/1 to 1/1) to obtain compound 036-1. LC/MS(ESI+) m/z: [M+H]⁺ =615.10.

### 36.2 Synthesis of compounds 036-A and 036-B

The synthesis of compound 036 refers to the synthesis method of steps 1.6 and 1.7 in Example 1. Compound 036 was further subjected to chiral resolution by supercritical fluid chromatography (Method: OD-MeOH(DEA)-40-3 mL-35 T) to obtain 036-A (retention time Rt = 0.912 min) and 036-B (retention time Rt = 1.354 min).

### 036-A (retention time Rt = 0.912 min):

¹H NMR (400 MHz, DMSO-*d₆*): δ 8.24 (s, 1H), 7.67 (d, *J* = 7.8 Hz, 1H), 7.47 (s, 1H), 7.39 (t, *J* = 7.8 Hz, 1H), 7.27 (d, *J* = 7.6 Hz, 1H), 7.11 (dd, *J* = 12.8, 7.0 Hz, 3H), 7.00 (s, 1H), 6.92 (d, *J =* 8.4 Hz, 1H), 6.81 (t, *J* = 7.4 Hz, 1H), 5.11 (s, 2H), 4.27 (q, *J* = 6.4 Hz, 1H), 3.77 (t, *J =* 7.2 Hz, 2H), 3.40 (dd, *J =* 18.2, 10.8 Hz, 2H), 3.26 (ddt, *J =* 18.2, 12.0, 6.0 Hz, 4H), 1.76 (t, *J =* 7.2 Hz, 2H), 1.71 - 1.59 (m, 4H), 1.50 (d, *J =* 6.8 Hz, 3H); LC/MS(ESI+) m/z: [M+H]⁺ =501.10.

### 036-B (retention time Rt = 1.354 min):

¹H NMR (400 MHz, DMSO-*d₆*): δ 8.26 (s, 1H), 7.67 (d, *J* = 7.8 Hz, 1H), 7.49 (s, 1H), 7.38 (t, *J* = 7.6 Hz, 1H), 7.26 (d, *J* = 7.6 Hz, 1H), 7.11 (dd, *J* = 14.8, 8.4 Hz, 3H), 6.99 (s, 1H), 6.91 (d, *J =* 8.6 Hz, 1H), 6.81 (d, *J* = 7.4 Hz, 1H), 5.11 (s, 2H), 4.25 (d, *J* = 6.8 Hz, 1H), 3.77 (t, *J* = 7.0 Hz, 2H), 3.50 (s, 2H), 3.45 - 3.33 (m, 2H), 3.33 - 3.18 (m, 4H), 1.76 (t, *J =* 7.2 Hz, 2H), 1.65 (d, *J* = 5.0 Hz, 4H), 1.50 (d, *J* = 6.8 Hz, 3H); LC/MS(ESI+) m/z: [M+H]⁺ =501.10.

### Example 37. Synthesis of 2-(2-((3'-(1-aminoethyl)-5-(6-azaspiro[2.5]octan-6-yl)-[1,1'-biphenyl]-3-yl)methoxy)phenyl)acetic acid (compound 037)

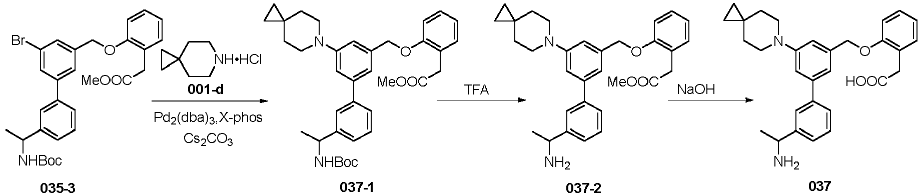

### 37.1 Synthesis of compound 037-1

035-3 (0.5 g, 0.90 mmol), 001-d (160 mg, 1.08 mmol), tris(dibenzylideneacetone)dipalladium (50 mg, 0.09 mmol), 2-dicyclohexylphosphino-2',4',6'-triisopropylbiphenyl (25 mg, 0.09 mmol), and cesium carbonate (0.6 g, 3.61 mmol) were dissolved in 1,4-dioxane (5 mL). The system was replaced with nitrogen three times, and the reaction was carried out at 100°C for 16 hours. The reaction mixture was cooled to room temperature, diluted with water (15 mL), and extracted with ethyl acetate (10 mL × 3). The combined organic phases were washed with saturated brine (10 mL), dried over anhydrous sodium sulfate, filtered, and the filtrate was concentrated under reduced pressure. The residue was purified by silica gel column chromatography (petroleum ether/ethyl acetate = 10/1 to 1/1) to obtain compound 037-1. LC/MS(ESI+) m/z: [M+H]⁺ =585.15.

### 37.2 Synthesis of compound 037

The synthesis of compound 037 refers to the synthesis method of steps 1.6 and 1.7 in Example 1.

¹H NMR (400 MHz, DMSO-*d₆*): δ 8.32 (s, 2H), 7.78 (s, 1H), 7.69 (d, *J* = 7.8 Hz, 1H), 7.52 (t, *J =* 7.8 Hz, 1H), 7.45 (d, *J* = 7.8 Hz, 1H), 7.26 (d, *J =* 10.0 Hz, 2H), 7.22 (dd, *J* = 7.6, 1.3 Hz, 2H), 7.19 (s, 1H), 7.04 (d, *J =* 8.0 Hz, 1H), 6.90 (dd, *J* = 11.6, 4.1 Hz, 1H), 5.14 (s, 2H), 4.51 - 4.45 (m, 1H), 3.60 (s, 2H), 3.40 - 3.33 (m, 4H), 1.55 (d, *J* = 6.8 Hz, 3H), 1.52 (d, *J =* 5.4 Hz, 4H), 0.37 (s, 4H); LC/MS(ESI+) m/z: [M+H]⁺ =471.05.

### Example 38. Synthesis of (S)-2-(2-((3'-(1-aminoethyl)-5-(3-oxa-9-azaspiro[5.5]undecan-9-yl)-[1,1'-biphenyl]-3-yl)methoxy)phenyl)acetic acid (compound 038-A or 038-B) and (R)-2-(2-((3'-(1-aminoethyl)-5-(3-oxa-9-azaspiro[5.5]undecan-9-yl)-[1,1'-biphenyl]-3-yl)methoxy)phenyl)acetic acid (compound 038-B or 038-A)

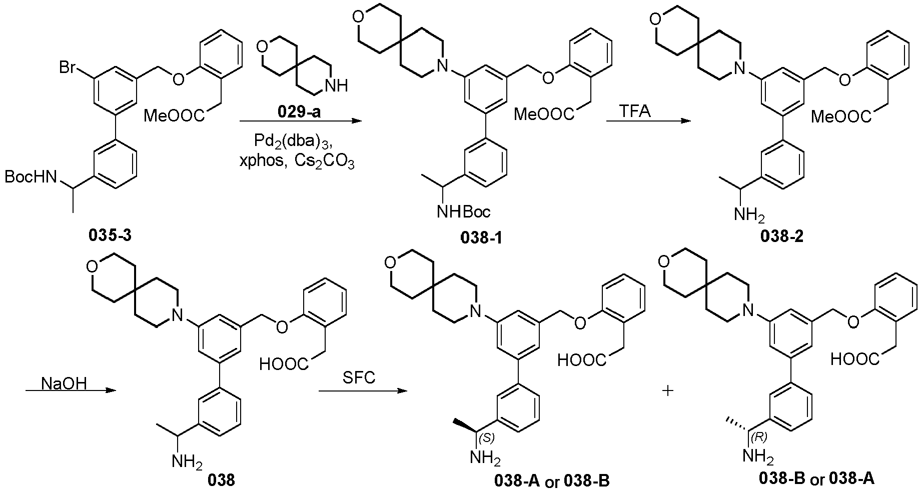

### 38.1 Synthesis of compound 038-1

035-3 (300 mg, 0.54 mmol) was weighed into a reaction flask, then 029-a (93 mg, 0.60 mmol), cesium carbonate (705 mg, 2.16 mmol), 1,4-dioxane (3 mL), tris(dibenzylideneacetone)dipalladium (49 mg, 0.05 mmol), and 2-dicyclohexylphosphino-2',4',6'-triisopropylbiphenyl (25 mg, 0.05 mmol) were added thereto. After the system was replaced with nitrogen, the reaction was carried out at 100°C for 5 hours. The reaction mixture was cooled to room temperature, diluted with water (10 mL), and then extracted with ethyl acetate (20 mL × 3). The organic phases were combined and washed with saturated brine (10 mL), dried over anhydrous sodium sulfate, filtered, and the filtrate was concentrated under reduced pressure. The residue was purified by silica gel column chromatography to obtain compound 038-1. LC/MS(ESI+) m/z: [M+H]⁺ =629.15.

### 38.2 Synthesis of compounds 038-A and 038-B

The synthesis of compound 038 refers to the synthesis method of steps 1.6 and 1.7 in Example 1. Compound 038 was further subjected to chiral resolution by supercritical fluid chromatography (Method: OD-EtOH(DEA)-40-3 mL-35 T) to obtain 038-A (retention time Rt = 1.079 min) and 038-B (retention time Rt = 1.627 min).

### 038-A (retention time Rt = 1.079 min):

¹H NMR (400 MHz, DMSO-*d₆*): δ 8.25 (s, 1H), 7.66 (d, *J* = 7.4 Hz, 1H), 7.47 (s, 1H), 7.37 (t, *J =* 7.4 Hz, 1H), 7.26 (d, *J =* 7.2 Hz, 1H), 7.16 - 7.03 (m, 3H), 6.98 (s, 1H), 6.90 (d, *J* = 8.8 Hz, 1H), 6.79 (d, *J =* 7.2 Hz, 1H), 5.10 (s, 2H), 4.24 (s, 1H), 3.59 (s, 4H), 3.35 (s, 2H), 3.25 (s, 4H), 1.64 (s, 4H), 1.49 (s, 3H), 1.49 - 1.41 (m, 4H); LC/MS(ESI+) m/z: [M+H]⁺ =515.05.

### 038-B (retention time Rt = 1.627 min):

¹H NMR (400 MHz, DMSO-*d₆*): δ 8.32 (s, 2H), 7.79 (s, 1H), 7.67 (d, *J* = 7.6 Hz, 1H), 7.50 (t, *J =* 7.6 Hz, 1H), 7.44 (d, *J* = 7.8 Hz, 1H), 7.22 (d, *J* = 7.6 Hz, 2H), 7.08 (s, 1H), 7.07 - 6.96 (m, 3H), 6.90 (t, *J* = 7.4 Hz, 1H), 5.12 (s, 2H), 4.50 (s, 1H), 3.60 (s, 4H), 3.57 (s, 2H), 3.28 - 3.23 (m, 4H), 1.73 - 1.58 (m, 4H), 1.55 (d, *J* = 6.8 Hz, 3H), 1.51 - 1.44 (m, 4H); LC/MS(ESI+) m/z: [M+H]⁺ =515.05.

### Example 39. Synthesis of (S)-2-(2-((3'-(1-aminoethyl)-5-(2-oxa-9-azaspiro[5.5]undecan-9-yl)-[1,1'-biphenyl]-3-yl)methoxy)phenyl)acetic acid (compound 039-A or 039-B) and (R)-2-(2-((3'-(1-aminoethyl)-5-(2-oxa-9-azaspiro[5.5]undecan-9-yl)-[1,1'-biphenyl]-3-yl)methoxy)phenyl)acetic acid (compound 039-B or 039-A)

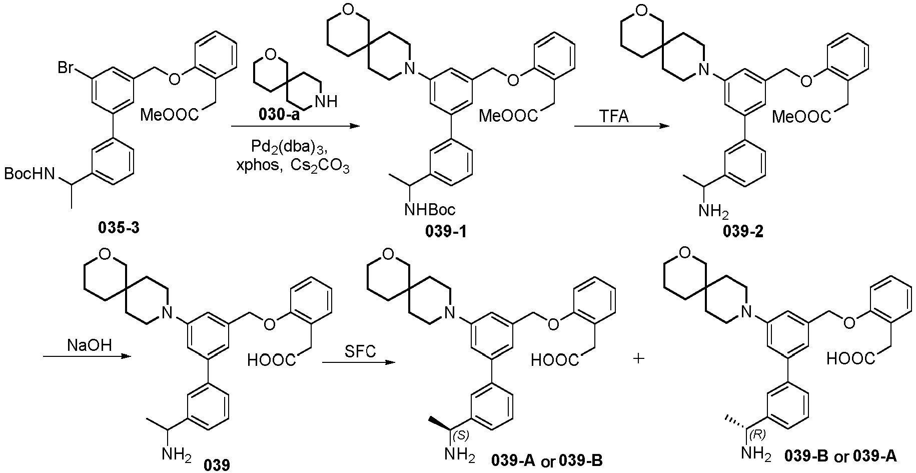

### 39.1 Synthesis of compound 039-1

035-3 (300 mg, 0.54 mmol) was weighed into a reaction flask, then 030-a (93 mg, 0.60 mmol), cesium carbonate (705 mg, 2.16 mmol), 1,4-dioxane (3 mL), tris(dibenzylideneacetone)dipalladium (49 mg, 0.05 mmol), and 2-dicyclohexylphosphino-2',4',6'-triisopropylbiphenyl (25 mg, 0.05 mmol) were sequentially added thereto. After the system was replaced with nitrogen, the reaction mixture was reacted at 100°C for 5 hours, then diluted with water (10 mL), and extracted with ethyl acetate (20 mL × 3). The combined organic phases were washed with saturated brine (10 mL), dried over anhydrous sodium sulfate, filtered, and the filtrate was concentrated under reduced pressure. The residue was purified by silica gel column chromatography to obtain compound 039-1. LC/MS(ESI+) m/z: [M+H]⁺ =629.15.

### 39.2 Synthesis of compounds 039-A and 039-B

The synthesis of compound 039 refers to the synthesis method of steps 1.6 and 1.7 in Example 1. Compound 039 was further subjected to chiral resolution by supercritical fluid chromatography (Method: OD-MeOH(DEA)-40-3 mL-35 T) to obtain 039-A (retention time Rt = 0.935 min) and 039-B (retention time Rt = 1.302 min).

### 039-A (retention time Rt = 0.935 min):

¹H NMR (400 MHz, DMSO-*d₆*): δ 8.28 (s, 2H), 7.79 (s, 1H), 7.68 (d, *J* = 7.6 Hz, 1H), 7.51 (t, *J =* 7.6 Hz, 1H), 7.45 (d, *J* = 8.0 Hz, 1H), 7.21 (d, *J* = 7.6 Hz, 2H), 7.16 (s, 2H), 7.10 (s, 1H), 7.03 (d, *J* = 8.0 Hz, 1H), 6.92 - 6.88 (m, 1H), 5.13 (s, 2H), 4.51 - 4.48 (m, 1H), 3.60 (s, 2H), 3.58 (s, 2H), 3.40 (s, 2H), 3.27 - 3.25 (m, 4H), 1.58 - 1.54 (m, 11H); LC/MS(ESI+) m/z: [M+H]⁺ =515.05.

### 039-B (retention time Rt = 1.302 min):

¹H NMR (400 MHz, DMSO-*d₆*): δ 8.27 (s, 1H), 7.66 (d, *J* = 7.8 Hz, 1H), 7.48 (s, 1H), 7.37 (t, *J =* 7.6 Hz, 1H), 7.26 (d, *J* = 7.6 Hz, 1H), 7.16 - 7.04 (m, 3H), 6.97 (s, 1H), 6.90 (d, *J* = 8.4 Hz, 1H), 6.80 (t, *J =* 7.2 Hz, 1H), 5.10 (s, 2H), 4.24 (d, *J* = 6.6 Hz, 1H), 3.55 (s, 2H), 3.41 (d, *J* = 13.0 Hz, 2H), 3.33 (d, *J* = 15.0 Hz, 2H), 3.24 (t, *J* = 5.6 Hz, 4H), 1.65 - 1.52 (m, 8H), 1.50 (d, *J =* 6.6 Hz, 3H); LC/MS(ESI+) m/z: [M+H]⁺ =515.05.

### Example 40. Synthesis of (S)-2-(2-((3'-(1-aminoethyl)-5-(6-oxa-2-azaspiro[3.4]octan-2-yl)-[1,1'-biphenyl]-3-yl)methoxy)phenyl)acetic acid (compound 040-A or 040-B) and (R)-2-(2-((3'-(1-aminoethyl)-5-(6-oxa-2-azaspiro[3.4]octan-2-yl)-[1,1'-biphenyl]-3-yl)methoxy)phenyl)acetic acid (compound 040-B or 040-A)

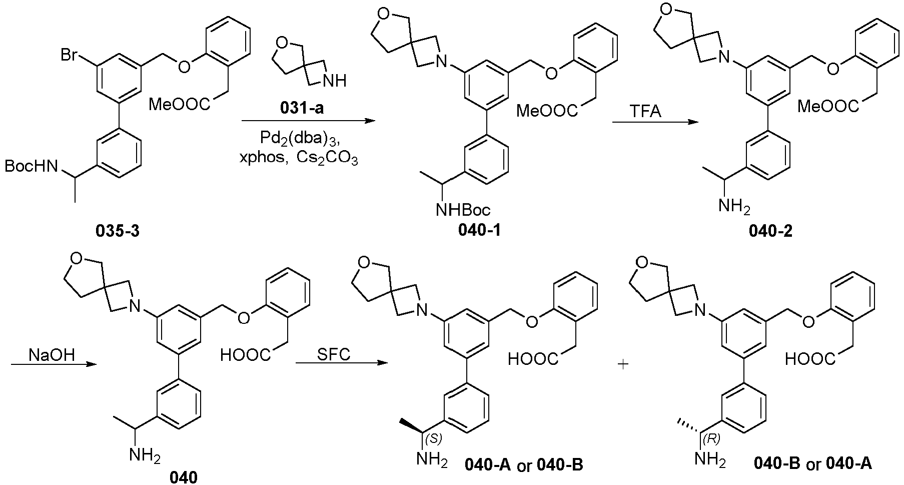

### 40.1 Synthesis of compound 040-1

035-3 (300 mg, 0.54 mmol) was weighed and dissolved in 1,4-dioxane (5 mL), then 031-a (67 mg, 0.60 mmol), cesium carbonate (706 mg, 2.16 mmol), tris(dibenzylideneacetone)dipalladium (30 mg, 10 wt%), and 2-dicyclohexylphosphino-2',4',6'-triisopropylbiphenyl (30 mg, 10 wt%) were added thereto. The reaction mixture was reacted at 100°C for 2 hours under nitrogen atmosphere, filtered, then the filtrate was concentrated, and the residue was purified by silica gel column chromatography to obtain compound 040-1.

### 40.2 Synthesis of compounds 040-A and 040-B

The synthesis of compound 040 refers to the synthesis method of steps 1.6 and 1.7 in Example 1. Compound 040 was further subjected to chiral resolution by supercritical fluid chromatography (Method: Cellulose-2-3-MeOH + CAN(DEA)-50-3 mL-35 T) to obtain 040-A (retention time Rt = 1.272 min) and 040-B (retention time Rt = 1.982 min).

### 040-A (retention time Rt = 1.272 min):

¹H NMR (400 MHz, DMSO-*d₆*): δ 8.24 (s, 1H), 7.62 (d, *J* = 7.8 Hz, 1H), 7.39 (dd, *J* = 18.0, 10.4 Hz, 2H), 7.26 (d, *J* = 7.4 Hz, 1H), 7.09 (t, *J* = 6.4 Hz, 2H), 6.90 (d, *J* = 8.6 Hz, 1H), 6.80 (t, *J =* 7.4 Hz, 1H), 6.64 (s, 1H), 6.48 (s, 1H), 5.09 (s, 2H), 4.24 (d, *J =* 6.6 Hz, 1H), 3.87 (s, 2H), 3.83 (s, 2H), 3.75 (s, 2H), 3.37 (dd, *J* = 28.2, 15.0 Hz, 4H), 2.16 (t, *J* = 6.8 Hz, 2H), 1.49 (d, *J* = 6.4 Hz, 3H); LC/MS(ESI+) m/z: [M+H]⁺ =473.10.

### 040-B (retention time Rt = 1.982 min):

¹H NMR (400 MHz, DMSO-*d₆*): δ 8.21 (s, 1H), 7.60 (d, *J* = 7.4 Hz, 1H), 7.38 (dd, *J* = 16.8, 9.2 Hz, 2H), 7.25 (d, *J* = 6.8 Hz, 1H), 7.08 (d, *J* = 6.8 Hz, 2H), 6.90 (d, *J* = 8.2 Hz, 1H), 6.80 (t, *J =* 7.2 Hz, 1H), 6.64 (s, 1H), 6.48 (s, 1H), 5.09 (s, 2H), 4.22 (d, *J =* 5.4 Hz, 1H), 3.87 (s, 2H), 3.83 (s, 2H), 3.75 (s, 2H), 3.37 (dt, *J* = 24.8, 12.3 Hz, 4H), 2.16 (t, *J* = 6.8 Hz, 2H), 1.48 (d, *J =* 4.8 Hz, 3H); LC/MS(ESI+) m/z: [M+H]⁺ =473.10.

### Example 41. Synthesis of 2-(2-((3'-(1-aminoethyl)-5-(1-oxa-6-azaspiro[3.4]octan-6-yl)-[1,1'-biphenyl]-3-yl)methoxy)phenyl)acetic acid (compound 041)

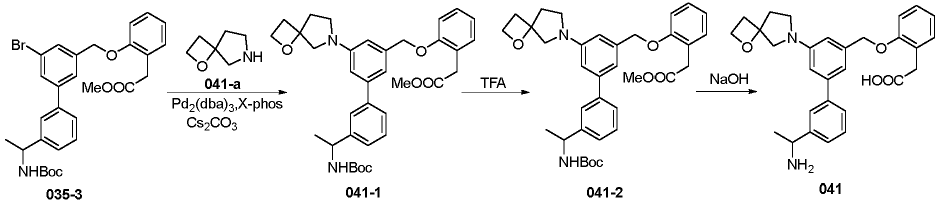

### 41.1 Synthesis of compound 041-1

035-3 (0.5 g, 0.90 mmol), 041-a (175 mg, 0.58 mmol), tris(dibenzylideneacetone)dipalladium (50 mg, 0.09 mmol), 2-dicyclohexylphosphino-2',4',6'-triisopropylbiphenyl (25 mg, 0.09 mmol), and cesium carbonate (1.2 g, 3.6 mmol) were dissolved in 1,4-dioxane (5 mL). The system was replaced with nitrogen three times, and the reaction was carried out at 100°C for 16 hours. The reaction mixture was diluted with water (15 mL) and extracted with ethyl acetate (10 mL × 3). The combined organic phases were washed with saturated brine (10 mL), dried over anhydrous sodium sulfate, filtered, and the filtrate was concentrated under reduced pressure. The residue was purified by silica gel column chromatography (petroleum ether/ethyl acetate = 10/1 to 1/1) to obtain compound 041-1. LC/MS(ESI+) m/z: [M+H]⁺ =587.10.

### 41.2 Synthesis of compound 041

The synthesis of compound 041 refers to the synthesis method of steps 1.6 and 1.7 in Example 1.

¹H NMR (400 MHz, DMSO-*d₆*): δ 7.95 (d, *J* = 42.0 Hz, 1H), 7.54 (d, *J* = 7.4 Hz, 1H), 7.33 (dt, *J* = 16.0, 7.7 Hz, 2H), 7.14 (d, *J* = 6.6 Hz, 2H), 7.06 (t, *J* = 7.2 Hz, 1H), 6.91 (d, *J* = 8.0 Hz, 1H), 6.79 (t, *J =* 7.4 Hz, 1H), 6.72 - 6.54 (m, 2H), 5.07 (s, 2H), 4.43 (t, *J* = 6.6 Hz, 2H), 4.11 (q, *J* = 6.6 Hz, 1H), 3.57 (d, *J* = 40.0 Hz, 2H), 3.39 - 3.28 (m, 4H), 2.80 - 2.61 (m, 2H), 2.41 - 2.12 (m, 3H), 1.37 (d, *J* = 6.6 Hz, 3H); LC/MS(ESI+) m/z: [M+H]⁺ =473.10.

### Example 42. Synthesis of (S)-2-(2-((3'-(1-aminoethyl)-5-(2-oxa-7-azaspiro[3.5]nonan-7-yl)-[1,1'-biphenyl]-3-yl)methoxy)phenyl)acetic acid (compound 042-A or 042-B) and (R)-2-(2-((3'-(1-aminoethyl)-5-(2-oxa-7-azaspiro[3.5]nonan-7-yl)-[1,1'-biphenyl]-3-yl)methoxy)phenyl)acetic acid (compound 042-B or 042-A)

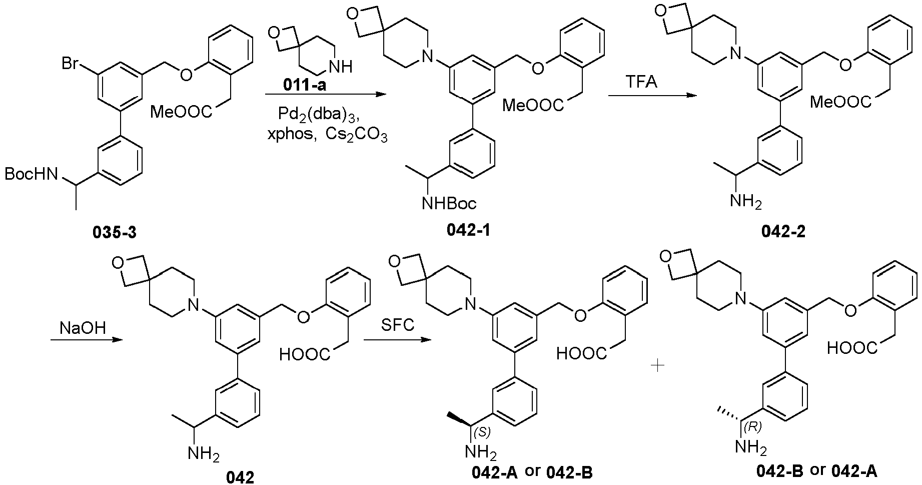

### 42.1 Synthesis of compound 042-1

035-3 (500 mg, 0.9 mmol) was weighed into a reaction flask, then 011-a (130 mg, 0.99 mmol), cesium carbonate (1.2 g, 3.6 mmol), 1,4-dioxane (5 mL), tris(dibenzylideneacetone)dipalladium (100 mg, 0.056 mmol), and 2-dicyclohexylphosphino-2',4',6'-triisopropylbiphenyl (60 mg, 0.056 mmol) were added thereto. The reaction mixture was reacted at 100°C for 24 hours, then filtered, and the filtrate was concentrated under reduced pressure. The residue was purified by silica gel column chromatography (petroleum ether/ethyl acetate = 1/1) to obtain compound 042-1. LC/MS(ESI+) m/z: [M+H]⁺ =601.05.

### 42.2 Synthesis of compounds 042-A and 042-B

The synthesis of compound 042 refers to the synthesis method of steps 1.6 and 1.7 in Example 1. 042 was further subjected to chiral resolution by supercritical fluid chromatography (Method: OD-MeOH(DEA)-40-3 mL-35 T) to obtain 042-A (retention time Rt = 0.913 min) and 042-B (retention time Rt = 1.444 min).

### 042-A (retention time Rt = 0.913 min):

¹H NMR (400 MHz, DMSO-*d₆*): δ 8.26 (s, 1H), 7.67 (dd, *J* = 8.0, 8.0 Hz, 1H), 7.50 (s, 1H), 7.37 (t, *J* = 16.0 Hz, 1H), 7.27 (t, *J* = 16.0 Hz, 1H), 7.11( m, 2H), 7.09(s, 1H), 7.00 (s, 1H), 6.90 (d, *J* = 8.0 Hz, 1H), 6.80 (m, 1H), 5.11 (m, 2H), 4.36 (s, 4H), 4.23 (d, *J* = 12.0 Hz, 1H), 3.43 - 3.31 (m, 2H), 3.19(m, 4H), 1.91 (m, 4H), 1.49 (d, *J* = 4.0 Hz, 3H); LC/MS(ESI+) m/z: [M+H]⁺ =487.10.

### 042-B (retention time Rt = 1.444 min):

¹H NMR (400 MHz, DMSO-*d₆*): δ 8.26 (s, 1H), 7.67 (dd, *J* = 8.0, 8.0 Hz, 1H), 7.50 (s, 1H), 7.37 (t, *J* = 16.0 Hz, 1H), 7.27 (8.10 (m, 1H), 7.61 (dd, *J* = 8.0, 8.0 Hz, 1H), 7.45 (s, 1H), 7.37 (t, *J* = 16.0 Hz, 1H), 7.27 (t, *J* = 16.0 Hz, 1H), 7.09 m, 3H), 7.00 (s, 1H), 6.90 (d, *J* = 8.0 Hz, 1H), 6.80 (m, 1H), 5.09 (m, 2H), 4.35 (s, 4H), 4.16 (d, *J* = 12.0 Hz, 1H), 3.35 - 3.32 (m, 2H), 3.20 - 3.17 (m, 4H), 1.91 (m, 4H), 1.43 (d, *J* = 8.0 Hz, 3H); LC/MS(ESI+) m/z: [M+H]⁺ =487.10.

### Example 43. Synthesis of (S)-2-(2-((3'-(1-aminoethyl)-5-(1-oxa-7-azaspiro[3.5]nonan-7-yl)-[1,1'-biphenyl]-3-yl)methoxy)phenyl)acetic acid (compound 043-A or 043-B) and (R)-2-(2-((3'-(1-aminoethyl)-5-(1-oxa-7-azaspiro[3.5]nonan-7-yl)-[1,1'-biphenyl]-3-yl)methoxy)phenyl)acetic acid (compound 043-B or 043-A)

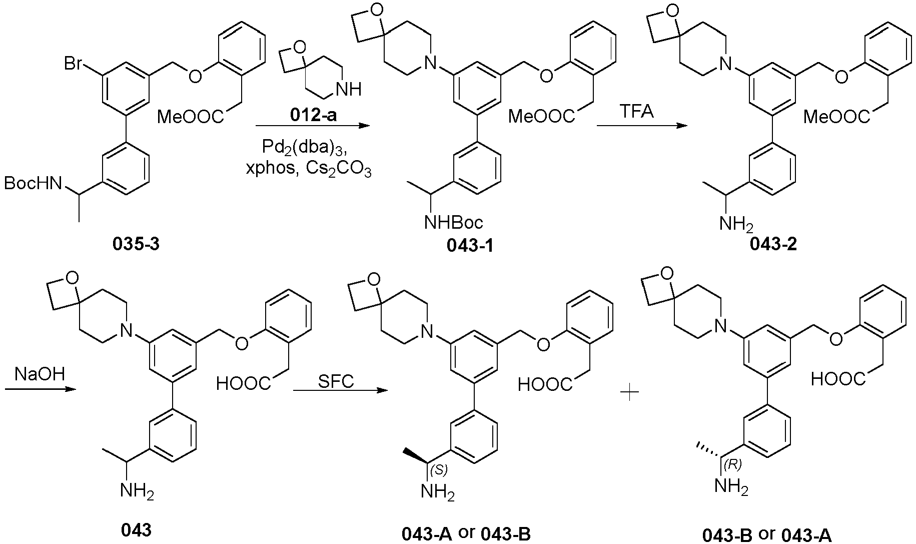

### 43.1 Synthesis of compound 043-1

035-3 (600 mg, 1.08 mmol) was weighed into a reaction flask, then 012-a (160 mg, 1.2 mmol), cesium carbonate (1.4 g, 4.32 mmol), 1,4-dioxane (3 mL), tris(dibenzylideneacetone)dipalladium (100 mg, 0.11 mmol), and 2-dicyclohexylphosphino-2',4',6'-triisopropylbiphenyl (50 mg, 0.11 mmol) were added thereto. The reaction mixture was reacted at 100°C for 5 hours under nitrogen atmosphere. The reaction mixture was cooled to room temperature, diluted with water (10 mL), and extracted with ethyl acetate (20 mL × 3). The combined organic phases were washed with saturated brine (10 mL), dried over anhydrous sodium sulfate, filtered, and the filtrate was concentrated under reduced pressure. The residue was purified by silica gel column chromatography to obtain compound 043-1. LC/MS(ESI+) m/z: [M+H]⁺ =601.10.

### 43.2 Synthesis of compounds 043-A and 043-B

The synthesis of compound 043 refers to the synthesis method of steps 1.6 and 1.7 in Example 1. 043 was further subjected to chiral resolution by supercritical fluid chromatography to obtain 043-A (retention time Rt = 1.54 min) and 043-B (retention time Rt = 2.386 min).

### 043-A (retention time Rt = 1.54 min):

¹H NMR (400 MHz, DMSO-*d₆*): δ 8.25 (s, 1H), 7.67 (d, *J* = 8.0 Hz, 1H), 7.49 (s, 1H), 7.37 (t, *J =* 8.0 Hz, 1H), 7.25 (d, *J* = 8.0 Hz, 1H), 7.13 (s, 1H), 7.08 (d, *J* = 4.0 Hz, 2H), 6.99 (s, 1H), 6.90 (d, *J* = 8.0 Hz, 1H), 6.80 (t, *J* = 8.0 Hz, 1H), 5.10 (s, 2H), 4.42 (s, 1H), 4.23 (d, *J* = 8.0 Hz, 1H), 3.38-3.35 (m, 4H), 3.18 (d, *J* = 4.0 Hz, 2H), 2.39 (t, *J* = 8.0 Hz, 2H), 1.90 (d, *J* = 4.0 Hz, 4H), 1.49 (d, *J* = 8.0 Hz, 3H); LC/MS(ESI+) m/z: [M+H]⁺ =487.10.

### 043-B (retention time Rt = 2.386 min):

¹H NMR (400 MHz, DMSO-*d₆*): δ 8.00-7.93 (d, *J =* 8.0 Hz, 1H), 7.55(s, 1H), 7.43-7.29 (m, 3H), 7.16-6.97 (m, 5H), 5.10 (s, 2H), 4.42 (s, 1H), 4.23 (d, *J* = 8.0 Hz, 1H), 3.38-3.35 (m, 4H), 3.18 (d, *J* = 4.0 Hz, 2H), 2.39 (t, *J* = 8.0 Hz, 2H), 1.90 (d, *J* = 4.0 Hz, 4H), 1.49 (d, *J* = 8.0 Hz, 3H); LC/MS(ESI+) m/z: [M+H]⁺ =487.10.

### Example 44. Synthesis of 2-(2-((3'-(1-amino-2-fluoroethyl)-5-(2-oxa-7-azaspiro[3.5]nonan-7-yl)-[1,1'-biphenyl]-3-yl)methoxy)phenyl)acetic acid (compound 044)

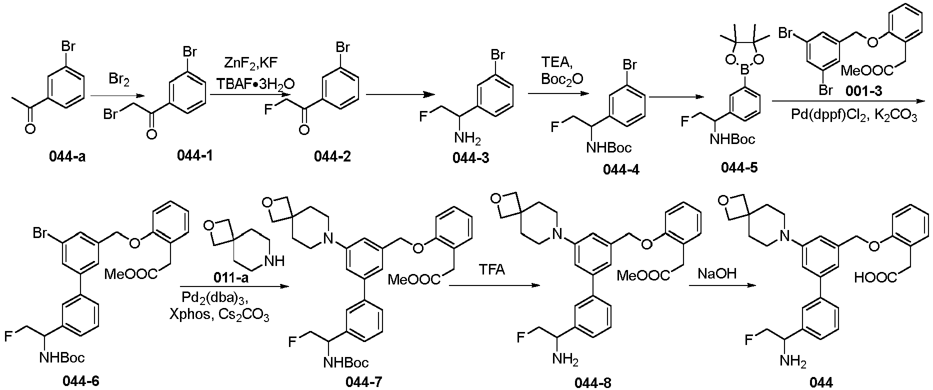

### 44.1 Synthesis of compound 044-1

044-a (4 g, 20.1 mmol) was dissolved in dichloromethane (25 mL), then bromine (1.2 mL, 20.1 mmol) was added thereto at 0°C, and the reaction mixture was reacted at 0°C for 0.5 hours. The reaction was quenched by the addition of saturated sodium sulfite aqueous solution, then the reaction mixture was filtered, and the solid was dried to obtain compound 044-1.

### 44.2 Synthesis of compound 044-2

Zinc fluoride (1.12 g, 10.8 mol), potassium fluoride (315 mg, 5.04 mol), and tetrabutylammonium fluoride trihydrate (2.55 g, 7.2 mol) were sequentially dissolved in acetonitrile (20 mL), and the reaction mixture was refluxed at 80°C for 1 hour. Then 044-1 (3.0 g, 10.8 mol) was dissolved in 5 mL of acetonitrile, and slowly added dropwise to the above system, and the reaction was carried out at 80°C for 12 hours. The reaction mixture was added with saturated ammonium chloride aqueous solution to quench the reaction, and extracted with ethyl acetate (30 mL × 3). The organic phases were combined, washed with saturated brine, dried over anhydrous sodium sulfate, filtered, and the filtrate was concentrated under reduced pressure. The residue was purified by silica gel column chromatography to obtain compound 044-2.

### 44.3 Synthesis of compound 044-3

To ammonia in methanol (1N, 20 mL) of 044-2 (2.4 g, 4.61 mol) was added dropwise titanium tetraisopropoxide (2.6 g, 9.22 mol) under nitrogen atmosphere, and the reaction was carried out at 25°C for 2 hours. Then sodium borohydride (262.3 mg, 6.91 mol) was added thereto and the reaction was carried out at room temperature for 2 hours. The reaction mixture was added with 6 N hydrochloric acid to adjust the pH to 2, and extracted with ethyl acetate. The aqueous phase was added with 6 N sodium hydroxide solution to adjust the pH to 10, and extracted with ethyl acetate. The organic phases were combined, dried over anhydrous sodium sulfate, filtered, and the filtrate was concentrated under reduced pressure to obtain the crude product of compound 044-3, which could be used directly in the next step. LC/MS(ESI+) m/z: [M+H]⁺ =218.00.

### 44.4 Synthesis of compound 044

The synthesis of compound 044 refers to the synthesis method of compound 001, which only needs to replace 001-a with 044-3 and replace 001-d with 011-a in the synthesis step.

¹H NMR (400 MHz, DMSO-*d₆*): δ 8.75 (s, 2H), 7.83 (s, 1H), 7.74 (d, J = 8.0 Hz, 1H), 7.54 (t, J = 8.0 Hz, 1H), 7.48 (d, J = 8.0 Hz, 1H), 7.25-7.21 (m, 2H), 7.16 (d, J = 8.0 Hz, 2H), 7.10 (s, 1H), 7.03 (d, J = 8.0 Hz, 1H), 6.90 (t, J = 8.0 Hz, 1H), 5.12 (s, 2H), 4.87 - 4.72 (m, 3H), 4.36 (s, 4H), 3.60 (s, 2H), 3.26 - 3.15 (m, 4H), 1.94 - 1.90 (m, 4H); LC/MS(ESI+) m/z: [M+H]⁺ =505.05.

### Example 45. Synthesis of 2-(2-((3'-(aminomethyl)-6-fluoro-5-(6-azaspiro[2.5]octan-6-yl)-[1,1'-biphenyl]-3-yl)methoxy)phenyl)acetic acid (compound 045)

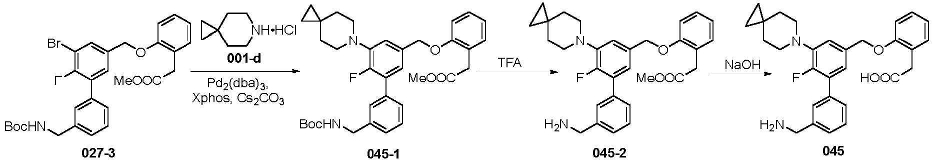

### 45.1 Synthesis of compound 045-1

027-3 (100 mg, 0.17 mmol) was weighed and dissolved in 1,4-dioxane (5 mL), then 001-d (31.7 mg, 0.21 mmol), cesium carbonate (166 mg, 0.51 mmol), tris(dibenzylideneacetone)dipalladium (10 mg, 10 wt%), and 2-dicyclohexylphosphino-2',4',6'-triisopropylbiphenyl (10 mg, 10 wt%) were added thereto. The reaction mixture was reacted at 100°C for 2 hours under nitrogen atmosphere, filtered, then the filtrate was concentrated under reduced pressure, and the residue was purified by silica gel column chromatography to obtain compound 045-1. LC/MS(ESI+) m/z: [M+H]⁺ =589.10.

### 45.2 Synthesis of compound 045

The synthesis of compound 045 refers to the synthesis method of steps 1.6 and 1.7 in Example 1.

¹H NMR (400 MHz, DMSO-*d₆*): δ 7.79 (s, 1H), 7.74 (s, 1H), 7.40-7.40(m, 2H), 7.28-7.12(m, 1H), 7.07-7.05(m, 3H),6.89 (d, *J* = 7.6 Hz, 1H), 6.78 (t, *J* = 7.6Hz, 1H), 5.10 (s, 2H), 3.93 (s, 2H), 3.34 (s, 2H), 3.15 (s, 4H), 1.50 (s, 4H), 0.31 (s, 4H); LC/MS(ESI+) m/z: [M+H]⁺ =475.10.

### Example 46. Synthesis of (S)-2-(2-((3'-(1-amino-2-hydroxyethyl)-5-(8-azaspiro[4.5]decan-8-yl)-[1,1'-biphenyl]-3-yl)methoxy)phenyl)acetic acid (compound 046)

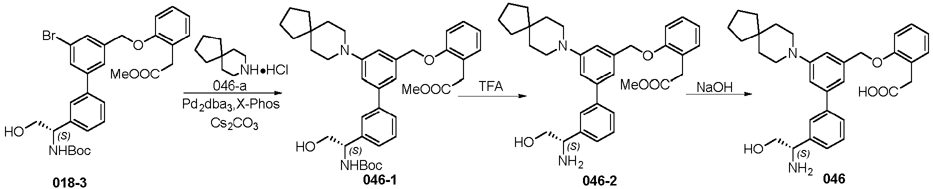

The synthesis of compound 046 refers to the synthesis method of compound 018, which only needs to replace the raw material 001-d with the hydrochloride of 8-azaspiro[4.5]decane (046-a).

¹H NMR (400 MHz, DMSO-*d₆*): δ 8.05 (d, *J* = 63.0 Hz, 1H), 7.60 (dd, *J* = 46.0, 7.6 Hz, 1H), 7.38 (dd, *J =* 16.8, 9.6 Hz, 2H), 7.23 (dd, *J* = 20.4, 7.4 Hz, 1H), 7.19 - 6.89 (m, 5H), 6.88 - 6.70 (m, 1H), 5.11 (s, 2H), 4.12 (s, 1H), 3.67 (s, 2H), 3.37 (d, *J* = 6.8 Hz, 2H), 3.22 (dd, *J* = 16.8, 11.3 Hz, 4H), 1.71 - 1.48 (m, 8H), 1.46 (d, *J* = 6.6 Hz, 4H); LC/MS(ESI+) m/z: [M+H]⁺ =515.15.

### Example 47. Synthesis of (R)-2-(2-((3'-(1-amino-2-hydroxyethyl)-5-(3-azaspiro[5.5]undecan-3-yl)-[1,1'-biphenyl]-3-yl)methoxy)phenyl)acetic acid (compound 047-A) trifluoroacetate and synthesis of (S)-2-(2-((3'-(1-amino-2-hydroxyethyl)-5-(3-azaspiro[5.5]undecan-3-yl)-[1,1'-biphenyl]-3-yl)methoxy)phenyl)acetic acid (compound 047-B) trifluoroacetate

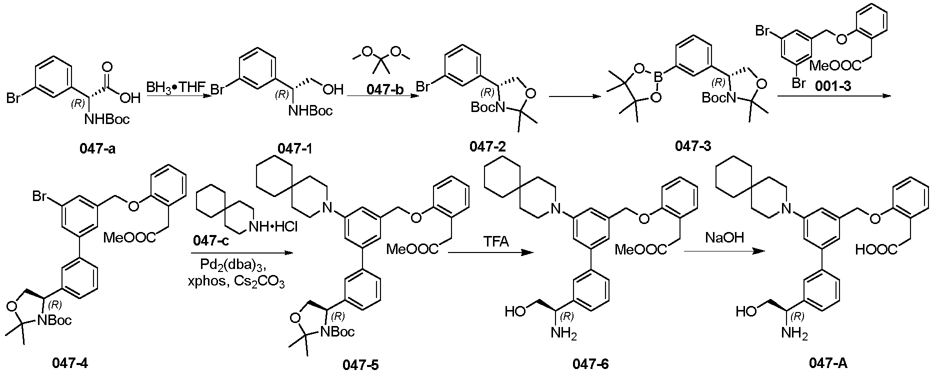

### 47.1 Synthesis of compound 047-1

047-a (1 g, 3.03 mmol) was weighed into a reaction flask, then tetrahydrofuran (5 mL) was added thereto, cooled to 0°C with an ice-water bath. Borane tetrahydrofuran complex (1 M, 9.1 mL, 9.1 mmol) was slowly added thereto, and the reaction mixture was maintained at 0°C and reacted for 3 hours after the addition. The reaction was quenched by slow dropwise addition of methanol (10 mL). After quenching was complete, the reaction mixture was concentrated under reduced pressure, and the residue was purified by silica gel column chromatography to obtain compound 047-1. LC/MS(ESI+) m/z: [M+H-tBu]⁺ =260.00.

### 47.2 Synthesis of compound 047-2

047-1 (700 mg, 2.21 mmol) was weighed into a reaction flask, then 2,2-dimethoxypropane (461 mg, 4.43 mmol), p-toluenesulfonic acid monohydrate (21 mg, 0.11 mmol), and toluene (5 mL) were added thereto, and the reaction mixture was heated to 50°C and reacted for 15 hours. The reaction system was added with saturated sodium bicarbonate aqueous solution to adjust the pH to 8, and extracted with ethyl acetate (10 mL × 3). The combined organic phases were washed with saturated brine (10 mL), dried over anhydrous sodium sulfate, and filtered. The filtrate was concentrated under reduced pressure, and the residue was purified by silica gel column chromatography to obtain compound 047-2.

### 47.3 Synthesis of compound 047-3

047-2 (400 mg, 1.12 mmol) was weighed into a reaction flask, then bis(pinacolato)diboron (314 mg, 1.24 mmol), potassium acetate (221 mg, 2.25 mmol), [1,1'-bis(diphenylphosphino)ferrocene]dichloropalladium(II) (80 mg, 0.11 mmol), and 1,4-dioxane (5 mL) were added thereto. After the system was replaced with nitrogen three times, the reaction mixture was reacted at 100°C for 2 hours and then directly concentrated under reduced pressure. The residue was purified by silica gel column chromatography to obtain compound 047-3.

### 47.4 Synthesis of compound 047-4

001-3 (400 mg, 0.96 mmol) was weighed into a reaction flask, then 047-3 (389 mg, 0.96 mmol), potassium carbonate (267 mg, 1.93 mmol), 1,4-dioxane (5 mL), and tetrakis(triphenylphosphine)palladium (111 mg, 0.09 mmol) were added thereto. After the system was replaced with nitrogen three times, the reaction mixture was heated to 100°C and reacted for 2 hours. The reaction mixture was diluted with water (10 mL) and extracted with ethyl acetate (10 mL × 3). The combined organic phases were washed with saturated brine (10 mL), dried over anhydrous sodium sulfate, filtered, and the filtrate was concentrated under reduced pressure. The residue was purified by silica gel column chromatography to obtain compound 047-4.

### 47.5 Synthesis of compound 047-5

047-4 (150 mg, 0.25 mmol) was weighed into a reaction flask, then 047-c (51 mg, 0.27 mmol), cesium carbonate (320 mg, 0.98 mmol), 1,4-dioxane (2 mL), tris(dibenzylideneacetone)dipalladium (22 mg, 0.02 mmol), and 2-dicyclohexylphosphino-2',4',6'-triisopropylbiphenyl (12 mg, 0.02 mmol) were added thereto. The system was replaced with nitrogen three times, and the reaction mixture was reacted at 100°C for 2 hours. The reaction mixture was cooled to room temperature, diluted with water (10 mL), and extracted with ethyl acetate (10 mL × 3). The organic phases were combined and washed with saturated brine (10 mL), dried over anhydrous sodium sulfate, filtered, and the filtrate was concentrated under reduced pressure. The residue was purified by silica gel column chromatography to obtain compound 047-5.

### 47.6 Synthesis of trifluoroacetate of compound 047-A

The synthesis of compound 047-A refers to the synthesis method of steps 1.6 and 1.7 in Example 1. The preparation process was carried out under the condition of trifluoroacetic acid, and finally the trifluoroacetate of compound 047-A was obtained.

¹H NMR (400 MHz, DMSO-*d₆*): δ 8.39 (s, 2H), 7.78 (s, 1H), 7.68 (d, *J* = 7.6 Hz, 1H), 7.49 (t, *J* = 7.6 Hz, 1H), 7.42 (d, *J* = 7.6 Hz, 1H), 7.21 (d, *J* = 7.6 Hz, 2H), 7.14 (s, 2H), 7.09 (s, 1H), 7.02 (d, *J =* 8.0 Hz, 1H), 6.89 (t, *J* = 7.6 Hz, 1H), 5.12 (s, 2H), 4.37 (s, 1H), 3.82 - 3.67 (m, 2H), 3.59 (s, 2H), 3.24 (s, 4H), 1.55 (s, 4H), 1.40 (d, *J =* 14.4 Hz, 10H); LC/MS(ESI+) m/z: [M+H]⁺ =529.10.

### 47.7 Synthesis of trifluoroacetate of compound 047-B

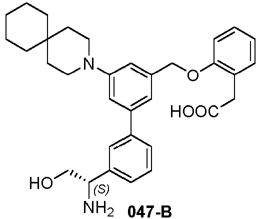

The synthesis of compound 047-B refers to the synthesis method of compound 047-A, which only needs to replace the raw material 047-a (R configuration) with (S)-2-(3-bromophenyl)-2-((tert-butoxycarbonyl)amino)acetic acid (S configuration).

¹H NMR (400 MHz, DMSO-*d₆*): δ 8.40 (s, 2H), 7.79 (s, 1H), 7.69 (d, *J* = 7.6 Hz, 1H), 7.50 (t, *J =* 7.6 Hz, 1H), 7.43 (d, *J* = 7.6 Hz, 1H), 7.22 (d, *J* = 7.6 Hz, 2H), 7.16 (s, 2H), 7.11 (s, 1H), 7.03 (d, *J* = 8.2 Hz, 1H), 6.90 (t, *J =* 7.4 Hz, 1H), 5.13 (s, 2H), 4.38 (s, 1H), 3.81 - 3.68 (m, 2H), 3.60 (s, 2H), 3.26 (s, 4H), 1.56 (s, 4H), 1.41 (d, *J =* 13.8 Hz, 10H); LC/MS(ESI+) m/z: [M+H]⁺ =529.10.

### Example 48. Synthesis of (R)-2-(2-((3'-(1-amino-2-hydroxyethyl)-5-(2-azaspiro[3.4]octan-2-yl)-[1,1'-biphenyl]-3-yl)methoxy)phenyl)acetic acid (compound 048-A) and synthesis of (S)-2-(2-((3'-(1-amino-2-hydroxyethyl)-5-(2-azaspiro[3.4]octan-2-yl)-[1,1'-biphenyl]-3-yl)methoxy)phenyl)acetic acid (compound 048-B)

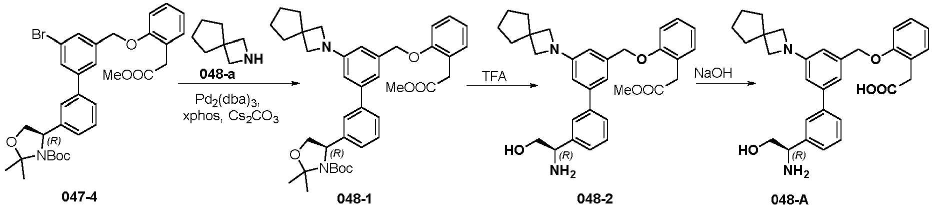

### 48.1 Synthesis of compound 048-A

The synthesis of compound 048-A refers to the synthesis method of compound 047-A in Example 47, which only needs to replace the raw material 047-c with 2-azaspiro[3.4]octane (048-a).

¹H NMR (400 MHz, DMSO-*d₆*): δ 9.51 (s, 1H), 7.96 (s, 1H), 7.49 (d, *J* = 7.6 Hz, 1H), 7.35 (s, 1H), 7.26 (t, *J =* 7.6 Hz, 1H), 7.16 - 7.02 (m, 3H), 6.83 (d, *J =* 8.4 Hz, 1H), 6.78 (t, *J* = 7.2 Hz, 1H), 6.59 (s, 1H), 6.45 (s, 1H), 5.22 - 5.01 (m, 2H), 4.69 - 4.56 (m, 1H), 3.80 - 3.69 (m, 4H), 3.48 - 3.41 (m, 1H), 3.38 (s, 2H), 2.04 - 1.93 (m, 1H), 1.87 - 1.76 (m, 4H), 1.68 - 1.56 (m, 4H); LC/MS(ESI+) m/z: [M+H]⁺ =487.10.

### 48.2 Synthesis of compound 048-B

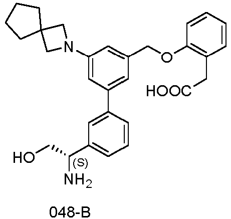

The synthesis of compound 048-B refers to the synthesis method of 048-A, which only needs to change the raw material from 047-4 (R configuration) to S configuration.

¹H NMR (400 MHz, DMSO-*d₆*): 9.49 (d, *J* = 8.8 Hz, 1H), 7.93 (d, *J* = 30.0 Hz, 1H), 7.51 (t, *J=* 8.4 Hz, 1H), 7.34 (d, *J =* 11.2 Hz, 1H), 7.31 - 7.03 (m, 4H), 6.95 - 6.73 (m, 2H), 6.58 (d, *J* = 6.8 Hz, 1H), 6.47 (d, *J* = 15.6 Hz, 1H), 5.21 - 5.04 (m, 2H), 4.63 (d, *J* = 8.0 Hz, 1H), 3.80 - 3.69 (m, 5H), 3.58 (s, 1H), 3.53 - 3.42 (m, 2H), 3.34 (d, *J =* 8.8 Hz, 1H), 1.82 (s, 4H), 1.66 - 1.56 (m, 4H); LC/MS(ESI+) m/z: [M+H]⁺ =487.10.

### Example 49. Synthesis of (R)-2-(2-((3'-(1-amino-2-hydroxyethyl)-5-(2-azaspiro[4.4]nonan-2-yl)-[1,1'-biphenyl]-3-yl)methoxy)phenyl)acetic acid (compound 049-A) and synthesis of (S)-2-(2-((3'-(1-amino-2-hydroxyethyl)-5-(2-azaspiro[4.4]nonan-2-yl)-[1,1'-biphenyl]-3-yl)methoxy)phenyl)acetic acid (compound 049-B)

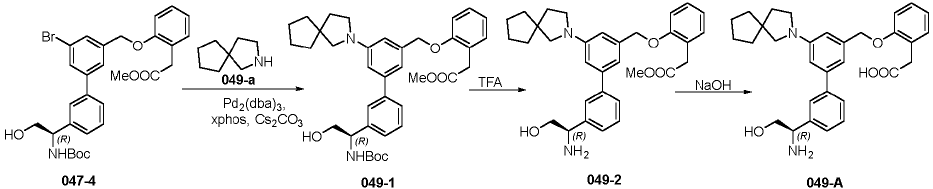

### 49.1 Synthesis of compound 049-A

The synthesis of compound 049-A refers to the synthesis method of compound 047-A in Example 47, which only needs to replace the raw material 047-c with 2-azaspiro[4.4]nonane (049-a).

¹H NMR (400 MHz, DMSO-*d₆*): δ 9.49 (d, *J* = 9.2 Hz, 1H), 8.02 (d, *J* = 42.4 Hz, 1H), 7.59 (dd, *J* = 35.2, 7.6 Hz, 1H), 7.36 (t, *J* = 7.6 Hz, 1H), 7.28 - 7.24 (m, 1H), 7.20 (s, 1H), 7.13 - 7.04 (m, 2H), 6.97 - 6.73 (m, 2H), 6.67 (s, 1H), 6.62 - 6.51 (m, 1H), 5.23 - 5.03 (m, 2H), 4.70 - 4.51 (m, 1H), 4.12 - 4.07 (m, 1H), 3.68 - 3.61 (m, 2H), 3.49 - 3.41 (m, 2H), 3.37 (d, *J* = 6.8 Hz, 2H), 3.25 - 3.18 (m, 2H), 1.88 (t, *J* = 6.8 Hz, 2H), 1.70 - 1.65 (m, 4H), 1.63 - 1.53 (m, 4H); LC/MS(ESI+) m/z: [M+H]⁺ =501.10.

### 49.2 Synthesis of compound 049-B

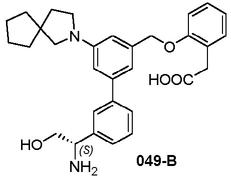

The synthesis of compound 049-B refers to the synthesis method of 049-A, which only needs to change the raw material from 047-4 (R configuration) to S configuration.

¹H NMR (400 MHz, DMSO-*d₆*): 9.50 (d, *J* = 9.2 Hz, 1H), 8.04 (d, *J* = 64.8 Hz, 1H), 7.60 (dd, *J =* 44.4, 7.8 Hz, 1H), 7.36 (t, *J* = 7.6 Hz, 1H), 7.28 - 7.21 (m, 2H), 7.14 - 7.06 (m, 2H), 6.94 - 6.76 (m, 2H), 6.68 (s, 1H), 6.59 - 6.53 (m, 1H), 5.11 (s, 2H), 4.15 - 4.09 (m, 1H), 3.69 - 3.66 (m, 2H), 3.47 (d, *J* = 15.2 Hz, 2H), 3.39 - 3.36 (m, 2H), 3.22 (s, 2H), 1.88 (t, *J* = 6.8 Hz, 2H), 1.70 - 1.66 (m, 4H), 1.63 - 1.56 (m, 4H); LC/MS(ESI+) m/z: [M+H]⁺ =501.10.

### Example 50. Synthesis of 2-(2-((3'-(1-amino-2-fluoroethyl)-2'-fluoro-5-(1-oxa-6-azaspiro[3.4]octan-6-yl)-[1,1'-biphenyl]-3-yl)methoxy)phenyl)acetic acid (compound 050)

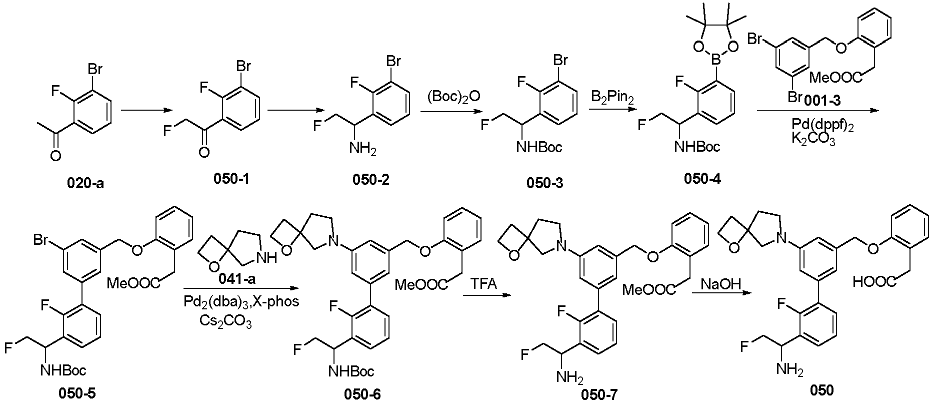

### 50.1 Synthesis of compound 050-1

020-a (2.0 g, 9.2 mmol) was weighed and dissolved in tetrahydrofuran (20 mL), and lithium bis(trimethylsilyl)amide (1 M, 10.1 mL, 10.1 mmol) was added thereto under nitrogen atmosphere at -78°C. After the reaction was carried out for 1 hour, chlorotrimethylsilane (1.08 g, 10.1 mmol) was added thereto at -78°C, and the reaction mixture was slowly warmed to room temperature and reacted for 1 hour. The reaction mixture was concentrated, added with acetonitrile (20 mL) and 1-chloromethyl-4-fluoro-1,4-diazoniabicyclo[2.2.2]octane bis(tetrafluoroborate) (4.7 g, 11.4 mmol), and reacted at room temperature for 2 hours. The reaction mixture was slowly quenched with saturated ammonium chloride aqueous solution (15 mL) and extracted with ethyl acetate (50 mL × 3). The combined organic phases were washed with saturated brine (50 mL), dried over anhydrous sodium sulfate, filtered, and the filtrate was concentrated under reduced pressure. The residue was purified by silica gel column chromatography to obtain compound 050-1. LC/MS(ESI+) m/z: [M+H]⁺ = 234.1.

### 50.2 Synthesis of compound 050-2

050-1 (600 mg, 5.19 mmol) was weighed and dissolved in ammonia in ethanol (2 M, 3.8 mL), then tetraisopropyl titanate (1.08 g, 3.8 mmol) was added thereto, and the reaction was carried out at 25°C for 2 hours. Then sodium borohydride (116 mg, 3.06 mmol) was added thereto and the reaction was carried out for 2 hours. The reaction mixture was slowly quenched with saturated ammonium chloride aqueous solution (5 mL) and extracted with ethyl acetate (10 mL × 3). The combined organic phases were washed with saturated brine (10 mL), dried over anhydrous sodium sulfate, and filtered. The filtrate was concentrated under reduced pressure, and the residue was purified by silica gel column chromatography to obtain compound 050-2. LC/MS(ESI+) m/z: [M+H]⁺ = 236.1.

### 50.3 Synthesis of compound 050

The synthesis of compound 050 refers to the synthesis method of compound 001, which only needs to replace 001-a with 050-2 and replace 001-d with 041-a in the synthesis step.

¹H NMR (400 MHz, DMSO-*d₆*): δ 7.61 (t, *J* = 7.2 Hz, 1H), 7.41-7.38 (m, 1H), 7.24 (t, *J* = 8.4 Hz, 1H), 7.17-8.15 (m, 1H) , 7.05-7.01 (m, 1H) , 6.90 (d, *J* = 8.4 Hz, 1H), 6.81-6.71 (m, 3H), 6.51 (d, *J =* 12.8 Hz, 1H), 5.01 (s, 2H), 4.52-4.38 (m, 5H), 3.57 (d, *J =* 11.2 Hz, 1H), 3.46 (d, *J =* 10.8 Hz, 1H),3.3 (s, 2H), 3.2 (s, 2H), 2.76-2.48 (m, 2H), 2.34-2.41 (m, 1H), 2.16-1.97 (m, 1H); LC/MS(ESI+) m/z: [M+H]⁺ =509.10.

### Example 51. Synthesis of 2-(2-((3'-(1-amino-3-hydroxypropyl)-5-(6-azaspiro[2.5]octan-6-yl)-[1,1'-biphenyl]-3-yl)methoxy)phenyl)acetic acid (compound 051)

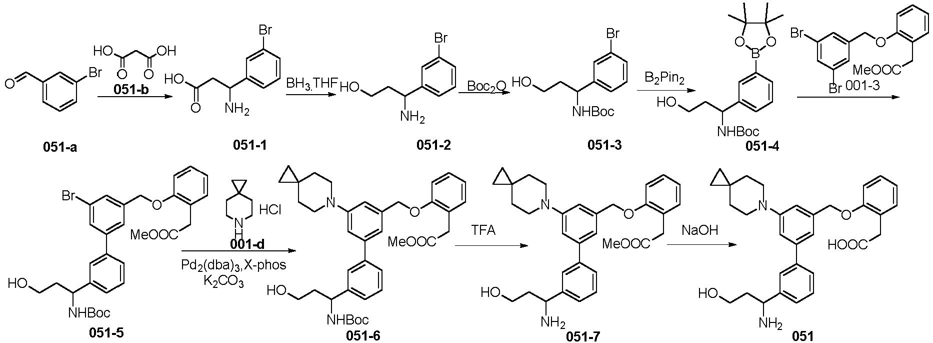

### 51.1 Synthesis of compound 051-1

051-a (5 g, 2.7 mmol), 051-b (2.8 g, 2.7 mmol), and ammonium acetate (4.2 g, 5.4 mmol) were sequentially dissolved in ethanol (100 mL), and the reaction mixture was reacted at 80°C for 24 hours. The reaction mixture was filtered, rinsed with ethanol, and the filter cake was dried under reduced pressure to obtain compound 051-1. LC/MS(ESI+) m/z: [M+H]⁺ = 246.00.

### 51.2 Synthesis of compound 051-2

051-1 (3.7 g, 15.2 mmol) was dissolved in tetrahydrofuran (70 mL), then the system was replaced with nitrogen, and borane tetrahydrofuran (10 mL) was added dropwise thereto at 0°C. The reaction mixture was reacted at 45°C for 5 hours. The reaction was quenched by slow dropwise addition of methanol, and the reaction mixture was concentrated under reduced pressure, added with water (50 mL), and then extracted with ethyl acetate (30 mL × 2). The organic phases were combined, then dried over anhydrous sodium sulfate, filtered, and the filtrate was concentrated under reduced pressure. The residue was purified by silica gel column chromatography (dichloromethane/methanol = 5/1) to obtain compound 051-2. LC/MS(ESI+) m/z: [M+H]⁺ = 230.00.

### 51.3 Synthesis of compound 051

The synthesis of compound 050 refers to the synthesis method of compound 001, which only needs to replace 001-a in the synthesis step with 051-2.

¹H NMR (400 MHz, DMSO-*d₆*): δ 8.26 (s, 1H), 7.68 (d, *J* = 8.0 Hz, 1H), 7.46 (s, 1H), 7.37 (t, *J* = 16.0 Hz, 1H), 7.22 (d, *J* = 8.0 Hz, 1H), 7.15 (s, 1H), 7.09 (m, 2H), 7.01 (s, 1H), 6.91 (d, *J =* 12.0 Hz, 1H), 6.79 (d, *J* =8.0Hz, 1H), 5.11 (s, 2H), 4.19 (s, 1H), 3.40 (t, *J* = 8.0 Hz, 2H), 3.34 (s, 2H), 3.32 - 3.27 (m, 4H), 2.16 - 1.94 (m, 2H), 1.53 - 1.46 (m, 4H), 0.35 (s, 4H); LC/MS(ESI+) m/z: [M+H]⁺ =501.10.

### Example 52. Synthesis of 2-(2-((3'-(1-amino-2-hydroxyethyl)-2'-fluoro-5-(6-azaspiro[2.5]octan-6-yl)-[1,1'-biphenyl]-3-yl)methoxy)phenyl)acetic acid (compound 052)

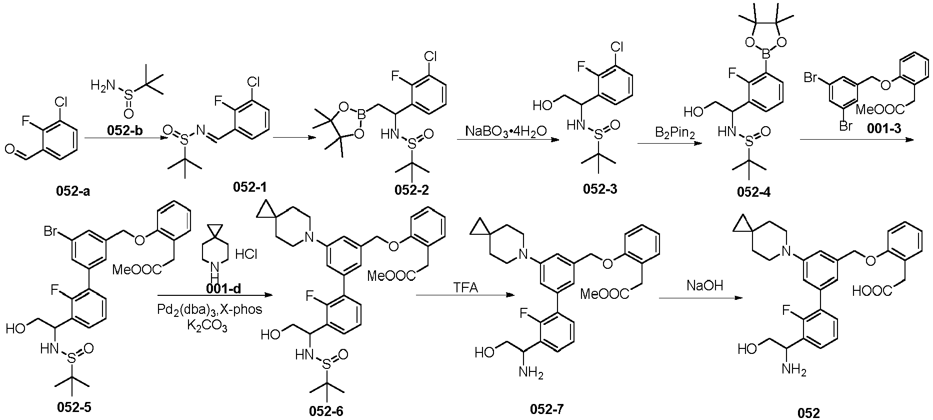

### 52.1 Synthesis of compound 052-1

052-a (3.0 g, 18.9 mmol) was dissolved in dichloromethane (50 mL), then cesium carbonate (12.4 g, 37.8 mmol) and 023-b (2.3 g, 18.9 mmol) were added thereto. The reaction mixture was reacted at 25°C for 2 hours, diluted with water (50 mL), and extracted with dichloromethane (30 mL × 2). The organic phase was dried over anhydrous sodium sulfate, filtered, and the filtrate was concentrated under reduced pressure. The residue was purified by silica gel column chromatography (petroleum ether/ethyl acetate = 3/1 to 1/1) to obtain compound 052-1. LC/MS(ESI+) m/z: [M+H]⁺ =262.00.

### 52.2 Synthesis of compound 052-2

052-1 (4.5 g, 25 mmol) was weighed and dissolved in a solution of toluene (30 mL), then bis[(pinacolato)boryl]methane (10 g, 37.3 mmol), 1,2-bis(diphenylphosphino)benzene (1.11 g, 2.5 mmol), cuprous bromide (360 mg, 2.5 mmol), and lithium tert-butoxide (6 g, 74.5 mmol) were added thereto. The reaction mixture was reacted at 50°C overnight under nitrogen atmosphere, then diluted with water (30 mL), and extracted with ethyl acetate (30 mL × 3). The organic phase was dried over anhydrous sodium sulfate, filtered, and the filtrate was concentrated under reduced pressure. The residue was purified by silica gel column chromatography (petroleum ether/ethyl acetate = 3/1 to 1/1) to obtain compound 052-2. LC/MS(ESI+) m/z: [M+H]⁺ = 404.05.

### 52.3 Synthesis of compound 052-3

052-2 (4.2 g, 20 mmol) was dissolved in toluene (20 mL), then sodium perborate tetrahydrate (11.2 g, 40 mmol) was added thereto. The reaction mixture was reacted at room temperature for 2 hours, then diluted with water (50 mL), and extracted with ethyl acetate (10 mL × 3). The combined organic phases were dried over anhydrous sodium sulfate, filtered, and the filtrate was concentrated under reduced pressure. The residue was purified by silica gel column chromatography (petroleum ether/ethyl acetate = 3/1 to 1/1) to obtain compound 052-3.

### 52.4 Synthesis of compound 052-4

052-3 (2.0 g, 6.8 mmol), bis(pinacolato)diboron (2.6 g, 10.2 mmol), potassium acetate (1.36 g, 13.6 mmol), and [1,1'-bis(diphenylphosphino)ferrocene]dichloropalladium(II) (460 mg, 0.68 mmol) were dissolved in 1,4-dioxane (20 mL) under nitrogen atmosphere. The reaction mixture was reacted at 100°C for 2 hours, then diluted with water (20 mL), and extracted with ethyl acetate (10 mL × 3). The combined organic phases were dried over anhydrous sodium sulfate, filtered, and the filtrate was concentrated under reduced pressure. The residue was purified by silica gel column chromatography to obtain compound 052-4. LC/MS(ESI+) m/z: [M+H]⁺ = 386.05.

### 52.5 Synthesis of compound 052

The synthesis of compound 052 refers to the synthesis method of compound 001, which only needs to replace 001-2 with 052-4.

¹H NMR (400 MHz, DMSO-*d₆*): δ 8.48 (s, 2H), 7.61 - 7.51 (m, 2H), 7.37 (t, *J* = 8.0 Hz, 1H), 7.25 - 7.20 (m, 2H), 7.17 (s, 1H), 7.05- 7.00(m, 3H), 6.90 (t, *J* =8.0 Hz, 1H), 5.13 (s, 2H), 4.58 (d, *J* = 8.0 Hz, 1H), 3.83-3.79 (m, 1H), 3.76-3.71 (m, 1H), 3.58 (s, 2H), 3.40 - 3.22 (m, 4H), 1.56 - 1.39 (m, 4H), 0.35 (s, 4H); LC/MS(ESI+) m/z: [M+H]⁺ =505.10.

### Example 53. Synthesis of 2-(2-((3'-(1-aminoethyl)-2'-fluoro-5-(2-oxa-7-azaspiro[3.5]nonan-7-yl)-[1,1'-biphenyl]-3-yl)methoxy)phenyl)acetic acid (compound 053)

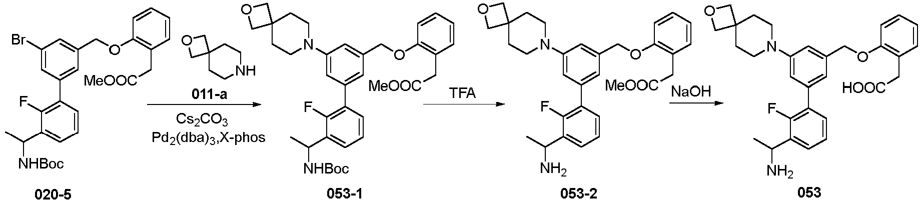

### 53.1 Synthesis of compound 053-1

020-5 (300 mg, 0.52 mmol) was weighed into a reaction flask, then 011-a (99 mg, 0.58 mmol), cesium carbonate (683 mg, 2.1 mmol), 1,4-dioxane (3 mL), tris(dibenzylideneacetone)dipalladium (48 mg, 0.05 mmol), and 2-dicyclohexylphosphino-2',4',6'-triisopropylbiphenyl (25 mg, 0.05 mmol) were added thereto. After the system was replaced with nitrogen, the reaction mixture was reacted at 100°C for 2 hours, then diluted with water (10 mL), and extracted with ethyl acetate (20 mL × 3). The organic phases were combined and washed with saturated brine (10 mL), dried over anhydrous sodium sulfate, filtered, and the filtrate was concentrated under reduced pressure. The residue was purified by silica gel column chromatography to obtain compound 053-1. LC/MS(ESI+) m/z: [M+H]⁺ =619.05.

### 53.2 Synthesis of compound 053

The synthesis of compound 053 refers to the synthesis method of steps 1.6 and 1.7 in Example 1.

¹H NMR (400 MHz, DMSO-*d₆*): δ 8.07 (d, *J* = 8.0 Hz, 1H), 7.54 (t, *J* = 6.8 Hz, 1H), 7.38 - 7.30 (m, 1H), 7.29 - 7.17 (m, 2H), 7.16 - 7.07 (m, 2H), 7.05 - 6.91 (m, 3H), 6.85 (t, *J* = 7.2 Hz, 1H), 5.11 (d, *J* = 28.4 Hz, 2H), 4.35 (s, 1H), 4.34 - 4.27 (m, 4H), 3.44 (s, 2H), 3.20 - 3.10 (m, 4H), 1.93 - 1.84 (m, 4H), 1.32 (dd, *J* = 16.8, 6.8 Hz, 3H); LC/MS(ESI+) m/z: [M+H]⁺ =505.10.

### Example 54. Synthesis of 2-(2-((3'-(1-aminoethyl)-2'-fluoro-5-(1-oxa-7-azaspiro[3.5]nonan-7-yl)-[1,1'-biphenyl]-3-yl)methoxy)phenyl)acetic acid (compound 054)

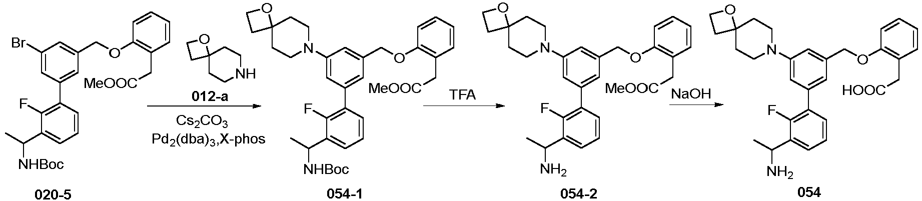

The synthesis of compound 054 refers to the synthesis method of compound 053, which only needs to replace the raw material 011-a with 012-a.

¹H NMR (400 MHz, DMSO-*d₆*): δ 8.12 (s, 1H), 7.55 (t, *J* = 6.8 Hz, 1H), 7.33 (td, *J* = 7.6, 1.6 Hz, 1H), 7.29 - 7.16 (m, 2H), 7.16 - 7.05 (m, 2H), 7.05 - 6.91 (m, 3H), 6.82 (t, *J =* 7.6 Hz, 1H), 5.10 (d, J = 40.4 Hz, 2H), 4.46 - 4.37 (m, 2H), 4.31 (q, *J* = 6.8 Hz, 1H), 3.36 - 3.29 (m, 4H), 3.18 - 3.11 (m, 2H), 2.37 (t, *J* = 7.6 Hz, 2H), 1.94 - 1.79 (m, 4H), 1.31 (dd, *J* = 21.4, 6.7 Hz, 3H); LC/MS(ESI+) m/z: [M+H]⁺ =505.10.

### Example 55. Synthesis of 2-(2-((3'-(1-aminoethyl)-2'-fluoro-5-(3-oxa-9-azaspiro[5.5]undecan-9-yl)-[1,1'-biphenyl]-3-yl)methoxy)phenyl)acetic acid (compound 055)

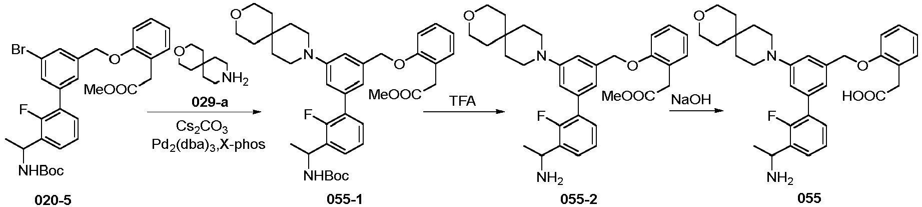

The synthesis of compound 055 refers to the synthesis method of compound 053, which only needs to replace the raw material 011-a with 029-a.

¹H NMR (400 MHz, DMSO-*d₆*): δ 7.54 (s, 1H), 7.33 (m, 1H), 7.27 - 7.16 (m, 2H), 7.08 (m, 2H), 6.95 (m, 3H), 6.82 (t, *J* = 16.0 Hz, 1H), 5.05 (s, 2H), 4.31 (d, *J* = 8.0Hz, 1H), 3.59 - 3.54 (m, 4H), 3.31 (s, 2H), 3.24 - 3.19 (m, 4H), 1.70 - 1.55 (m, 4H), 1.49 - 1.41 (m, 4H), 1.29 (d, *J* = 4.0 Hz, 3H); LC/MS(ESI+) m/z: [M+H]⁺ =533.10.

### Example 56. Synthesis of 2-(2-((3'-(1-aminoethyl)-2'-fluoro-5-(2-oxa-9-azaspiro[5.5]undecan-9-yl)-[1,1'-biphenyl]-3-yl)methoxy)phenyl)acetic acid (compound 056)

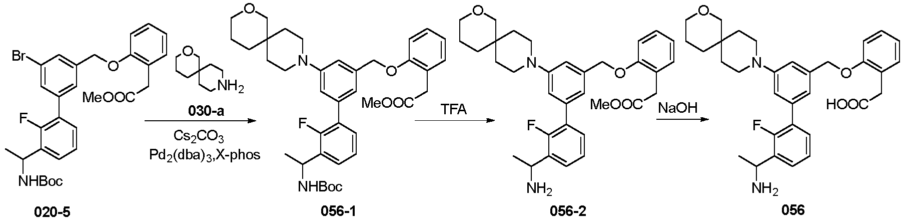

The synthesis of compound 056 refers to the synthesis method of compound 053, which only needs to replace the raw material 011-a with 030-a.

¹H NMR (400 MHz, DMSO-*d₆*): δ 8.38 (d, *J =* 4.0 Hz, 2H), 7.56 (t, *J =* 16.0 Hz, 2H), 7.38 (t, *J* = 12.0 Hz, 1H), 7.23 (t, *J* =16.0 Hz, 2H), 7.15 (s, 1H), 7.03 (d, *J* = 8.0 Hz, 3H), 6.90 (t, *J* =16.0 Hz, 1H), 5.12 (s, 2H), 4.73 - 4.69 (m, 1H), 3.56 (d, *J* = 12.0 Hz, 4H), 3.39 (s, 2H), 3.24 (t, *J* = 12.0 Hz, 4H), 1.58 (d, *J* = 8.0 Hz, 4H), 1.55 (s, 3H), 1.53 (s, 4H); LC/MS(ESI+) m/z: [M+H]⁺ =533.10.

### <Biological activity test assay>

### 1. Complement factor D inhibitory activity (C3b method) in vitro screening experiment

### 1.1 Experimental materials and instruments

V-bottom plate (AXYGEN), DMSO (Sigma), MicroVue Bb Plus ELISA kit (Quidel), complement factor C3b (abbreviated as factor C3b, Complement tech), complement factor B (abbreviated as factor B, Complement tech), complement factor D (abbreviated as factor D, Complement tech), EDTA (MACKLIN), GVB° (Complement tech), EGTA (Aladdin), MgCl₂ (Aladdin), NaOH (Sinopharm), microplate reader (Molecular Devices, SpectraMax i3x), microplate thermostatic shaker (Thermo, MB100-2A), pipette (Gilson)

### 1.2 Preparation before experiment

### 1.2.1 Preparation of Mg-EGTA

0.1 M Mg-EGTA: 3.8 g of EGTA, 0.9521 g of MgCl₂, and 0.7 g of NaOH were weighed, and the pH was adjusted to 7.5 with NaOH. Distilled water was added to make up to 100 mL, and the mixture was filtered with a 0.2 µm sterile needle filter , stored in portions at 4°C.

### 1.2.2 Preparation of working solution

Buffer: 0.1 M Mg-EGTA solution was diluted 10-fold to 10 mM with GVB° buffer;
factor B working solution: 1 mg/mL factor B solution (10.75 µM) was diluted 6.7-fold to 1.6 µM with buffer;
factor C3b working solution: 1 mg/mL factor C3b solution (5.68 µM) was diluted 5-fold to 1.12 µM with buffer;
factor D working solution: 0.1 mg/mL factor D solution (4.17 µM) was diluted 1303-fold to 3.2 nM with buffer.
Note: a. The above dilution multiples are for reference only and are adjusted according to the true concentration labeled on the reagent;
b. factor B: 93 KDa, factor D: 24 KDa, factor C3b: 176 KDa.

### 1.2.3 Preparation of stop solution

Stop solution: An appropriate amount of EDTA powder was weighed, dissolved in a certain amount of GVB° buffer, and the pH was adjusted to 7.5 with NaOH. The mixture was stirred until clear and prepared into a 10 mM solution.

### 1.2.4 Preparation of compounds

Compound stock solution: 40 mM compound solution was diluted with DMSO into 1 mM stock solution, and the 1 mM stock solution was diluted 3-fold with DMSO at 8 points to prepare compound stock solutions of various concentrations.

Compound working solution: diluted 250-fold with buffer to obtain compound solutions of various concentrations.

### 1.3 Experimental steps

In the V-bottom plate, the experimental group was added with 10 µL of factor D solution and 10 µL of compound solution; the positive control group was added with 10 µL of factor D solution and buffer containing 0.4% DMSO; the blank control group was added with 20 µL of buffer containing 0.2% DMSO; incubated at 37°C for 15 min; the factor B working solution and the factor C3b working solution were mixed homogeneously at a ratio of 1:1; 20 µL of the mixture was added to each well and incubated at 37°C for 30 min; the reaction was stopped by adding 40 µL of stop solution; the production of product Bb was detected by MicroVue Bb Plus ELISA kit.

### 1.4 ELISA assay

1.4.1 The required wells of the ELISA plate were taken out and allowed to return to room temperature, and the remaining ones were repackaged and stored at 4°C;
1.4.2 the wells were washed twice with 300 µL of 1× wash buffer, and incubated for 1 min at 25°C for the first wash;
1.4.3 samples were diluted 8-fold with complement specimen diluent, and 100 µL of sample was added to each well and incubated at 25°C for 30 min;
1.4.4 the liquid in the wells was discarded, and the wells were washed 5 times with 300 µL of 1× wash buffer, and incubated at room temperature for 1 min for the first wash;
1.4.5 50 µL of Bb Plus Conjugate was added to each well and incubated at 25°C for 30 min;
1.4.6 the liquid in the wells was discarded, and the wells were washed 5 times with 300 µL of 1× wash buffer, and incubated at room temperature for 1 min for the first wash;
1.4.7 100 µL of compound working solution (Substrate Solution) was added to each well and incubated at 25°C for 15 min;
1.4.8 100 µL of stop solution was added to each well, and the absorbance was detected at 450 nm within 30 min.

### 1.5 Data analysis

1.5.1 Inhibition rate of each drug concentration: $Inhibition rate \left(%\right) = \frac{OD value of PC group - OD value of administration group}{OD value of PC group - OD value of NC group} \times 100 %$

The PC group represents 0% inhibition rate, and the NC group represents 100% inhibition rate.

### 1.5.2 Calculation of signal-to-background ratio (S/B):

Average value of OD in PC group / average value of OD in NC group represents the size of the signal window.

### 1.5.3 Z' factor:

Calculation formula: $Z ′ factor = 1 - \frac{3 \times standard deviation of OD in PC group + 3 \times standard deviation of OD in NC group}{\left|Average value of OD in PC group-average value of OD in NC group\right|}$

The Z' factor should be greater than 0.4.

### 1.5.4 Compound IC₅₀:

IC₅₀: half-maximal inhibitory concentration, representing the concentration at which the compound inhibits 50% of the enzymatic activity of complement factor D;
data were collected to calculate the log value of inhibition rate versus compound concentration, and IC₅₀ values were calculated using GraphPad Prism software. The inhibitory activity of the compounds of the present disclosure on complement factor D is shown in Table 1.

**Table 1: Inhibitory activity of compounds of the present disclosure on complement factor D**

| Compound number | C3b IC₅₀ (nM) |
|---|---|
| 001 | 10 |
| 003 | 3 |
| 004 | 19 |
| 006 | 4 |
| 007 | 12 |
| 008 | 18 |
| 011 | 18 |
| 012 | 6 |
| 013 | 11 |
| 014 | 14 |
| 016 | 26 |
| 017 | 6 |
| 021 | 11 |
| 023 | 15 |
| 024 | 4 |
| 025 | 7 |
| 029 | 6 |
| 030 | 4 |
| 031 | 3 |
| 032 | 10 |
| 033 | 2 |
| 034 | 1 |

Experimental conclusion: The compounds of the present disclosure have a good inhibitory effect on complement factor D.

### 2. Rabbit erythrocyte hemolysis assay to evaluate the inhibitory activity of compounds on the bypass pathway

### 2.1 Experimental materials and instruments

Normal Human Serum, NHS (collected from healthy people), Normal Human Plasma, NHP (Shanghai Yuduo), 96-well ELISA plate (Jet Biofil), Japanese big-eared white rabbit (Wuhan WQJX), Alsever's solution (Procell), centrifuge (Thermo, PICO17), thermostatic shaker (Shanghai Fuma), destaining shaker (Beijing Liuyi), cell counter (Invitrogen, Counter Countess II) (GVB^{o}, EGTA, MgCl₂, NaOH, microplate reader, microplate thermostatic shaker, and pipette are equivalent to those in the experiment 1).

### 2.2 Preparation of working solution

48% NHP: 100% NHP was diluted to 48% with buffer;
26.4% NPS: 100% NHS was diluted to 26.4% with buffer;
rabbit erythrocyte suspension: blood was taken from rabbit ear marginal vein, anticoagulated with Alsever's solution at a ratio of 1:1, stored in portions at 4°C, and could be stored for 4 weeks. Before use, the suspension was centrifuged at 500 g for 5 min to discard the Alsever's solution, washed three times with an equal volume of buffer and centrifuged at 500 g for 5 min, and finally the density was adjusted to 6 × 10⁸ cells/mL with buffer.

### 2.3 Experimental steps

In the 96-well ELISA plate, the experimental group was added with 50 µL of 48% NHP or 26.4% NHS and 50 µL of compound solution; the positive control group was added with 50 µL of 48% NHP or 26.4% NHS and 50 µL of buffer containing 0.2% DMSO; the blank control group was added with 50 µL of 48% inactivated NHP or 26.4% inactivated NHS and 0.2% DMSO buffer; the H₂O group was added with 100 µL of double-distilled water; incubated at 37°C for 15 min; 20 µL of rabbit erythrocyte suspension was added to each well and incubated on a shaker at 37°C for 30 min; centrifuged at 2000 g (3380 rpm) for 5 min, and 100 µL of supernatant was transferred to a new 96-well ELISA plate, and the absorbance was detected at 415 nm.

### 2.4 Data analysis

2.4.1 Hemolysis percentage for each drug concentration: $Hemolysis percentage \left(%\right) = \frac{OD value of administration group - OD value of NC group}{OD value of PC group - OD value of NC group} \times 100 %$

The PC group represents 100% hemolysis, and the NC group represents 0% hemolysis.

### 2.4.2 Calculation of signal-to-background ratio (S/B):

Average value of OD in PC group / average value of OD in NC group represents the size of the signal window.

### 2.4.3 Z' factor:

Calculation formula: $Z ′ factor = 1 - \frac{3 \times standard deviation of OD in PC group + 3 \times standard deviation of OD in NC group}{\left|Average value of OD in PC group-average value of OD in NC group\right|}$

### 2.4.4 Compound IC₅₀:

IC₅₀: half-maximal inhibitory concentration. Data were collected to calculate the log value of hemolysis percentage versus compound concentration, and IC₅₀ values were calculated using GraphPad Prism software. The inhibitory activity of the compounds of the present disclosure on hemolysis of rabbit erythrocytes is shown in Table 2.

**Table 2: Inhibitory activity of the compounds of the present disclosure on hemolysis of rabbit erythrocytes**

| Compound number | Rabbit erythrocyte hemolysis IC₅₀ (nM) |
|---|---|
| 001 | 41 |
| 002 | 98 |
| 003 | 43 |
| 004 | 36 |
| 005 | 73 |
| 006 | 54 |
| 007 | 59 |
| 008 | 196 |
| 009 | 218 |
| 010 | 159 |
| 011 | 13 |
| 012 | 4 |
| 013 | 28 |
| 014 | 10 |
| 015 | 7 |
| 016 | 19 |
| 017 | 65 |
| 021 | 65 |
| 023 | 76 |
| 024 | 27 |
| 025 | 12 |
| 026 | 16 |
| 028 | 58 |
| 029 | 10 |
| 030 | 22 |
| 031 | 6 |
| 032 | 8 |
| 033 | 3 |
| 034 | 11 |
| 035 | 183 |
| 036 | 44 |
| 038 | 206 |
| 039 | 127 |
| 040 | 138 |
| 041 | 151 |
| 042 | 50 |
| 043 | 93 |
| 044 | 133 |
| 055 | 117 |
| 056 | 70 |

Experimental conclusion: The compounds of the present disclosure have a good inhibitory effect on the hemolysis of rabbit erythrocytes.

### 3. In vivo pharmacokinetic study of the compounds of the present disclosure

After adaptive domestication, SPF grade SD rats were administered 1 or 3 mg/kg the compounds of the present disclosure by single gavage and tail vein injection, respectively. Plasma was collected at specific time points after administration, and the concentration of compounds in plasma was detected by LC-MS/MS (AB SCIEX Qtrap4500). The PK parameters of each compound were calculated using software to reflect the pharmacokinetic properties of the compounds of the present disclosure *in vivo* in animals. The PK parameters of the compounds of the present disclosure are shown in Table 3.

**Table 3: PK assay of compounds of the present disclosure on SD rats**

| Compound | Administration dosage mg/kg | Administration mode | Cₘₐₓ (ng/mL) | T_{1/2} (hr) | AUCₗₐₛₜ (ng/mL * hr) | Oral bioavailability F (%) |
|---|---|---|---|---|---|---|
| 001 | 1 | PO (3% DMSO + 4% Tween) | 577 ± 12.3 | 4.13 1.38 | 3238 ± 115 | 40.3% |
| | | IV | 1803 ± 227 | 3.59 ± 0.18 | 8031 ± 574 | / |
| 007 | 1 | PO (3% DMSO + 4% Tween) | 936 ± 184 | 1.46 ± 0.02 | 2312 ± 385 | 70.2% |
| | | IV | 3273 ± 680 | 0.64 ± 0.06 | 3291 ± 215 | / |
| 010 | 1 | PO (3% DMSO + 4% Tween) | 349 ± 34.4 | 3.18 ± 0.24 | 1751 ± 137 | 30.9% |
| | | IV | 1840 ± 171 | 3.31 ± 0.44 | 5659 ± 394 | / |
| 023 | 3 | PO (3% DMSO + 4% Tween) | 1017 ± 132 | 4.10 ± 0.37 | 7478 ± 974 | 35.3% |
| | 1 | IV | 3407 ± 344 | 4.58 ± 0.08 | 7058 ± 1335 | / |

Experimental conclusion: The compounds of the present disclosure can achieve higher *in vivo* exposure and higher oral bioavailability at lower doses, and has excellent overall pharmacokinetic properties.

## Claims

1. A compound of formula (I), a tautomer thereof, a stereoisomer thereof, or a pharmaceutically acceptable salt of any one of the foregoing, or a solvate of any one of the foregoing: 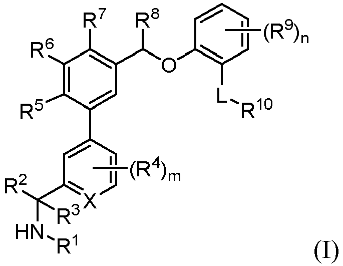 wherein
R¹ is H, D, C₁₋₆ alkyl, or C₁₋₆ alkyl substituted by 1, 2, or 3 R¹⁻¹; each R¹⁻¹ is independently halogen, -CN, -OH, C₁₋₆ alkoxy, or -NH₂;
R² and R³ are each independently H, D, halogen, C₁₋₆ alkyl, or C₁₋₆ alkyl substituted by 1, 2, or 3 R²⁻¹; each R²⁻¹ is independently halogen, -CN, -OH, C₁₋₆ alkoxy, or -NH₂;
R⁴ is H or halogen; m is 0, 1, 2, or 3;
R⁵ and R⁷ are each independently H, halogen, -CN, C₁₋₆ alkyl, or C₁₋₆ alkoxy;
R⁶ is a 8- to 11-membered heterocycloalkyl with 1, 2, or 3 heteroatoms selected from 1, 2, or 3 types of N, O, and S, or a 8- to 11-membered heterocycloalkyl with 1, 2, or 3 heteroatoms selected from 1, 2, or 3 types of N, O, and S substituted by 1, 2, or 3 R⁶⁻²;
R⁶⁻² are each independently hydroxyl, oxo (=O), halogen, C₁₋₆ alkyl, C₁₋₆ alkoxy, C₁₋₆ haloalkyl, C₁₋₆ haloalkoxy, C₃₋₆ cycloalkyl, or -(CH₂)ₚ-C₃₋₆ cycloalkyl; p is 1, 2, 3, or 4;
in R⁶⁻², the cycloalkyl is independently a monocyclic ring, a bridged ring, or a spiro ring;
in R⁶, the heterocycloalkyl is a bridged ring, or a spiro ring;
R⁸ is H, halogen, or C₁₋₆ alkyl;
R⁹ is H, halogen, C₁₋₆ alkyl, or C₁₋₆ alkyl substituted by 1, 2, or 3 R⁹⁻¹;
each R⁹⁻¹ is independently halogen, -CN, -OH, or -NH₂; n is 0, 1, 2, 3, or 4;
L is -(CR^{a}R^{b})_{q}-; q is 0, 1, 2, or 3;
R^{a} and R^{b} are each independently H, D, or halogen, or R^{a} and R^{b} are connected together to form a C₃₋₆ cycloalkylene, and the formed cycloalkylene is a monocyclic ring, a bridged ring, or a spiro ring;
R¹⁰ is -COOH;
X is CR^{d} or N; R^{d} is H, halogen, or C₁₋₆ alkyl.

2. The compound of formula (I), the tautomer thereof, the stereoisomer thereof, or the pharmaceutically acceptable salt of any one of the foregoing, or the solvate of any one of the foregoing according to claim 1, wherein the compound of formula (I) satisfies one or more than one of the following conditions:
(1) R¹ is H;
(2) R² and R³ are each independently H, C₁₋₃ alkyl, or C₁₋₃ alkyl substituted by 1, 2, or 3 R²⁻¹; each R²⁻¹ is independently halogen or -OH, and the halogen is preferably F;
(3) m is 0 or 1;
(4) R⁵ and R⁷ are each independently H or halogen, and the halogen is preferably F;
(5) in R⁶, the 8- to 11-membered heterocycloalkyl is 6-azaspiro[2.5]octyl, 5-azaspiro[2.5]octyl, 6-azaspiro[3.4]octyl, 2-azaspiro[3.4]octyl, 2-oxa-6-azaspiro[3.4]octyl, 6-oxa-2-azaspiro[3.4]octyl, 4-oxa-7-azaspiro[2.5]octyl, 2-azaspiro[4.4]nonyl, 2-azaspiro[3.5]nonyl, 2-oxa-7-azaspiro[3.5]nonyl, 1-oxa-7-azaspiro[3.5]nonyl, 7-azaspiro[3.5]nonyl, 2,7-diazaspiro[3.5]nonyl, 2-oxa-8-azaspiro[4.5]decyl, 3-oxa-9-azaspiro[5.5]undecyl, 2-oxa-9-azaspiro[5.5]undecyl, 3,9-diazaspiro[5.5]undecyl, 3-azabicyclo[3.2.1]octyl, 3-azaspiro[5,5]undecyl, 8-azaspiro[4.5]decyl, or 1-oxa-6-azaspiro[3,4]octyl;
(6) each R⁶⁻² is independently hydroxyl or C₁₋₃ alkyl, preferably, each R⁶⁻² is independently hydroxyl, methyl, ethyl, n-propyl, or isopropyl;
(7) R⁸ is H;
(8) R⁹ is H or halogen, and the halogen is preferably F;
(9) n is 0 or 1;
(10) q is 1;
(11) R^{a} and R^{b} are each independently H;
(12) R^{d} is H.

3. The compound of formula (I), the tautomer thereof, the stereoisomer thereof, or the pharmaceutically acceptable salt of any one of the foregoing, or the solvate of any one of the foregoing according to claim 1, wherein
R⁶ is a 8- to 10-membered heterocycloalkyl with 1, 2, or 3 heteroatoms selected from 1, 2, or 3 types of N, O, and S or a 8- to 10-membered heterocycloalkyl with 1, 2, or 3 heteroatoms selected from 1, 2, or 3 types of N, O, and S substituted by 1, 2, or 3 R⁶⁻²

4. The compound of formula (I), the tautomer thereof, the stereoisomer thereof, or the pharmaceutically acceptable salt of any one of the foregoing, or the solvate of any one of the foregoing according to claim 1, wherein R¹ is H;
R² and R³ are each independently H, C₁₋₃ alkyl, or C₁₋₃ alkyl substituted by 1, 2, or 3 R²⁻¹; each R²⁻¹ is independently halogen or -OH;
R⁴ is H or halogen; m is 0 or 1;
R⁵ and R⁷ are each independently H or halogen;
R⁶ is a 8- to 11-membered heterocycloalkyl with 1, 2, or 3 heteroatoms selected from 1, 2, or 3 types of N, O, and S or a 8- to 11-membered heterocycloalkyl with 1, 2, or 3 heteroatoms selected from 1, 2, or 3 types of N, O, and S substituted by 1, 2, or 3 R⁶⁻², wherein the heterocycloalkyl is a bridged ring or a spiro ring;
each R⁶⁻² is independently hydroxyl or C₁₋₃ alkyl;
R⁸ is H;
R⁹ is H or halogen; n is 0 or 1;
L is -(CR^{a}R^{b})_{q}-, q is 1; R^{a} and R^{b} are each independently H;
R¹⁰ is -COOH;
X is CR^{d} or N; R^{d} is H.

5. The compound of formula (I), the tautomer thereof, the stereoisomer thereof, or the pharmaceutically acceptable salt of any one of the foregoing, or the solvate of any one of the foregoing according to claim 1, wherein the compound of formula (I) satisfies one or more than one of the following conditions:
(1) in R¹, R², R³, R⁵, R⁷, R⁶⁻², R⁸, R⁹, and R^{d}, each alkyl is independently methyl, ethyl, n-propyl, isopropyl, n-butyl, isobutyl, or tert-butyl, preferably methyl or ethyl;
(2) in R¹⁻¹, R²⁻¹, R⁵, R⁷, and R⁶⁻², each alkoxy is independently methoxy, ethoxy, n-propoxy, isopropoxy, n-butoxy, isobutoxy, or tert-butoxy;
(3) in R¹⁻¹, R², R³, R²⁻¹, R⁴, R⁵, R⁷, R⁶⁻², R⁸, R⁹, R⁹⁻¹, R^{a}, R^{b}, and R^{d}, each halogen is independently F, Cl, Br, or I, preferably F;
(4) in R⁶, the heterocycloalkyl is connected to the parent through an N atom; the heterocycloalkyl is preferably

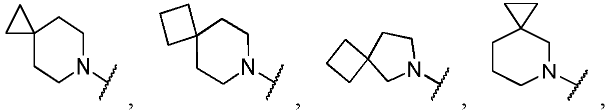

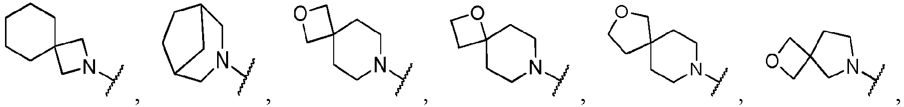

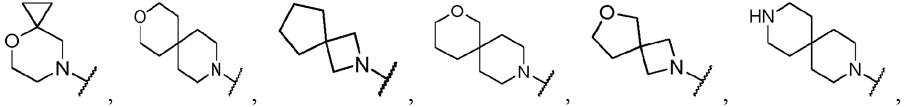

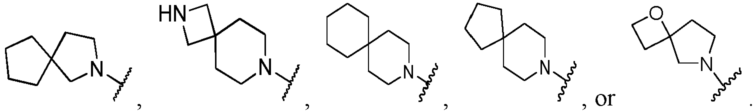

6. The compound of formula (I), the tautomer thereof, the stereoisomer thereof, or the pharmaceutically acceptable salt of any one of the foregoing, or the solvate of any one of the foregoing according to claim 1, wherein the compound of formula (I) satisfies one or more than one of the following conditions:
(1) R² is H, R³ is H, -CH₃, -CH₂OH, -CH₂CH₂OH, -CH₂F, or -CF₂H;
(2) R⁶ is 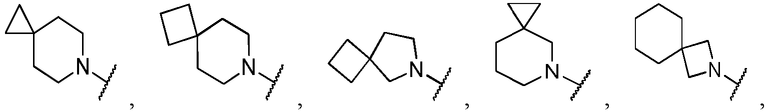 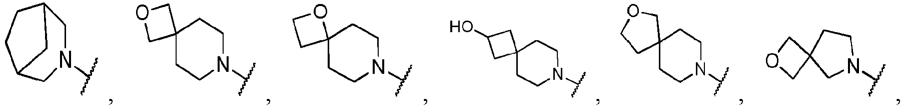 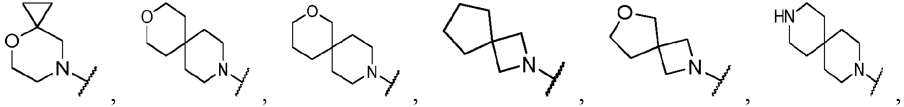 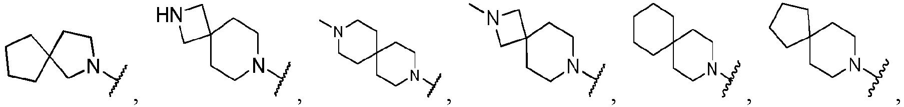 or

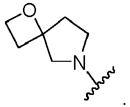

7. The compound of formula (I), the tautomer thereof, the stereoisomer thereof, or the pharmaceutically acceptable salt of any one of the foregoing, or the solvate of any one of the foregoing according to claim 1, wherein the compound of formula (I) satisfies any one of the following conditions:
(1) R¹ is H;
R² is H, R³ is H, -CH₃, or -CH₂F;
R⁴ is H or halogen, m is 0 or 1;
R⁵ and R⁷ are each independently H or F;
R⁶ is a 8- to 11-membered heterocycloalkyl with 1, 2, or 3 heteroatoms selected from 1, 2, or 3 types of N, O, and S or a 8- to 11-membered heterocycloalkyl with 1, 2, or 3 heteroatoms selected from 1, 2, or 3 types of N, O, and S substituted by 1, 2, or 3 R⁶⁻²;
each R⁶⁻² is independently hydroxyl or C₁₋₃ alkyl;
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d} or N; R^{d} is H;
(2) R¹ is H;
R² is H, R³ is H, -CH₃, or -CH₂F;
R⁴ is H or halogen, m is 0 or 1;
R⁵ and R⁷ are each independently H or F;
R⁶ is a 8- to 10-membered heterocycloalkyl with 1, 2, or 3 heteroatoms selected from 1, 2, or 3 types of N, O, and S or a 8- to 10-membered heterocycloalkyl with 1, 2, or 3 heteroatoms selected from 1, 2, or 3 types of N, O, and S substituted by 1, 2, or 3 R⁶⁻²;
each R⁶⁻² is independently hydroxyl or C₁₋₃ alkyl;
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d} or N; R^{d} is H;
(3) R¹ is H;
R² is H, R³ is H, -CH₃, or -CH₂F;
R⁴ is H or halogen, m is 0 or 1;
R⁵ and R⁷ are each independently H or F;
R⁶ is 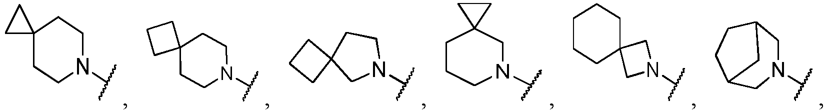 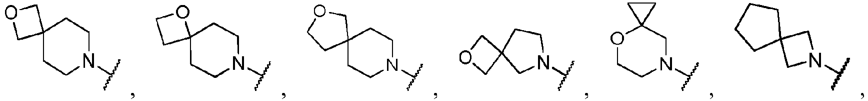 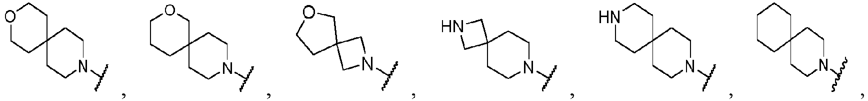 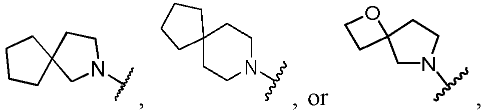 or the following group substituted by 1 R⁶⁻²: 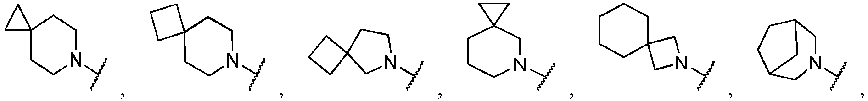 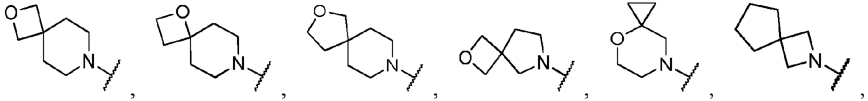 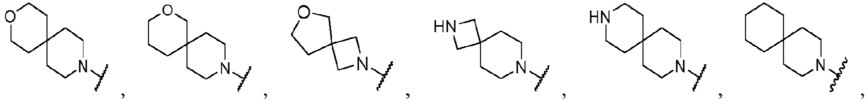 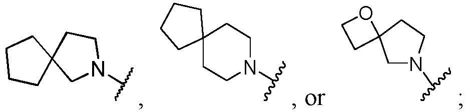
R⁶⁻² is hydroxyl or methyl;
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d} or N; R^{d} is H;
(4) R¹ is H;
R² is H, R³ is H, -CH₃, or -CH₂F;
R⁴ is H or F, m is 0 or 1;
R⁵ and R⁷ are each independently H or F;
R⁶ is 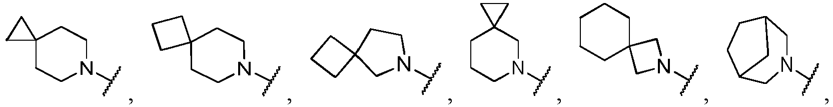 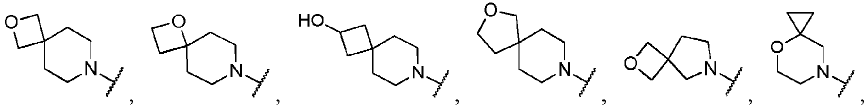 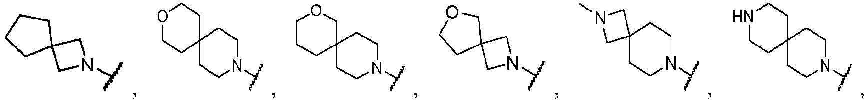 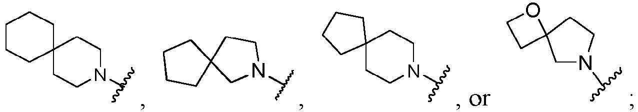
R⁸ is H;
R⁹ is H or F; n is 0 or 1;
L is -(CR^{a}R^{b})_{q}-; q is 1; R^{a} and R^{b} are each independently H;
R¹⁰ is -COOH;
X is CR^{d} or N; R^{d} is H.

8. The compound of formula (I), the tautomer thereof, the stereoisomer thereof, or the pharmaceutically acceptable salt of any one of the foregoing, or the solvate of any one of the foregoing according to at least one of claims 1 to 7, wherein the compound of formula (I) has a structure of formula (I-1) to (I-4): 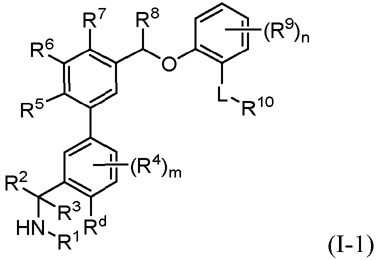
wherein R¹, R², R³, R⁴, R⁵, R⁶, R⁷, R⁸, R⁹, R¹⁰, R^{d}, L, m, and n are defined as in at least one of claims 1 to 7; 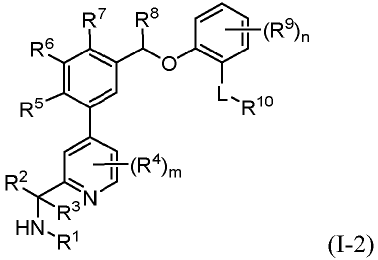
wherein R¹, R², R³, R⁴, R⁵, R⁶, R⁷, R⁸, R⁹, R¹⁰, L, m, and n are defined as in at least one of claims 1 to 7; 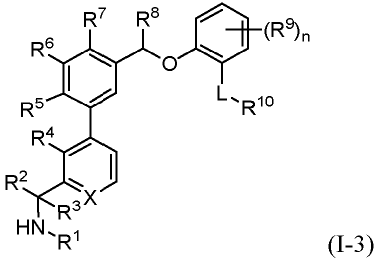
wherein X, R¹, R², R³, R⁴, R⁵, R⁶, R⁷, R⁸, R⁹, R¹⁰, L, and n are defined as in at least one of claims 1 to 7; 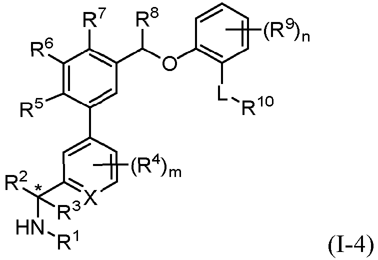
wherein X, R¹, R², R³, R⁴, R⁵, R⁶, R⁷, R⁸, R⁹, R¹⁰, L, m, and n are defined as in at least one of claims 1 to 7; when a carbon atom marked with "*" is a chiral carbon atom, it represents an R configuration, an S configuration, or a mixture thereof.

9. The compound of formula (I), the tautomer thereof, the stereoisomer thereof, or the pharmaceutically acceptable salt of any one of the foregoing, or the solvate of any one of the foregoing according to claim 1, wherein the compound of formula (I) is selected from any one of the following compounds:

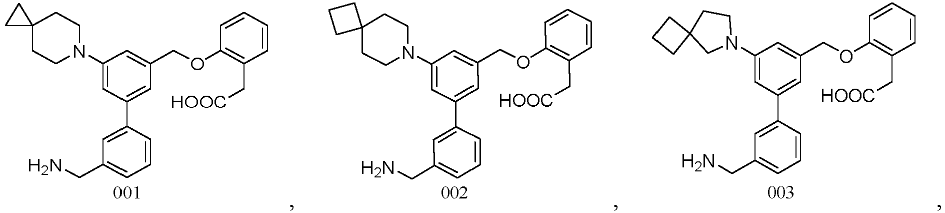

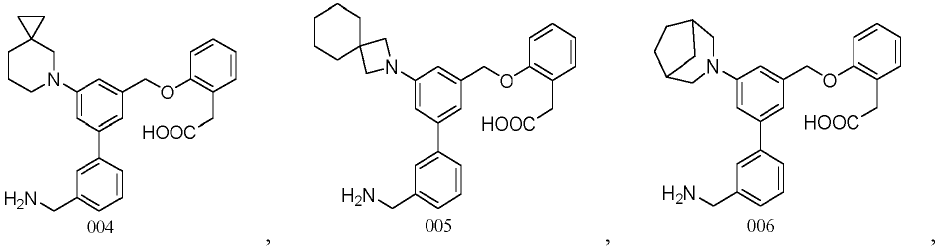

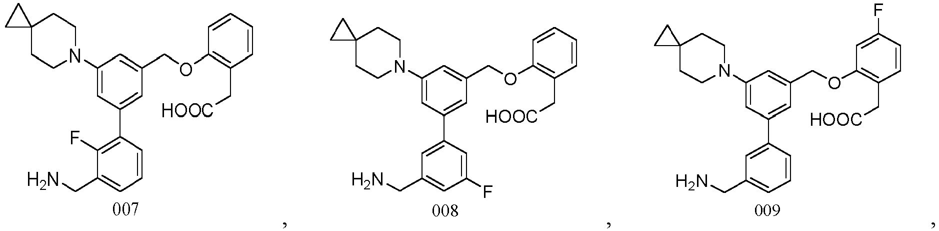

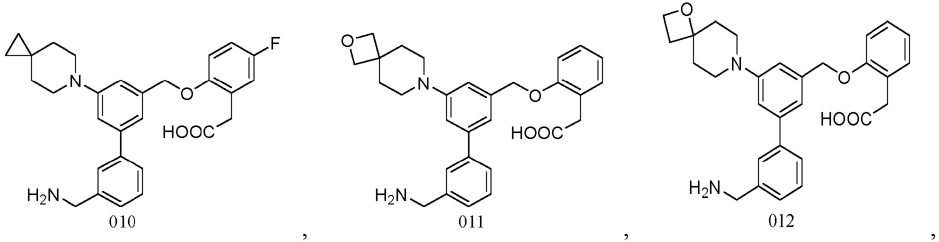

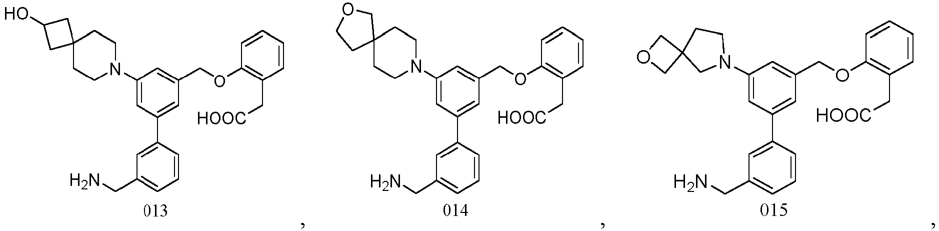

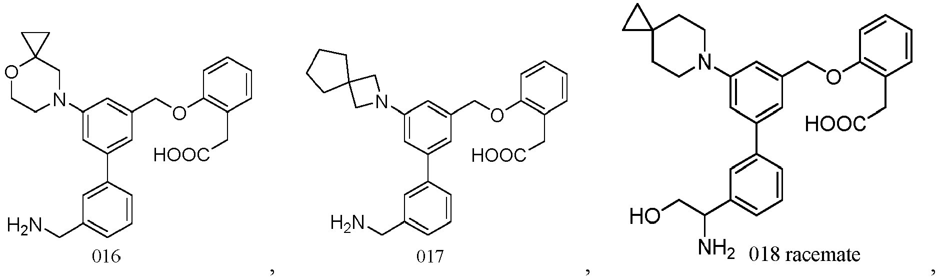

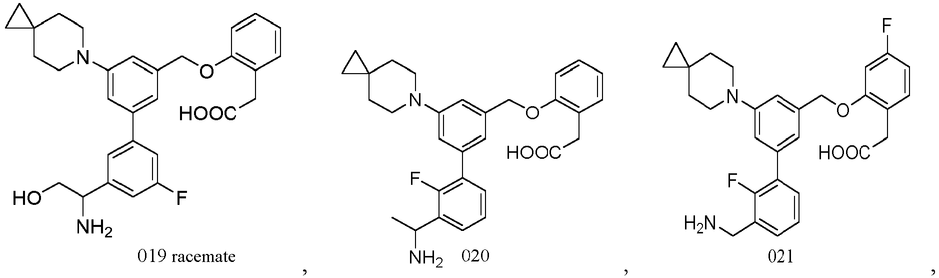

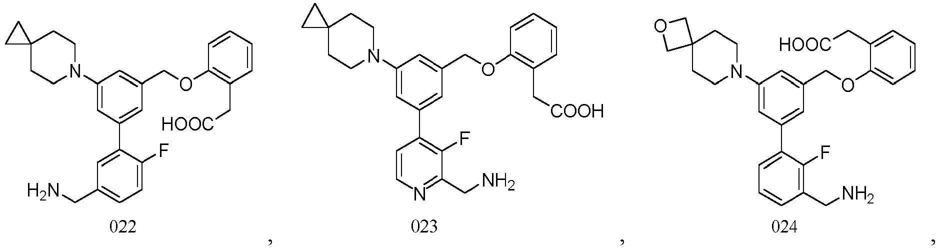

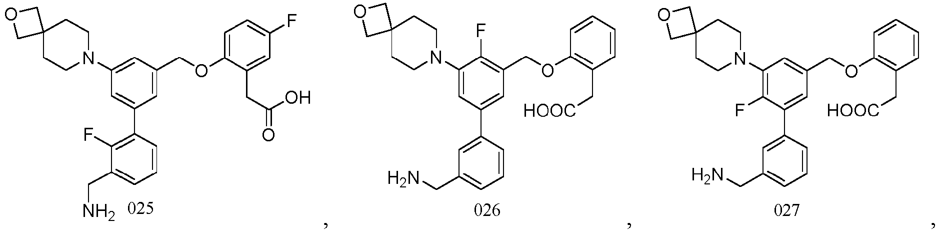

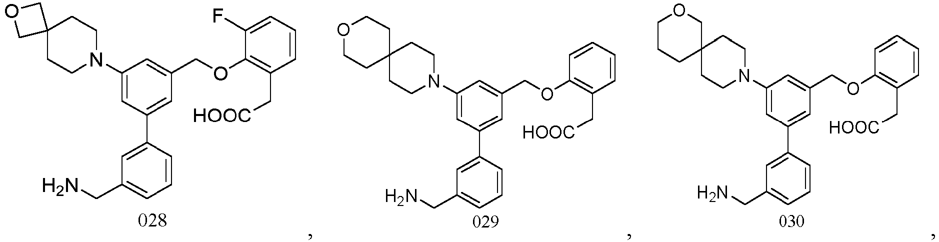

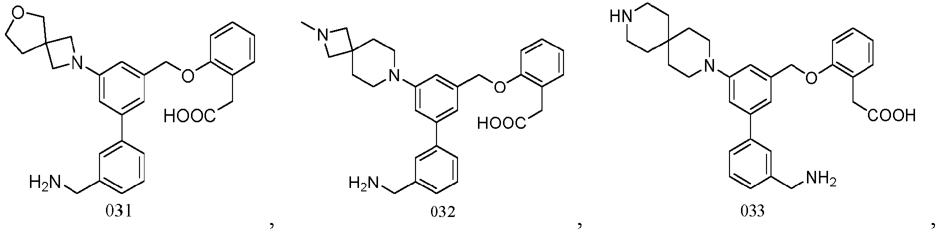

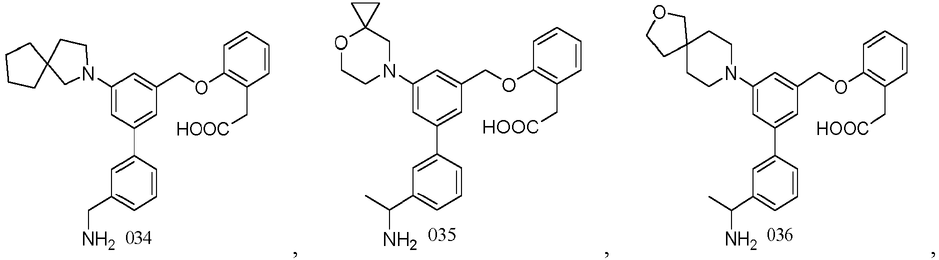

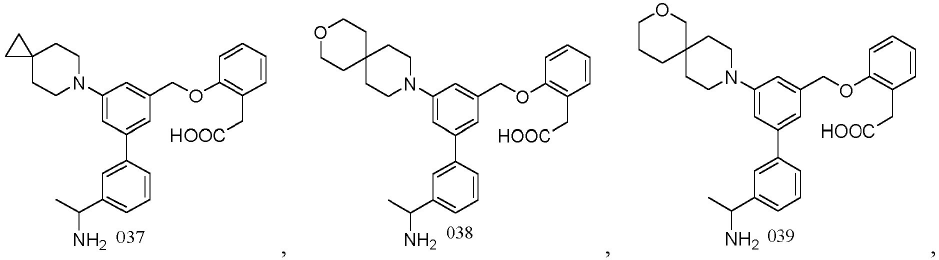

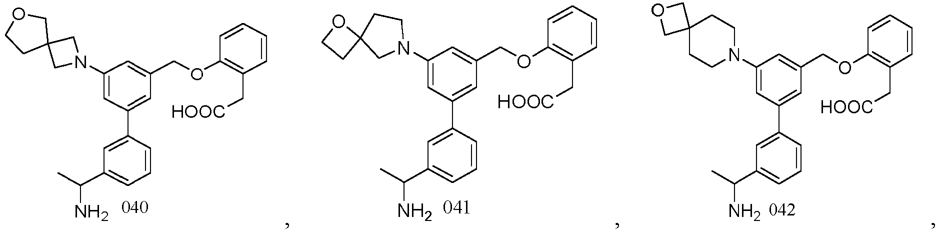

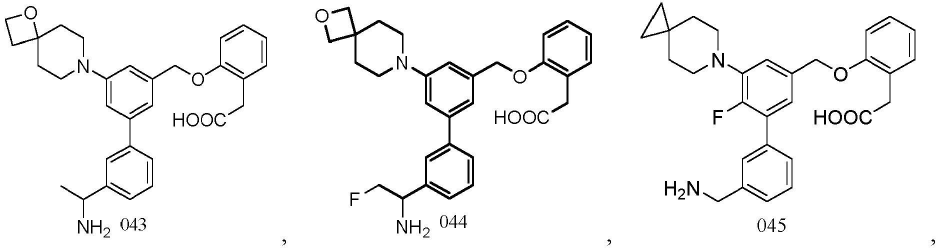

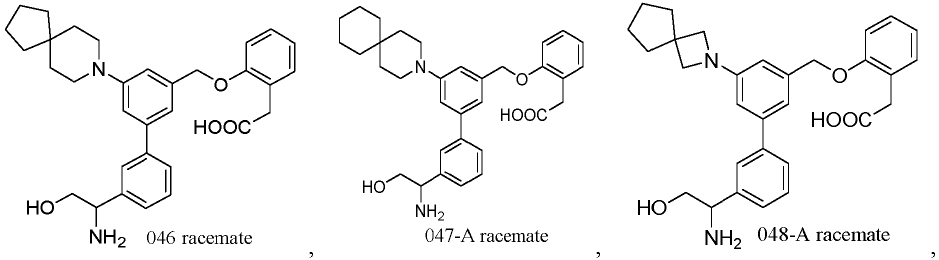

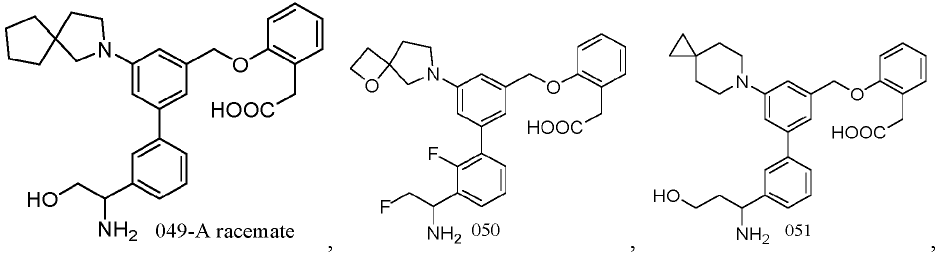

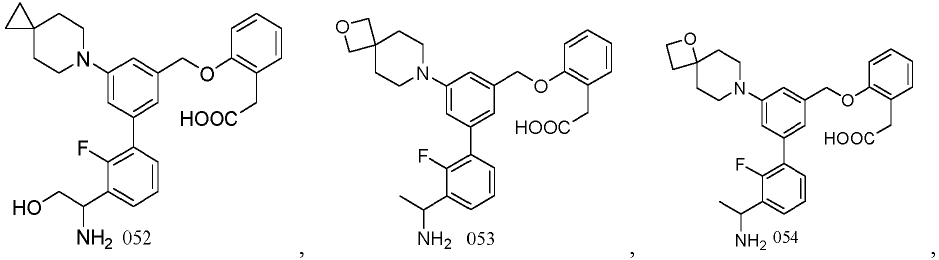

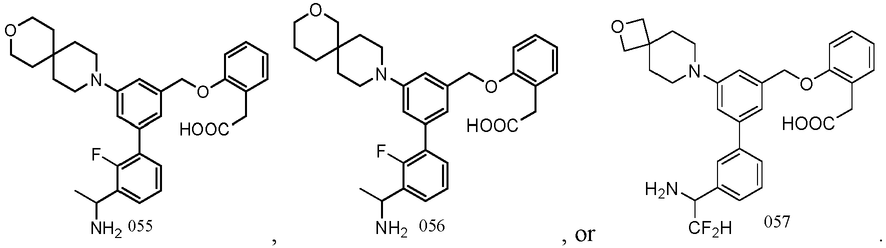

10. The compound of formula (I), the tautomer thereof, the stereoisomer thereof, or the pharmaceutically acceptable salt of any one of the foregoing, or the solvate of any one of the foregoing according to claim 9, wherein the compound of formula (I) is selected from any one of the following compounds: 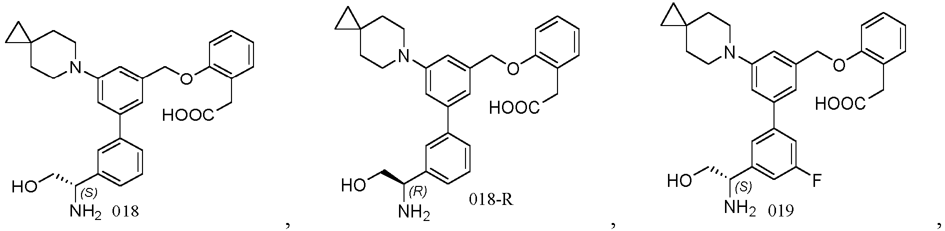 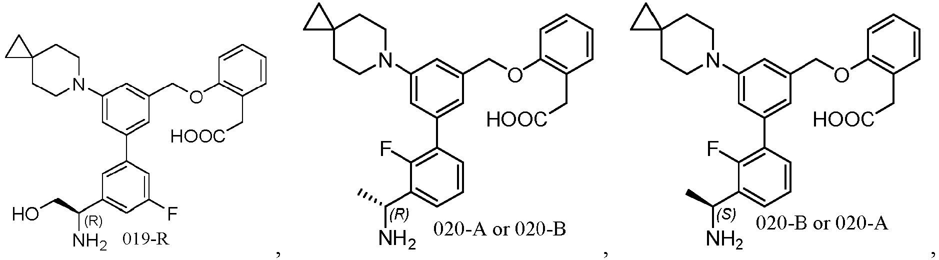 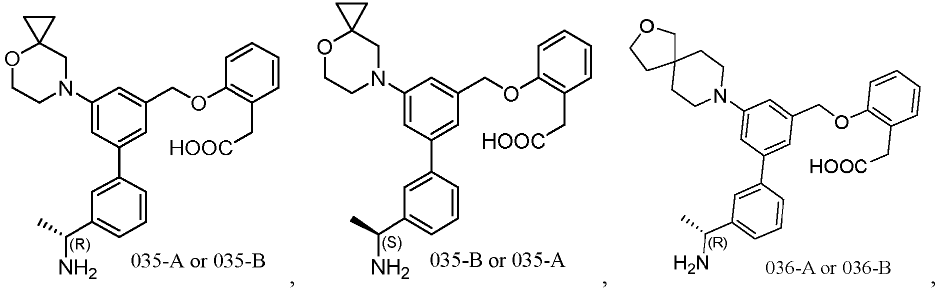 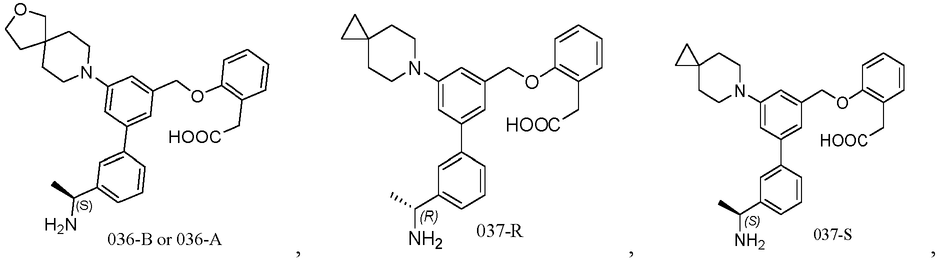 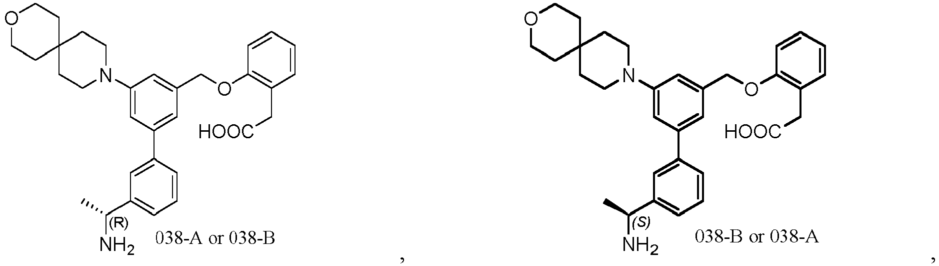 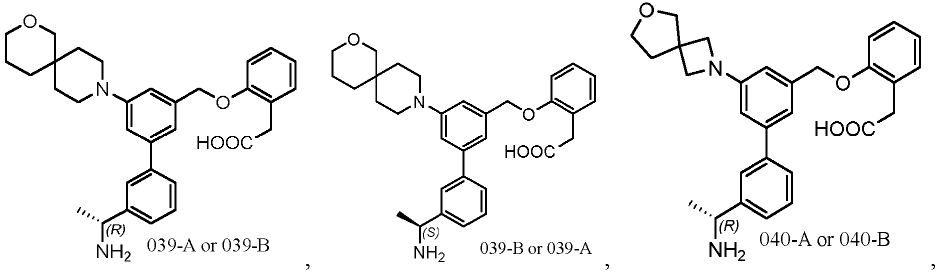 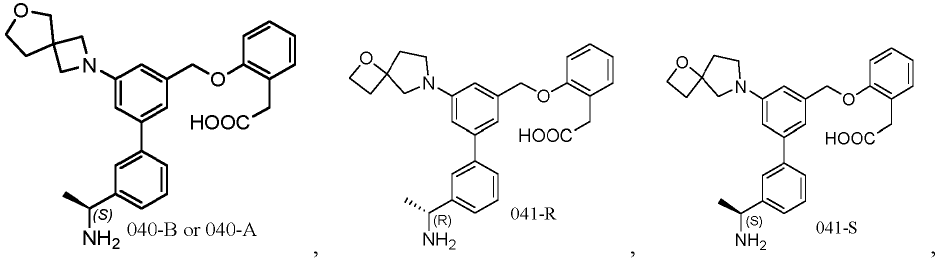 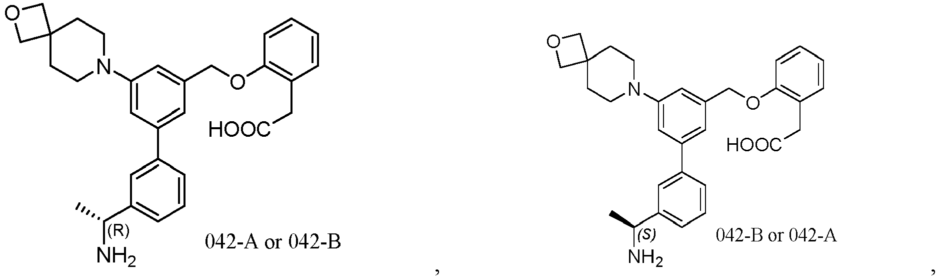 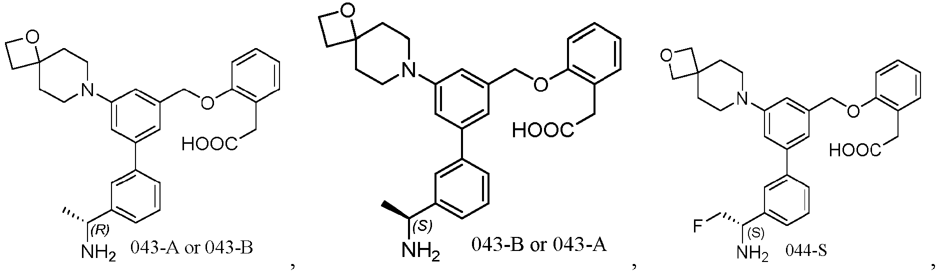 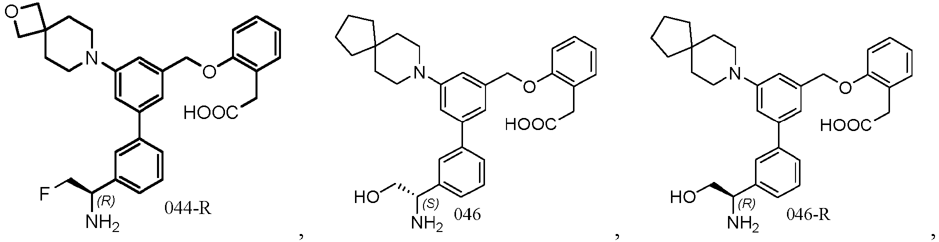 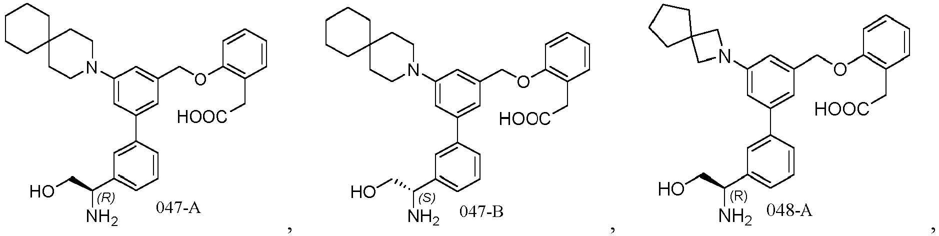 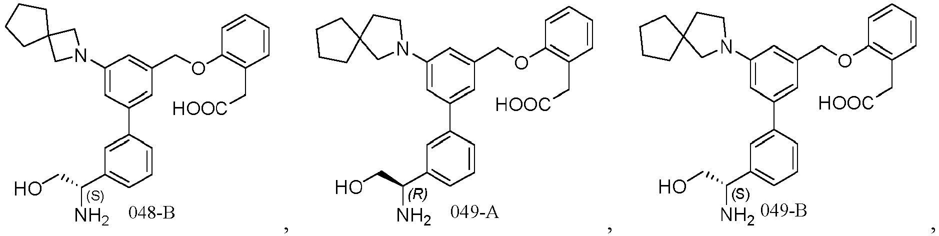 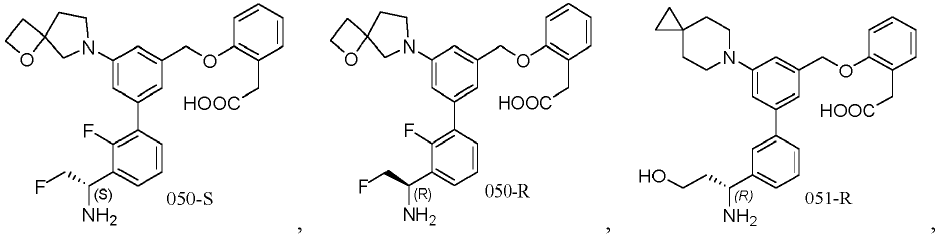 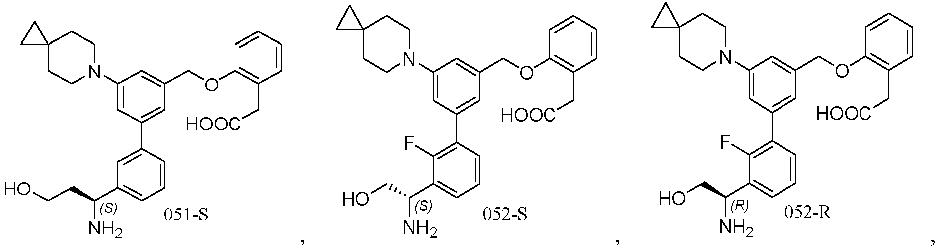 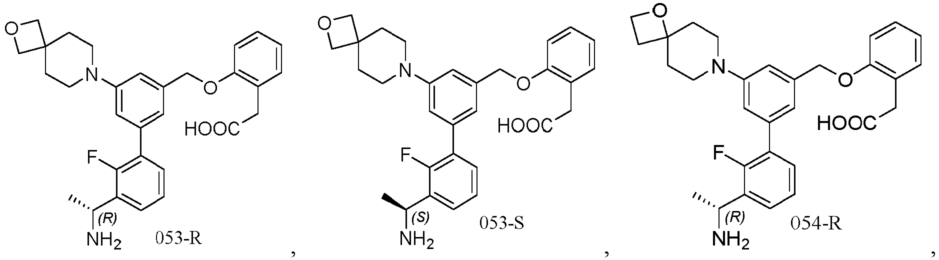 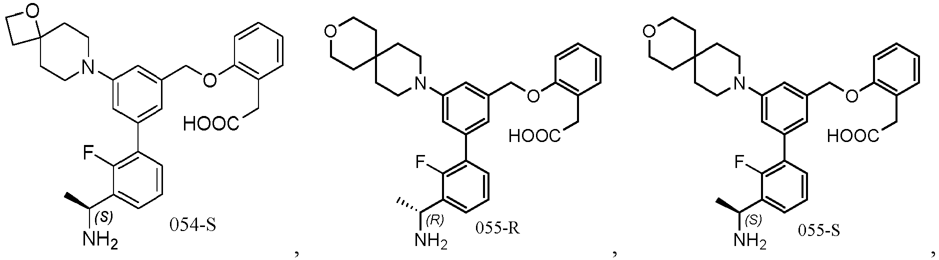 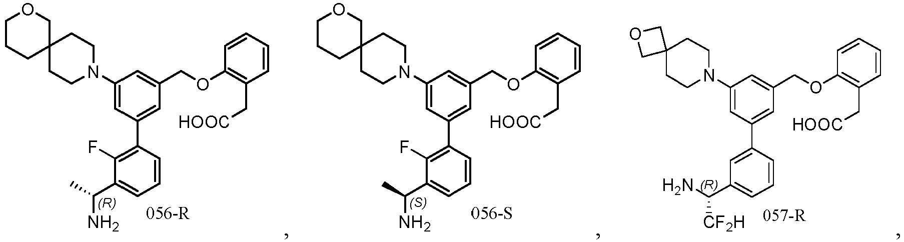 or

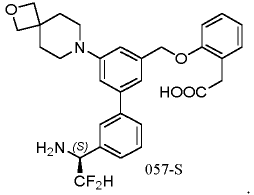

11. A preparation method for a compound of formula (I), comprising the following steps:
(1) subjecting a compound of formula II-3 to a deprotection reaction to obtain a compound of formula II-4; 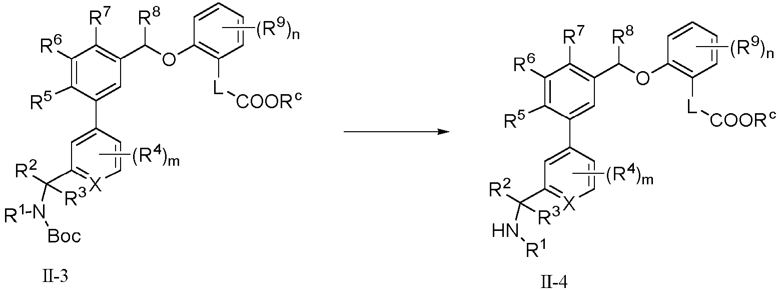
(2) subjecting the compound of formula II-4 to a hydrolysis reaction to obtain the compound of formula (I); 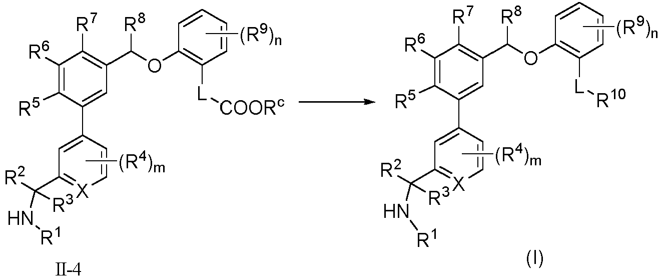 wherein R¹⁰ is -COOH, R^{c} is C₁₋₆ alkyl or C₁₋₆ alkyl substituted by 1, 2, or 3 R^{c-1}; each R^{c-1} is independently halogen, -OH, or -C(=O)OC(CH₃)₃, and R¹, R², R³, R⁴, R⁵, R⁶, R⁷, R⁸, R⁹, X, L, m, and n are defined as in at least one of claims 1 to 10; preferably, R¹ is H.

12. A compound of formula II-3 or formula II-4: 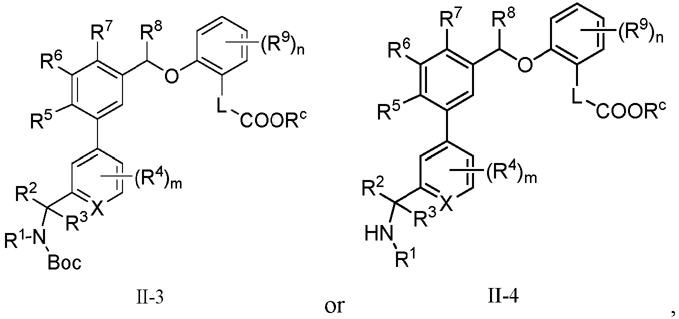
Wherein, R^{c} is C₁₋₆ alkyl or C₁₋₆ alkyl substituted by 1, 2, or 3 R^{c-1}; each R^{c-1} is independently halogen, -OH, or -C(=O)OC(CH₃)₃;
R¹, R², R³, R⁴, R⁵, R⁶, R⁷, R⁸, R⁹, X, L, m, and n are defined as in at least one of claims 1 to 10; preferably, R¹ is H.

13. The compound according to claim 12, wherein, the compound is selected from any one of the following: 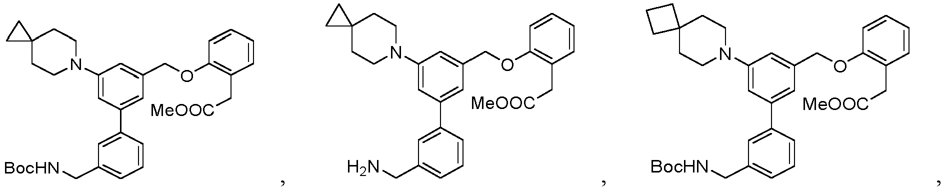 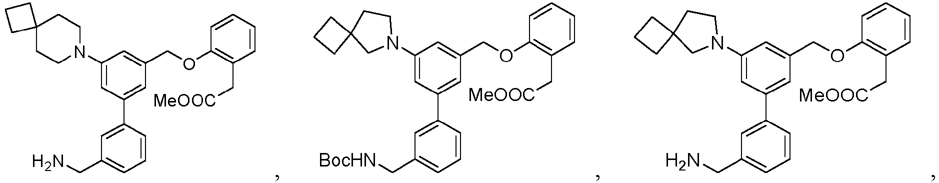 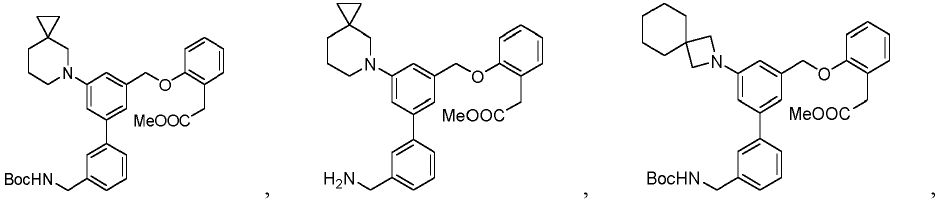 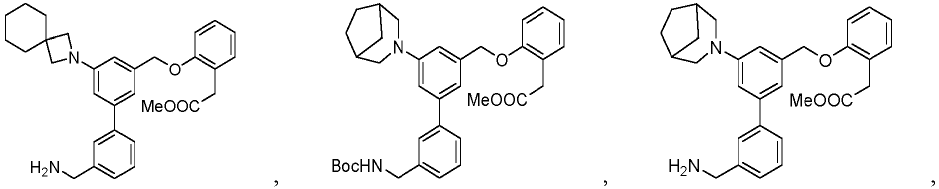 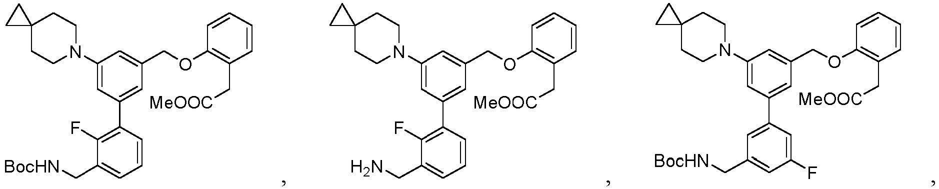 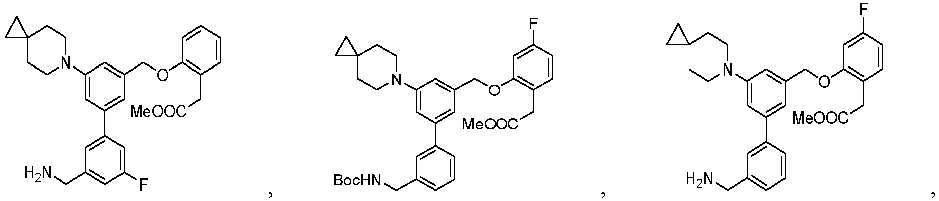 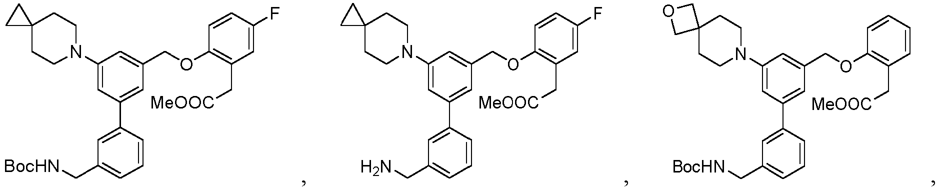 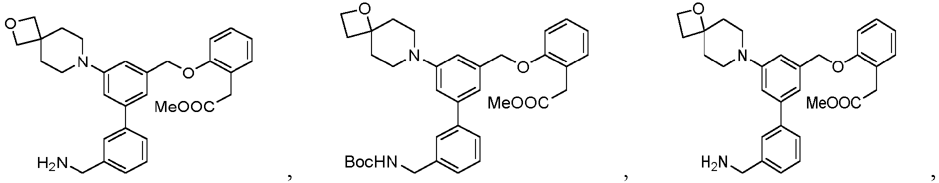 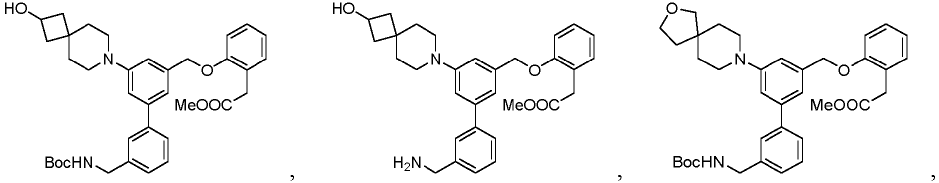 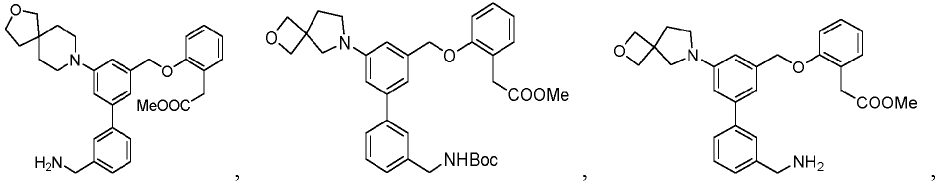 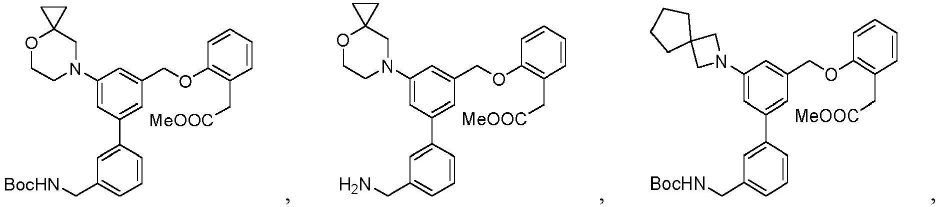 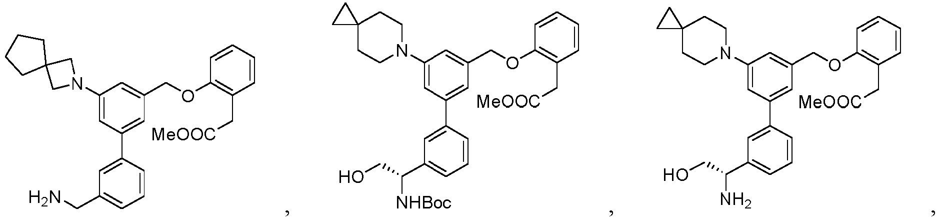 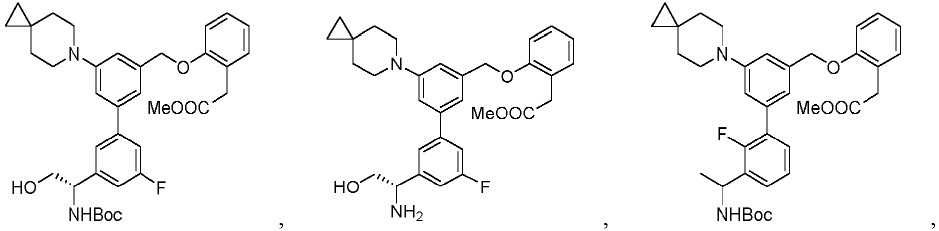 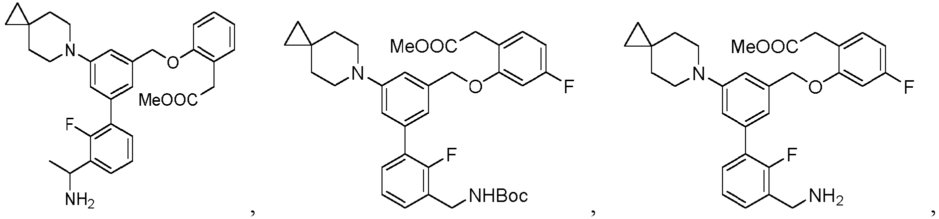 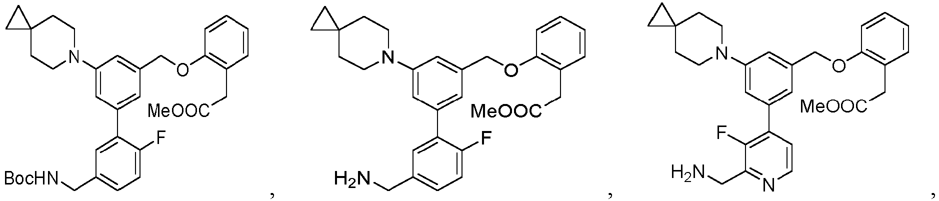 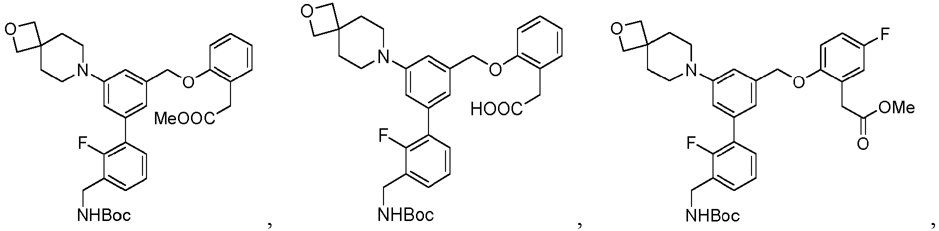 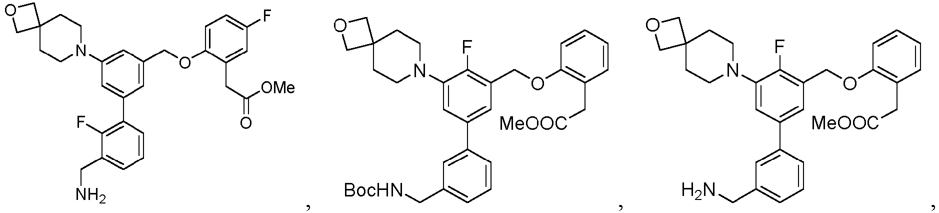 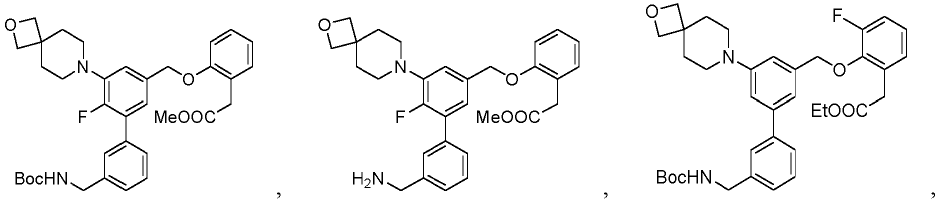 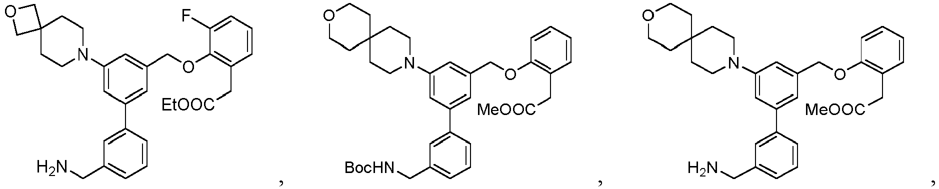 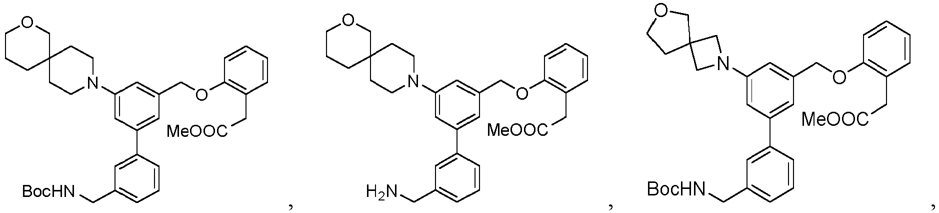 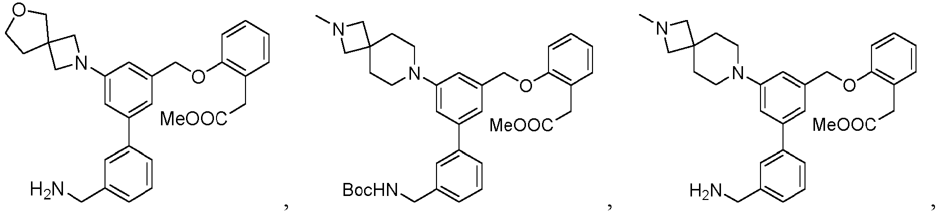 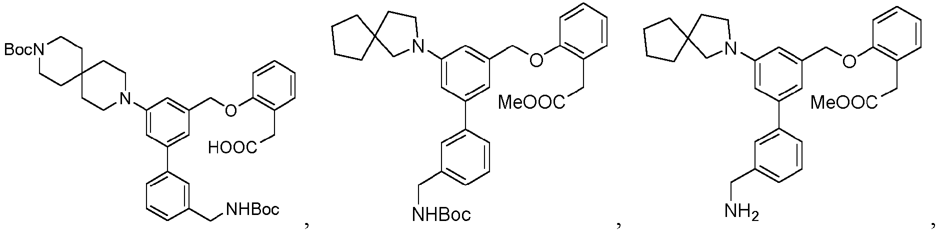 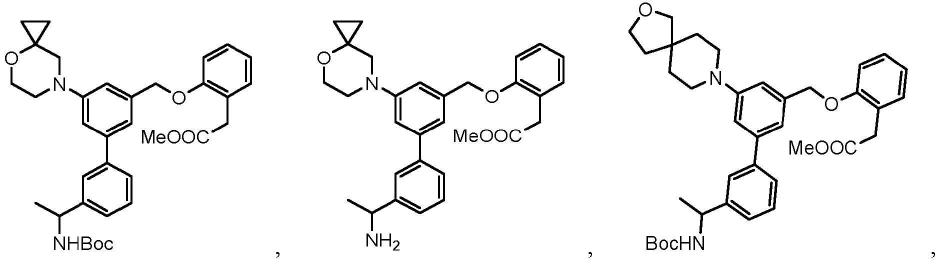 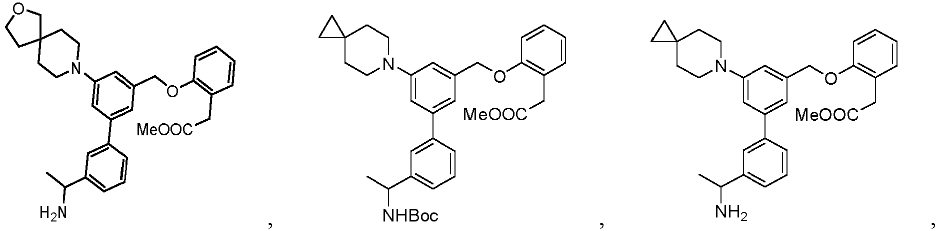 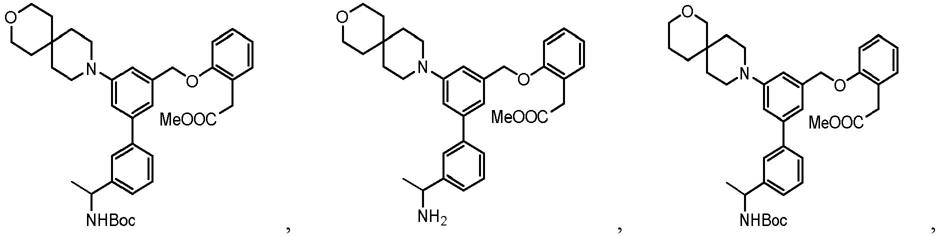 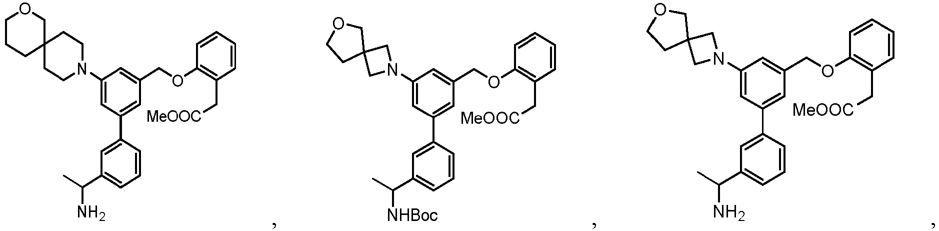 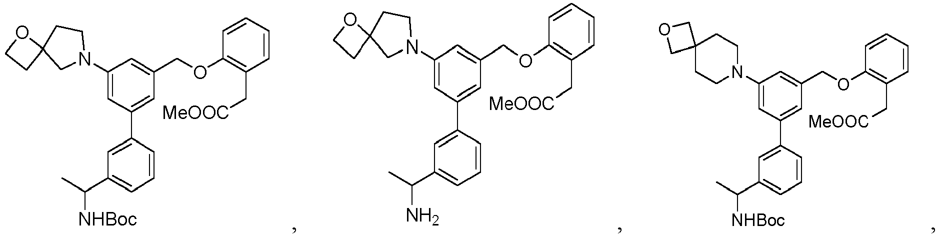 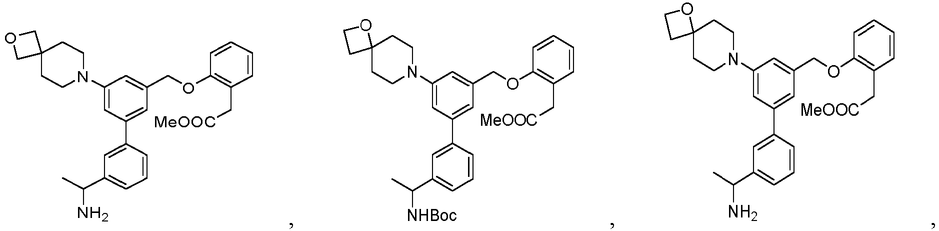 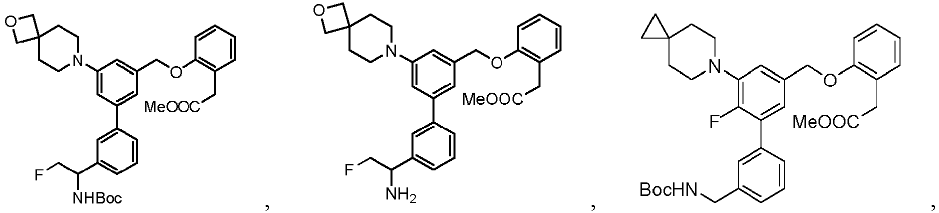 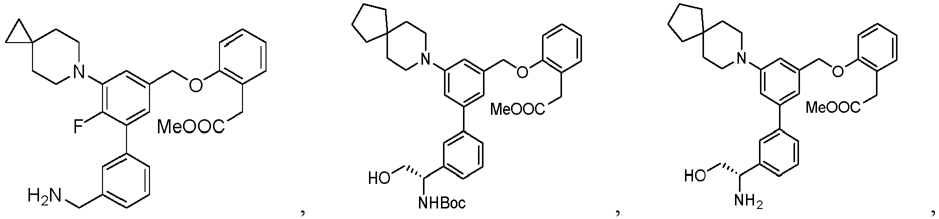 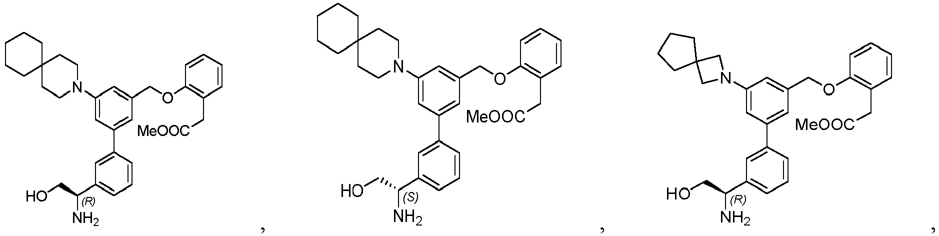 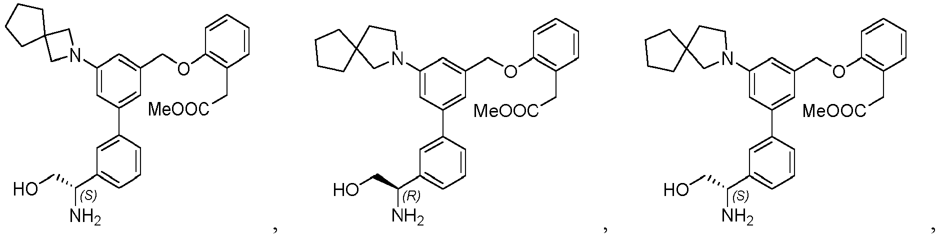 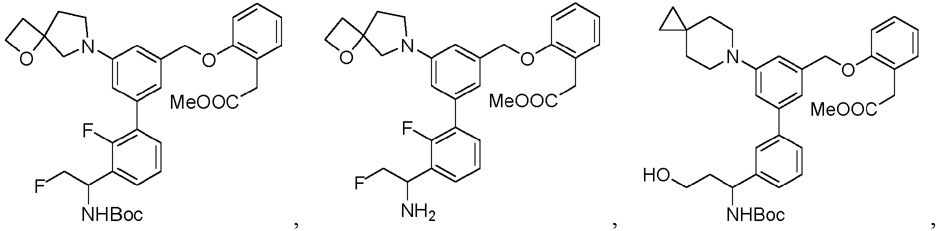 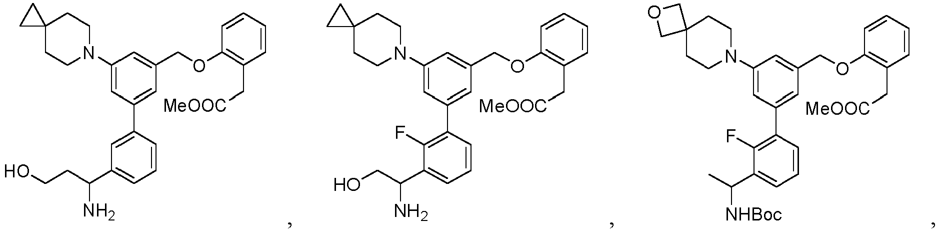 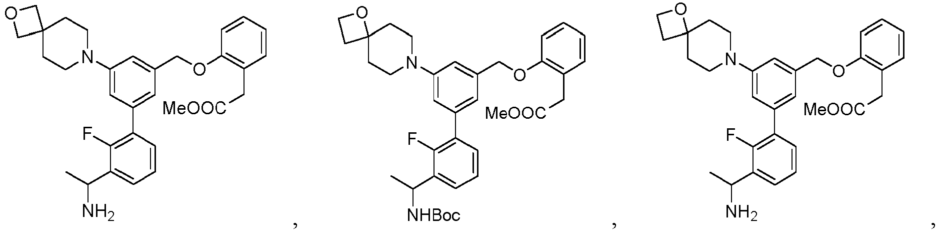 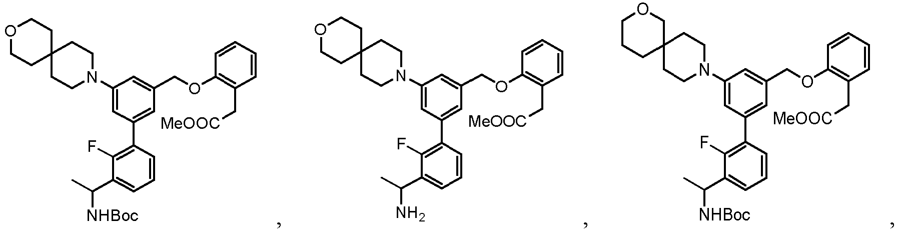 or

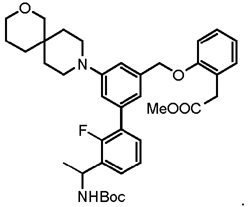

14. A pharmaceutical composition, comprising:
(1) the compound of formula (I), the tautomer thereof, the stereoisomer thereof, or the pharmaceutically acceptable salt of any one of the foregoing, or the solvate of any one of the foregoing according to at least one of claims 1 to 10; and
(2) a pharmaceutically acceptable carrier.

15. The compound of formula (I), the tautomer thereof, the stereoisomer thereof, or the pharmaceutically acceptable salt of any one of the foregoing, or the solvate of any one of the foregoing according to at least one of claims 1 to 10, or the pharmaceutical composition according to claim 14 for use as a complement factor D inhibitor medicament or a medicament for the treatment and/or prevention of a disease mediated by complement factor D, wherein the disease mediated by complement factor D is a hematological disorder, a renal disease, a cardiovascular disease, an immunological disorder, a disease of the central nervous system, a respiratory disease, a urogenital system disease, or an ocular disorder;
preferably, the disease mediated by complement factor D is cold agglutinin disease, catastrophic antiphospholipid syndrome, hemolytic anemia, antineutrophil cytoplasmic antibody-associated vasculitis, warm autoimmune hemolytic anemia, paroxysmal nocturnal hemoglobinuria, IgA nephropathy, lupus nephritis, atypical hemolytic uremic syndrome, membranoproliferative glomerulonephritis, dense-deposit disease, C3 glomerulonephritis, focal segmental glomerulosclerosis, diabetic nephropathy, systemic lupus or lupus erythematosus, rheumatoid arthritis, inflammatory bowel disease, psoriasis, multiple sclerosis, organ transplant rejection, myasthenia gravis, Alzheimer's disease, respiratory distress syndrome, asthma, chronic obstructive pulmonary disease, emphysema, coronavirus infection, macular degeneration, age-related macular degeneration, macular edema, diabetic macular edema, choroidal neovascularization, uveitis, Behcet's uveitis, proliferative diabetic retinopathy, non-proliferative diabetic retinopathy, glaucoma, hypertensive retinopathy, corneal neovascularization disease, post-corneal transplant rejection, corneal dystrophy disease, autoimmune dry eye disease, Stevens-Johnson syndrome, Sjögren's syndrome, environmental dry eye disease, Fuchs' endothelial dystrophy, retinal vein occlusion, or post-operative inflammation;
preferably, coronavirus infection is SARS-CoV infection, MERS-CoV infection, or SARS-CoV-2 infection.

## Patentansprüche

1. Eine Verbindung der Formel (I), ein Tautomer davon, ein Stereoisomer davon oder ein pharmazeutisch akzeptables Salz eines der Vorstehenden oder ein Solvat eines der Vorstehenden: 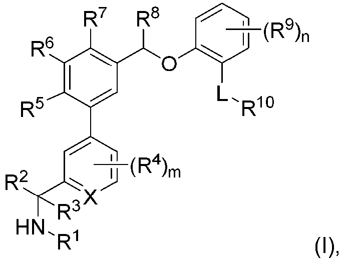 wobei
R¹ H, D, C₁₋₆-Alkyl oder C₁₋₆-Alkyl, substituiert mit 1, 2 oder 3 R¹⁻¹, ist; jedes R¹⁻¹ unabhängig Halogen, -CN, -OH, C₁₋₆-Alkoxy oder -NH₂ ist;
R² und R³ jeweils unabhängig H, D, Halogen, C₁₋₆-Alkyl oder C₁₋₆-Alkyl, substituiert mit 1, 2 oder 3 R²⁻¹, sind; jedes R²⁻¹ unabhängig Halogen, -CN, -OH, C₁₋₆-Alkoxy oder -NH₂ ist;
R⁴ H oder Halogen ist; m 0, 1, 2 oder 3 ist;
R⁵ und R⁷ jeweils unabhängig H, Halogen, -CN, C₁₋₆-Alkyl oder C₁₋₆-Alkoxy sind;
R⁶ ein 8- bis 11-gliedriges Heterocycloalkyl mit 1, 2 oder 3 Heteroatomen, ausgewählt aus 1, 2 oder 3 Typen von N, O und S, oder ein 8- bis 11-gliedriges Heterocycloalkyl mit 1, 2 oder 3 Heteroatomen, ausgewählt aus 1, 2 oder 3 Typen von N, O und S, substituiert mit 1, 2 oder 3 R⁶⁻², ist;
R⁶⁻² jeweils unabhängig Hydroxyl, Oxo (=O), Halogen, C₁₋₆-Alkyl, C₁₋₆-Alkoxy, C₁₋₆-Halogenalkyl, C₁₋₆-Halogenalkoxy, C₃₋₆-Cycloalkyl oder -(CH₂)ₚ-C₃₋₆-Cycloalkyl sind; p 1, 2, 3 oder 4 ist;
in R⁶⁻² das Cycloalkyl unabhängig ein monocyclischer Ring, ein verbrückter Ring oder ein Spiroring ist;
in R⁶ das Heterocycloalkyl ein verbrückter Ring oder ein Spiroring ist;
R⁸ H, Halogen oder C₁₋₆-Alkyl ist;
R⁹ H, Halogen, C₁₋₆-Alkyl oder C₁₋₆-Alkyl, substituiert mit 1, 2 oder 3 R⁹⁻¹, ist;
jedes R⁹⁻¹ unabhängig Halogen, -CN, -OH oder -NH₂ ist; n 0, 1, 2, 3 oder 4 ist;
L -(CR^{a}R^{b})_{q}- ist; q 0, 1, 2 oder 3 ist;
R^{a} und R^{b} jeweils unabhängig H, D oder Halogen sind oder R^{a} und R^{b} miteinander verbunden sind, um ein C₃₋₆-Cycloalkylen zu bilden, und das gebildete Cycloalkylen ein monocyclischer Ring, ein verbrückter Ring oder ein Spiroring ist;
R¹⁰ -COOH ist;
X CR^{d} oder N ist; R^{d} H, Halogen oder C₁₋₆-Alkyl ist.

2. Verbindung der Formel (I), das Tautomer davon, das Stereoisomer davon oder das pharmazeutisch akzeptable Salz eines der Vorstehenden oder das Solvat eines der Vorstehenden gemäß Anspruch 1, wobei die Verbindung der Formel (I) eine oder mehr als eine der folgenden Bedingungen erfüllt:
(1) R¹ ist H;
(2) R² und R³ sind jeweils unabhängig H, C₁₋₃-Alkyl oder C₁₋₃-Alkyl, substituiert mit 1, 2 oder 3 R²⁻¹; jedes R²⁻¹ ist unabhängig Halogen oder -OH und das Halogen ist vorzugsweise F;
(3) m ist 0 oder 1;
(4) R⁵ und R⁷ sind jeweils unabhängig H oder Halogen und das Halogen ist vorzugsweise F;
(5) in R⁶ ist das 8- bis 11-gliedrige Heterocycloalkyl 6-Azaspiro[2.5]octyl, 5-Azaspiro[2.5]octyl, 6-Azaspiro[3.4]octyl, 2-Azaspiro[3.4]octyl, 2-Oxa-6-azaspiro[3.4]octyl, 6-Oxa-2-azaspiro[3.4]octyl, 4-Oxa-7-azaspiro[2.5]octyl, 2-Azaspiro[4.4]nonyl, 2-Azaspiro[3.5]nonyl, 2-Oxa-7-azaspiro[3.5]nonyl, 1-Oxa-7-azaspiro[3.5]nonyl, 7-Azaspiro[3.5]nonyl, 2,7-Diazaspiro[3.5]nonyl, 2-Oxa-8-azaspiro[4.5]decyl, 3-Oxa-9-azaspiro[5.5]undecyl, 2-Oxa-9-azaspiro[5.5]undecyl, 3,9-Diazaspiro[5.5]undecyl, 3-Azabicyclo[3.2.1]octyl, 3-Azaspiro[5.5]undecyl, 8-Azaspiro[4.5]decyl oder 1-Oxa-6-azaspiro[3.4]octyl;
(6) jedes R⁶⁻² ist unabhängig Hydroxyl oder C₁₋₃-Alkyl, vorzugsweise ist jedes R⁶⁻² unabhängig Hydroxyl, Methyl, Ethyl, n-Propyl oder Isopropyl;
(7) R⁸ ist H;
(8) R⁹ ist H oder Halogen und das Halogen ist vorzugsweise F;
(9) n ist 0 oder 1;
(10) q ist 1;
(11) R^{a} und R^{b} sind jeweils unabhängig H;
(12) R^{d} ist H.

3. Verbindung der Formel (I), das Tautomer davon, das Stereoisomer davon oder das pharmazeutisch akzeptable Salz eines der Vorstehenden oder das Solvat eines der Vorstehenden gemäß Anspruch 1, wobei
R⁶ ein 8- bis 10-gliedriges Heterocycloalkyl mit 1, 2 oder 3 Heteroatomen, ausgewählt aus 1, 2 oder 3 Typen von N, O und S, oder ein 8- bis 10-gliedriges Heterocycloalkyl mit 1, 2 oder 3 Heteroatomen, ausgewählt aus 1, 2 oder 3 Typen von N, O und S, substituiert mit 1, 2 oder 3 R⁶⁻², ist.

4. Verbindung der Formel (I), das Tautomer davon, das Stereoisomer davon oder das pharmazeutisch akzeptable Salz eines der Vorstehenden oder das Solvat eines der Vorstehenden gemäß Anspruch 1, wobei R¹ H ist;
R² und R³ jeweils unabhängig H, C₁₋₃-Alkyl oder C₁₋₃-Alkyl, substituiert mit 1, 2 oder 3 R²⁻¹, sind; jedes R²⁻¹ unabhängig Halogen oder -OH ist;
R⁴ H oder Halogen ist; m 0 oder 1 ist;
R⁵ und R⁷ jeweils unabhängig H oder Halogen sind;
R⁶ ein 8- bis 11-gliedriges Heterocycloalkyl mit 1, 2 oder 3 Heteroatomen, ausgewählt aus 1, 2 oder 3 Typen von N, O und S, oder ein 8- bis 11-gliedriges Heterocycloalkyl mit 1, 2 oder 3 Heteroatomen, ausgewählt aus 1, 2 oder 3 Typen von N, O und S, substituiert mit 1, 2 oder 3 R⁶⁻², ist, wobei das Heterocycloalkyl ein verbrückter Ring oder ein Spiroring ist;
jedes R⁶⁻² unabhängig Hydroxyl oder C₁₋₃-Alkyl ist;
R⁸ H ist;
R⁹ H oder Halogen ist; n 0 oder 1 ist;
L -(CR^{a}R^{b})_{q}- ist, q 1 ist; R^{a} und R^{b} jeweils unabhängig H sind;
R¹⁰ -COOH ist;
X CR^{d} oder N ist; R^{d} H ist.

5. Verbindung der Formel (I), das Tautomer davon, das Stereoisomer davon oder das pharmazeutisch akzeptable Salz eines der Vorstehenden oder das Solvat eines der Vorstehenden gemäß Anspruch 1, wobei die Verbindung der Formel (I) eine oder mehr als eine der folgenden Bedingungen erfüllt:
(1) in R¹, R², R³, R⁵, R⁷, R⁶⁻², R⁸, R⁹ und R^{d} ist jedes Alkyl unabhängig Methyl, Ethyl, n-Propyl, Isopropyl, n-Butyl, Isobutyl oder tert-Butyl, vorzugsweise Methyl oder Ethyl;
(2) in R¹⁻¹, R²⁻¹, R⁵, R⁷ und R⁶⁻² ist jedes Alkoxy unabhängig Methoxy, Ethoxy, n-Propoxy, Isopropoxy, n-Butoxy, Isobutoxy oder tert-Butoxy;
(3) in R¹⁻¹, R², R³, R²⁻¹, R⁴, R⁵, R⁷, R⁶⁻², R⁸, R⁹, R⁹⁻¹, R^{a}, R^{b} und R^{d} ist jedes Halogen unabhängig F, Cl, Br oder I, vorzugsweise F;
(4) in R⁶ ist das Heterocycloalkyl über ein N-Atom mit der Muttersubstanz verbunden; das Heterocycloalkyl ist vorzugsweise

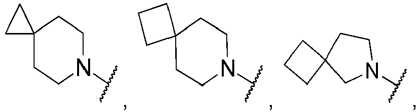

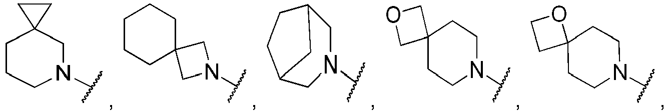

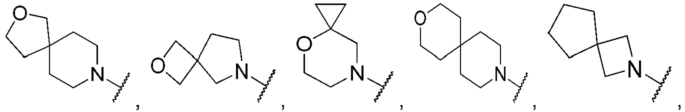

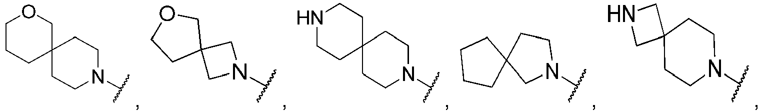

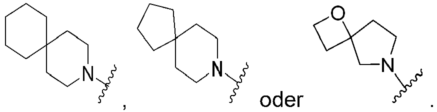

6. Verbindung der Formel (I), das Tautomer davon, das Stereoisomer davon oder das pharmazeutisch akzeptable Salz eines der Vorstehenden oder das Solvat eines der Vorstehenden gemäß Anspruch 1, wobei die Verbindung der Formel (I) eine oder mehr als eine der folgenden Bedingungen erfüllt:
(1) R² ist H, R³ ist H, -CH₃, -CH₂OH, -CH₂CH₂OH, -CH₂F oder -CF₂H;
(2) R⁶ ist

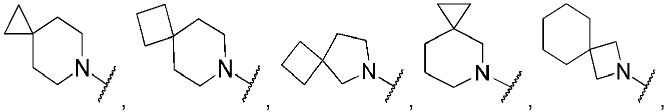

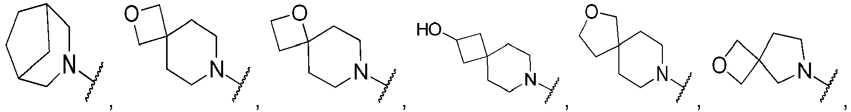

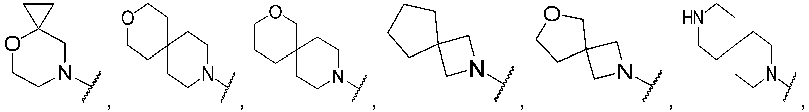

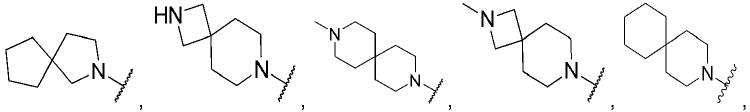

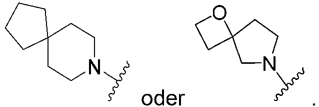

7. Verbindung der Formel (I), das Tautomer davon, das Stereoisomer davon oder das pharmazeutisch akzeptable Salz eines der Vorstehenden oder das Solvat eines der Vorstehenden gemäß Anspruch 1, wobei die Verbindung der Formel (I) eine der folgenden Bedingungen erfüllt:
(1) R¹ ist H;
R² ist H, R³ ist H, -CH₃ oder -CH₂F;
R⁴ ist H oder Halogen, m ist 0 oder 1;
R⁵ und R⁷ sind jeweils unabhängig H oder F;
R⁶ ist ein 8- bis 11-gliedriges Heterocycloalkyl mit 1, 2 oder 3 Heteroatomen, ausgewählt aus 1, 2 oder 3 Typen von N, O und S, oder ein 8- bis 11-gliedriges Heterocycloalkyl mit 1, 2 oder 3 Heteroatomen, ausgewählt aus 1, 2 oder 3 Typen von N, O und S, substituiert mit 1, 2 oder 3 R⁶⁻²;
jedes R⁶⁻² ist unabhängig Hydroxyl oder C₁₋₃-Alkyl;
R⁸ ist H;
R⁹ ist H oder F; n ist 0 oder 1;
L ist -(CR^{a}R^{b})_{q}-; q ist 1; R^{a} und R^{b} sind jeweils unabhängig H;
R¹⁰ ist -COOH;
X ist CR^{d} oder N; R^{d} ist H;
(2) R¹ ist H;
R² ist H, R³ ist H, -CH₃ oder -CH₂F;
R⁴ ist H oder Halogen, m ist 0 oder 1;
R⁵ und R⁷ sind jeweils unabhängig H oder F;
R⁶ ist ein 8- bis 10-gliedriges Heterocycloalkyl mit 1, 2 oder 3 Heteroatomen, ausgewählt aus 1, 2 oder 3 Typen von N, O und S, oder ein 8- bis 10-gliedriges Heterocycloalkyl mit 1, 2 oder 3 Heteroatomen, ausgewählt aus 1, 2 oder 3 Typen von N, O und S, substituiert mit 1, 2 oder 3 R⁶⁻²;
jedes R⁶⁻² ist unabhängig Hydroxyl oder C₁₋₃-Alkyl;
R⁸ ist H;
R⁹ ist H oder F; n ist 0 oder 1;
L ist -(CR^{a}R^{b})_{q}-; q ist 1; R^{a} und R^{b} sind jeweils unabhängig H;
R¹⁰ ist -COOH;
X ist CR^{d} oder N; R^{d} ist H;
(3) R¹ ist H;
R² ist H, R³ ist H, -CH₃ oder -CH₂F;
R⁴ ist H oder Halogen, m ist 0 oder 1;
R⁵ und R⁷ sind jeweils unabhängig H oder F;
R⁶ ist 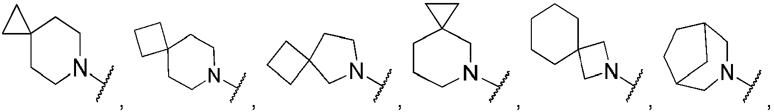 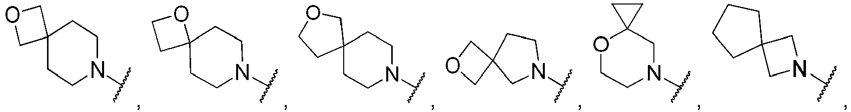 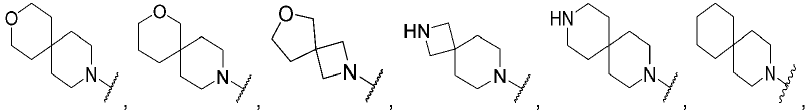 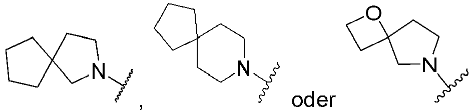 oder die folgende Gruppe, substituiert mit 1 R⁶⁻²: 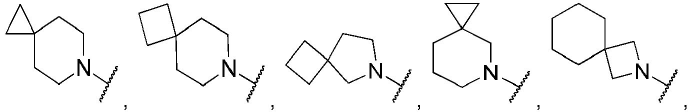 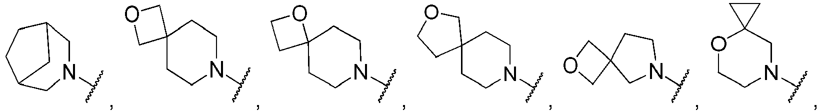 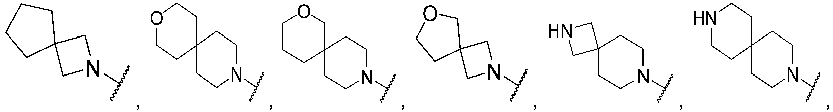 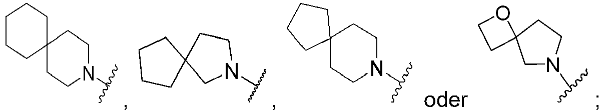
R⁶⁻² ist Hydroxyl oder Methyl;
R⁸ ist H;
R⁹ ist H oder F; n ist 0 oder 1;
L ist -(CR^{a}R^{b})_{q}-; q ist 1; R^{a} und R^{b} sind jeweils unabhängig H;
R¹⁰ ist -COOH;
X ist CR^{d} oder N; R^{d} ist H;
(4) R¹ ist H;
R² ist H, R³ ist H, -CH₃ oder -CH₂F;
R⁴ ist H oder F, m ist 0 oder 1;
R⁵ und R⁷ sind jeweils unabhängig H oder F;
R⁶ ist 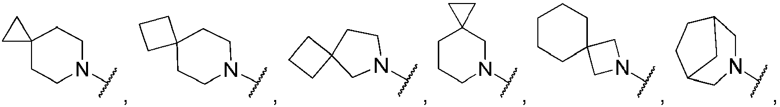 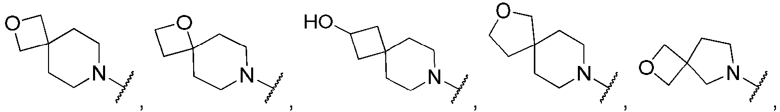 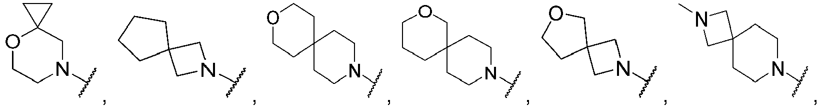 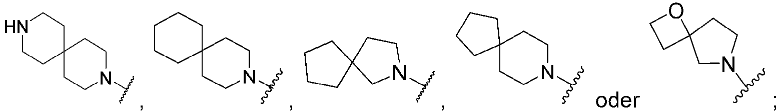
R⁸ ist H;
R⁹ ist H oder F; n ist 0 oder 1;
L ist -(CR^{a}R^{b})_{q}-; q ist 1; R^{a} und R^{b} sind jeweils unabhängig H;
R¹⁰ ist -COOH;
X ist CR^{d} oder N; R^{d} ist H.

8. Verbindung der Formel (I), das Tautomer davon, das Stereoisomer davon oder das pharmazeutisch akzeptable Salz eines der Vorstehenden oder das Solvat eines der Vorstehenden gemäß mindestens einem der Ansprüche 1 bis 7, wobei die Verbindung der Formel (I) eine Struktur der Formel (I-1) bis (I-4) aufweist: 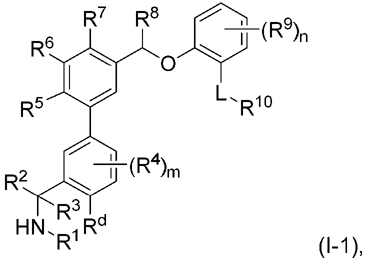
wobei R¹, R², R³, R⁴, R⁵, R⁶, R⁷, R⁸, R⁹, R¹⁰, R^{d}, L, m und n wie in mindestens einem der Ansprüche 1 bis 7 definiert sind; 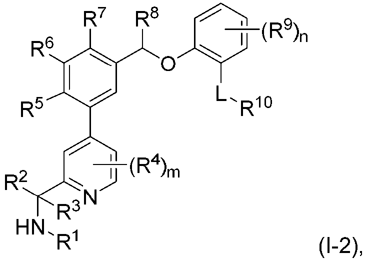
wobei R¹, R², R³, R⁴, R⁵, R⁶, R⁷, R⁸, R⁹, R¹⁰, L, m und n wie in mindestens einem der Ansprüche 1 bis 7 definiert sind; 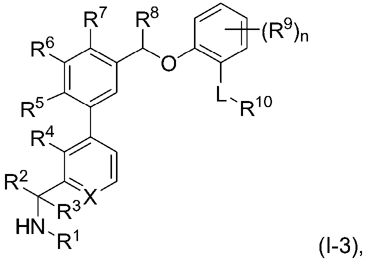
wobei X, R¹, R², R³, R⁴, R⁵, R⁶, R⁷, R⁸, R⁹, R¹⁰, L und n wie in mindestens einem der Ansprüche 1 bis 7 definiert sind; 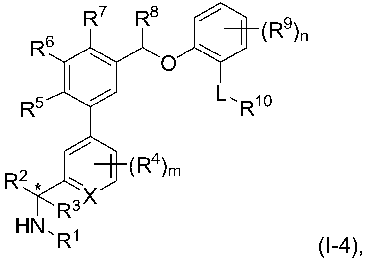
wobei X, R¹, R², R³, R⁴, R⁵, R⁶, R⁷, R⁸, R⁹, R¹⁰, L, m und n wie in mindestens einem der Ansprüche 1 bis 7 definiert sind; wenn ein mit "*" markiertes Kohlenstoffatom ein chirales Kohlenstoffatom ist, es eine R-Konfiguration, eine S-Konfiguration oder eine Mischung davon darstellt.

9. Verbindung der Formel (I), das Tautomer davon, das Stereoisomer davon oder das pharmazeutisch akzeptable Salz eines der Vorstehenden oder das Solvat eines der Vorstehenden gemäß Anspruch 1, wobei die Verbindung der Formel (I) aus einer der folgenden Verbindungen ausgewählt ist:

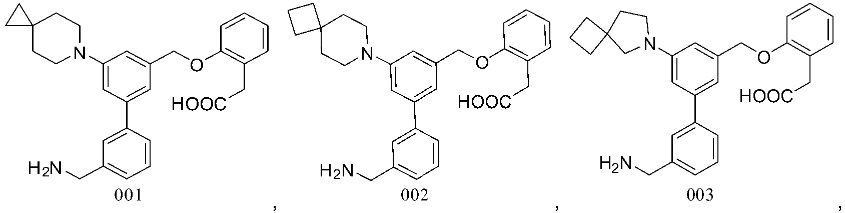

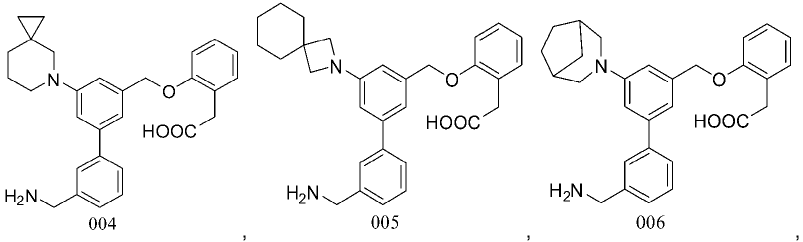

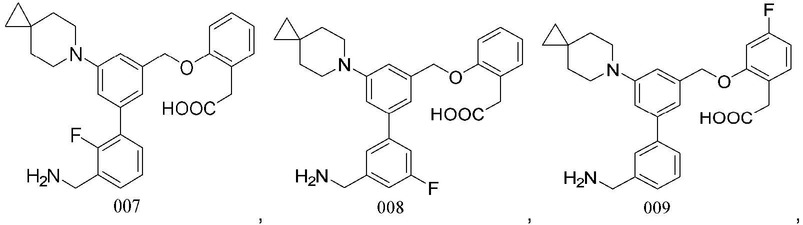

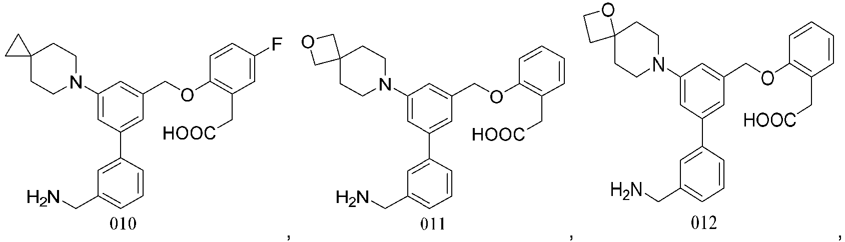

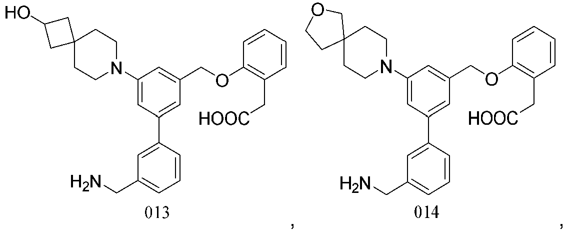

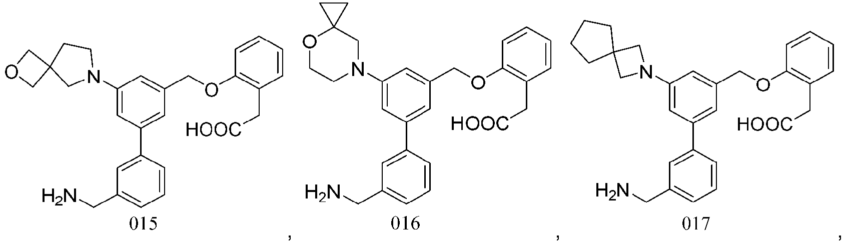

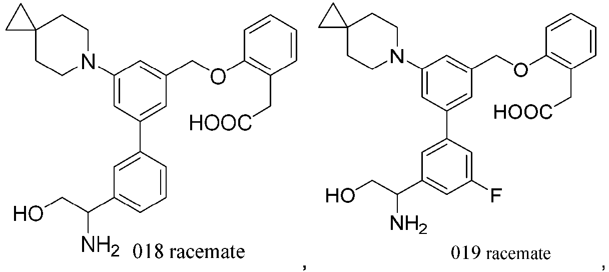

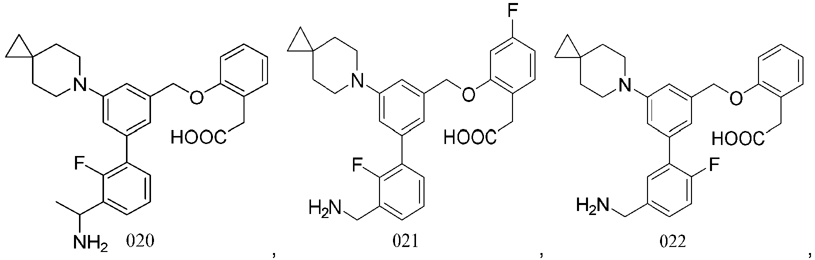

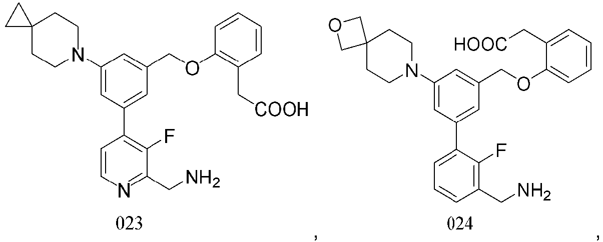

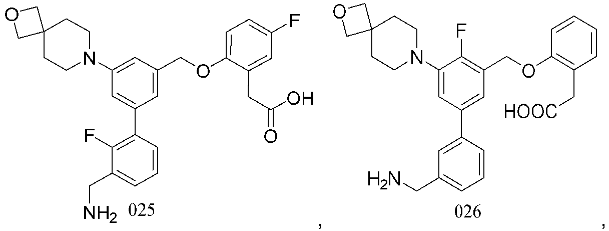

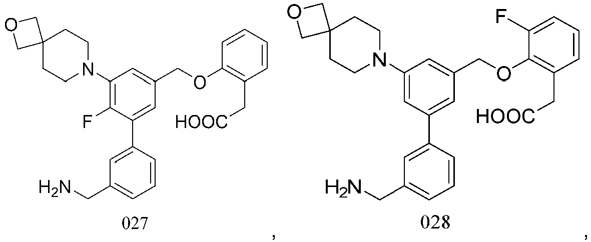

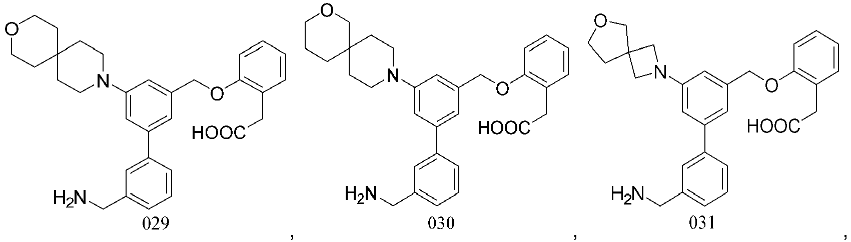

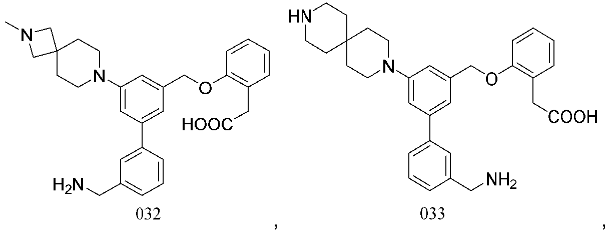

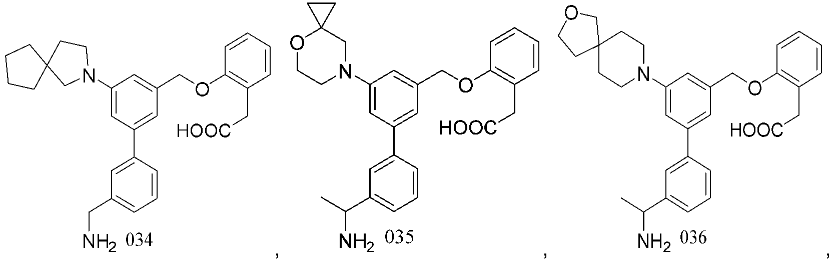

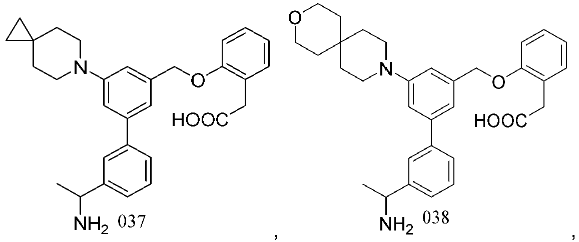

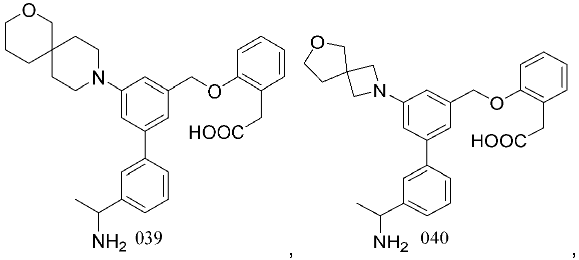

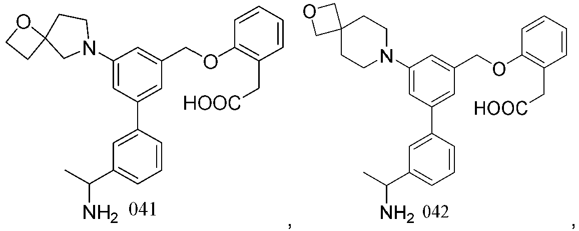

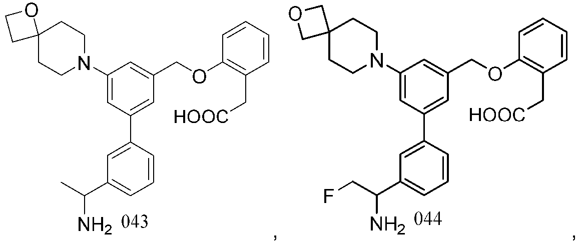

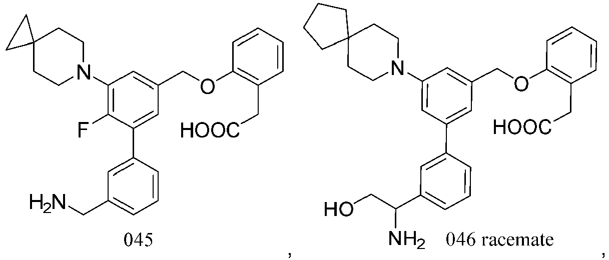

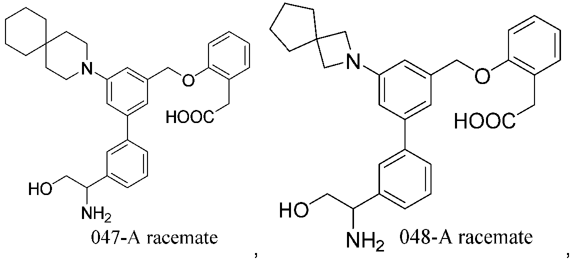

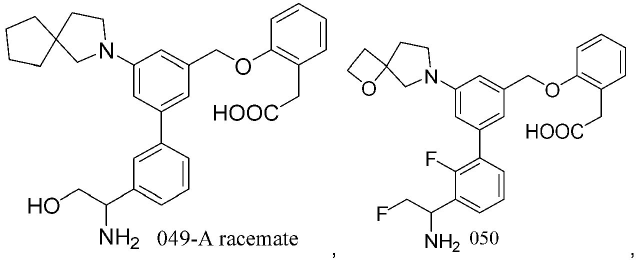

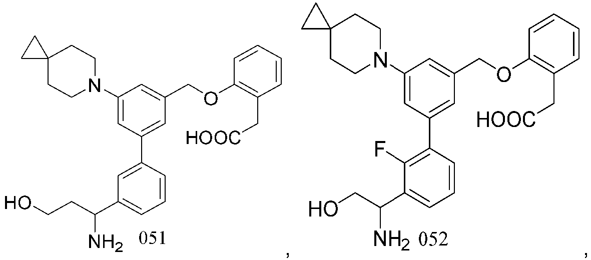

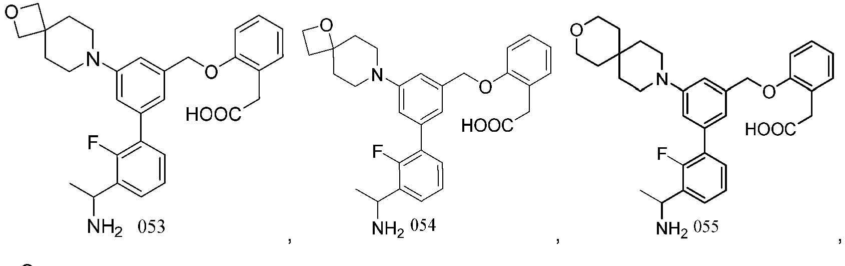

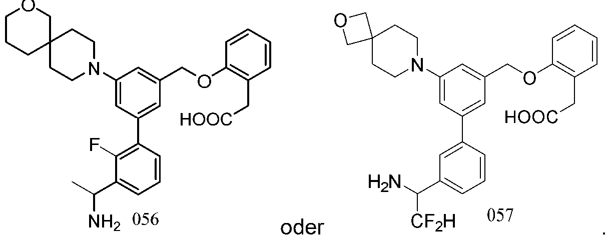

10. Verbindung der Formel (I), das Tautomer davon, das Stereoisomer davon oder das pharmazeutisch akzeptable Salz eines der Vorstehenden oder das Solvat eines der Vorstehenden gemäß Anspruch 9, wobei die Verbindung der Formel (I) aus einer der folgenden Verbindungen ausgewählt ist: 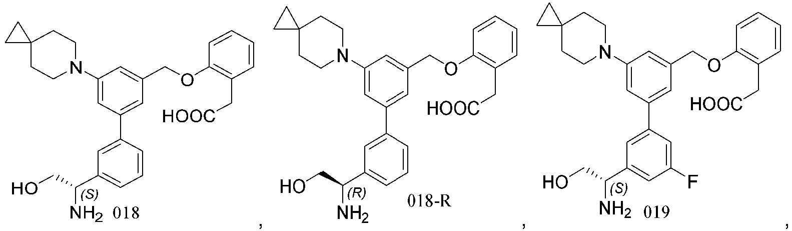 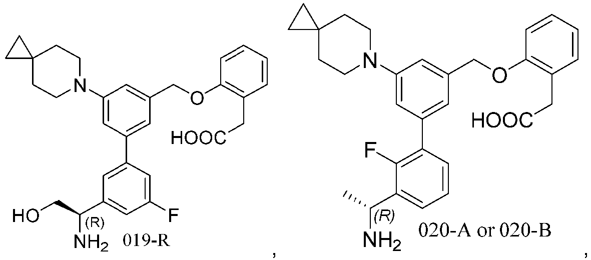 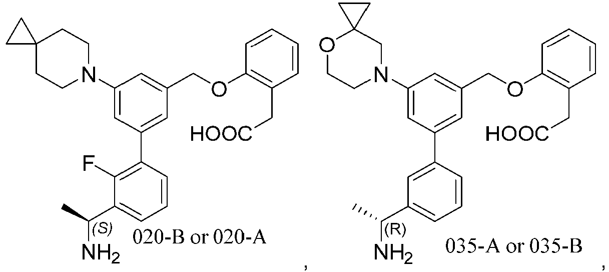 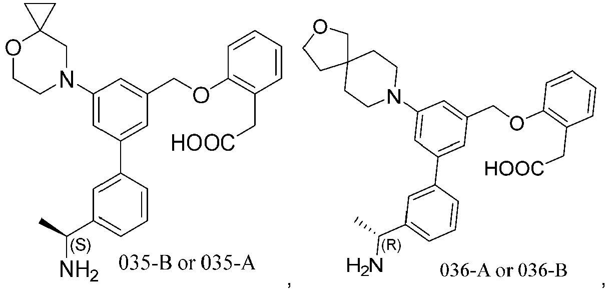 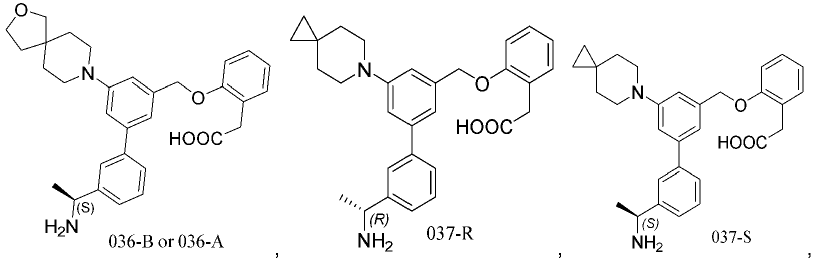 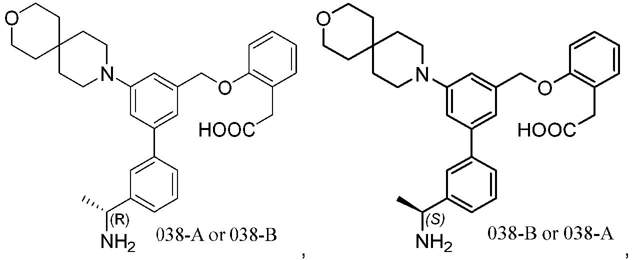 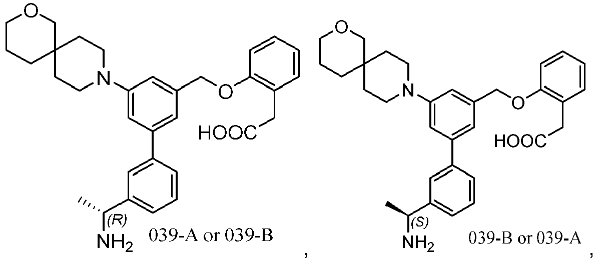 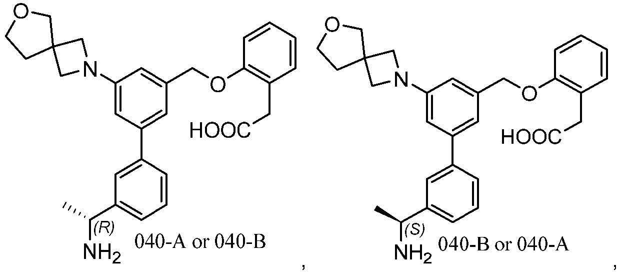 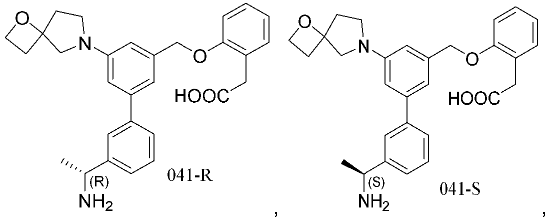 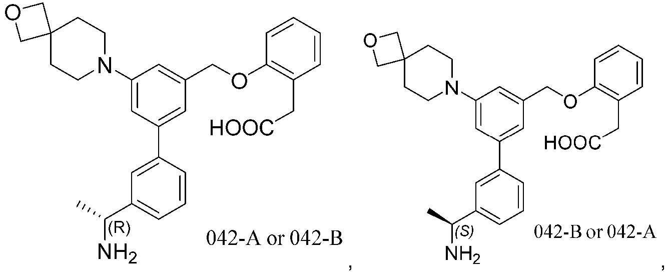 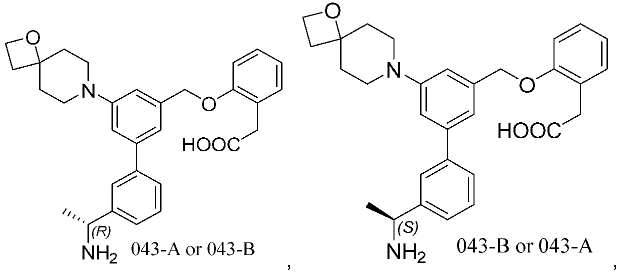 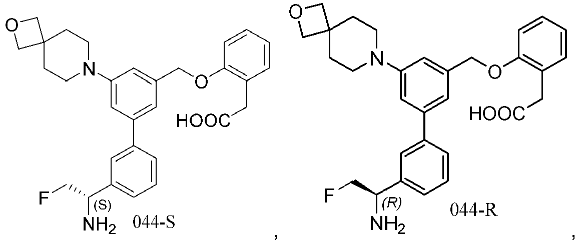 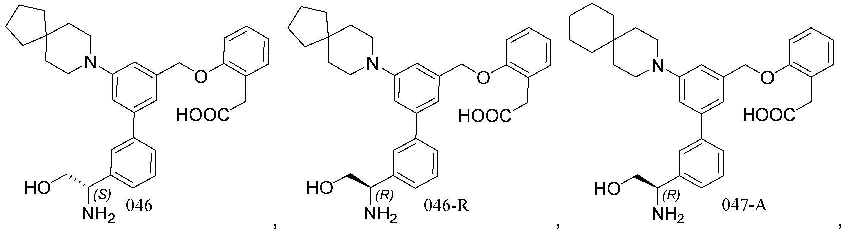 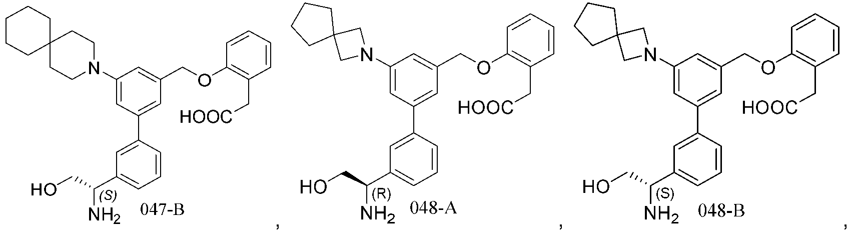 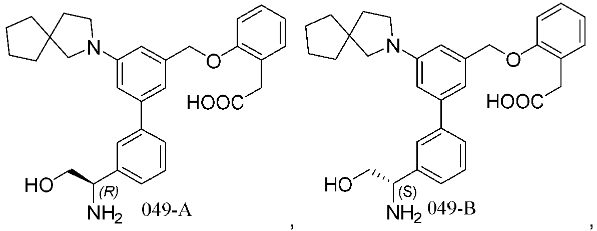 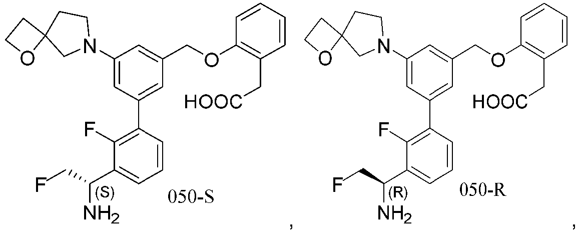 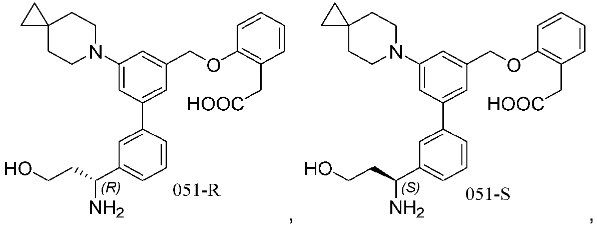 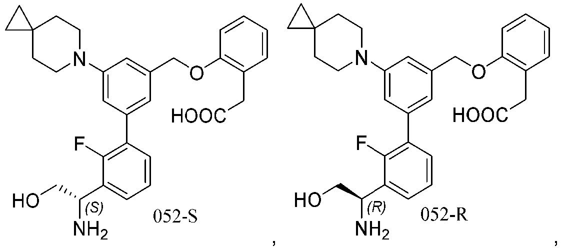 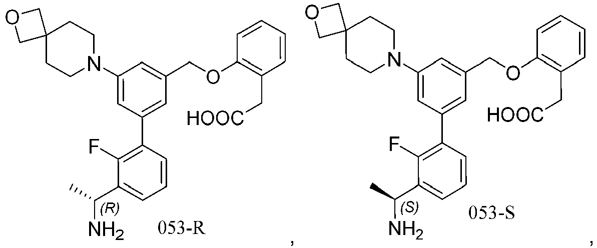 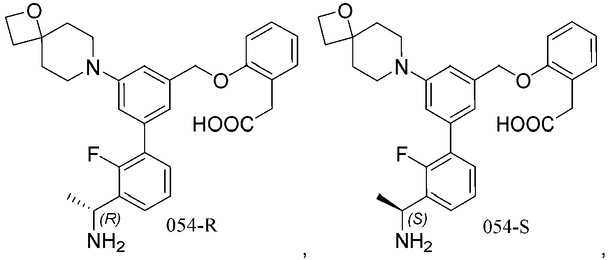 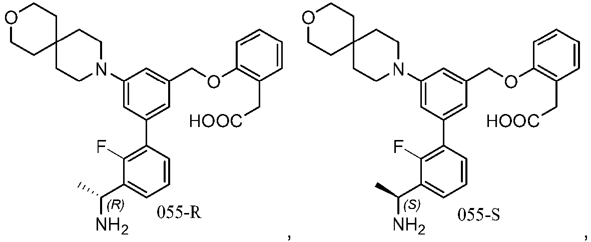 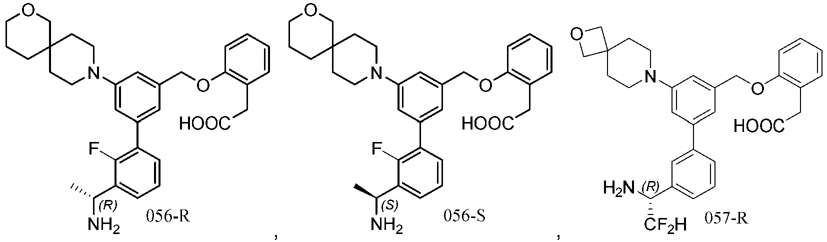 oder

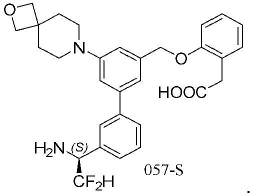

11. Ein Herstellungsverfahren für eine Verbindung der Formel (I), das die folgenden Schritte beinhaltet:
(1) Unterziehen einer Verbindung der Formel II-3 einer Entschützungsreaktion, um eine Verbindung der Formel II-4 zu erhalten; 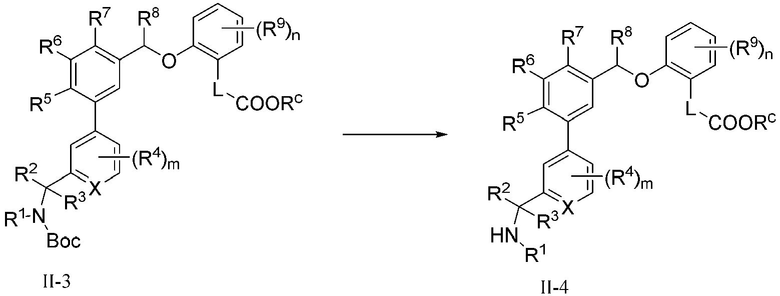
(2) Unterziehen der Verbindung der Formel II-4 einer Hydrolysereaktion, um die Verbindung der Formel (I) zu erhalten; 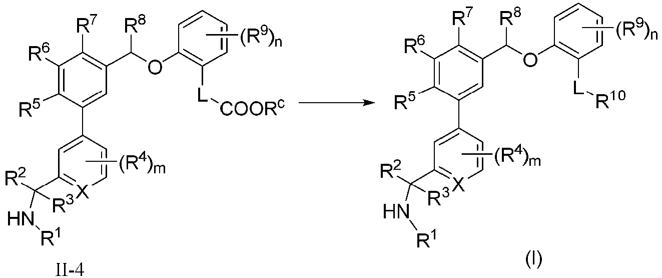 wobei R¹⁰ -COOH ist, R^{c} C₁₋₆-Alkyl oder C₁₋₆-Alkyl, das durch 1, 2 oder 3 R^{c-1} substituiert ist, ist; R^{c-1} jeweils unabhängig Halogen, -OH oder -C(=O)OC(CH₃)₃ ist und R¹, R², R³, R⁴, R⁵, R⁶, R⁷, R⁸, R⁹, X, L, m und n wie in mindestens einem der Ansprüche 1 bis 10 definiert sind; vorzugsweise R¹ H ist.

12. Verbindung der Formel II-3 oder Formel II-4: 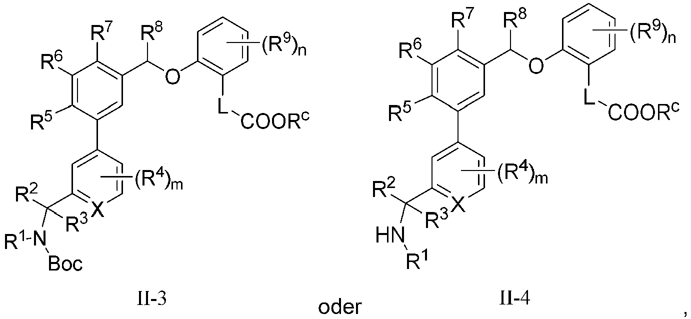
wobei R^{c} C₁₋₆-Alkyl oder C₁₋₆-Alkyl, das durch 1, 2 oder 3 R^{c-1} substituiert ist, ist; R^{c-1} jeweils unabhängig Halogen, -OH oder -C(=O)OC(CH₃)₃ ist;
R¹, R², R³, R⁴, R⁵, R⁶, R⁷, R⁸, R⁹, X, L, m und n wie in mindestens einem der Ansprüche 1 bis 10 definiert sind; vorzugsweise R¹ H ist.

13. Verbindung gemäß Anspruch 12, wobei die Verbindung aus einer der folgenden Verbindungen ausgewählt ist: 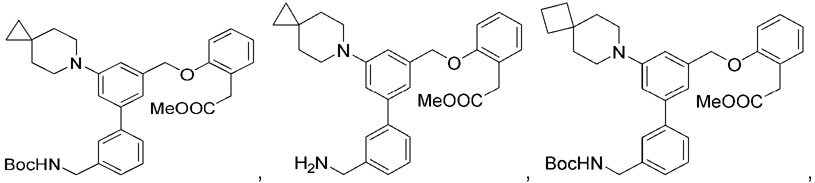 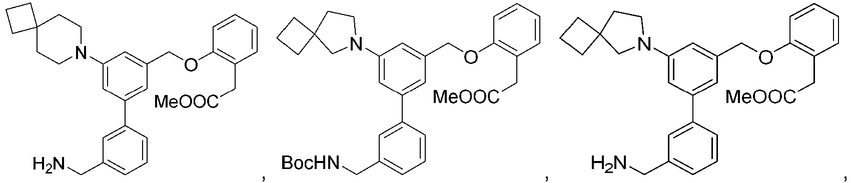 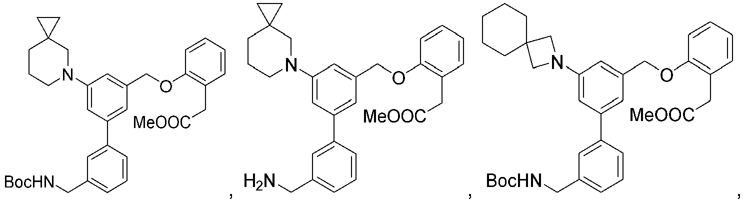 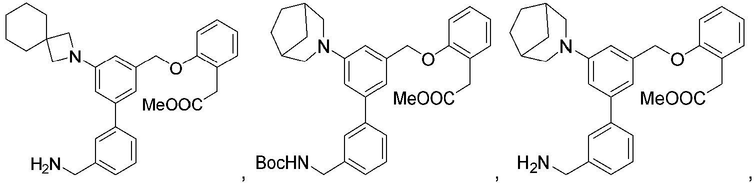 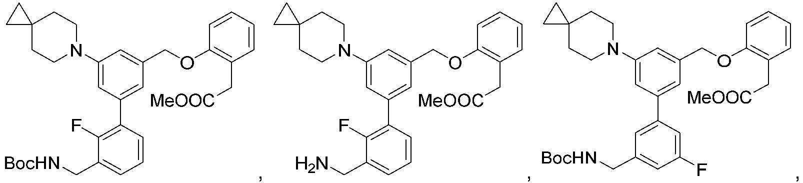 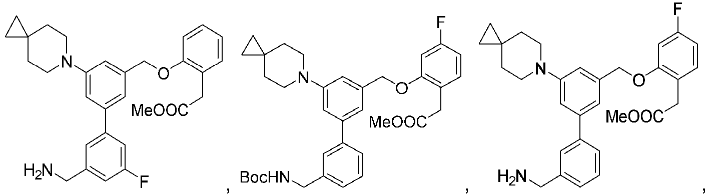 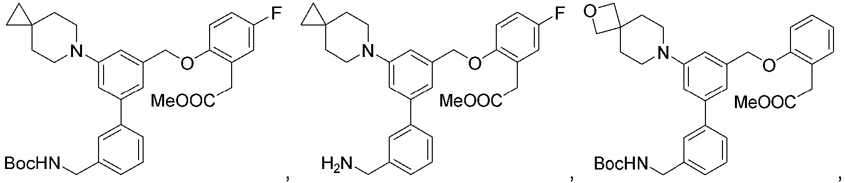 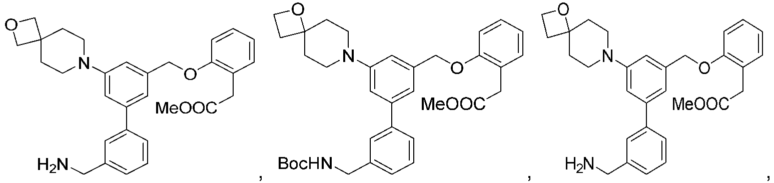 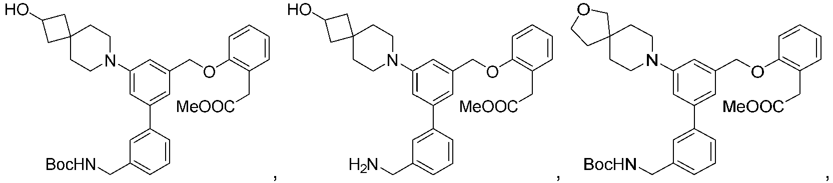 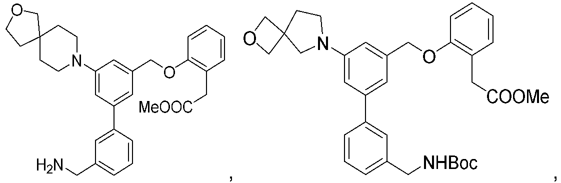 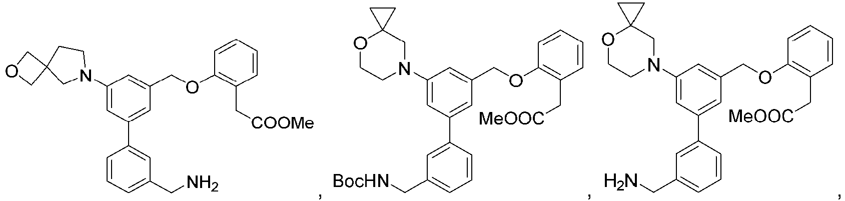 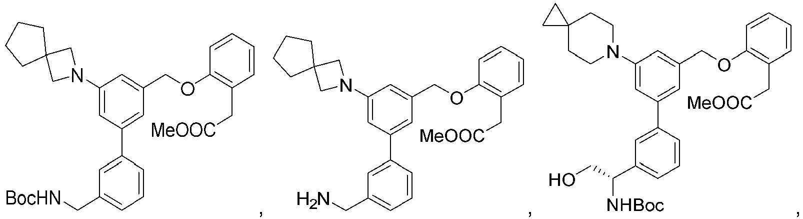 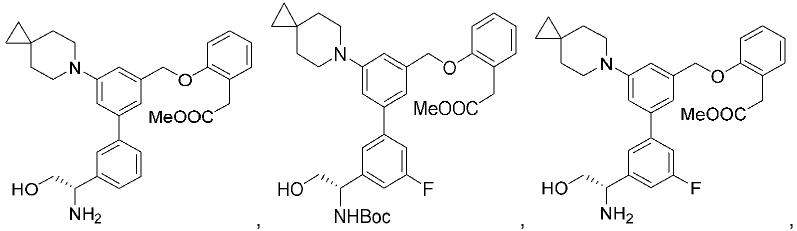 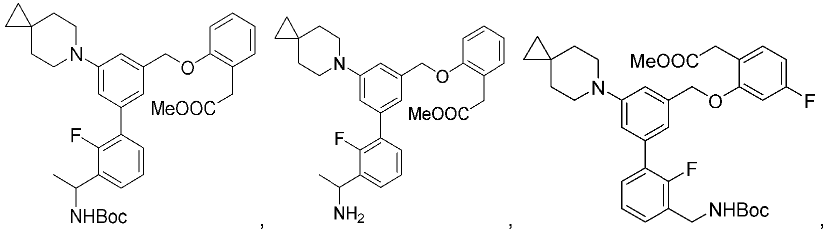 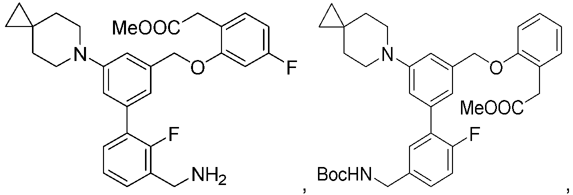 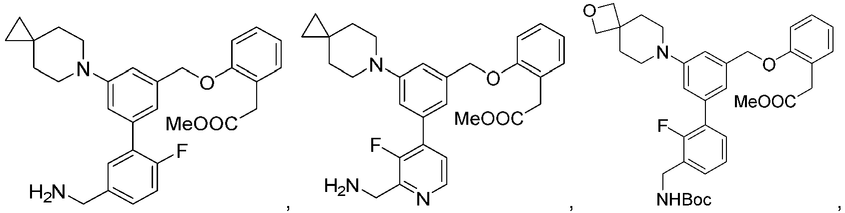 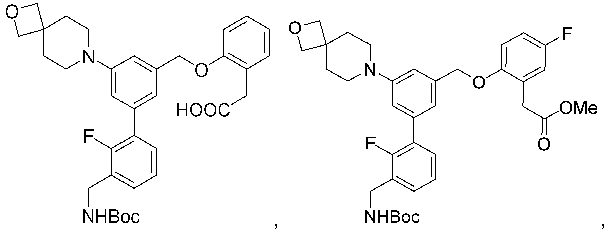 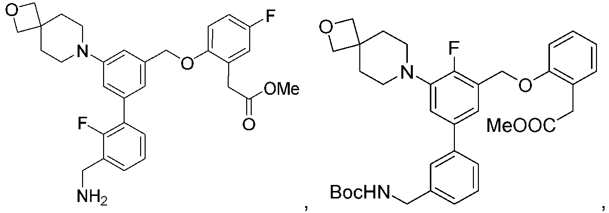 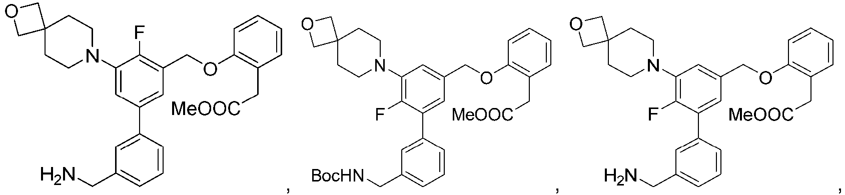 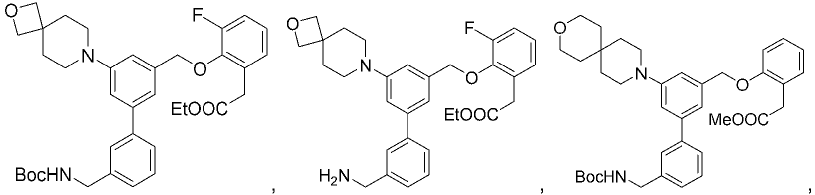 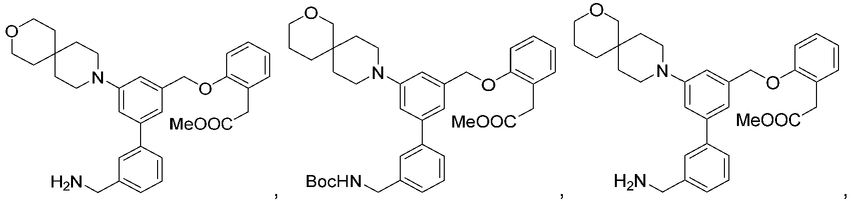 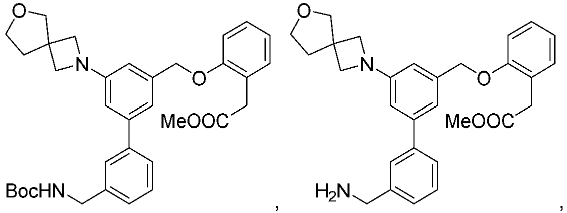 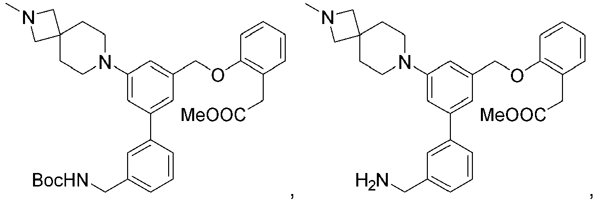 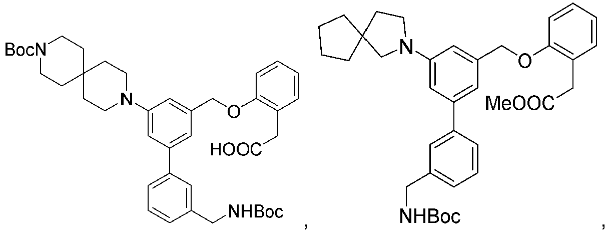 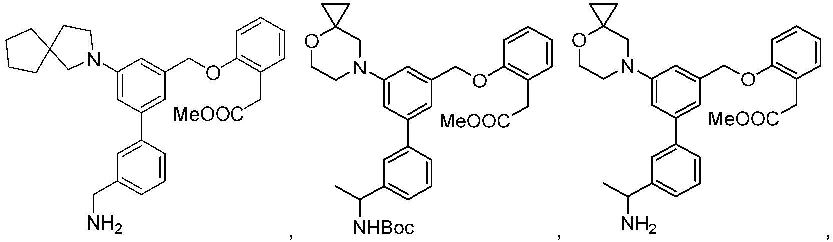 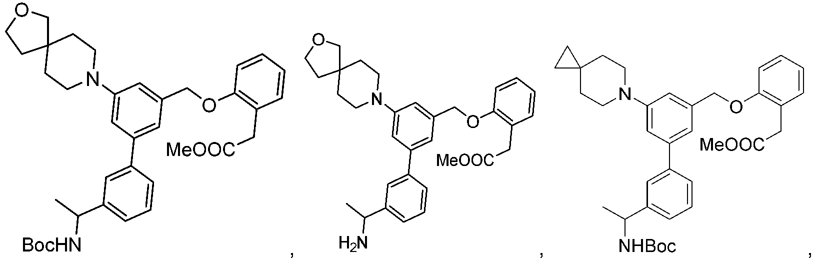 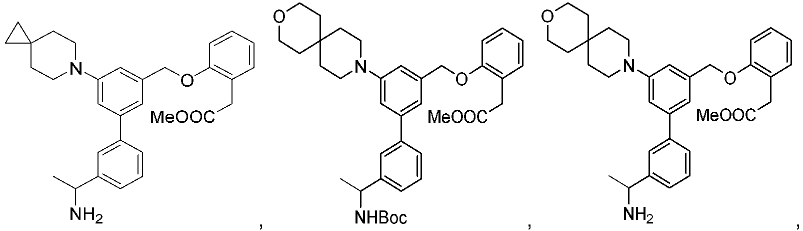 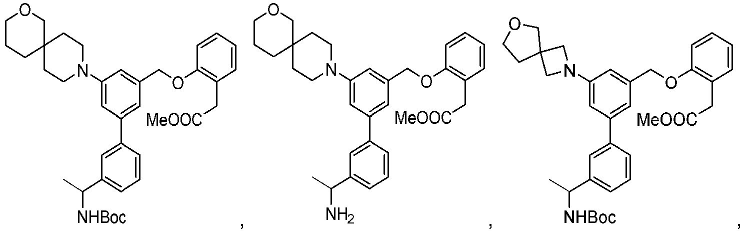 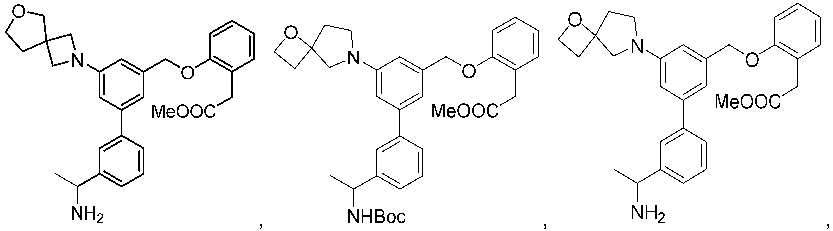 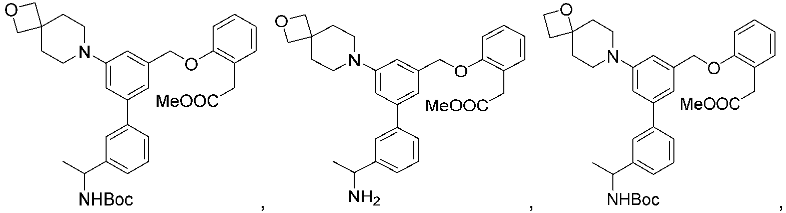 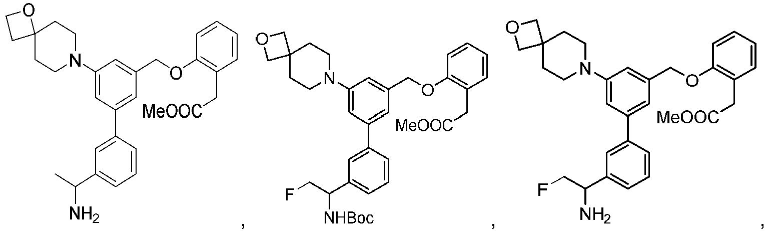 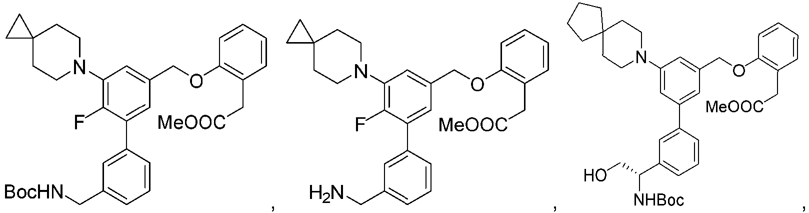 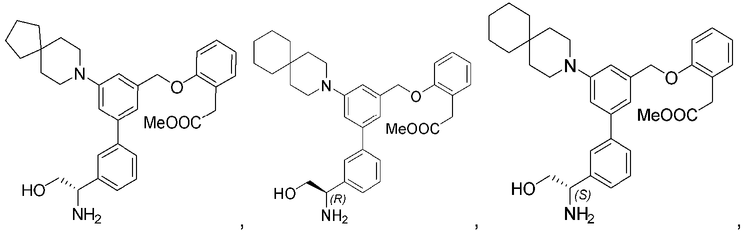 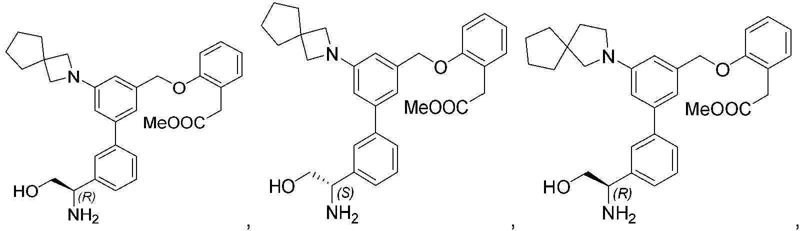 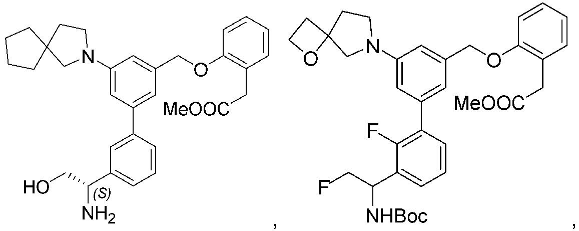 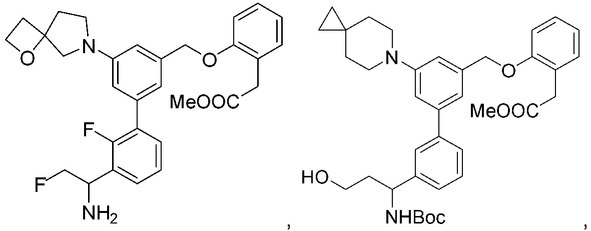 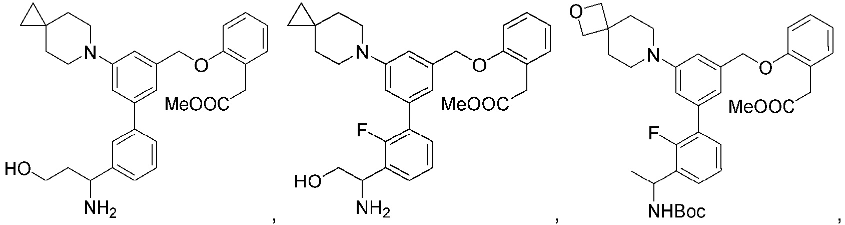 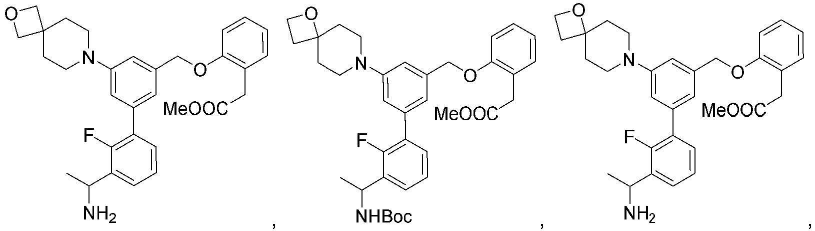 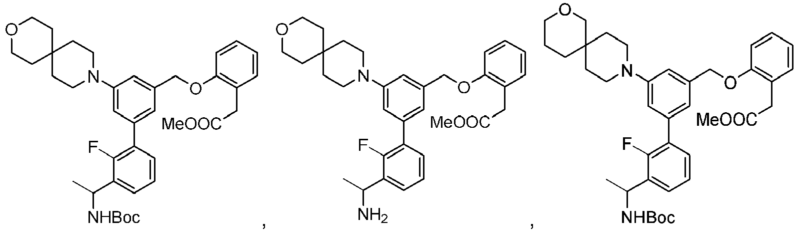 oder

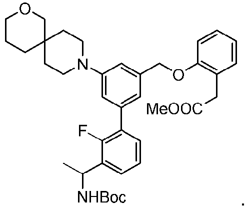

14. Eine pharmazeutische Zusammensetzung, die Folgendes beinhaltet:
(1) die Verbindung der Formel (I), das Tautomer davon, das Stereoisomer davon oder das pharmazeutisch akzeptable Salz eines der Vorstehenden oder das Solvat eines der Vorstehenden gemäß mindestens einem der Ansprüche 1 bis 10; und
(2) einen pharmazeutisch akzeptablen Träger.

15. Verbindung der Formel (I), das Tautomer davon, das Stereoisomer davon oder das pharmazeutisch akzeptable Salz eines der Vorstehenden oder das Solvat eines der Vorstehenden gemäß mindestens einem der Ansprüche 1 bis 10 oder die pharmazeutische Zusammensetzung gemäß Anspruch 14 zur Verwendung als ein Komplementfaktor-D-Inhibitor-Medikament oder ein Medikament zur Behandlung und/oder Prävention einer durch Komplementfaktor D vermittelten Krankheit, wobei es sich bei der durch Komplementfaktor D vermittelten Krankheit um eine hämatologische Störung, eine Nierenkrankheit, eine Herz-Kreislauf-Krankheit, eine immunologische Störung, eine Krankheit des Zentralnervensystems, eine Atemwegskrankheit, eine Krankheit des Urogenitalsystems oder eine Augenstörung handelt; vorzugsweise es sich bei der durch Komplementfaktor D vermittelten Krankheit um Kälteagglutininkrankheit, katastrophales Antiphospholipid-Syndrom, hämolytische Anämie, mit antineutrophilen zytoplasmatischen Antikörpern assoziierte Vaskulitis, warme autoimmunhämolytische Anämie, paroxysmale nächtliche Hämoglobinurie, IgA-Nephritis, Lupus-Nephritis, atypisches hämolytisch-urämisches Syndrom, membranoproliferative Glomerulonephritis, Dense-Deposit-Krankheit, C3-Glomerulonephritis, fokal segmentale Glomerulosklerose, diabetische Nephropathie, systemischen Lupus oder Lupus erythematodes, rheumatoide Arthritis, chronischentzündliche Darmerkrankung, Psoriasis, multiple Sklerose, Organtransplantatabstoßung, Myasthenia gravis, Alzheimer-Krankheit, Atemnotsyndrom, Asthma, chronisch obstruktive Lungenerkrankung, Emphysem, Coronavirusinfektion, Makuladegeneration, altersbedingte Makuladegeneration, Makulaödem, diabetisches Makulaödem, choroidale Neovaskularisation, Uveitis, Behcet-Uveitis, proliferative diabetische Retinopathie, nichtproliferative diabetische Retinopathie, Glaukom, hypertensive Retinopathie, Hornhautneovaskularisationskrankheit, posthornhautige Transplantatabstoßung, Hornhautdystrophiekrankheit, autoimmune Trockenaugenkrankheit, Stevens-Johnson-Syndrom, Sjögren-Syndrom, Umwelttrockenaugenkrankheit, Fuchs-Endotheldystrophie, retinalen Venenverschluss oder postoperative Entzündung handelt; vorzugsweise es sich bei der Coronavirusinfektion um SARS-CoV-Infektion, MERS-CoV-Infektion oder SARS-CoV-2-Infektion handelt.

## Revendications

1. Un composé de formule (I), un tautomère de celui-ci, un stéréoisomère de celui-ci, ou un sel pharmaceutiquement acceptable de l'un quelconque des précédents, ou un solvate de l'un quelconque des précédents : 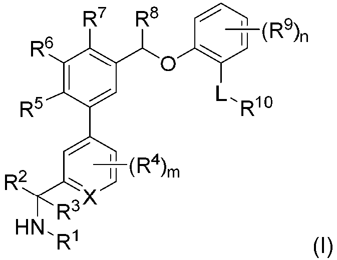 où
R¹ est H, D, un alkyle en C₁₋₆, ou un alkyle en C₁₋₆ substitué par 1, 2, ou 3 R¹⁻¹ ; chaque R¹⁻¹ est indépendamment un halogène, -CN, -OH, un alcoxy en C₁₋₆, ou -NH₂ ;
R² et R³ sont chacun indépendamment H, D, un halogène, un alkyle en C₁₋₆, ou un alkyle en C₁₋₆ substitué par 1, 2, ou 3 R²⁻¹ ; chaque R²⁻¹ est indépendamment un halogène, -CN, -OH, un alcoxy en C₁₋₆, ou -NH₂ ;
R⁴ est H ou un halogène ; m est 0, 1, 2, ou 3 ;
R⁵ et R⁷ sont chacun indépendamment H, un halogène, -CN, un alkyle en C₁₋₆, ou un alcoxy en C₁₋₆ ;
R⁶ est un hétérocycloalkyle de 8 à 11 chaînons avec 1, 2, ou 3 hétéroatomes sélectionnés parmi 1, 2, ou 3 types de N, O, et S, ou un hétérocycloalkyle de 8 à 11 chaînons avec 1, 2, ou 3 hétéroatomes sélectionnés parmi 1, 2, ou 3 types de N, O, et S substitués par 1, 2, ou 3 R⁶⁻² ;
R⁶⁻² sont chacun indépendamment un hydroxyle, un oxo (=O), un halogène, un alkyle en C₁₋₆, un alcoxy en C₁₋₆, un haloalkyle en C₁₋₆, un haloalcoxy en C₁₋₆, un cycloalkyle en C₃₋₆, ou un -(CH₂)ₚ-cycloalkyle en C₃₋₆ ; p est 1, 2, 3, ou 4 ;
dans R⁶⁻², le cycloalkyle est indépendamment un cycle monocyclique, un cycle ponté, ou un cycle spiro ;
dans R⁶, l'hétérocycloalkyle est un cycle ponté, ou un cycle spiro ;
R⁸ est H, un halogène, ou un alkyle en C₁₋₆ ;
R⁹ est H, un halogène, un alkyle en C₁₋₆, ou un alkyle en C₁₋₆ substitué par 1, 2, ou 3 R⁹⁻¹ ;
chaque R⁹⁻¹ est indépendamment un halogène, -CN, -OH, ou -NH₂ ; n est 0, 1, 2, 3, ou 4 ;
L est -(CR^{a}R^{b})_{q}- ; q est 0, 1, 2, ou 3 ;
R^{a} et R^{b} sont chacun indépendamment H, D, ou un halogène, ou R^{a} et R^{b} sont reliés ensemble pour former un cycloalkylène en C₃₋₆, et le cycloalkylène formé est un cycle monocyclique, un cycle ponté, ou un cycle spiro ;
R¹⁰ est -COOH ;
X est CR^{d} ou N ; R^{d} est H, un halogène, ou un alkyle en C₁₋₆.

2. Le composé de formule (I), le tautomère de celui-ci, le stéréoisomère de celui-ci, ou le sel pharmaceutiquement acceptable de l'un quelconque des précédents, ou le solvate de l'un quelconque des précédents selon la revendication 1, où le composé de formule (I) répond à une ou plus d'une des conditions suivantes :
(1) R¹ est H ;
(2) R² et R³ sont chacun indépendamment H, un alkyle en C₁₋₃, ou un alkyle en C₁₋₃ substitué par 1, 2, ou 3 R²⁻¹ ; chaque R²⁻¹ est indépendamment un halogène ou -OH, et l'halogène est de préférence F ;
(3) m est 0 ou 1 ;
(4) R⁵ et R⁷ sont chacun indépendamment H ou un halogène, et l'halogène est de préférence F ;
(5) dans R⁶, l'hétérocycloalkyle de 8 à 11 chaînons est le 6-azaspiro[2,5]octyle, le 5-azaspiro[2,5]octyle, le 6-azaspiro[3,4]octyle, le 2-azaspiro[3,4]octyle, le 2-oxa-6-azaspiro[3,4]octyle, le 6-oxa-2-azaspiro[3,4]octyle, le 4-oxa-7-azaspiro[2,5]octyle, le 2-azaspiro[4,4]nonyle, le 2-azaspiro[3,5]nonyle, le 2-oxa-7-azaspiro[3,5]nonyle, le 1-oxa-7-azaspiro[3,5]nonyle, le 7-azaspiro[3,5]nonyle, le 2,7-diazaspiro[3,5]nonyle, le 2-oxa-8-azaspiro[4,5]décyle, le 3-oxa-9-azaspiro[5,5]undécyle, le 2-oxa-9-azaspiro[5,5]undécyle, le 3,9-diazaspiro[5,5]undécyle, le 3-azabicyclo[3,2,1]octyle, le 3-azaspiro[5,5]undécyle, le 8-azaspiro[4,5]décyle, ou le 1-oxa-6-azaspiro[3,4]octyle ;
(6) chaque R⁶⁻² est indépendamment un hydroxyle ou un alkyle en C₁₋₃, de préférence, chaque R⁶⁻² est indépendamment un hydroxyle, un méthyle, un éthyle, un n-propyle, ou un isopropyle ;
(7) R⁸ est H ;
(8) R⁹ est H ou un halogène, et l'halogène est de préférence F ;
(9) n est 0 ou 1 ;
(10) q est 1 ;
(11) R^{a} et R^{b} sont chacun indépendamment H ;
(12) R^{d} est H.

3. Le composé de formule (I), le tautomère de celui-ci, le stéréoisomère de celui-ci, ou le sel pharmaceutiquement acceptable de l'un quelconque des précédents, ou le solvate de l'un quelconque des précédents selon la revendication 1, où
R⁶ est un hétérocycloalkyle de 8 à 10 chaînons avec 1, 2, ou 3 hétéroatomes sélectionnés parmi 1, 2, ou 3 types de N, O, et S, ou un hétérocycloalkyle de 8 à 10 chaînons avec 1, 2, ou 3 hétéroatomes sélectionnés parmi 1, 2, ou 3 types de N, O, et S substitués par 1, 2, ou 3 R⁶⁻².

4. Le composé de formule (I), le tautomère de celui-ci, le stéréoisomère de celui-ci, ou le sel pharmaceutiquement acceptable de l'un quelconque des précédents, ou le solvate de l'un quelconque des précédents selon la revendication 1, où R¹ est H ;
R² et R³ sont chacun indépendamment H, un alkyle en C₁₋₃, ou un alkyle en C₁₋₃ substitué par 1, 2, ou 3 R²⁻¹ ; chaque R²⁻¹ est indépendamment un halogène ou -OH ; R⁴ est H ou un halogène ; m est 0 ou 1 ;
R⁵ et R⁷ sont chacun indépendamment H ou un halogène ;
R⁶ est un hétérocycloalkyle de 8 à 11 chaînons avec 1, 2, ou 3 hétéroatomes sélectionnés parmi 1, 2, ou 3 types de N, O, et S, ou un hétérocycloalkyle de 8 à 11 chaînons avec 1, 2, ou 3 hétéroatomes sélectionnés parmi 1, 2, ou 3 types de N, O, et S substitués par 1, 2, ou 3 R⁶⁻², où l'hétérocycloalkyle est un cycle ponté ou un cycle spiro ;
chaque R⁶⁻² est indépendamment un hydroxyle ou un alkyle en C₁₋₃ ;
R⁸ est H ;
R⁹ est H ou un halogène ; n est 0 ou 1 ;
L est -(CR^{a}R^{b})_{q}-, q est 1 ; R^{a} et R^{b} sont chacun indépendamment H ;
R¹⁰ est -COOH ;
X est CR^{d} ou N ; R^{d} est H.

5. Le composé de formule (I), le tautomère de celui-ci, le stéréoisomère de celui-ci, ou le sel pharmaceutiquement acceptable de l'un quelconque des précédents, ou le solvate de l'un quelconque des précédents selon la revendication 1, où le composé de formule (I) répond à une ou plus d'une des conditions suivantes :
(1) dans R¹, R², R³, R⁵, R⁷, R⁶⁻², R⁸, R⁹, et R^{d}, chaque alkyle est indépendamment un méthyle, un éthyle, un n-propyle, un isopropyle, un n-butyle, un isobutyle, ou un tert-butyle, de préférence un méthyle ou un éthyle ;
(2) dans R¹⁻¹, R²⁻¹, R⁵, R⁷, et R⁶⁻², chaque alcoxy est indépendamment un méthoxy, un éthoxy, un n-propoxy, un isopropoxy, un n-butoxy, un isobutoxy, ou un tert-butoxy ;
(3) dans R¹⁻¹, R², R³, R²⁻¹, R⁴, R⁵, R⁷, R⁶⁻², R⁸, R⁹, R⁹⁻¹, Ra, R^{b}, et R^{d}, chaque halogène est indépendamment F, CI, Br, ou I, de préférence F ;
(4) dans R⁶, l'hétérocycloalkyle est relié au parent par l'intermédiaire d'un atome N ; l'hétérocycloalkyle est de préférence

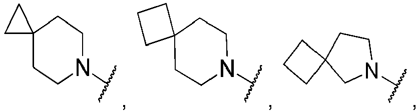

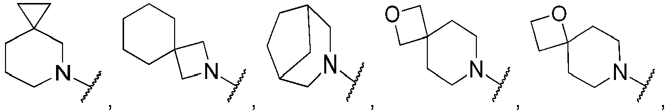

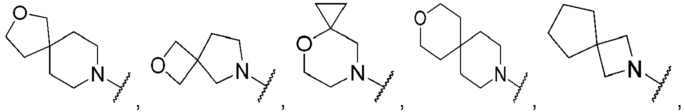

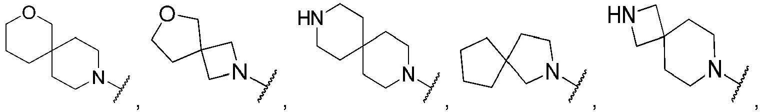

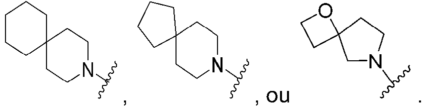

6. Le composé de formule (I), le tautomère de celui-ci, le stéréoisomère de celui-ci, ou le sel pharmaceutiquement acceptable de l'un quelconque des précédents, ou le solvate de l'un quelconque des précédents selon la revendication 1, où le composé de formule (I) répond à une ou plus d'une des conditions suivantes :
(1) R² est H, R³ est H, -CH₃, -CH₂OH, -CH₂CH₂OH, -CH₂F, ou -CF₂H ;
(2) R⁶ est

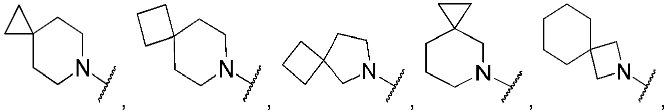

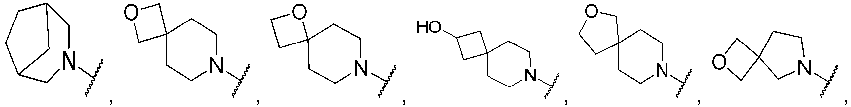

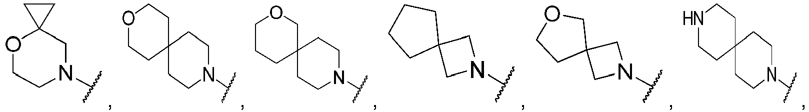

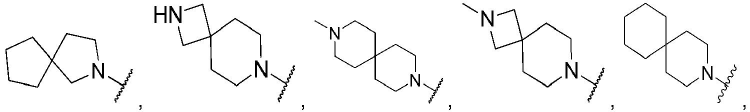

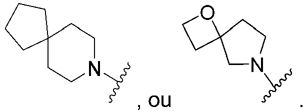

7. Le composé de formule (I), le tautomère de celui-ci, le stéréoisomère de celui-ci, ou le sel pharmaceutiquement acceptable de l'un quelconque des précédents, ou le solvate de l'un quelconque des précédents selon la revendication 1, où le composé de formule (I) répond à l'une quelconque des conditions suivantes :
(1) R¹ est H ;
R² est H, R³ est H, -CH₃, ou -CH₂F ;
R⁴ est H ou un halogène, m est 0 ou 1 ;
R⁵ et R⁷ sont chacun indépendamment H ou F ;
R⁶ est un hétérocycloalkyle de 8 à 11 chaînons avec 1, 2, ou 3 hétéroatomes sélectionnés parmi 1, 2, ou 3 types de N, O, et S, ou un hétérocycloalkyle de 8 à 11 chaînons avec 1, 2, ou 3 hétéroatomes sélectionnés parmi 1, 2, ou 3 types de N, O, et S substitués par 1, 2, ou 3 R⁶⁻² ;
chaque R⁶⁻² est indépendamment un hydroxyle ou un alkyle en C₁₋₃ ;
R⁸ est H ;
R⁹ est H ou F ; n est 0 ou 1 ;
L est -(CR^{a}R^{b})_{q}- ; q est 1 ; R^{a} et R^{b} sont chacun indépendamment H ;
R¹⁰ est -COOH ;
X est CR^{d} ou N ; R^{d} est H ;
(2) R¹ est H ;
R² est H, R³ est H, -CH₃, ou -CH₂F ;
R⁴ est H ou un halogène, m est 0 ou 1 ;
R⁵ et R⁷ sont chacun indépendamment H ou F ;
R⁶ est un hétérocycloalkyle de 8 à 10 chaînons avec 1, 2, ou 3 hétéroatomes sélectionnés parmi 1, 2, ou 3 types de N, O, et S, ou un hétérocycloalkyle de 8 à 10 chaînons avec 1, 2, ou 3 hétéroatomes sélectionnés parmi 1, 2, ou 3 types de N, O, et S substitués par 1, 2, ou 3 R⁶⁻² ;
chaque R⁶⁻² est indépendamment un hydroxyle ou un alkyle en C₁₋₃ ;
R⁸ est H ;
R⁹ est H ou F ; n est 0 ou 1 ;
L est -(CR^{a}R^{b})_{q}- ; q est 1 ; R^{a} et R^{b} sont chacun indépendamment H ;
R¹⁰ est -COOH ;
X est CR^{d} ou N ; R^{d} est H ;
(3) R¹ est H ;
R² est H, R³ est H, -CH₃, ou -CH₂F ;
R⁴ est H ou un halogène, m est 0 ou 1 ;
R⁵ et R⁷ sont chacun indépendamment H ou F ;
R⁶ est 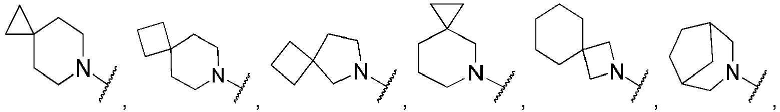 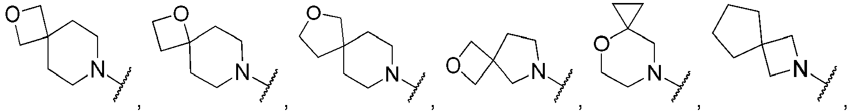 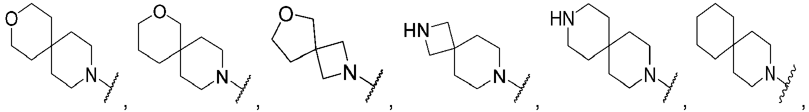 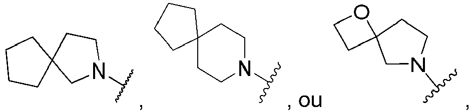 ou le groupe suivant substitué par 1
R⁶⁻² : 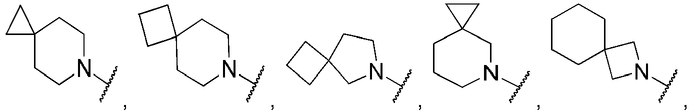 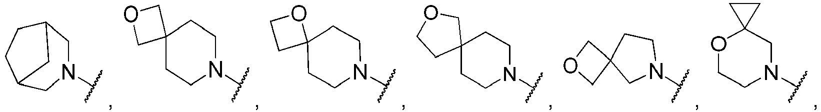 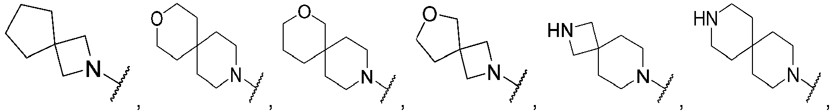 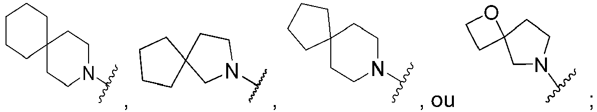
R⁶⁻² est un hydroxyle ou un méthyle ;
R⁸ est H ;
R⁹ est H ou F ; n est 0 ou 1 ;
L est -(CR^{a}R^{b})_{q}- ; q est 1 ; R^{a} et R^{b} sont chacun indépendamment H ;
R¹⁰ est -COOH ;
X est CR^{d} ou N ; R^{d} est H ;
(4) R¹ est H ;
R² est H, R³ est H, -CH₃, ou -CH₂F ;
R⁴ est H ou F, m est 0 ou 1 ;
R⁵ et R⁷ sont chacun indépendamment H ou F ;
R⁶ est 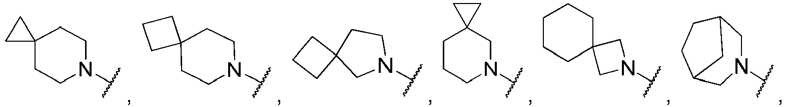 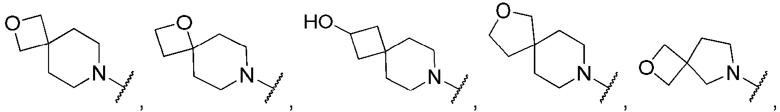 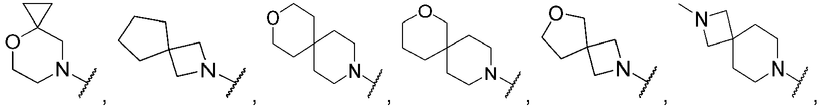 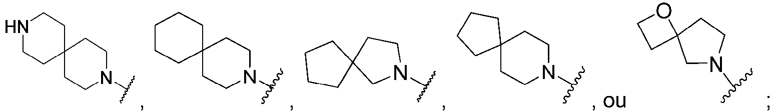
R⁸ est H ;
R⁹ est H ou F ; n est 0 ou 1 ;
L est -(CR^{a}R^{b})_{q}- ; q est 1 ; R^{a} et R^{b} sont chacun indépendamment H ;
R¹⁰ est -COOH ;
X est CR^{d} ou N ; R^{d} est H.

8. Le composé de formule (I), le tautomère de celui-ci, le stéréoisomère de celui-ci, ou le sel pharmaceutiquement acceptable de l'un quelconque des précédents, ou le solvate de l'un quelconque des précédents selon au moins une des revendications 1 à 7, où le composé de formule (I) a une structure de formule (I-1) à (I-4) : 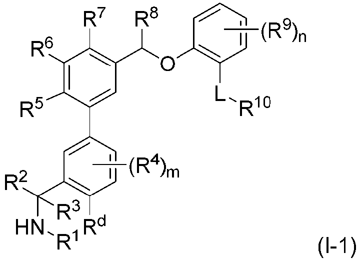
où R¹, R², R³, R⁴, R⁵, R⁶, R⁷, R⁸, R⁹, R¹⁰, R^{d}, L, m, et n sont définis comme dans au moins une des revendications 1 à 7 ; 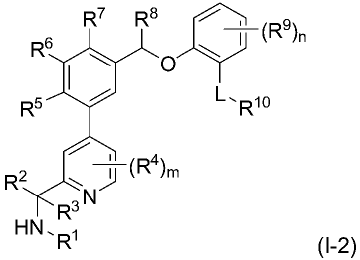
où R¹, R², R³, R⁴, R⁵, R⁶, R⁷, R⁸, R⁹, R¹⁰, L, m, et n sont définis comme dans au moins une des revendications 1 à 7 ; 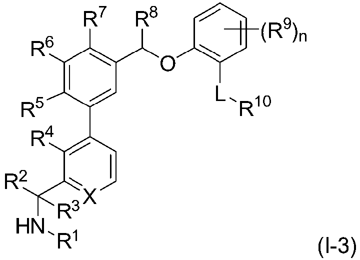
où X, R¹, R², R³, R⁴, R⁵, R⁶, R⁷, R⁸, R⁹, R¹⁰, L, et n sont définis comme dans au moins une des revendications 1 à 7 ; 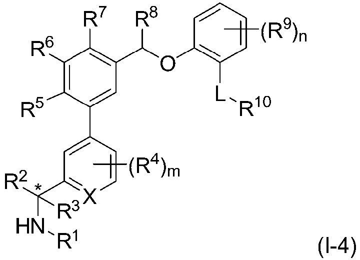
où X, R¹, R², R³, R⁴, R⁵, R⁶, R⁷, R⁸, R⁹, R¹⁰, L, m, et n sont définis comme dans au moins une des revendications 1 à 7 ; lorsqu'un atome de carbone marqué par « * » est un atome de carbone chiral, il représente une configuration R, une configuration S, ou un mélange de celles-ci.

9. Le composé de formule (I), le tautomère de celui-ci, le stéréoisomère de celui-ci, ou le sel pharmaceutiquement acceptable de l'un quelconque des précédents, ou le solvate de l'un quelconque des précédents selon la revendication 1, où le composé de formule (I) est sélectionné parmi l'un quelconque des composés suivants :

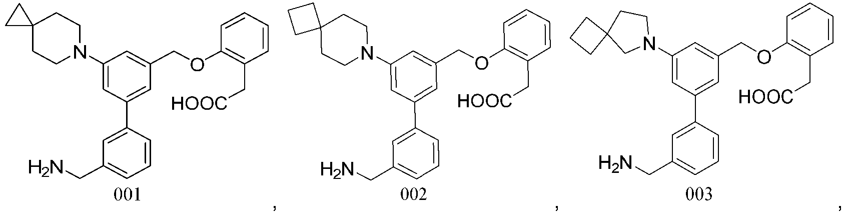

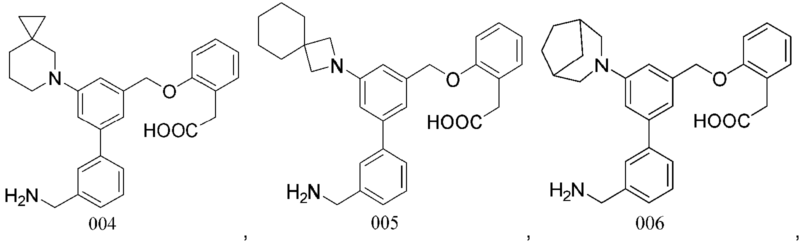

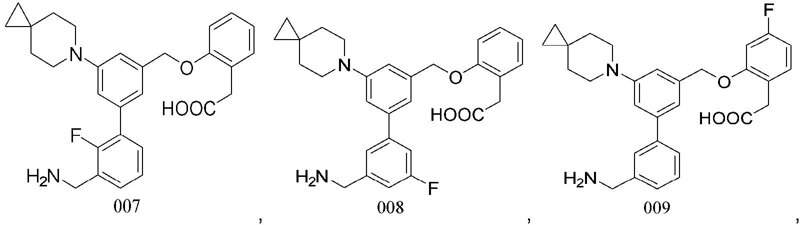

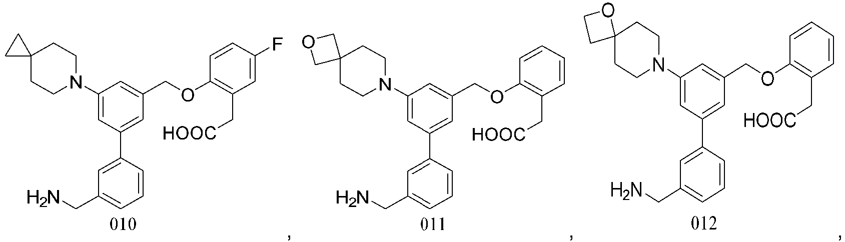

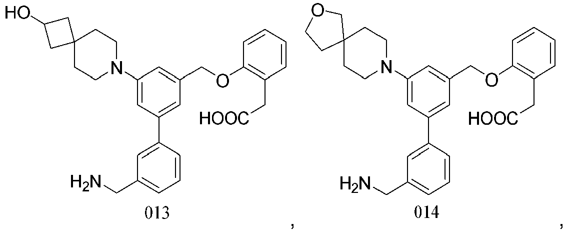

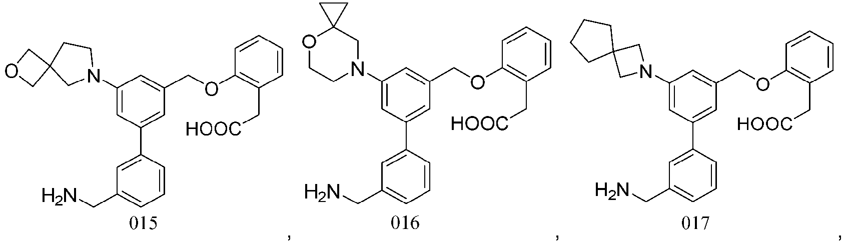

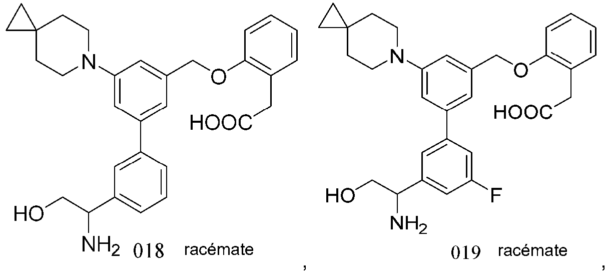

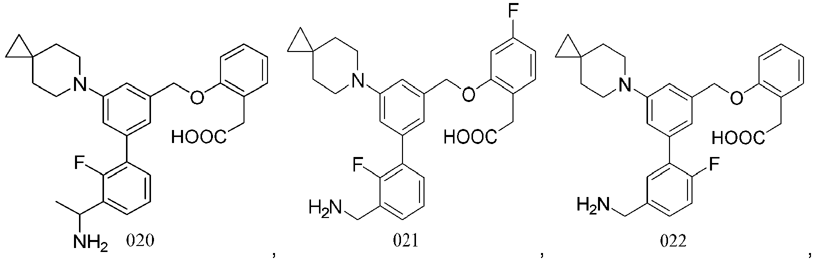

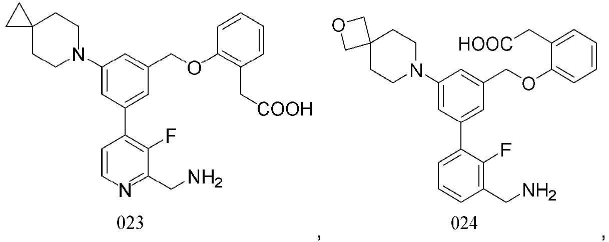

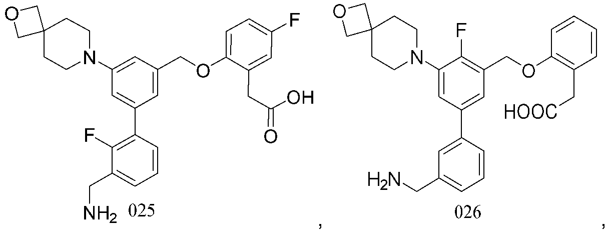

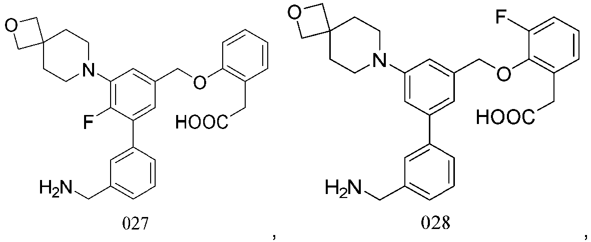

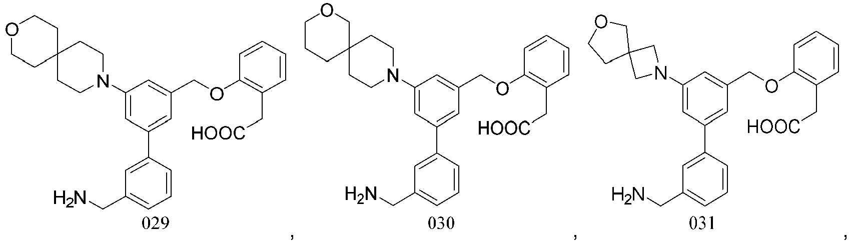

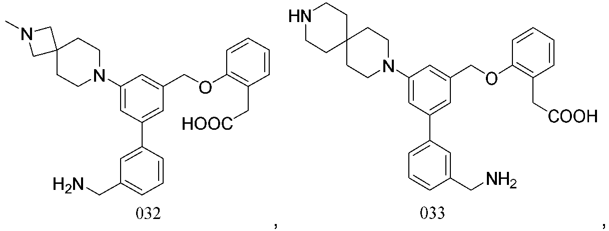

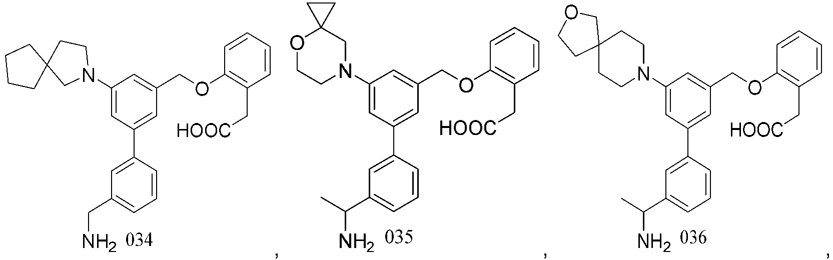

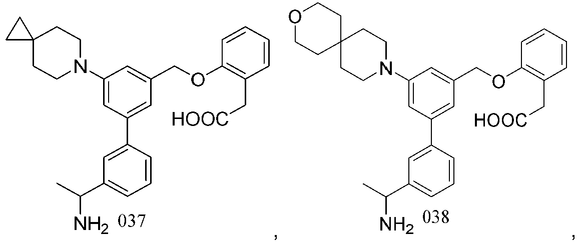

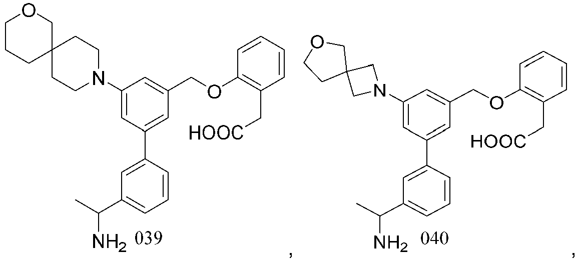

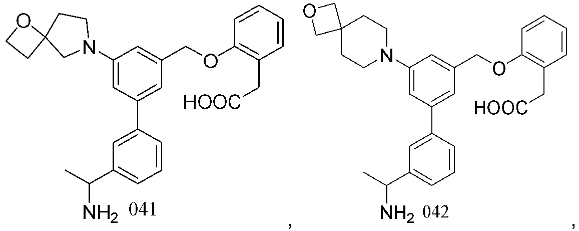

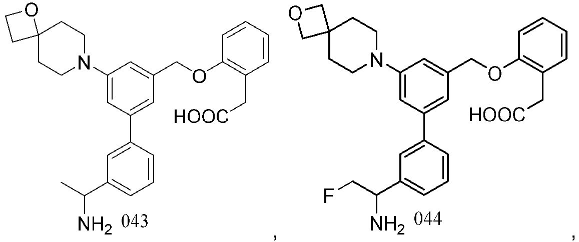

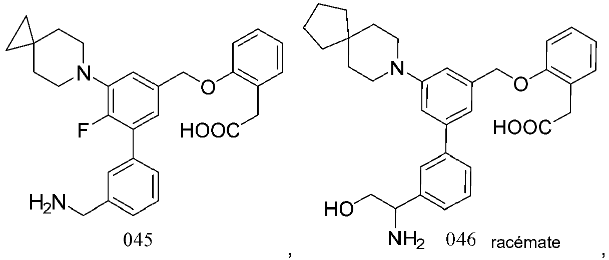

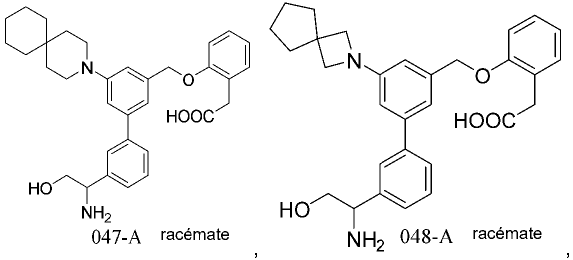

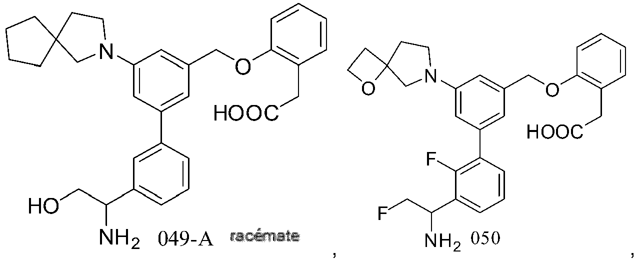

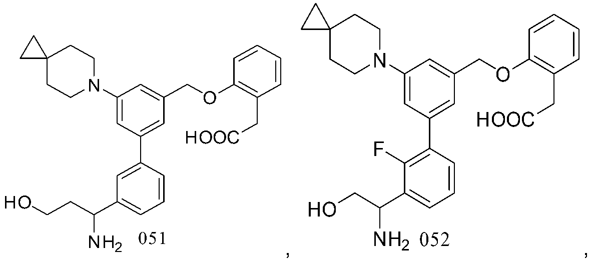

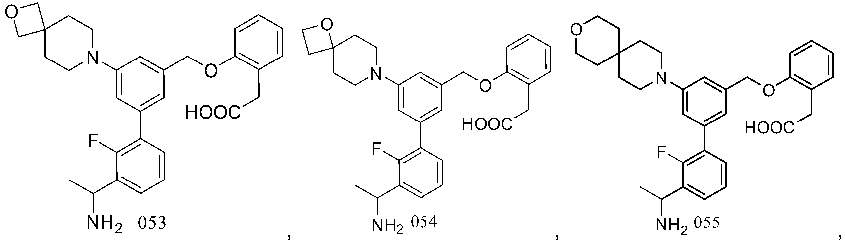

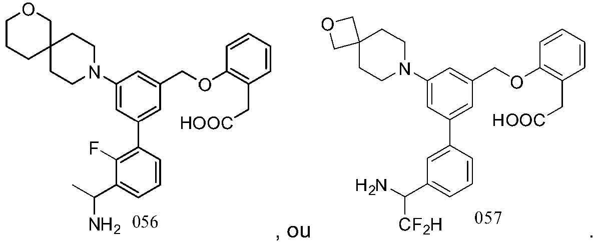

10. Le composé de formule (I), le tautomère de celui-ci, le stéréoisomère de celui-ci, ou le sel pharmaceutiquement acceptable de l'un quelconque des précédents, ou le solvate de l'un quelconque des précédents selon la revendication 9, où le composé de formule (I) est sélectionné parmi l'un quelconque des composés suivants : 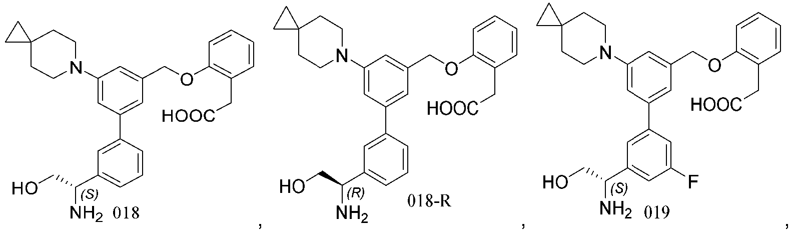 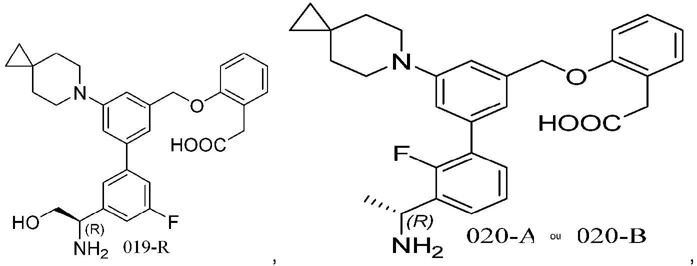 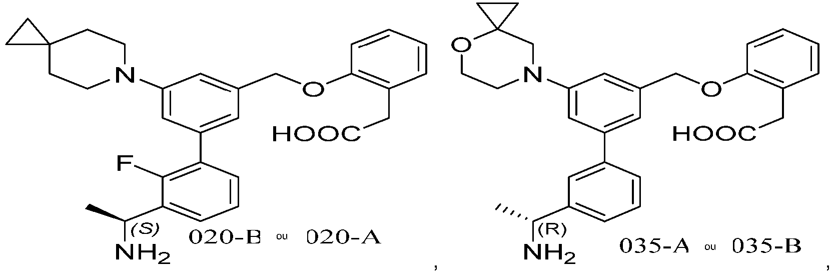 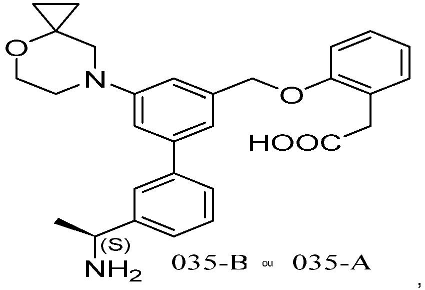 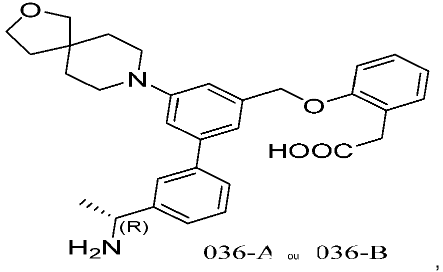 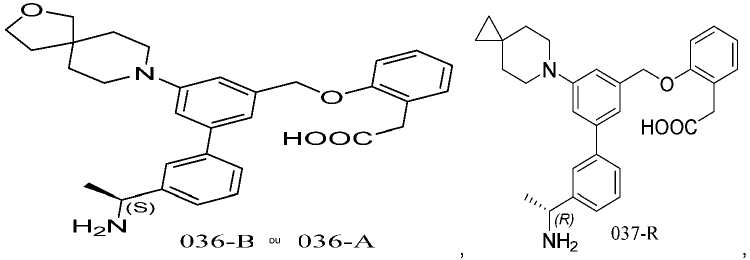 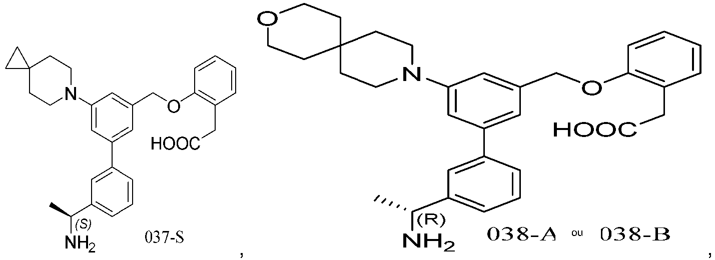 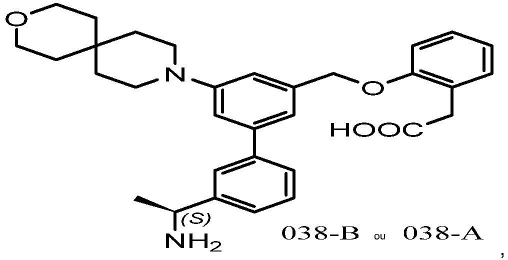 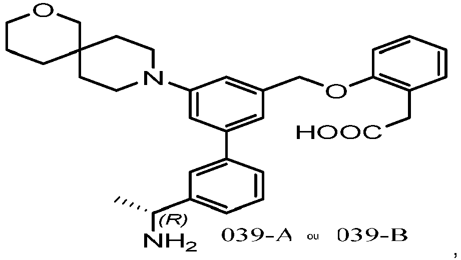 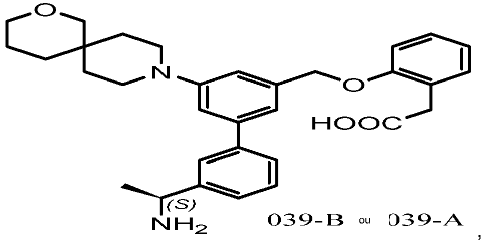 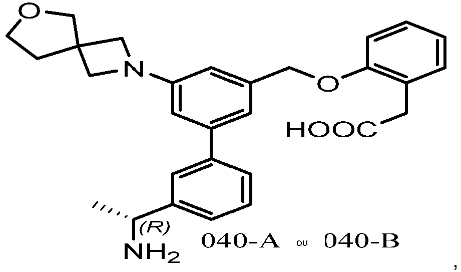 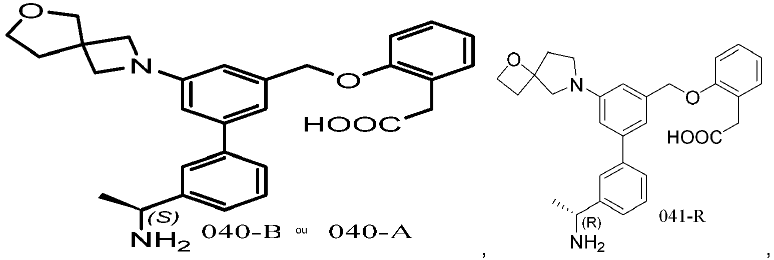 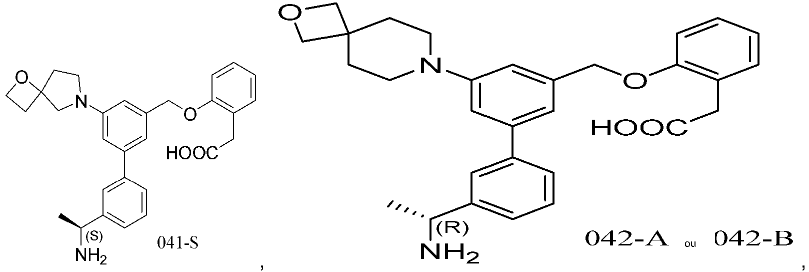 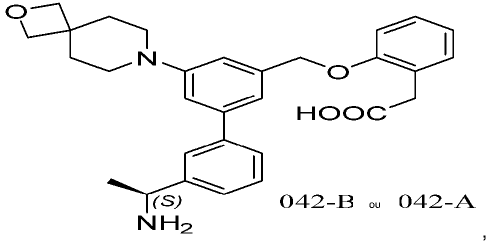 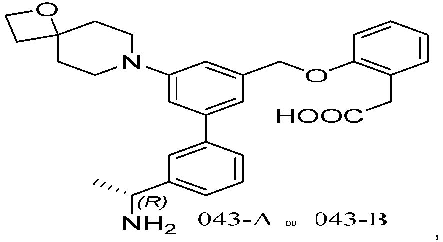 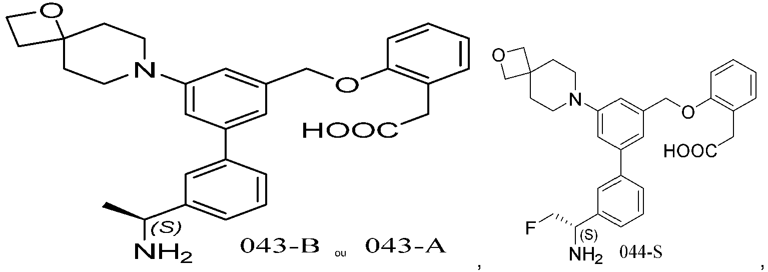 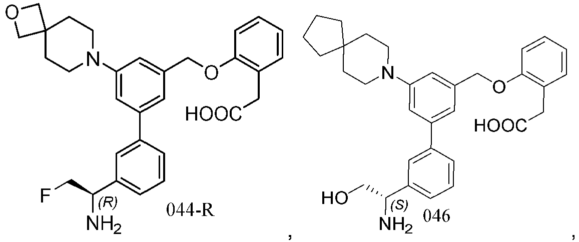 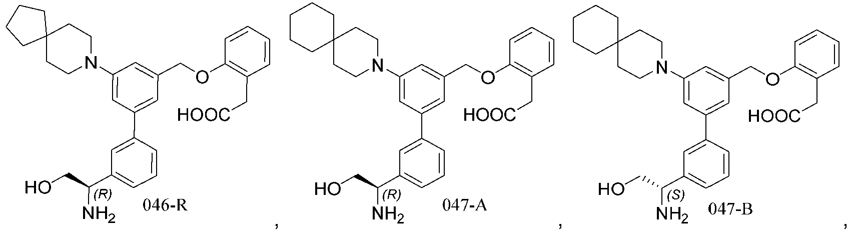 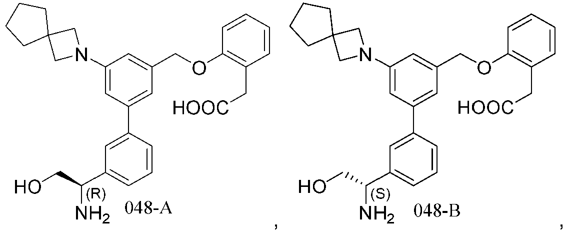 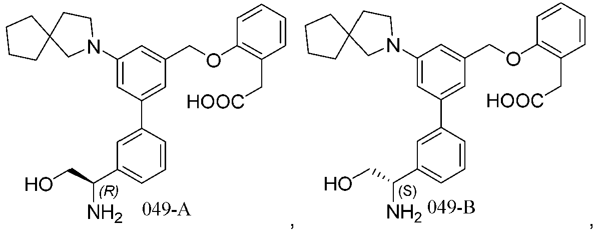 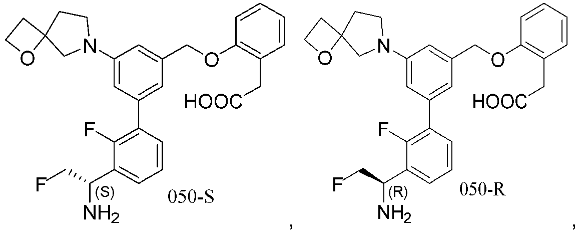 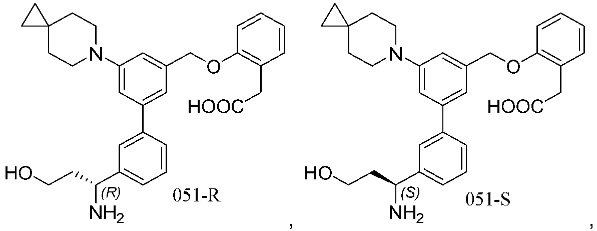 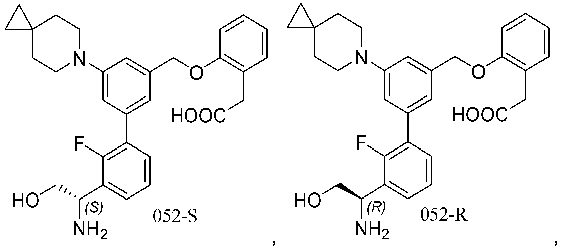 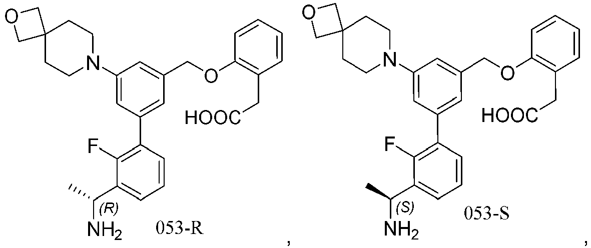 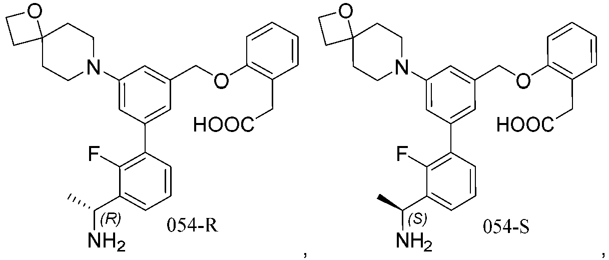 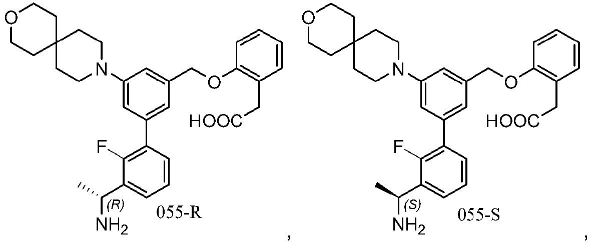 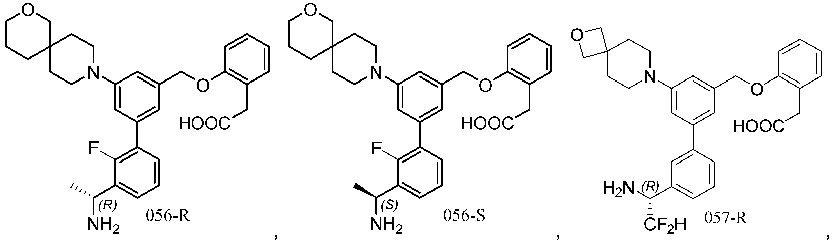 ou

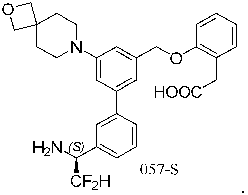

11. Un procédé de préparation pour un composé de formule (I), comprenant les étapes suivantes :
(1) soumettre un composé de formule II-3 à une réaction de déprotection pour obtenir un composé de formule II-4 ; 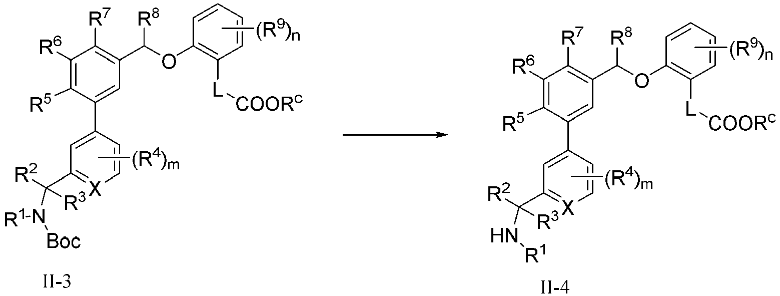
(2) soumettre le composé de formule II-4 à une réaction d'hydrolyse pour obtenir le composé de formule (I) ; 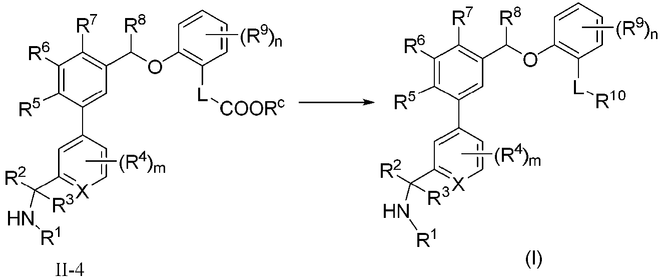 où R¹⁰ est -COOH, R^{c} est un alkyle en C₁₋₆ ou un alkyle en C₁₋₆ substitué par 1, 2, ou 3 R^{c-1} ; chaque R^{c-1} est indépendamment un halogène, -OH, ou -C(=O)OC(CH₃)₃, et R¹, R², R³, R⁴, R⁵, R⁶, R⁷, R⁸, R⁹, X, L, m, et n sont définis comme dans au moins l'une des revendications 1 à 10 ; de préférence, R¹ est H.

12. Un composé de formule II-3 ou de formule II-4 : 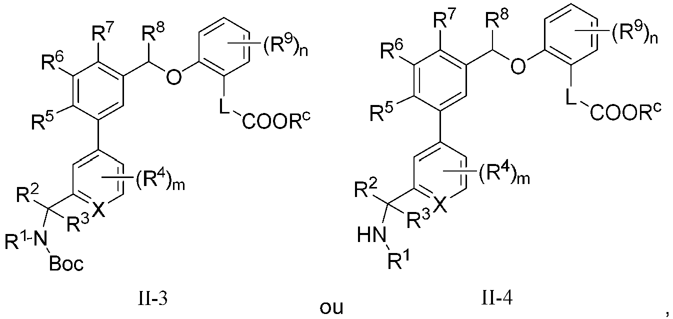
où, R^{c} est un alkyle en C₁₋₆ ou un alkyle en C₁₋₆ substitué par 1, 2, ou 3 R^{c-1} ; chaque R^{c-1} est indépendamment un halogène, -OH, ou -C(=O)OC(CH₃)₃ ;
R¹, R², R³, R⁴, R⁵, R⁶, R⁷, R⁸, R⁹, X, L, m, et n sont définis comme dans au moins l'une des revendications 1 à 10 ; de préférence, R¹ est H.

13. Le composé selon la revendication 12, où le composé est sélectionné parmi l'un quelconque des suivants : 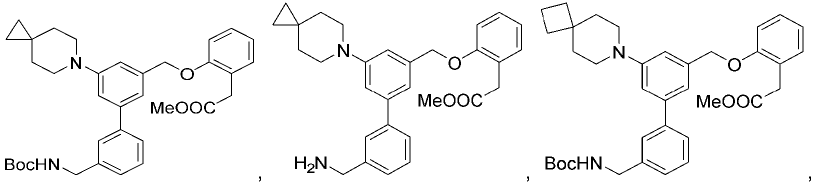 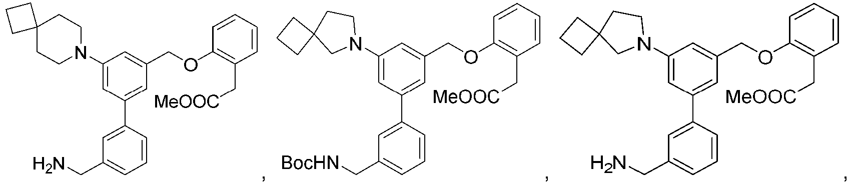 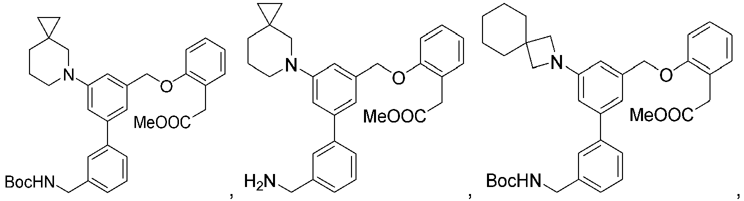 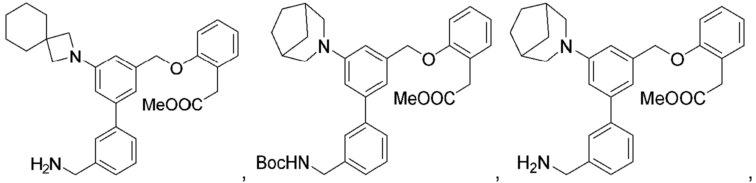 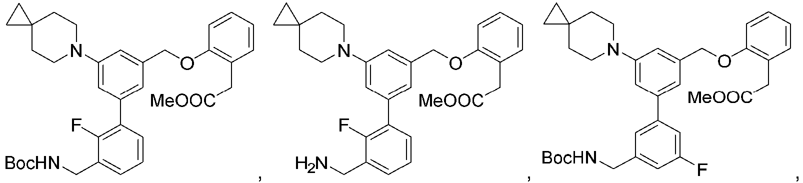 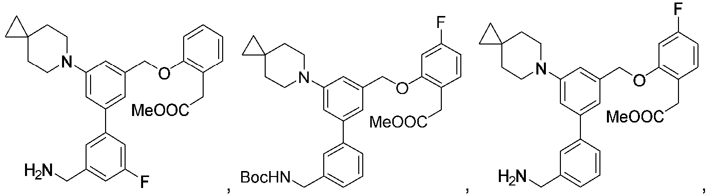 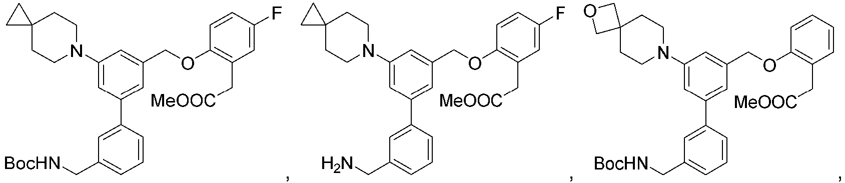 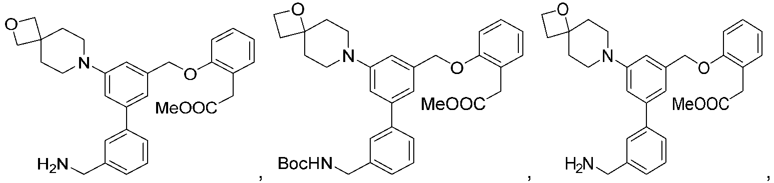 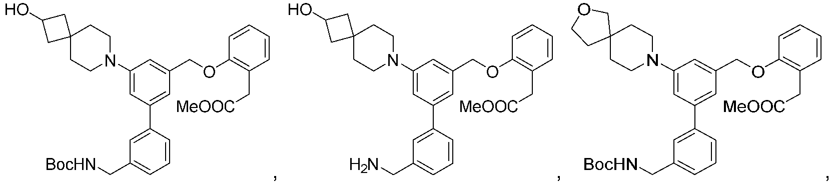 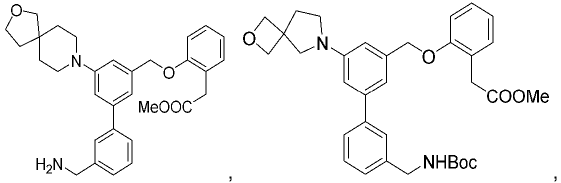 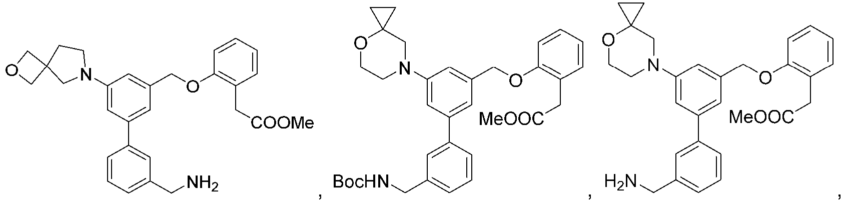 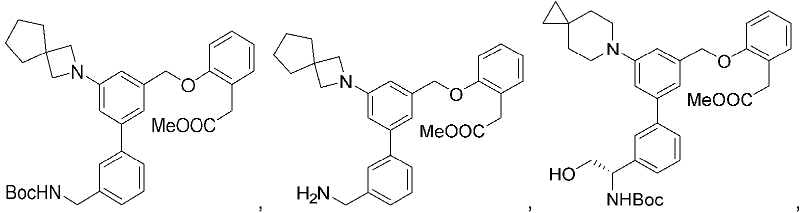 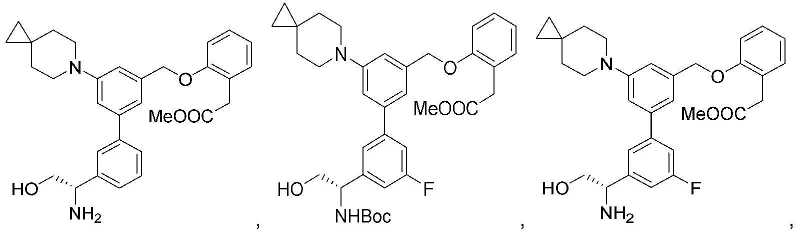 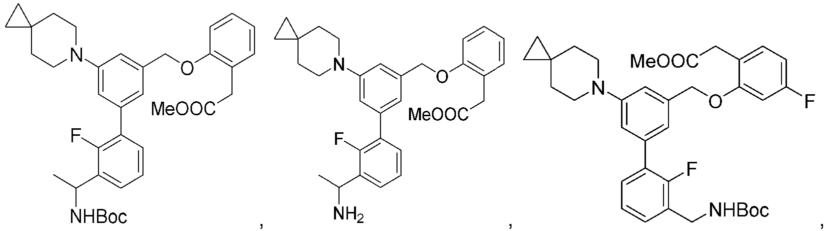 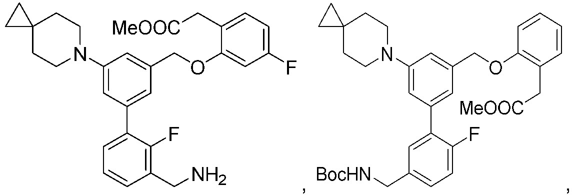 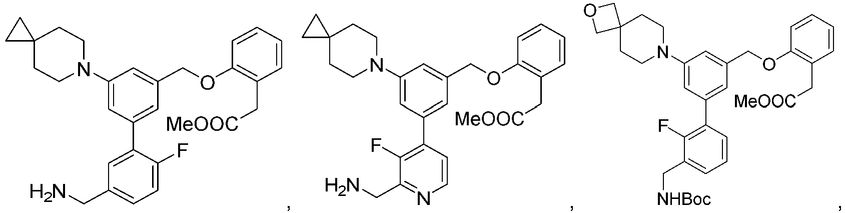 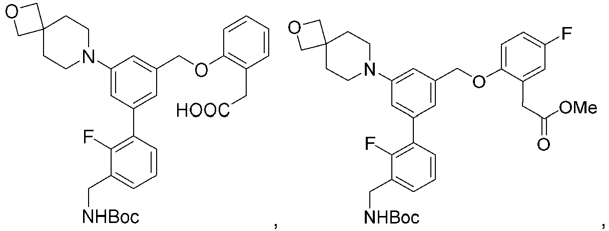 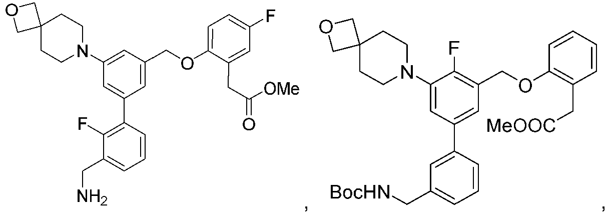 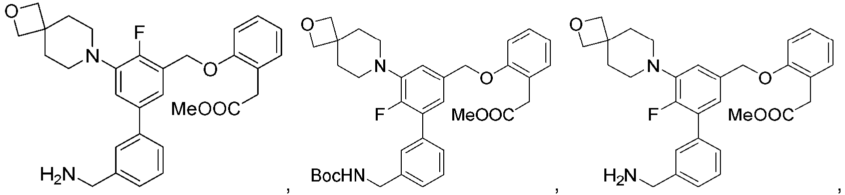 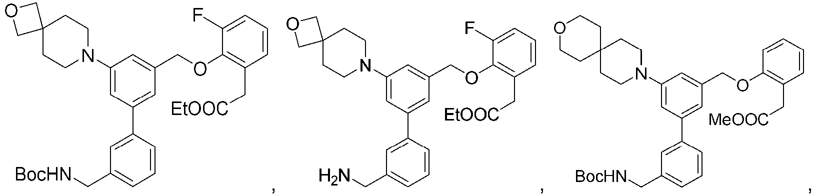 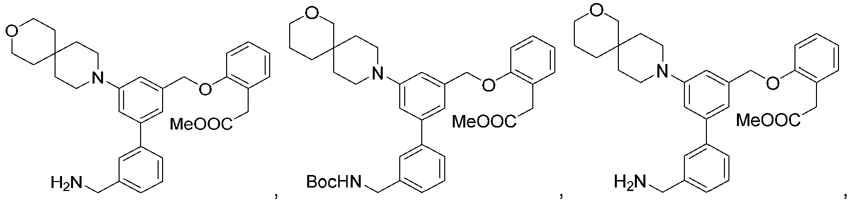 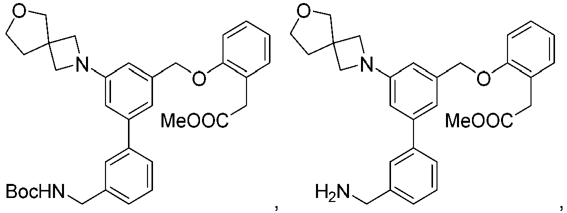 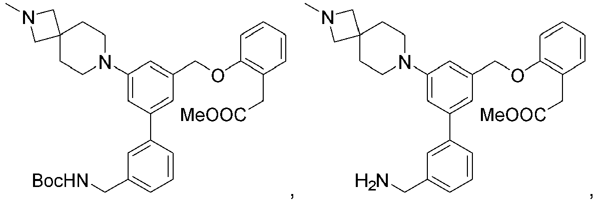 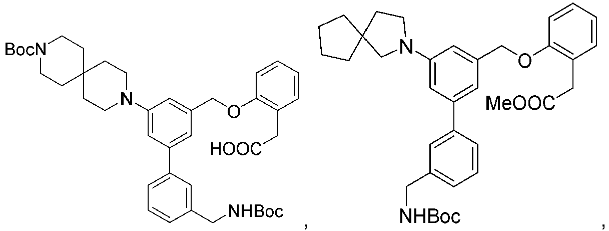 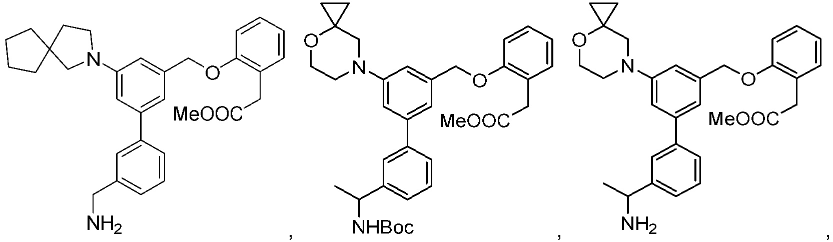 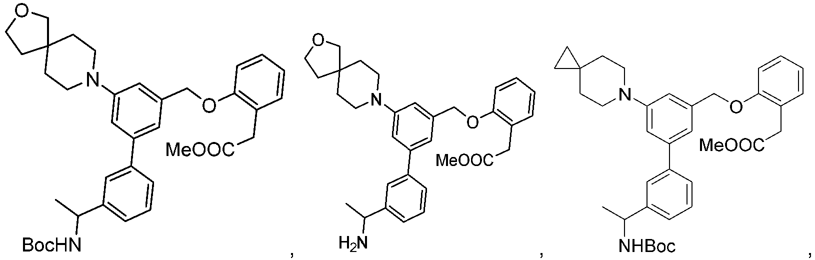 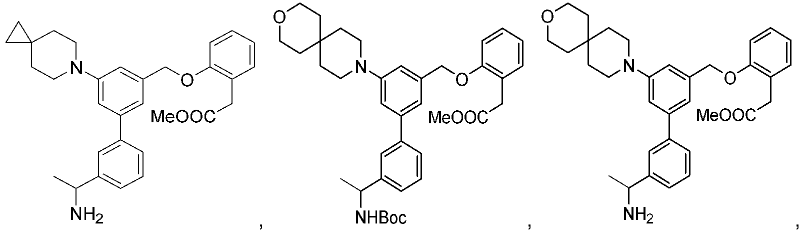 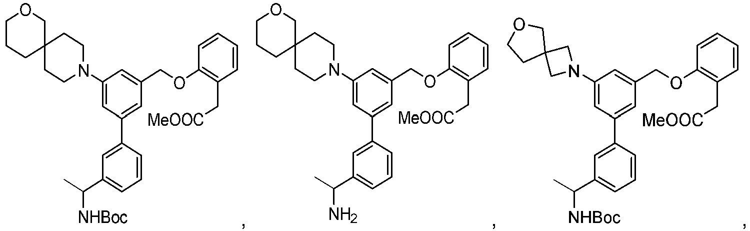 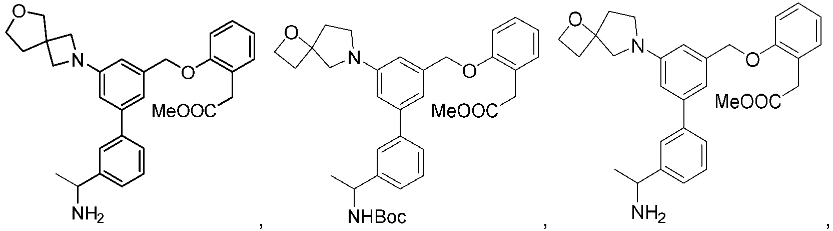 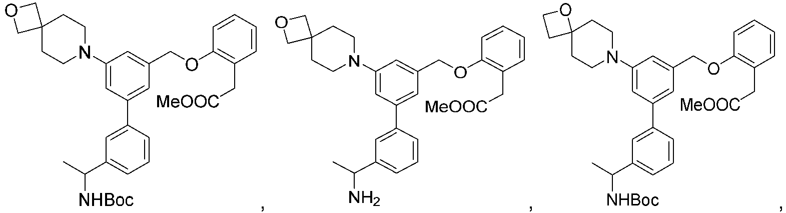 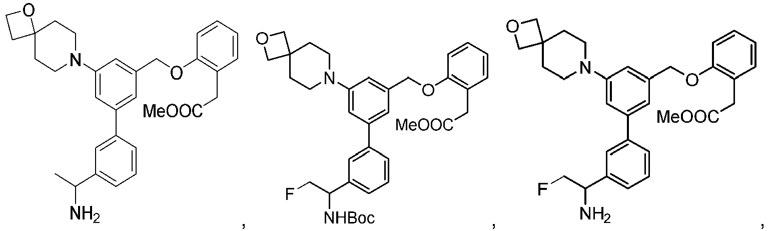 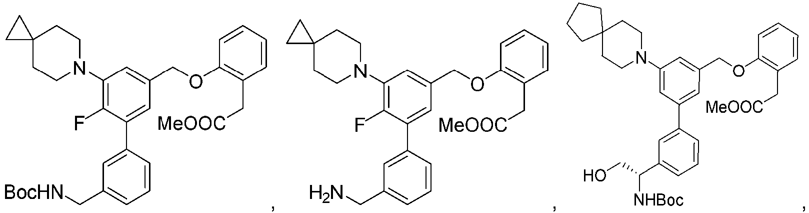 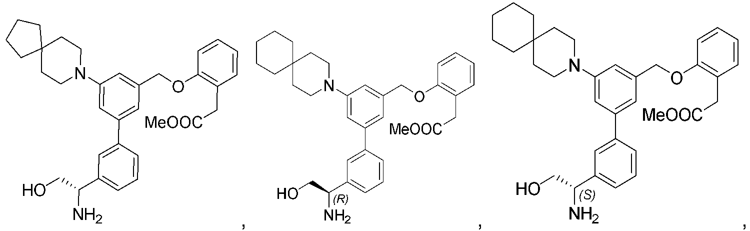 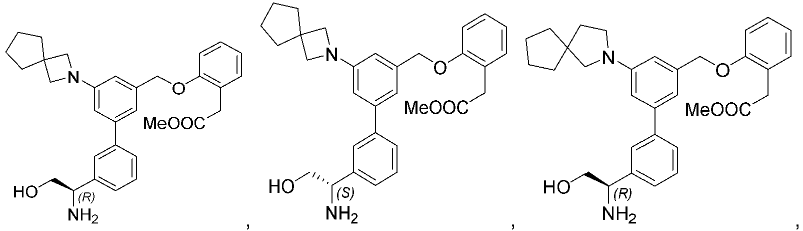 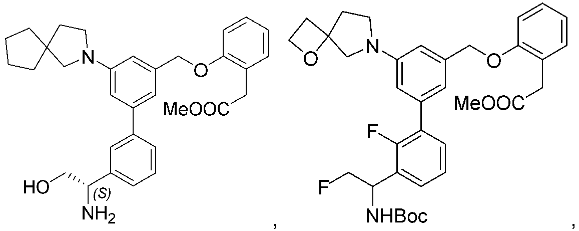 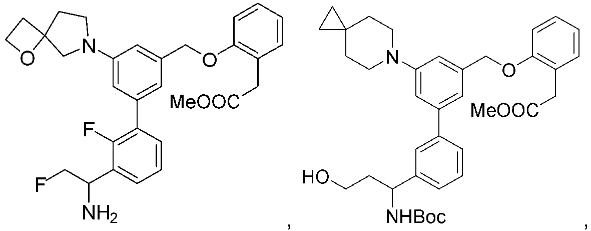 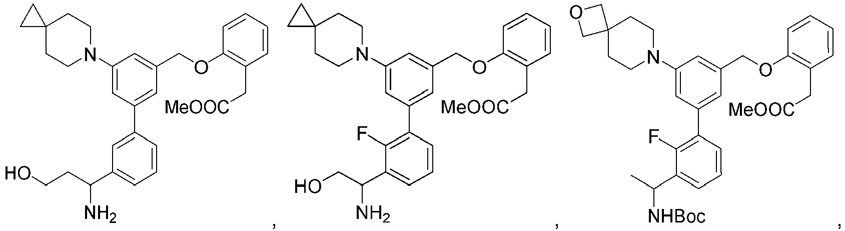 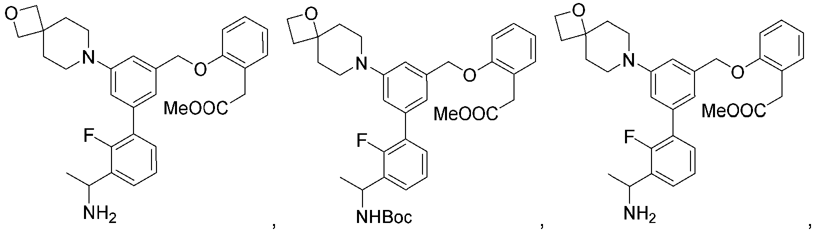 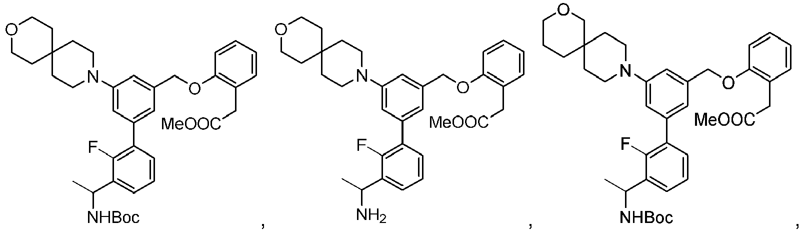 ou

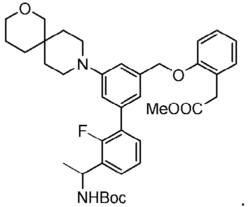

14. Une composition pharmaceutique, comprenant :
(1) le composé de formule (I), le tautomère de celui-ci, le stéréoisomère de celui-ci, ou le sel pharmaceutiquement acceptable de l'un quelconque des précédents, ou le solvate de l'un quelconque des précédents selon au moins une des revendications 1 à 10 ; et
(2) un vecteur pharmaceutiquement acceptable.

15. Le composé de formule (I), le tautomère de celui-ci, le stéréoisomère de celui-ci, ou le sel pharmaceutiquement acceptable de l'un quelconque des précédents, ou le solvate de l'un quelconque des précédents selon au moins une des revendications 1 à 10, ou la composition pharmaceutique selon la revendication 14 pour une utilisation en tant que médicament inhibiteur du facteur D du complément ou médicament pour le traitement et/ou la prévention d'une maladie médiée par le facteur D du complément, où la maladie médiée par le facteur D du complément est un trouble hématologique, une maladie rénale, une maladie cardiovasculaire, un trouble immunologique, une maladie du système nerveux central, une maladie respiratoire, une maladie du système urogénital, ou un trouble oculaire ;
de préférence, la maladie médiée par le facteur D du complément est la maladie des agglutinines froides, le syndrome catastrophique des antiphospholipides, l'anémie hémolytique, la vascularite associée aux anticorps cytoplasmiques antineutrophiles, l'anémie hémolytique auto-immune chaude, l'hémoglobinurie paroxystique nocturne, la néphropathie à immunoglobuline IgA, la néphrite lupique, le syndrome hémolytique urémique atypique, la glomérulonéphrite membranoproliférative, la maladie des dépôts denses, la glomérulonéphrite C3, la glomérulosclérose segmentaire focale, la néphropathie diabétique, le lupus systémique ou le lupus érythémateux, la polyarthrite rhumatoïde, la maladie inflammatoire de l'intestin, le psoriasis, la sclérose en plaques, le rejet de greffe d'organe, la myasthénie grave, la maladie d'Alzheimer, le syndrome de détresse respiratoire, l'asthme, la bronchopneumopathie chronique obstructive, l'emphysème, l'infection par un coronavirus, la dégénérescence maculaire, la dégénérescence maculaire liée à l'âge, l'œdème maculaire, l'œdème maculaire diabétique, la néovascularisation choroïdienne, l'uvéite, l'uvéite de Behçet, la rétinopathie diabétique proliférative, la rétinopathie diabétique non proliférative, le glaucome, la rétinopathie hypertensive, la maladie de néovascularisation cornéenne, le rejet de greffe post-cornéenne, la dystrophie cornéenne, la maladie auto-immune de l'œil sec, le syndrome de Stevens-Johnson, le syndrome de Sjögren, la maladie environnementale de l'œil sec, la dystrophie endothéliale de Fuchs, l'occlusion veineuse rétinienne, ou l'inflammation postopératoire ;
de préférence, l'infection par un coronavirus est une infection par SARS-CoV, une infection par MERS-CoV, ou une infection par SARS-CoV-2.
